# EUROPEAN PATENT APPLICATION

(11) **EP 2 526 945 A1**
(43) Date of publication of application: **28.11.2012**
(21) Application number: 11382169.8
(22) Date of filing: 25.05.2011
(51) Int. Cl.: A61K 31/437, A61K 31/4709, C07D 471/04, A61P 11/00, A61P 11/06, A61P 11/08, A61P 17/00, A61P 17/06

(54) **New CRTH2 Antagonists**

(71) Applicant: Almirall, S.A., 08022 Barcelona (ES)
(72) Inventor: Roberts, Richard Spurring, 08980 Sant Feliú de Llobregat (ES); Forns Berenguel, Maria Pilar, 08980 Sant Feliú de Llobregat (ES); Eastwood, Paul Robert, 08980 Sant Feliú de Llobregat (ES); Sevilla Gomez, Sara, 08980 Sant Feliú de Llobregat (ES); Buil Albero, Maria Antonia, 08980 Sant Feliú de Llobregat (ES); Moreno Mollo, Inmaculada Montserrat, 08980 Sant Feliú de Llobregat (ES); Matassa, Victor Giulio, 08980 Sant Feliú de Llobregat (ES); Casado Vilches, Oscar, 08980 Sant Feliú de Llobregat (ES)
(74) Representative: Elzaburu Marquez, Alberto

(57) **Abstract**

The present invention relates to a compound of formula (I), to the process for preparing such compounds and to their use in the treatment of a pathological condition or disease susceptible to amelioration by CRTh2 antagonist activity.

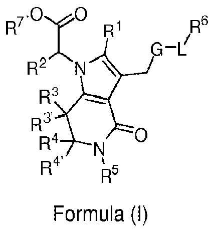

## Description

### FIELD OF THE INVENTION

The present invention relates to novel compounds having CRTh2 activity. This invention also relates to pharmaceutical compositions containing them, processes for their preparation and their use in respiratory therapies.

### BACKGROUND OF THE INVENTION

Prostaglandin D2 (PGD2) is a prostaglandin derived from the metabolism of arachidonic acid by cyclooxygenases and downstream PGD2 synthases. In the immune system, PGD2 is mainly produced by mast cells (Lewis et al., J. Immunol., 1982, 129, 1627-1631) but also although at lower levels, by macrophages and Th2 lymphocytes (Urade; J. Immunol, 1989, 143, 2982-2989. Tanaka; J. Immunol, 2000, 164, 2277-2280) in response to local tissue damage and allergic inflammation in diseases such as asthma (Murray et al., N. Engl. J. Med., 1986, 315, 800-804; Liu et al; Am. Rev. Respir. Dis., 1990, 142, 126-132; Wenzel et al., J. Allergy Clin. Immunol., 1991, 87, 540-548), rhinitis (Naclerio et al., Am. Rev. Respir. Dis., 1983, 128, 597-602; Brown et al., Arch. Otolarynol. Head Neck Surg., 1987, 113, 179-183; Lebel et al; J. Allergy Clin. Immunol., 1988, 82, 869-877) and atopic dermatitis among others. Exogenous PGD2 applied to bronchial airways elucidates many characteristics of asthmatic response suggesting that this prostaglandin plays an important pro-inflammatory role in allergic diseases (Hardy; N. Engl. J. Med., 1980, 311, 209-213. Sampson; Thorax, 1997, 52, 513-518). PGD2 binds to three different receptors, the thromboxane-type prostanoid receptor (TP) (Coleman; Br. J. Pharmacol., 1989, 96, 688-692. Francis; Br. J. Pharmacol., 1991, 104, 596-602), the PGD2 receptor (DP also known as DP1) (Boie et al; J. Biol. Chem., 1995, 270, 18910-18916) and the chemoattractant receptor-homologous molecule expressed on Th2 (CRTh2 also known as DP2) (Nagata et al; J. Immunol., 1999, 162, 1278-1289; Powell; Prostaglandins Leukot. Essent. Fatty Acids, 2003, 69, 179-185). CRTh2 is a Gi-coupling GPCR signalling through reduction of intracellular cAMP and calcium mobilization and it is involved in the chemotaxis of Th2 lymphocytes, eosinophils and basophils (Hirai, J. Exp. Med. 2001, 193, 255-261. Shichijo; J. Pharmacol. Exp. Ther., 2003, 307, 518-525). CRTh2 also inhibits the apoptosis of Th2 lymphocytes (Xue; J. Immunol., 2009, 182, 7580-7586) and stimulates the production of IL4, IL5, and IL13 (Xue; J. Immunol., 2005, 175, 6531-6536) which are cytokines involved in important biological responses such as eosinophil recruitment and survival, mucus secretion, airway hyperresponsiveness and immunoglobulin E production among others. Therefore, molecules that antagonize the mentioned pro-inflammatory PGD2 effects mediated by CRTh2 on key cell types associated with allergic inflammation like basophils, eosinophils and Th2 lymphocytes would have a potential benefit in related pathologies.

In view of these physiological effects, CRTh2 inhibitors of varied chemical structures have been recently disclosed for the treatment or prevention of chronic and acute inflammatory diseases and other pathological conditions, diseases and disorders known to be susceptible to amelioration by inhibition of CRTh2. Several structural families of antagonists are observed.

In general antagonists can be grouped into one of the following families: indole acetic acids, phenylacetic acids, phenoxyacetic acids and tetrahydroquinolines derivatives.

Examples of indole acetic acids derivatives are those described in GB2407318, WO2003101981, WO2005019171, WO2006036994, WO2007022501, WO2008012511, WO2009063215, US2010063103.

Structurally similar analogues of the indole group have also been reported. Compounds disclosed include structural variations such as: azaindoles (for example: WO2007068418), indolizines (for example: WO2009044147), tetrahydoindazoles (for example: W02010006944), tetrahydroindoles (for example: WO2010039982), benzimidazoles (for example: WO2006034418), oxindoles (for example: WO2009061676) and thienopyrroles (for example: W02009102462).

Additionally, tricyclic variations of some of the above structures have been disclosed. For example: WO2010008864, W02010031183.

For examples of phenylacetic acids and structurally similar analogues, see: WO2004058164, WO2007039736, WO2008024746, WO2009061730 and W02010003120.

Structurally similar analogues of the phenylacetic group have also been reported. Compounds disclosed include structural variations such as: naphthalene acetic acids (for example: WO2010055006), pyrimidine acetic acids (for example: WO2010027448), pyrrole acetic acids (for example: WO2007144127), thiophene acetic acids (for example: WO2007062797) and other heterocycle-acetic acids (for example: W02010057118).

For examples of phenoxyacetic acids see: WO2004089884, WO2005018529, WO2006005909, WO2007036743, WO2008119917, WO2009004379 and W02010018109.

For examples of tetrahydroquinolines see: WO2004032848, W02006091674.

Ramatroban, {(3R)-3-[(4-fluorophenyl)sulphonylamino]-1,2,3,4-tetrahydro-9H-carbazole-9-propanoic acid}, a thromboxane A2 receptor antagonist is also known to be a CRTh2 antagonist (Sugimoto et al; J. Pharmacol. Exp. Ther., 2003, 305, 347-352). A few compounds having the capacity to selectively inhibit CRTh2 are in active development. Examples of these compounds are ODC-9101, AZD-1981, ACT-129968, AP-761, AM211, AM461, ADC-3680, ARRY-502, OC-459, MK-7246 and RG-7185.

In addition, compounds having the capacity to selectively inhibit both DP1 and CRTh2 are in active development. An example of this type of compound is AMG-853.

These observations suggest that CRTh2 antagonists may have valuable properties for the treatment of diseases mediated by PGD2.

### SUMMARY OF THE INVENTION

The invention provides a compound of formula (I), or a pharmaceutically acceptable salt, N-oxide, solvate, stereiosomer or deuterated derivative thereof: 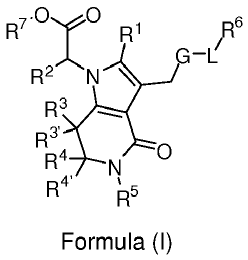 wherein:
- R¹ is selected from the group consisting of a linear or branched C₁₋₄ alkyl group and a C₃₋₇ cycloalkyl group;
- R² is selected from the group consisting of a hydrogen atom and a linear or branched C₁₋₄ alkyl group;
- R³, R^{3'}, R⁴ and R^{4'} are independently selected from the group consisting of a hydrogen atom and a linear or branched C₁₋₄ alkyl group;
   or R³ and R^{3'} together with the carbon atom to which they are attached form a C₃₋₇ cycloalkyl group and R⁴ and R^{4 '}are independently selected from the group consisting of a hydrogen atom and a linear or branched C₁₋₄ alkyl group;
   or R⁴ and R^{4'} together with the carbon atom to which they are attached form a C₃₋₇ cycloalkyl group and R³ and R^{3'} are independently selected from the group consisting of a hydrogen atom and a linear or branched C₁₋₄ alkyl group;
   or R³ together with R⁴ form an additional bond and R^{3'} and R^{4'} are independently selected from the group consisting of a hydrogen atom and a linear or branched C₁₋₄ alkyl group;
- R⁵ is selected from the group consisting of a hydrogen atom, a linear or branched C₁₋₄ alkyl group; a linear or branched C₁₋₄ haloalkyl group and a benzyl group;
- R⁶ is selected from the group consisting of a hydrogen atom, a linear or branched C₁₋₄ alkyl group, a C₃₋₇ cycloalkyl group, a benzyl group, a -N(R^{b})COR^{a} group, a C₆₋₁₀ aryl group, a 5- to 10-membered, mono- or bicyclic heteroaryl group containing at least one heteroatom selected from N, S and O, and a 5- to 10-membered, mono- or bicyclic heterocyclyl group containing at least one heteroatom selected from N, S and O;
   wherein the aryl, heteroaryl and heterocyclyl groups independently are optionally substituted by one or more substituents selected from the group consisting of a halogen atom, a cyano group, a nitro group, a -CF₃ group, a linear or branched C₁₋₄ alkyl group, a C₃₋₇ cycloalkyl group, a -CO₂H group, a -CO₂R^{a} group, a -CONR^{d}R^{e} group, a -NR^{d}R^{e} group, a -NCOR^{a} group, a -NCO₂R^{a} group, a -NCONR^{d}R^{e} group, a -NSO₂R^{a} group, a -NSO₂NR^{d}R^{e} group, a -OH group, a -OR^{a} group, -SR^{a} group, a -SOR^{a} group, a -SO₂R^{a} group and a -SO₂NR^{d}R^{e} group;
- L is selected from the group consisting of a direct bond, a -CR^{b}R^{c}-, -O-, -CH(OH)-, -CO-, -S-, -SO-, -SO₂- and -SO₂N(CH₃)-;
- G is selected from a C₆₋₁₀ aryl group and a 5- to 10-membered, mono- or bicyclic heteroaryl group containing at least one heteroatom selected from N, S and O; wherein the aryl group and the heteroaryl groups independently are optionally substituted by one or more substituents selected from the group consisting of a halogen atom, a cyano group, a nitro group, a -CF₃ group, a linear or branched C₁₋₄ alkyl group, a C₃₋₇ cycloalkyl group, a -CO₂H group, a -CO₂R^{a} group, a -CONR^{d}R^{e} group, a -NR^{d}R^{e} group, a -NCOR^{a} group, a -NCO₂R^{a} group, a -NCONR^{d}R^{e} group, a -NSO₂R^{a} group, a -NSO₂NR^{d}R^{e} group, a -OH group, a -OR^{a} group, -SR^{a} group, a -SOR^{a} group, a -SO₂R^{a} group and a -SO₂NR^{d}R^{e} group;
- R⁷ is selected from the group consisting of a hydrogen atom, a linear or branched C₁₋₄ alkyl group, a mono- or bicyclic saturated or unsaturated C₃₋₁₀ cycloalkyl group, a C₁₋₄ alkoxy-C₁₋₄ alkyl, a -(CH₂)₂O(CH₂)₂OCH₃ group, a -CH₂CH(OH)CH₂OH group, a phenyl group, a benzyl group, a -CH₂(CHR^{a})₀₋₁CO₂H group, a -CH₂CONR^{d}R^{e} group, a -CHR^{b}OC(O)R^{a} group, a -CHR^{b}OC(O)O-cyclohexyl group, a -(CH₂)₂NR^{f}R^{g} group, a -(CH₂)₀₋₁(CR^{b}R^{c})CH₂NR^{f}R^{g} and a -(CH₂)₀₋₁-saturated or unsaturated 5- to 10-membered heterocyclyl) group;
   wherein the cycloalkyl, phenyl, benzyl and heterocyclyl groups are optionally substituted by one or more substituents selected from the group consisting of a C₁₋₄ alkyl group, a C₁₋₄ alkoxy group, an oxo group, a halogen atom, a carboxy group and a benzyl group;
- R^{a} is selected from the group consisting of a linear or branched C₁₋₄ alkyl group, a -CF₃ group, a C₃₋₇ cycloalkyl group, a 3 to 7-membered heterocycyl group containing at least one heteroatom selected from N, S and O, a -(C₁₋₄ alkyl)-(C₆₋₁₀ aryl) group, a C₆₋₁₀ aryl group and a 5- to 10-membered heteroaryl group containing at least one heteroatom selected from N, S and O; wherein the aryl and the heteroaryl groups are optionally substituted by one or more substituents selected from halogen atom, a C₁₋₂ alkyl group or a cyano group;
- R^{b}, R^{c}, R^{d} and R^{e} are independently selected from the group consisting of a hydrogen atom, a linear or branched C₁₋₄ alkyl group, a C₃₋₇ cycloalkyl group and a 3- to 7-membered heterocyclyl group containing at least one heteroatom selected from N, S and O;
   or R^{b} and R^{c} together with the carbon atom to which they are attached form a 3-C₃₋₇ cycloalkyl or a 3- to 7-membered heterocycyl containing one additional heteroatom selected from N, S and O;
   or R^{d} and R^{e} together with the nitrogen atom to which they are attached form a 3- to 7-membered N-containing heterocyclyl group and optionally containing one additional heteroatom selected from N, S and O;
- R^{f} and R^{g} are independently selected from the group consisting of a hydrogen atom, a C₁₋₄ alkyl group, a phenyl group, a diphenylpropyl group, a -CO-pyridyl group and a benzyl group optionally substituted by a halogen atom; or R^{f} and R^{g} together with the nitrogen atom to which they are attached form a 6-membered N-containing heterocyclyl group and optionally containing one additional heteroatom selected from N, S and O.

The invention provides synthetic processes and intermediates described herein, which are useful for preparing compounds of the invention.

The invention also provides a pharmaceutical composition comprising a compound of the invention and a pharmaceutically-acceptable carrier. The invention further provides combinations comprising a compound of the invention and one or more other therapeutic agents and pharmaceutical compositions comprising such combinations.

The invention also provides a compound of the invention for use in the treatment of the human or animal body by therapy.

The invention also provides a compound of the invention for use in the treatment of a disease or condition susceptible to amelioration by CRTh2 antagonists, in a mammal, in particular wherein the pathological condition or disease is selected from asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome (ARDS), myocardial infarction, aneurysm, stroke, cancer, wound healing, endotoxic shock, pain, inflammatory conditions, eosinophilic esophagitis, eosinophil-associated gastrointestinal disorders (EGID), idiopathic otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, irritable bowel disease and inflammatory bowel disease (such as Crohn's disease and ulcerative colitis).

The invention also provides the use of a compound of the invention in the manufacture of a formulation or medicament for treating a disease or condition susceptible to amelioration by CRTh2 antagonists, in particular wherein the condition or disease is as described above.

The invention also provides a method of treating a disease or condition as described above; comprising such method administering to the mammal, a therapeutically effective amount of a compound of the invention. The invention further provides a method of treatment comprising administering a therapeutically effective amount of a combination of a compound of the invention together with one or more other therapeutic agents.

### DETAILED DESCRIPTION OF THE INVENTION

When describing the compounds, compositions, combinations and methods of the invention, the following terms have the following meanings, unless otherwise indicated.

As used herein the term C₁₋₄ alkyl embraces optionally substituted, linear or branched radicals having 1 to 4 carbon atoms. Examples include methyl, ethyl, n-propyl, i-propyl, n-butyl, sec-butyl or t-butyl.

As used herein, the term C₁₋₄ alkoxy (or alkyloxy) embraces optionally substituted, linear or branched oxy-containing radicals each having alkyl portions of 1 to 4 carbon atoms. Examples of C₁₋₄ alkoxy radicals include methoxy, ethoxy, n-propoxy, i-propoxy, n-butoxy, sec-butoxy and t-butoxy.

As used herein, the term C₁-C₄ haloalkyl group is a linear or branched alkyl group, which is substituted by one or more, preferably 1, 2 or 3 halogen atoms. Examples include CCl₃, CF₃ and CHF₂.

As used herein, the term C₃₋₁₀ cycloalkyl group embraces saturated or unsaturated, monocyclic or polycyclic carbocyclic radicals having from 3 to 10 carbon atoms. Examples include cyclopropyl, cyclobutyl, cyclopentyl, cyclohexyl, cycloheptyl and 1H-2,3-dihydroindenyl.

As used herein, the term C₆₋₁₀ aryl radical embraces typically a C₆-C₁₀ monocyclic or polycyclic aryl radical such as phenyl and naphthyl.

As used herein, the term 5- to 10- membered heteroaryl radical embraces typically a 5-to 10- membered ring system comprising at least one heteroaromatic ring and containing at least one heteroatom selected from O, S and N. A 5- to 10- membered heteroaryl radical may be a single ring or two or more fused rings wherein at least one ring contains a heteroatom. Examples include pyridinyl, pyrazinyl, pyrimidinyl, pyridazinyl, furyl, benzofuranyl, oxadiazolyl, oxazolyl, isoxazolyl, benzoxazolyl, benzisoxazolyl, imidazolyl, benzimidazolyl, thiazolyl, thiadiazolyl, isothiazolyl, benzisothiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, 1H-pyrazolo[3,4-d]pyridinyl, thieno[2,3-d] pyrimidinyl and the various pyrrolopyridinyl radicals.

As used herein, the term 3 to 7-membered heterocyclyl radical embraces typically a non-aromatic, saturated or unsaturated C₃-C₇ carbocyclic ring system in which one or more carbon atoms are replaced by a heteroatom selected from N, O and S. Examples of 3 to 7-membered heterocyclic radicals include piperidinyl, pyrrolidinyl, pyrrolinyl, piperazinyl, morpholinyl, thiomorpholinyl, pyrazolinyl, azetidinyl, oxetanyl, tetrahydrofuranyl, tetrahydrothiophenyl, tetrahydropyranyl, tetrahydrothiopyranyl, 1,1-dioxotetrahydrothiopyranyl and dioxanyl.

As used herein, the term halogen atom embraces chlorine, fluorine, bromine or iodine atoms typically a fluorine, chlorine or bromine atom. The term halo when used as a prefix has the same meaning.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition associated with CRTh2 activity" includes all disease states and/or conditions that are acknowledged now, or that are found in the future, to be associated with CRTh2 activity. Such disease states include, but are not limited to, asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome (ARDS), myocardial infarction, aneurysm, stroke, cancer, wound healing, endotoxic shock, pain, inflammatory conditions, eosinophilic esophagitis, eosinophil-associated gastrointestinal disorders (EGID), idiopathic otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, irritable bowel disease and inflammatory bowel disease (such as Crohn's disease and ulcerative colitis). Preferably, such disease states are asthma or chronic obstructive pulmonary disease (COPD), rhinitis and atopic dermatitis.

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulfonic, benzoic, camphosulfonic, citric, ethanesulfonic, formic, fumaric, gluconic, glutamic, hydrobromic, hydrochloric, hydrofluoric, lactic, maleic, malic, mandelic, methanesulfonic, mucic, nitric, pantothenic, phosphoric, succinic, sulfuric, tartaric, p-toluenesulfonic, xinafoic (1-hydroxy-2-naphthoic acid), napadisilic (1,5-naphthalenedisulfonic acid), triphenyl acetic and the like. Particularly preferred are salts derived from formic, fumaric, hydrobromic, hydrochloric, hydrofluoric, acetic, sulfuric, methanesulfonic, xinafoic, tartaric, maleic, succinic and napadisilic acids. Salts derived from pharmaceutically-acceptable inorganic bases include aluminum, calcium, copper, ferric, ferrous, lithium, magnesium, manganic, manganous, potassium, sodium, zinc and the like. Particularly preferred are calcium, magnesium, potassium and sodium salts.

Salts derived from pharmaceutically-acceptable organic bases include salts of ammonia, primary, secondary and tertiary amines, including substituted amines, cyclic amines, naturally-occurring amines and the like, such as ammonia, arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term hydrate is used instead of solvate.

The invention also includes isotopically-labeled compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C, are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of the invention can generally be prepared by conventional techniques known to those skilled in the art or by processes analogous to those described herein, using an appropriate isotopically-labeled reagent in place of the non-labeled reagent otherwise employed.

Preferred isotopically-labeled compounds include deuterated derivatives of the compounds of the invention. As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

The term "amino-protecting group" refers to a protecting group suitable for preventing undesired reactions at amino nitrogen. Representative amino-protecting groups include, but are not limited to, formyl; acyl groups, for example alkanoyl groups such as acetyl, benzoyl, pivaloyl or trifluoroacetyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups such as benzyl (Bn), trityl (Tr), and 1,1-di-(4'-methoxyphenyl)methyl and the like.

The term "hydroxy-protecting group" refers to a protecting group suitable for preventing undesired reactions at a hydroxy group. Representative hydroxy-protecting groups include, but are not limited to, alkyl groups, such as methyl, ethyl, and tert-butyl; acyl groups, for example alkanoyl groups, such as acetyl, pivaloyl or trifluoroacetyl; arylcarbonyl groups, such as benzoyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

Compounds of the invention may be administered in prodrug form, such as esters. "Prodrug" means a compound that is convertible in vivo by metabolic means (for example, by hydrolysis, reduction or oxidation) to a compound of the invention. For example an ester prodrug of a compound of the invention may be convertible by hydrolysis in vivo to the parent molecule.

The compounds of the invention may contain one or more chiral centers. Accordingly, the invention includes racemic mixtures, enantiomers, and mixtures enriched in one or more stereoisomer. The scope of the invention as described and claimed encompasses the racemic forms of the compounds as well as the individual enantiomers, diastereomers, and stereoisomer-enriched mixtures.

Typically, R¹ is selected from the group consisting of a -CH₃ group and a cyclopropyl group.

Typically, R² is selected from the group consisting of a hydrogen atom and a -CH₃ group.

Typically, R³, R^{3'}, R⁴ and R^{4'} are independently selected from the group consisting of a hydrogen atom and a -CH₃ group; or R³ together with R⁴ form an additional bond and R³ and R^{4'} are independently selected from the group consisting of a hydrogen atom and a CH₃ group.

Typically, R⁵ represents a hydrogen atom, a -CH₃ group, an isopropyl group, a -CHF₂ group, a -CH(CH₃)CF₃ group or a benzyl group.

When R⁵ represents a hydrogen atom, the compounds of the invention may exhibit tautomerism. All such tautomeric forms are included within the scope of compounds of Formula I. Tautomers exist as mixtures of a tautomeric set in solution. In solid form, usually one tautomer predominates. Even though one tautomer may be described, the present invention includes all tautomers of the compounds of Formula I.

Typically, L represents a direct bond, -CH₂-, -O-, -CH(OH)-, -CO-, -SO₂- and - SO₂N(CH₃)-, preferably -SO₂-, -SO₂NCH₃ or -CH(OH)-, more preferably -SO₂-.

Typically, G represents a phenyl group, a pyrrolyl group or a quinolinyl group, wherein the phenyl, pyrrolyl and quinolinyl groups are optionally substituted by one or more substitutents, typically 1 or 2 substituents, preferably 1 substituent, which substituents are selected from the group consisting of a halogen atom, a cyano group, a -CF₃ group, a -CO₂H group, a -CO₂R^{a} group, a -CONR^{d}R^{e} group and a -OR^{a} group.

Typically, R⁶ represents a hydrogen atom, a phenyl group, a benzyl group, a cyclohexyl group or a morpholinyl group, wherein the phenyl, benzyl, cyclohexyl and morpholinyl groups are optionally substituted by one or more substituents, typically 1 2 or 3 substituents, preferably 1 or 2 substituents, which substituents are selected from the group consisting of a halogen atom a -OR^{a} group and a CONR^{d}R^{e} group.

Preferably, R⁶ represents a phenyl group, a benzyl group, a cyclohexyl group or a morpholinyl group, wherein the phenyl, benzyl, cyclohexyl and morpholinyl groups are optionally substituted by one or more substituents selected from the group consisting of a halogen atom and a -OR^{a} group.

Typically, R⁷ represents a hydrogen atom, a linear or branched C₁₋₄ alkyl group, a -CH₂CONR^{d}R^{e} group, a -(CH₂)₂NR^{f}R^{g} group, a -CHR^{b}OC(O)O-cyclohexyl group, a phenyl group, a mono- or bicyclic saturated or unsaturated C₆₋₁₀ cycloalkyl group and a -(CH₂)-(saturated or unsaturated 5-membered heterocyclyl) group, wherein the phenyl and heterocyclyl groups are optionally substituted by one or more substituents, typically 1 2 or 3 substituents, preferably 1 or 2 substituents, which substituents are selected from the group consisting of a C₁₋₄ alkyl group, a C₁₋₄alkoxy group and an oxo group.

In a preferred embodiment of the present invention, R¹ represents a -CH₃ group or a cyclopropyl group; R³, R^{3'}, R⁴ and R^{4'} are independently selected from the group consisting of a hydrogen atom and a -CH₃ group; or R³ together with R⁴ form an additional bond and R^{3'} and R^{4'} are independently selected from the group consisting of a hydrogen atom and a CH₃ group; R⁵ represents a hydrogen atom, a -CH₃ group, an isopropyl group, a -CHF₂ group, a -CH(CH₃)CF₃ group or a benzyl group; L represents -SO₂- -SO₂NCH₃ or -CH(OH)-, preferably -SO₂-; G represents a phenyl group, a pyrrolyl group or a quinolinyl group, wherein the phenyl, pyrrolyl and quinolinyl groups are optionally substituted by one or more substitutents selected from the group consisting of a halogen atom, a cyano group, a -CF₃ group, a -CO₂H group, a -CO₂R^{a} group, a -CONR^{d}R^{e} group and a -OR^{a} group; R⁶ represents a phenyl group, a benzyl group, a cyclohexyl group or a morpholinyl group, wherein the phenyl, benzyl, cyclohexyl and morpholinyl groups are optionally substituted by one or more substituents selected from the group consisting of a halogen atom and a -OR^{a} group; and R⁷ represents a hydrogen atom, a linear or branched C₁₋₄ alkyl group, a -CH₂CONR^{d}R^{e} group, a -(CH₂)₂NR^{f}R^{g} group, a -CHR^{b}OC(O)O-cyclohexyl group, a phenyl group, a mono- or bicyclic saturated or unsaturated C₆₋₁₀ cycloalkyl group and a -(CH₂)-(saturated or unsaturated 5-membered heterocyclyl) group, wherein the phenyl and heterocyclyl groups are optionally substituted by one or more substituents selected from the group consisting of a C₁₋₄ alkyl group, a C₁₋₄alkoxy group and an oxo group.

In another embodiment of the present invention, R¹ represents a -CH₃ group or a cyclopropyl group; R² represents a hydrogen atom or a -CH₃ group; R³, R^{3'}, R⁴ and R^{4'} are independently selected from the group consisting of a hydrogen atom and a -CH₃ group; or R³ together with R⁴ form an additional bond and R^{3'} and R^{4'} are independently selected from the group consisting of a hydrogen atom and a CH₃ group; R⁵ represents a hydrogen atom, a -CH₃ group, an isopropyl group, a -CHF₂ group, a -CH(CH₃)CF₃ group or a benzyl group; L represents a direct bond, -CH₂-, -O-, -CH(OH)-, -CO-, -SO₂- and -SO₂N(CH₃)-;

G represents a phenyl group, a pyrrolyl group or a quinolinyl group, wherein the phenyl, pyrrolyl and quinolinyl groups are optionally substituted by one or more substitutents selected from the group consisting of a fluorine atom, a bromine atom, a cyano group, a -CF₃ group, a -CO₂H group, a -CO₂CH₃ group, a -CONHCH₃ group and a -OCH₃ group; R⁶ represents a hydrogen atom, a -CH₃ group, a -CH₂CH₃ group, a phenyl group, a benzyl group, a cyclohexyl group, a morpholinyl group, a triazolyl group, a naphthyl group and a -N(Et)C(O)cyclopropyl group, wherein the phenyl group is optionally substituted by one or more substituents selected from the group consisting of a fluorine atom, a chlorine atom, a -OCF₃ group, a -OCH₃ group and a -CON(CH₃)₂ group; and R⁷ represents a hydrogen atom, a -CH₃ group, a -CH₂CH₃ group, a tert-butyl group, a -CH₂CON(CH₃)₂ group, a -(CH₂)₂N-morpholinyl group, -(CH₂)₂N(CH₃)₂ group, a -CH(CH₃)OC(O)O-cyclohexyl group, a 2-methoxyphenyl group, a 2,3-dihydro-1 H-inden-5-yl group and a (5-methyl-2-oxo-1,3-dioxol-4-yl)methyl group.

Particular individual compounds of the invention include:
Ethyl 2-(2-methyl-4-oxo-3-(2-(phenylsulfonyl)benzyl)-4,5,6,7-tetrahydro-1H-pyrrolo[3,2-c]pyridin-1-yl)acetate;
{2-Methyl-4-oxo-3-[2-(phenylsulfonyl)benzyl]-4,5,6,7-tetrahydro-1H-pyrrolo[3,2-c]pyridin-1-yl}acetic acid;
Ethyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
(3-{2-[(4-Fluorophenyl)sulfonyl]benzyl}-2-methyl-4-oxo-4,5,6,7-tetrahydro-1H-pyrrolo[3,2-c]pyridin-1-yl)acetic acid;
Ethyl {3-[5-methoxy-2-(phenylsulfonyl)benzyl]-2-methyl-4-oxo-4,5,6,7-tetrahydro-1H-pyrrolo[3,2-c]pyridin-1-yl}acetate;
{3-[5-Methoxy-2-(phenylsulfonyl)benzyl]-2-methyl-4-oxo-4,5,6,7-tetrahydro-1H-pyrrolo[3,2-c]pyridin-1-yl}acetic acid;
Ethyl (3-{2-[(3-chlorophenyl)sulfonyl]benzyl}-2-methyl-4-oxo-4,5,6,7-tetrahydro-1H-pyrrolo[3,2-c]pyridin-1-yl)acetate;
(3-{2-[(3-Chlorophenyl)sulfonyl]benzyl}-2-methyl-4-oxo-4,5,6,7-tetrahydro-1H-pyrrolo[3,2-c]pyridin-1-yl)acetic acid;
Ethyl {2-methyl-4-oxo-3-[2-(phenylsulfonyl)-4-(trifluoromethyl)benzyl]-4,5,6,7-tetrahydro-1H-pyrrolo[3,2-c]pyridin-1-yl}acetate;
{2-Methyl-4-oxo-3-[2-(phenylsulfonyl)-4-(trifluoromethyl)benzyl]-4,5,6,7-tetrahydro-1H-pyrrolo[3,2-c]pyridin-1-yl}acetic acid;
Ethyl (3-{2-[(3-fluorophenyl)sulfonyl]benzyl}-2-methyl-4-oxo-4,5,6,7-tetrahydro-1H-pyrrolo[3,2-c]pyridin-1-yl)acetate;
(3-{2-[(3-Fluorophenyl)sulfonyl]benzyl}-2-methyl-4-oxo-4,5,6,7-tetrahydro-1H-pyrrolo[3,2-c]pyridin-1-yl)acetic acid;
{2-Methyl-3-[4-(methylsulfonyl)benzyl]-4-oxo-4,5,6,7-tetrahydro-1H-pyrrolo[3,2-c]pyridin-1-yl}acetic acid;
(3-{2-[(4-Chlorophenyl)sulfonyl]benzyl}-2-methyl-4-oxo-4,5,6,7-tetrahydro-1H-pyrrolo[3,2-c]pyridin-1-yl)acetic acid;
(3-{2-[(4-Methoxyphenyl)sulfonyl]benzyl}-2-methyl-4-oxo-4,5,6,7-tetrahydro-1H-pyrrolo[3,2-c]pyridin-1-yl)acetic acid;
[2-Methyl-4-oxo-3-(2-{[4-(trifluoromethoxy)phenyl]sulfonyl}benzyl)-4,5,6,7-tetrahydro-1 H-pyrrolo[3,2-c]pyridin-1-yl]acetic acid;
(3-{2-[(2-Fluorophenyl)sulfonyl]benzyl}-2-methyl-4-oxo-4,5,6,7-tetrahydro-1H-pyrrolo[3,2-c]pyridin-1-yl)acetic acid;
{3-[2-({3-[(Dimethylamino)carbonyl]phenyl}sulfonyl)benzyl]-2-methyl-4-oxo-4,5,6,7-tetrahydro-1H-pyrrolo[3,2-c]pyridin-1-yl}acetic acid;
(2S)-2-{2-Methyl-4-oxo-3-[2-(phenylsulfonyl)benzyl]-4,5,6,7-tetrahydro-1H-pyrrolo[3,2-c]pyridin-1-yl}propanoic acid;
(2R)-2-{2-Methyl-4-oxo-3-[2-(phenylsulfonyl)benzyl]-4,5,6,7-tetrahydro-1H-pyrrolo[3,2-c]pyridin-1-yl}propanoic acid;
{3-[3-Methoxy-2-(phenylsulfonyl)benzyl]-2-methyl-4-oxo-4,5,6,7-tetrahydro-1H-pyrrolo[3,2-c]pyridin-1-yl}acetic acid;
{3-[2-(Ethylsulfonyl)benzyl]-2-methyl-4-oxo-4,5,6,7-tetrahydro-1H-pyrrolo[3,2-c]pyridin-1-yl}acetic acid;
{3-[2-(Cyclohexylsulfonyl)benzyl]-2-methyl-4-oxo-4,5,6,7-tetrahydro-1H-pyrrolo[3,2-c]pyridin-1-yl}acetic acid;
{2-Methyl-3-[2-(morpholin-4-ylsulfonyl)benzyl]-4-oxo-4,5,6,7-tetrahydro-1H-pyrrolo[3,2-c]pyridin-1-yl}acetic acid;
{2-Methyl-4-oxo-3-[2-(1H-1,2,4-triazol-1-ylmethyl)benzyl]-4,5,6,7-tetrahydro-1H-pyrrolo[3,2-c]pyridin-1-yl}acetic acid;
Ethyl 2-(2,5-dimethyl-4-oxo-3-(2-(phenylsulfonyl)benzyl)-4,5,6,7-tetrahydro-1H-pyrrolo[3,2-c]pyridin-1-yl)acetate;
{2,5-Dimethyl-4-oxo-3-[2-(phenylsulfonyl)benzyl]-4,5,6,7-tetrahydro-1H-pyrrolo[3,2-c]pyridin-1-yl}acetic acid;
{3-[4-(Methoxycarbonyl)-2-(phenylsulfonyl)benzyl]-2-methyl-4-oxo-4,5,6,7-tetrahydro-1H-pyrrolo[3,2-c]pyridin-1-yl}acetic acid;
4-{[1-(Carboxymethyl)-2-methyl-4-oxo-4,5,6,7-tetrahydro-1H-pyrrolo[3,2-c]pyridin-3-yl]methyl}-3-(phenylsulfonyl)benzoic acid;
Methyl 2-(2-methyl-3-(4-(methylcarbamoyl)-2-(phenylsulfonyl)benzyl)-4-oxo-4,5,6,7-tetrahydro-1H-pyrrolo[3,2-c]pyridin-1-yl)acetate;
{2-Methyl-3-[4-[(methylamino)carbonyl]-2-(phenylsulfonyl)benzyl]-4-oxo-4,5,6,7-tetrahydro-1H-pyrrolo[3,2-c]pyridin-1-yl}acetic acid;
Methyl {3-[2-methoxy-6-(phenylsulfonyl)benzyl]-2-methyl-4-oxo-4,5,6,7-tetrahydro-1H-pyrrolo[3,2-c]pyridin-1-yl}acetate;
{3-[2-Methoxy-6-(phenylsulfonyl)benzyl]-2-methyl-4-oxo-4,5,6,7-tetrahydro-1H-pyrrolo[3,2-c]pyridin-1-yl}acetic acid;
(3-{2-[(3-Methoxyphenyl)sulfonyl]benzyl}-2-methyl-4-oxo-4,5,6,7-tetrahydro-1H-pyrrolo[3,2-c]pyridin-1-yl)acetic acid;
{2-Cyclopropyl-4-oxo-3-[2-(phenylsulfonyl)benzyl]-4,5,6,7-tetrahydro-1H-pyrrolo[3,2-c]pyridin-1-yl}acetic acid;
{5-Benzyl-2-methyl-4-oxo-3-[2-(phenylsulfonyl)benzyl]-4,5,6,7-tetrahydro-1H-pyrrolo[3,2-c]pyridin-1-yl}acetic acid;
{3-[5-Fluoro-2-(phenylsulfonyl)benzyl]-2-methyl-4-oxo-4,5,6,7-tetrahydro-1H-pyrrolo[3,2-c]pyridin-1-yl}acetic acid;
{2-Methyl-4-oxo-3-[4-(phenylsulfonyl)benzyl]-4,5,6,7-tetrahydro-1H-pyrrolo[3,2-c]pyridin-1-yl}acetic acid;
{3-[4-(Benzylsulfonyl)benzyl]-2-methyl-4-oxo-4,5,6,7-tetrahydro-1H-pyrrolo[3,2-c]pyridin-1-yl}acetic acid;
[2-Methyl-4-oxo-3-(2-phenoxybenzyl)-4,5,6,7-tetrahydro-1H-pyrrolo[3,2-c]pyridin-1-yl]acetic acid;
Ethyl {3-[5-bromo-2-(phenylsulfonyl)benzyl]-2-methyl-4-oxo-4,5,6,7-tetrahydro-1H-pyrrolo[3,2-c]pyridin-1-yl}acetate;
{3-[5-Bromo-2-(phenylsulfonyl)benzyl]-2-methyl-4-oxo-4,5,6,7-tetrahydro-1H-pyrrolo[3,2-c]pyridin-1-yl}acetic acid;
Ethyl (2-methyl-4-oxo-3-{[1-(phenylsulfonyl)-1H-pyrrol-2-yl]methyl}-4,5,6,7-tetrahydro-1H-pyrrolo[3,2-c]pyridin-1-yl)acetate;
(2-Methyl-4-oxo-3-{[1-(phenylsulfonyl)-1H-pyrrol-2-yl]methyl}-4,5,6,7-tetrahydro-1H-pyrrolo[3,2-c]pyridin-1-yl)acetic acid;
{5-Isopropyl-2-methyl-4-oxo-3-[2-(phenylsulfonyl)benzyl]-4,5,6,7-tetrahydro-1H-pyrrolo[3,2-c]pyridin-1-yl}acetic acid;
Ethyl [2-methyl-4-oxo-3-(quinolin-2-ylmethyl)-4,5,6,7-tetrahydro-1H-pyrrolo[3,2-c]pyridi n-1-yl]acetate;
[2-Methyl-4-oxo-3-(quinolin-2-ylmethyl)-4,5,6,7-tetrahydro-1H-pyrrolo[3,2-c]pyridin-1-yl]acetic acid;
(3-{2-[(4-Fluorophenyl)sulfonyl]-5-methoxybenzyl}-2-methyl-4-oxo-4,5,6,7-tetrahydro-1H-pyrrolo[3,2-c]pyridin-1-yl)acetic acid;
Ethyl {2-methyl-3-[2-(1-naphthyl)benzyl]-4-oxo-4,5,6,7-tetrahydro-1H-pyrrolo[3,2-c]pyridin-1-yl}acetate;
{2-Methyl-3-[2-(1-naphthyl)benzyl]-4-oxo-4,5,6,7-tetrahydro-1H-pyrrolo[3,2-c]pyridin-1-yl}acetic acid;
Ethyl {3-[(2'-chloro-6'-methoxybiphenyl-2-yl)methyl]-2-methyl-4-oxo-4,5,6,7-tetrahydro-1 H-pyrrolo[3,2-c]pyridin-1-yl}acetate;
{3-[(2'-Chloro-6'-methoxybiphenyl-2-yl)methyl]-2-methyl-4-oxo-4,5,6,7-tetrahydro-1H-pyrrolo[3,2-c]pyridin-1-yl}acetic acid;
Ethyl {3-[(2'-methoxybiphenyl-2-yl)methyl]-2-methyl-4-oxo-4,5,6,7-tetrahydro-1H-pyrrolo[3,2-c]pyridin-1-yl}acetate;
{3-[(2'-Methoxybiphenyl-2-yl)methyl]-2-methyl-4-oxo-4,5,6,7-tetrahydro-1H-pyrrolo[3,2-c]pyridin-1-yl}acetic acid;
Ethyl [3-(2-benzoylbenzyl)-2-methyl-4-oxo-4,5,6,7-tetrahydro-1H-pyrrolo[3,2-c]pyridin-1-yl]acetate;
[3-(2-Benzoylbenzyl)-2-methyl-4-oxo-4,5,6,7-tetrahydro-1H-pyrrolo[3,2-c]pyridin-1-yl]acetic acid;
Ethyl {2,6,6-trimethyl-4-oxo-3-[2-(phenylsulfonyl)benzyl]-4,5,6,7-tetrahydro-1H-pyrrolo[3,2-c]pyridin-1-yl}acetate;
{2,6,6-Trimethyl-4-oxo-3-[2-(phenylsulfonyl)benzyl]-4,5,6,7-tetrahydro-1H-pyrrolo[3,2-c]pyridin-1-yl}acetic acid;
Ethyl (3-{2-[(4-fluorophenyl)sulfonyl]benzyl}-2,6,6-trimethyl-4-oxo-4,5,6,7-tetrahydro-1H-pyrrolo[3,2-c]pyridin-1-yl)acetate;
(3-{2-[(4-Fluorophenyl)sulfonyl]benzyl}-2,6,6-trimethyl-4-oxo-4,5,6,7-tetrahydro-1H-pyrrolo[3,2-c]pyridin-1-yl)acetic acid;
Ethyl (3-{2-[(4-fluorophenyl)sulfonyl]-5-methoxybenzyl}-2,6,6-trimethyl-4-oxo-4,5,6,7-tetrahydro-1H-pyrrolo[3,2-c]pyridin-1-yl)acetate;
(3-{2-[(4-Fluorophenyl)sulfonyl]-5-methoxybenzyl}-2,6,6-trimethyl-4-oxo-4,5,6,7-tetrahydro-1H-pyrrolo[3,2-c]pyridin-1-yl)acetic acid;
Ethyl {3-[5-cyano-2-(phenylsulfonyl)benzyl]-2-methyl-4-oxo-4,5,6,7-tetrahydro-1H-pyrrolo[3,2-c]pyridin-1-yl}acetate;
{3-[5-Cyano-2-(phenylsulfonyl)benzyl]-2-methyl-4-oxo-4,5,6,7-tetrahydro-1H-pyrrolo[3,2-c]pyridin-1-yl}acetic acid;
tert-Butyl [3-(2-{[benzyl(methyl)amino]sulfonyl}benzyl)-2-methyl-4-oxo-4,5,6,7-tetrahydro-1H-pyrrolo[3,2-c]pyridin-1-yl]acetate;
[3-(2-{[Benzyl(methyl)amino]sulfonyl}benzyl)-2-methyl-4-oxo-4,5,6,7-tetrahydro-1H-pyrrolo[3,2-c]pyridin-1-yl]acetic acid;
Ethyl [3-(2-benzylbenzyl)-2-methyl-4-oxo-4,5,6,7-tetrahydro-H-pyrrolo[3,2-c]pyridin-1-yl]acetate;
[3-(2-Benzylbenzyl)-2-methyl-4-oxo-4,5,6,7-tetrahydro-1H-pyrrolo[3,2-c]pyridin-1-yl]acetic acid;
(3-{2-[Hydroxy(phenyl)methyl]benzyl}-2-methyl-4-oxo-4,5,6,7-tetrahydro-1H-pyrrolo[3,2-c]pyridin-1-yl)acetic acid;
[3-(2-{[(Cyclopropylcarbonyl)(ethyl)amino]methyl}benzyl)-2-methyl-4-oxo-4,5,6,7-tetrahydro-1H-pyrrolo[3,2-c]pyridin-1-yl]acetic acid;
[2-Methyl-4-oxo-3-[2-(phenylsulfonyl)benzyl]-5-(2,2,2-trifluoro-1-methylethyl)-4,5,6,7-tetrahydro-1H-pyrrolo[3,2-c]pyridin-1-yl]acetic acid;
{2-Methyl-4-oxo-3-[2-(phenylsulfonyl)benzyl]-4,5-dihydro-1H-pyrrolo[3,2-c]pyridin-1-yl}acetic acid;
Ethyl {2-methyl-4-oxo-3-[2-(phenylsulfonyl)benzyl]-4,5-dihydro-1H-pyrrolo[3,2-c]pyridin-1-yl}acetate;
Ethyl {2,5-dimethyl-4-oxo-3-[2-(phenylsulfonyl)benzyl]-4,5-dihydro-1H-pyrrolo[3,2-c]pyridin-1-yl}acetate;
{2,5-Dimethyl-4-oxo-3-[2-(phenylsulfonyl)benzyl]-4,5-dihydro-1H-pyrrolo[3,2-c]pyridin-1-yl}acetic acid;
Ethyl {5-(difluoromethyl)-2-methyl-4-oxo-3-[2-(phenylsulfonyl)benzyl]-4,5-dihydro-1H-pyrrolo[3,2-c]pyridin-1-yl}acetate;
{5-(Difluoromethyl)-2-methyl-4-oxo-3-[2-(phenylsulfonyl)benzyl]-4,5-dihydro-1H-pyrrolo[3,2-c]pyridin-1-yl}acetic acid;
2-(Dimethylamino)-2-oxoethyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
2-Morpholin-4-ylethyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
2,3-Dihydro-1H-inden-5-yl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
1-{[(Cyclohexyloxy)carbonyl]oxy}ethyl(3-{2-[(4-fluorophenyl)sulfonyl]benzyl}-2-methyl-4-oxo-4,5,6,7-tetrahydro-1H-pyrrolo[3,2-c]pyridin-1-yl)acetate;
(5-Methyl-2-oxo-1,3-dioxol-4-yl)methyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
2-(Dimethylamino)ethyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
2-Methoxyphenyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
or a pharmaceutically acceptable salt, N-oxide, solvate, stereiosomer or deuterated derivative thereof.

Of particular interest are the compounds:
{2-Methyl-4-oxo-3-[2-(phenylsulfonyl)benzyl]-4,5,6,7-tetrahydro-1H-pyrrolo[3,2-c]pyridin -1-yl}acetic acid;
(3-{2-[(4-Fluorophenyl)sulfonyl]benzyl}-2-methyl-4-oxo-4,5,6,7-tetrahydro-1H-pyrrolo-[3,2-c]pyridin-1-yl)acetic acid;
{3-[5-Methoxy-2-(phenylsulfonyl)benzyl]-2-methyl-4-oxo-4,5,6,7 -tetrahydro-1 H-pyrrolo-[3,2-c]pyridin-1-yl}acetic acid;
(3-{2-[(3-Chlorophenyl)sulfonyl]benzyl}-2-methyl-4-oxo-4,5,6,7-tetrahydro-1H-pyrrolo-[3,2-c]pyridin-1-yl)acetic acid;
{2-Methyl-4-oxo-3-[2-(phenylsulfonyl)-4-(trifluoromethyl)benzyl]-4,5,6,7-tetrahydro-1H-pyrrolo[3,2-c]pyridin-1-yl}acetic acid;
(3-{2-[(3-Fluorophenyl)sulfonyl]benzyl}-2-methyl-4-oxo-4,5,6,7-tetrahydro-1H-pyrrolo-[3,2-c]pyridin-1-yl)acetic acid;
(3-{2-[(4-Chlorophenyl)sulfonyl]benzyl}-2-methyl-4-oxo-4,5,6,7-tetrahydro-1H-pyrrolo-[3,2-c]pyridin-1-yl)acetic acid;
(3-{2-[(4-Methoxyphenyl)sulfonyl]benzyl}-2-methyl-4-oxo-4,5,6,7-tetrahydro-1H-pyrrolo-[3,2-c]pyridin-1-yl)acetic acid;
[2-Methyl-4-oxo-3-(2-{[4-(trifluoromethoxy)phenyl]sulfonyl}benzyl)-4,5,6,7-tetrahydro-1 H-pyrrolo[3,2-c]pyridin-1-yl]acetic acid;
(3-{2-[(2-Fluorophenyl)sulfonyl]benzyl}-2-methyl-4-oxo-4,5,6,7-tetrahydro-1H-pyrrolo-[3,2-c]pyridin-1-yl)acetic acid;
(2R)-2-{2-Methyl-4-oxo-3-[2-(phenylsulfonyl)benzyl]-4,5,6,7-tetrahydro-1H-pyrrolo-[3,2-c]pyridin-1-yl}propanoic acid;
{3-[3-Methoxy-2-(phenylsulfonyl)benzyl]-2-methyl-4-oxo-4,5,6,7-tetrahydro-1H-pyrrolo-[3,2-c]pyridin-1-yl}acetic acid;
{3-[2-(Cyclohexylsulfonyl)benzyl]-2-methyl-4-oxo-4,5,6,7-tetrahydro-1H-pyrrolo[3,2-c]-pyridin-1-yl}acetic acid;
{2-Methyl-3-[2-(morpholin-4-ylsulfonyl)benzyl]-4-oxo-4,5,6,7-tetrahydro-1H-pyrrolo-[3,2-c]pyridin-1-yl}acetic acid;
{2,5-Dimethyl-4-oxo-3-[2-(phenylsulfonyl)benzyl]-4,5,6,7-tetrahydro-1H-pyrrolo[3,2-c]-pyridin-1-yl}acetic acid;
{3-[4-(Methoxycarbonyl)-2-(phenylsulfonyl)benzyl]-2-methyl-4-oxo-4,5,6,7-tetrahydro-1H-pyrrolo[3,2-c]pyridin-1-yl}acetic acid;
4-{[1-(Carboxymethyl)-2-methyl-4-oxo-4,5,6,7-tetrahydro-1H-pyrrolo[3,2-c]pyridin-3-yl]-methyl}-3-(phenylsulfonyl)benzoic acid;
(2-Methyl-3-[4-[(methylamino)carbonyl]-2-(phenylsulfonyl)benzyl]-4-oxo-4,5,6,7-tetrahydro-1H-pyrrolo[3,2-c]pyridin-1-yl}acetic acid;
(3-{2-[(3-Methoxyphenyl)sulfonyl]benzyl}-2-methyl-4-oxo-4,5,6,7-tetrahydro-1H-pyrrolo-[3,2-c]pyridin-1-yl)acetic acid;
{2-Cyclopropyl-4-oxo-3-[2-(phenylsulfonyl)benzyl]-4,5,6,7-tetrahydro-1H-pyrrolo[3,2-c]-pyridin-1-yl}acetic acid;
{5-Benzyl-2-methyl-4-oxo-3-[2-(phenylsulfonyl)benzyl]-4,5,6,7-tetrahydro-1H-pyrrolo-[3,2-c]pyridin-1-yl}acetic acid;
{3-[5-Fluoro-2-(phenylsulfonyl)benzyl]-2-methyl-4-oxo-4,5,6,7-tetrahydro-1H-pyrrolo-[3,2-c]pyridin-1-yl}acetic acid;
{3-[4-(Benzylsulfonyl)benzyl]-2-methyl-4-oxo-4,5,6,7-tetrahydro-1H-pyrrolo[3,2-c]-pyridin-1-yl}acetic acid;
{3-[5-Bromo-2-(phenylsulfonyl)benzyl]-2-methyl-4-oxo-4,5,6,7-tetrahydro-1H-pyrrolo-[3,2-c]pyridin-1-yl}acetic acid;
(2-Methyl-4-oxo-3-{[1-(phenylsulfonyl)-1H-pyrrol-2-yl]methyl}-4,5,6,7-tetrahydro-1H-pyrrolo[3,2-c]pyridin-1-yl)acetic acid;
{5-Isopropyl-2-methyl-4-oxo-3-[2-(phenylsulfonyl)benzyl]-4,5,6,7-tetrahydro-1H-pyrrolo-[3,2-c]pyridin-1-yl}acetic acid;
(3-{2-[(4-Fluorophenyl)sulfonyl]-5-methoxybenzyl}-2-methyl-4-oxo-4,5,6,7-tetrahydro-1H-pyrrolo[3,2-c]pyridin-1-yl)acetic acid;
{2,6,6-Trimethyl-4-oxo-3-[2-(phenylsulfonyl)benzyl]-4,5,6,7-tetrahydro-1H-pyrrolo-[3,2-c]pyridin-1-yl}acetic acid;
(3-{2-[(4-Fluorophenyl)sulfonyl]benzyl}-2,6,6-trimethyl-4-oxo-4,5,6,7-tetrahydro-1H-pyrrolo[3,2-c]pyridin-1-yl)acetic acid;
(3-{2-[(4-Fluorophenyl)sulfonyl]-5-methoxybenzyl}-2,6,6-trimethyl-4-oxo-4,5,6,7-tetrahydro-1H-pyrrolo[3,2-c]pyridin-1-yl)acetic acid;
{3-[5-Cyano-2-(phenylsulfonyl)benzyl]-2-methyl-4-oxo-4,5,6,7-tetrahydro-1H-pyrrolo-[3,2-c]pyridin-1-yl}acetic acid;
[3-(2-{[Benzyl(methyl)amino]sulfonyl}benzyl)-2-methyl-4-oxo-4,5,6,7-tetrahydro-1H-pyrrolo[3,2-c]pyridin-1-yl]acetic acid;
(3-{2-[Hydroxy(phenyl)methyl]benzyl}-2-methyl-4-oxo-4,5,6,7-tetrahydro-1H-pyrrolo-[3,2-c]pyridin-1-yl)acetic acid;
[2-Methyl-4-oxo-3-[2-(phenylsulfonyl)benzyl]-5-(2,2,2-trifluoro-1-methylethyl)-4,5,6,7-tetrahydro-1H-pyrrolo[3,2-c]pyridin-1-yl]acetic acid;
{2-Methyl-4-oxo-3-[2-(phenylsulfonyl)benzyl]-4,5-dihydro-1H-pyrrolo[3,2-c]pyridin-1-yl}-acetic acid;
{2,5-Dimethyl-4-oxo-3-[2-(phenylsulfonyl)benzyl]-4,5-dihydro-1H-pyrrolo[3,2-c]pyridin-1-yl}acetic acid; or
{5-(Difluoromethyl)-2-methyl-4-oxo-3-[2-(phenylsulfonyl)benzyl]-4,5-dihydro-1H-pyrrolo-[3,2-c]pyridin-1-yl}acetic acid.

### GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and de-protection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein.

Processes for preparing compounds of the invention are provided as further embodiments of the invention and are illustrated by the procedures below.

R¹ R², R³, R^{3'}, R⁴, R^{4'}, R⁵, R⁶, R⁷, R^{a}, R^{b}, R^{c}, R^{d}, R^{e}, R^{f}, R^{g}, L and G are defined according to claim 1.

Specific synthetic processes not covered by Schemes 1 - 9 are described in detail in the Experimental section.

Compounds of general formula (Iₐ) (wherein R⁷ is not a hydrogen atom) may be prepared following the synthetic scheme depicted in Scheme 1.

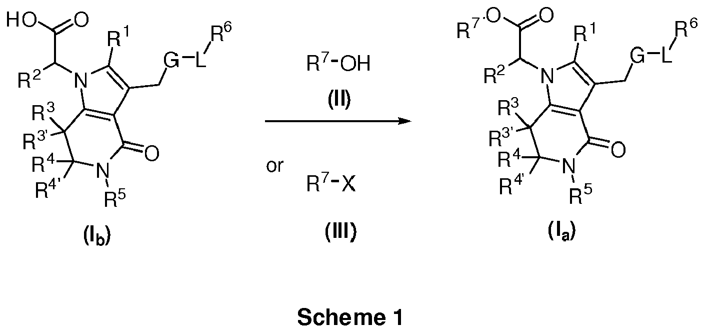

Compounds of general formula (Iₐ) may be prepared by the esterification of the carboxylic acid group of a compound of formula (I_{b}) with an alcohol of formula (II).

The reaction is carried out by mixing a compound of formula (I_{b}) with an alcohol of formula (II) in the presence of an acid such as sulphuric acid, methanesulphonic acid, tosic acid, hydrochloric acid or trifluoroacetic acid in the absence or presence of a solvent such as benzene or toluene at a temperature from 0ºC to the boiling point of the solvent to yield the compound of formula (Iₐ).

Alternatively, in some cases the reaction is carried out by mixing a compound of formula (I_{b}) with an alcohol of formula (II) in the presence of a dehydrating agent such as N,N'-dicyclohexylcarbodiimide (DCC) in the presence of a base such as 4-dimethylaminopyridine (DMAP), in a solvent such as dimethylformamide at a temperature from 0ºC to 50ºC to yield the compound of formula (Iₐ).

Alternatively, in some cases the reaction is carried out by mixing a compound of formula (I_{b}) with a halide of formula (III), wherein X represents a halogen atom such as chlorine, bromine or iodine.

The reaction is carried out by mixing a compound of formula (I_{b}) with a halide of formula (III), wherein X is hereinbefore defined in the presence of a base such as potassium carbonate in a solvent such as N,N-dimethylformamide at a temperature from 0ºC to 50 ºC to yield the compound of formula (Iₐ).

In the case where R⁵ and R⁷ are a hydrogen atom, compounds of the general formula (I_{c}) may be prepared following the synthetic scheme depicted in Scheme 2.

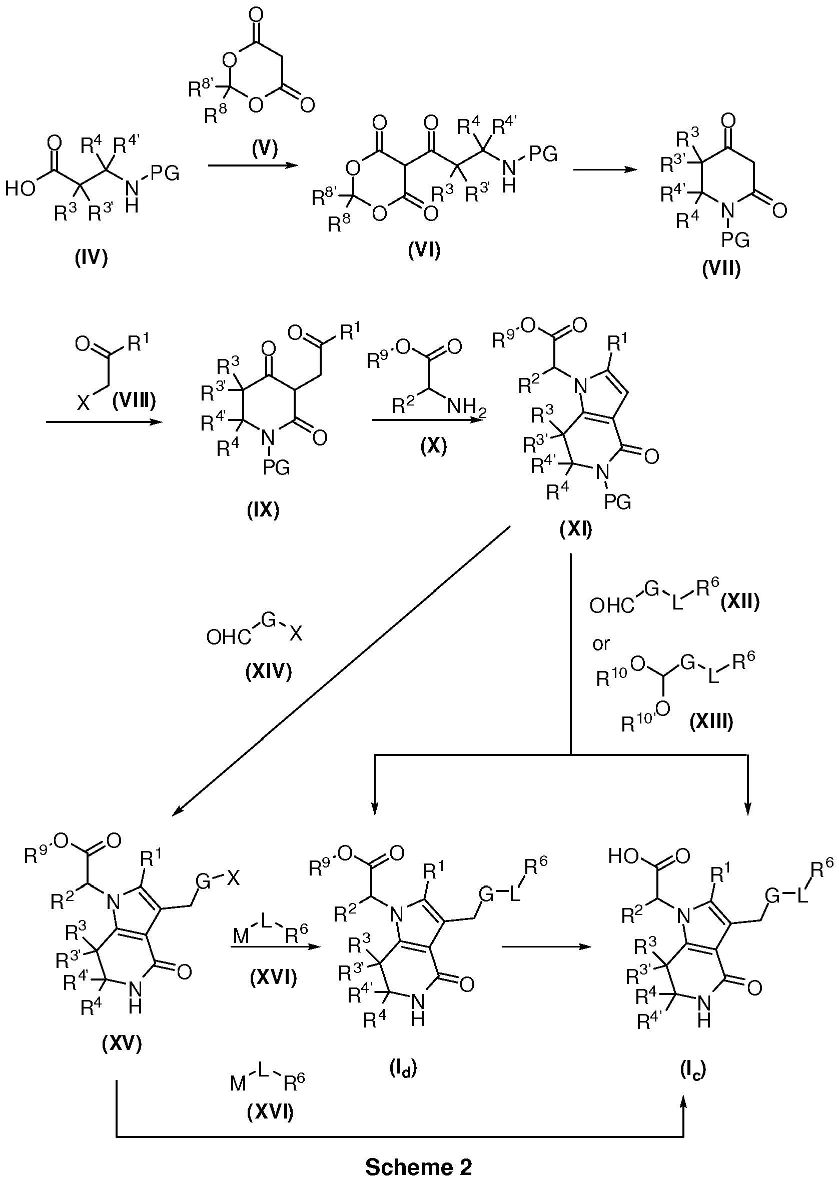

Compounds of general formula (VI) may be prepared from a compound of formula (IV) wherein PG represents a carbamate protecting group such as tert-butoxycarbonyl, methoxycarbonyl, ethoxycarbonyl or benzyloxycarbonyl with a cyclic diester of formula (V) wherein R⁸ and R^{8'} each independently represents an alkyl group such as methyl, ethyl or propyl, (Meldrum's acid refers to the compound of formula (V) where R⁸ and R^{8'} are both methyl) or R⁸ together with R^{8'} together form a carbocycle such as cyclopentyl.

The reaction is carried out by mixing a compound of formula (IV) with a Meldrum's acid derivative of formula (V), a peptide coupling reagent such as N,N'-dicyclohexylcarbodiimide or 1-(3-diaminopropyl)-3-ethylcarbodiimide hydrochloride and a base such as N,N-dimethylaminopyridine, in a solvent such as dichloromethane or chloroform at a temperature from -20ºC to 80ºC to yield the compound of formula (VI).

Compounds of general formula (VII) may be prepared by thermal rearrangement from a compound of formula (VI) wherein PG, R⁸ and R^{8'} are as hereinbefore defined. The reaction is carried out by heating a compound of formula (VI) in a solvent such as ethyl acetate at a temperature from 40ºC to the boiling point of the solvent to yield the compound of formula (VII).

Compounds of general formula (IX) may be prepared from compounds of formula (VII) wherein PG is as hereinbefore defined with an alkyl halide of formula (VIII) wherein X represents a chlorine, bromine or iodine atom. The reaction is carried out by treating a compound of formula (VII) with an alkyl halide of formula (VIII) in the presence of a base such as potassium carbonate in a solvent such as chloroform, dichloromethane, tetrahydrofuran or N,N-dimethylformamide at a temperature from 0ºC to the boiling point of the solvent to yield the compound of formula (IX).

Compounds of general formula (XI) may be prepared from compounds of formula (IX) with an amine of formula (X), wherein R⁹ represents an alkyl group such as methyl, ethyl, propyl, tert-butyl or benzyl. The reaction is carried out by treating a compound of formula (IX) with an amine of formula (X) or salt form thereof, in the presence or absence of a base such as sodium acetate, in a solvent such as ethanol at a temperature from 0ºC to the boiling point of the solvent to yield the compound of formula (XI).

Compounds of general formula (XV) may be prepared from compounds of formula (XI) by reaction with an aldehyde of formula (XIV) wherein X is as hereinbefore defined. The reaction is carried out by treating a compound of formula (XI) with an aldehyde of formula (XIV), in the presence of an acid such as trifluoroacetic acid or hydroiodic acid and in the presence of a reducing agent such as triethylsilane or hypophosphorous acid, in a solvent such as dichloromethane, chloroform, acetic anhydride, acetic acid, water or mixtures thereof at a temperature from 0ºC to the boiling point of the solvent to yield the compound of formula (XV).

In some instances, compounds of general formula (I_{d}) may be prepared from compounds of formula (XI) with an aldehyde of formula (XII) or an acetal of formula (XIII) wherein R¹⁰ and R^{10'} represent an alkyl group such as methyl, ethyl or propyl, or R¹⁰ and R^{10'} together represent an alkyl chain such as ethylene or propylene. The reaction is carried out by treating a compound of formula (XI) with an aldehyde of formula (XII) or an acetal of formula (XIII) in the presence of an acid such as trifluoroacetic acid or hydroiodic acid and in the presence of a reducing agent such as triethylsilane or hypophosphorous acid, in a solvent such as dichloromethane, chloroform, acetic anhydride, acetic acid, water or mixtures thereof at a temperature from 0ºC to the boiling point of the solvent to yield the compound of formula (I_{d}).

Additionally, compounds of general formula (I_{d}) may be prepared from compounds of formula (XV) with a suitable nucleophile of formula (XVI) wherein M represents hydrogen, or a metal such as lithium or caesium, or a metal salt such as magnesium chloride, magnesium bromide, zinc chloride, zinc bromide, or a group such as tributyltin, dihydroxyboron, 4,4,5,5-tetramethyl-1,3,2-dioxaborolane.

In the particular case where L is an oxygen atom or a sulphur atom, the reaction is carried out by treating a compound of formula (XV) with a nucleophile of formula (XVI) wherein M represents hydrogen in the presence of a base such as caesium carbonate, and a transition metal catalyst such as copper(I) iodide and a ligand such as N,N-dimethylglycine in a solvent such as dimethylformamide, dimethylsulfoxide or mixtures thereof at a temperature from ambient to the boiling point of the solvent to yield the compound of formula (I_{b}).

In the particular where L represents a CR^{b}R^{c} group or a direct bond, the reaction is carried out by treating a compound of formula (XV) with a nucleophile of formula (XVI) wherein M represents lithium or salts or complexes of magnesium, zinc, copper, tin or boron in the presence of a transition metal catalyst such as palladium, copper or nickel and suitable ligands and additives and in suitable solvents as recognised by one skilled in the art of transition metal catalysed coupling reactions to yield the compound of formula (I_{d}).

Compounds of general formula (I_{c}) may be prepared from compounds of formula (I_{d}) in the presence of either an acid such as trifluoroacetic acid or hydrochloric acid and in a solvent such as chloroform, dichloromethane or dioxane, or in the presence of a base such as lithium hydroxide in a solvent such as water, tetrahydrofuran, acetonitrile or mixtures thereof, at a temperature from 0ºC to the boiling point of the solvent. Additionally, in some instances compounds of general formula (I_{c}) may be prepared from compounds of formula (XI) with an aldehyde of formula (XII) or an acetal of formula (XIII) as hereinbefore defined. The reaction is carried out by treating a compound of formula (XI) with an aldehyde of formula (XIV) or an acetal of formula (XIII) in the presence of an acid such as trifluoroacetic acid or hydroiodic acid and in the presence of a reducing agent such as triethylsilane or hypophosphorous acid, in a solvent such as dichloromethane, chloroform, acetic anhydride, acetic acid, water or mixtures thereof at a temperature from 0ºC to the boiling point of the solvent to yield the compound of formula (I_{c}).

Additionally, in some instances compounds of general formula (I_{c}) may be prepared from compounds of formula (XV) with a suitable nucleophile of formula (XVI) wherein M represents hydrogen, or a metal such as lithium or caesium, or a metal salt such as magnesium chloride, magnesium bromide, zinc chloride, zinc bromide, or a group such as tributyltin, dihydroxyboron, 4,4,5,5-tetramethyl-1,3,2-dioxaborolane.

In the particular case where L represents a CR^{b}R^{c} group or a direct bond, the reaction is carried out by treating a compound of formula (XV) with a nucleophile of formula (XVI) wherein M represents lithium or salts or complexes of magnesium, zinc, copper, tin or boron in the presence of a transition metal catalyst such as palladium, copper or nickel and suitable ligands and additives and in suitable solvents as recognised by one skilled in the art of transition metal catalysed coupling reactions to yield the compound of formula (I_{c}).

In the particular case where L is a -SO₂- group, and R⁶ is not a -N(R^{b})COR^{a} group, intermediates of the general formula (XIIₐ) may be prepared following the synthetic scheme depicted in Scheme 3.

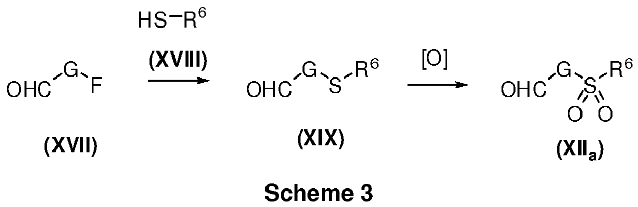

Compounds of formula (XIX) may be prepared from compounds of formula (XVII) with a thiol of formula (XVIII). The reaction is carried out by mixing a compound of formula (XVII) with a thiol of formula (XVIII) in the presence of a metal carbonate base such as potassium carbonate or sodium bicarbonate or a base such as sodium hydride, or a tertiary amine base such as triethylamine or diisopropylethylamine in a solvent such as dimethylsulfoxide, acetonitrile, tetrahydrofuran or N,N-dimethylformamide at a temperature from 0ºC to the boiling point of the solvent to yield the compound of formula (XIX).

Compounds of formula (XIIₐ) may be prepared from compounds of formula (XIX), by mixing a compound of formula (XIX) with an oxidising agent such as meta-chloroperbenzoic, peracetic acid, hydrogen peroxide or tert-butylhydrogen peroxide, or a high valent oxide salt such as permanganate or periodate, in a solvent such as dichloromethane, chloroform or water at a temperature from 0ºC to the boiling point of the solvent to yield the compound of formula (XIIₐ).

In the case where R⁵ is hydrogen, compounds of the general formula (I_{d}) may also be prepared following the synthetic scheme depicted in Scheme 4.

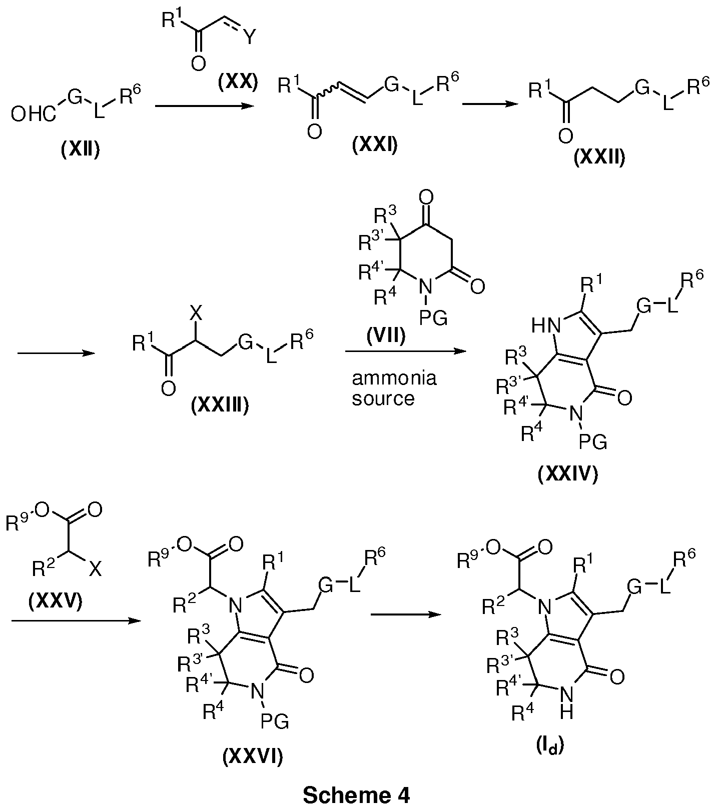

Compounds of general formula (XXI) may be prepared from compounds of formula (XII) and a compound of formula (XX) wherein Y is a phosphoranylidene such as triphenylphosphoranylidene, a triarylphosphonium salt such as triphenylphosphine chloride, triphenylphosphine bromide or triphenylphosphine iodide or a phosphonate ester such as diethyl phosphonate.

The reaction is carried out by mixing a compound of formula (XII) with a compound of formula (XX) in the absence or presence of a metal hydride base such as a sodium hydride, a metal alkoxide base such as potassium tert-butoxide or a metal carbonate base such as potassium carbonate or caesium carbonate, in a solvent such as toluene, benzene, tetrahydrofuran, dioxane or tert-butanol at a temperature from 0ºC to the boiling point of the solvent to yield the compound of formula (XXI).

Compounds of general formula (XXII) may be prepared from compounds of formula (XXI) under reducing conditions.

The reaction is carried out by treating a compound of formula (XXI) under conditions such as palladium on carbon under an atmosphere of hydrogen gas, or zinc metal in the presence of acetic acid, with suitable solvents as recognised by one skilled in the art of alkene reduction at a temperature from 0ºC to the boiling point of the solvent to yield the compound of formula (XXII).

Compounds of general formula (XXIII) wherein X represents a halogen atom such as chlorine, bromine or iodine may be prepared from compounds of formula (XXII) by halogenation.

The reaction is carried out by mixing a compound of formula (XXII) and a halogenating reagent such as triethylbromosilane in the presence of dimethylsulfoxide, N-chlorosuccinimide, N-bromosuccinimide or N-iodosuccinimide, in a solvent such as acetonitrile, dichloromethane or chloroform at a temperature from 0ºC to the boiling point of the solvent to yield the compound of formula (XXIII).

Compounds of general formula (XXIV) may be prepared from compounds of formula (VII) and a compound of formula (XXIII), wherein PG and X are as hereinbefore defined, in the presence of an ammonia source such as ammonium acetate in a solvent such as ethanol at a temperature from 0ºC to the boiling point of the solvent.

Compounds of general formula (XXVI) may be prepared from compounds of formula (XXIV) and a halide of formula (XXV) wherein X represents a halogen atom such as chlorine, bromine or iodine and R⁹ is hereinbefore defined.

The reaction is carried out by mixing a compound of formula (XXIV) with a halide of formula (XXV) in the presence of a base such as sodium hydride in a solvent such as dimethylformamide at a temperature from 0ºC to the boiling point of the solvent to yield the compound of formula (XXVI).

Compounds of general formula (I_{d}) may be prepared from compounds of formula (XXVI) by treating a compound of formula (XXVI) wherein PG and R²⁰ are hereinbefore defined either under acidic conditions such trifluoroacetic acid in dichloromethane or hydrochloric acid in dioxane, or under basic conditions such as lithium hydroxide in a mixture of water and tetrahydrofuran, at a temperature from 0ºC to the boiling point of the solvent to yield the compound of formula (I_{d}).

In the particular case where R⁵ is hydrogen, compounds of the general formula (I_{d}) may also be prepared following the synthetic scheme depicted in Scheme 5.

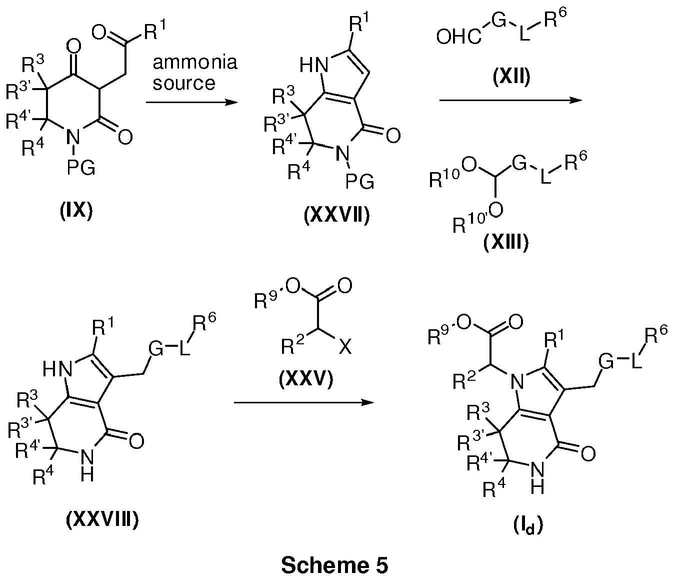

Compounds of general formula (XXVII) may be prepared from compounds of formula (IX) with an ammonia source, such as ammonium acetate in the presence or absence of an acid such as acetic acid, in a solvent such as ethanol at a temperature from 0ºC to the boiling point of the solvent.

Compounds of general formula (XXVIII) may be prepared from compounds of formula (XXVII) and either an aldehyde of formula (XII) or an acetal of formula (XIII) wherein R¹⁰ and R^{10'} represent an alkyl group such as methyl, ethyl or propyl, or R¹⁰ and R¹⁰' together represent an alkyl chain such as ethylene or propylene.

The reaction is carried out by treating a compound of formula (XXVII) with either an aldehyde of formula (XII) or an acetal of formula (XIII) in the presence of an acid such as trifluoroacetic acid or hydroiodic acid and in the presence of a reducing agent such as triethylsilane or hypophosphorous acid, in a solvent such as dichloromethane, chloroform, acetic anhydride, acetic acid, water or mixtures thereof at a temperature from 0ºC to the boiling point of the solvent to yield the compound of formula (XXVIII).

Compounds of general formula (I_{d}) may be prepared from compounds of formula (XXVIII) and a halide of formula (XXV), by mixing a compound of formula (XXVIII) with a halide of formula (XXV) in the presence of a base such as sodium hydride in a solvent such as dimethylformamide at a temperature from 0ºC to the boiling point of the solvent.

Compounds of the general formula (I_{b}) may also be prepared following the synthetic scheme depicted in Scheme 6.

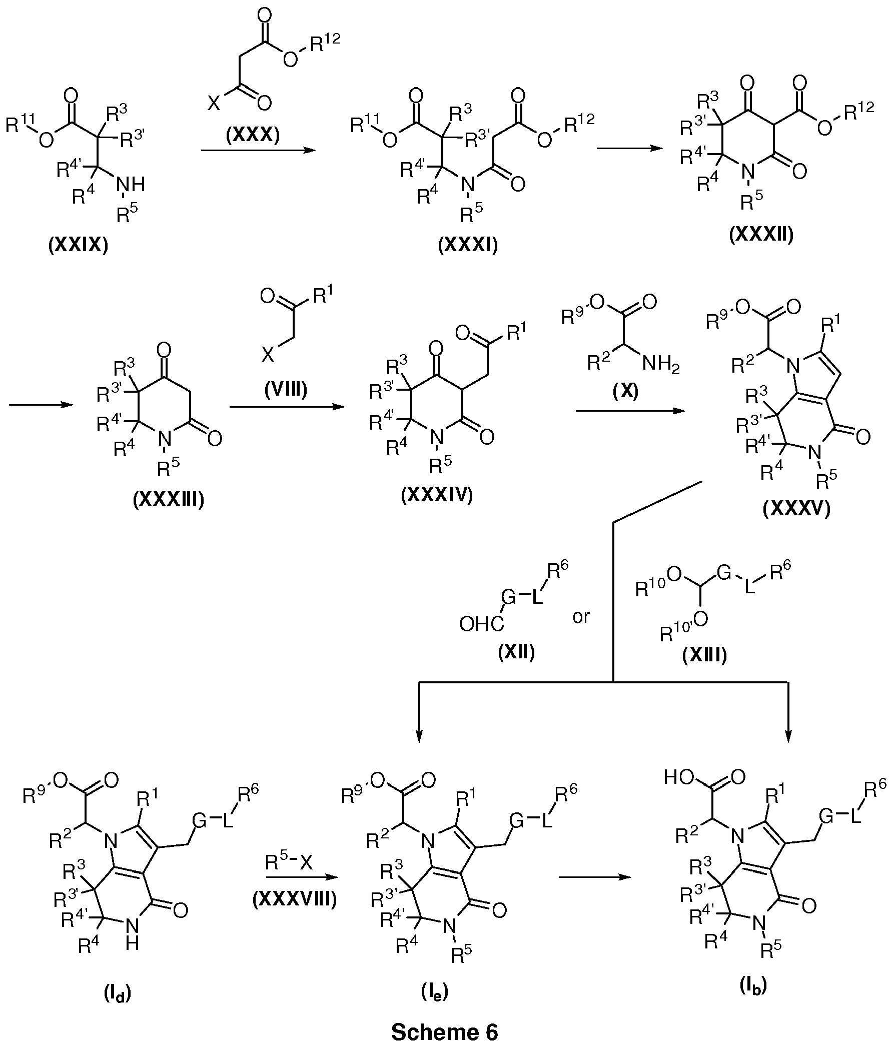

Compounds of general formula (XXXI) wherein R¹¹ and R¹² independently represent an alkyl group such as methyl, ethyl, propyl, tert-butyl or benzyl may be prepared from compounds of formula (XXIX) with and an acyl halide of formula (XXX) wherein X is as hereinbefore defined. The reaction is carried out by mixing a compound of formula (XXIX) with a compound of formula (XXX) in the presence of a base such as triethylamine, in a solvent such as tetrahydrofuran at a temperature from 0ºC to the boiling point of the solvent.

Compounds of general formula (XXXII) may be prepared from compounds of formula (XXXI) by treating the compound of formula formula (XXXI) with a base such as sodium methoxide, in a solvent such as methanol, toluene or mixtures thereof, at a temperature from ambient to the boiling point of the solvent.

Compounds of general formula (XXXIII) may be prepared from compounds of formula (XXXII) by thermal decarboxylation. The reaction is carried out by heating a compound of formula (XXXII) in a solvent such as acetonitrile, water or mixtures thereof at a temperature from 50ºC to the boiling point of the solvent to yield the compound of formula (XXXIII).

Compounds of general formula (XXXIV) may be prepared from compounds of formula (XXXIII) with a halide of formula (VIII), in the presence of a base such as potassium carbonate in a solvent such as chloroform at a temperature from 0ºC to the boiling point of the solvent to yield the compound of formula (XXXIV).

Compounds of general formula (XXXV) may be prepared from compounds of formula (XXXIV) and an amine of formula (X) wherein R⁹ is as hereinbefore defined. The reaction is carried out by treating a compound of formula (XXXIV) with an amine of formula (X) or salt form thereof, in the presence or absence of a base such as sodium acetate, in a solvent such as ethanol at a temperature from 0ºC to the boiling point of the solvent to yield the compound of formula (XXXV).

Compounds of general formula (Iₑ) may be prepared from compounds of formula (XXXV) with either an aldehyde of formula (XII) or an acetal of formula (XIII) wherein R¹⁰ and R^{10'} are as hereinbefore defined. The reaction is carried out by treating a compound of formula (XXXV) with either an aldehyde of formula (XII) or an acetal of formula (XIII) in the presence of an acid such as trifluoroacetic acid or hydroiodic acid and in the presence of a reducing agent such as triethylsilane or hypophosphorous acid, in a solvent such as dichloromethane, chloroform, acetic anhydride, acetic acid, water or mixtures thereof at a temperature from 0ºC to the boiling point of the solvent to yield the compound of formula (Iₑ).

Additionally, compounds of general formula (Iₑ) may be prepared from compounds of formula (I_{d}) with a halide of formula (XXXVIII) in the presence of a base such as lithium hexamethyldisilazide (LiHMDS) in a solvent such as tetrahydrofuran at a temperature from -78ºC to the boiling point of the solvent.

Compounds of general formula (I_{b}) may be prepared from compounds of formula (Iₑ), either in the presence of either an acid such as trifluoroacetic acid or hydrochloric acid and in a solvent such as chloroform, dichloromethane or dioxane, or in the presence of a base such as lithium hydroxide in a solvent such as water, tetrahydrofuran, acetonitrile or mixtures thereof, at a temperature from 0ºC to the boiling point of the solvent.

Alternatively, in the case where R⁵ is not hydrogen, compounds of the general formula (XXXIII) may be prepared following the synthetic scheme depicted in Scheme 7.

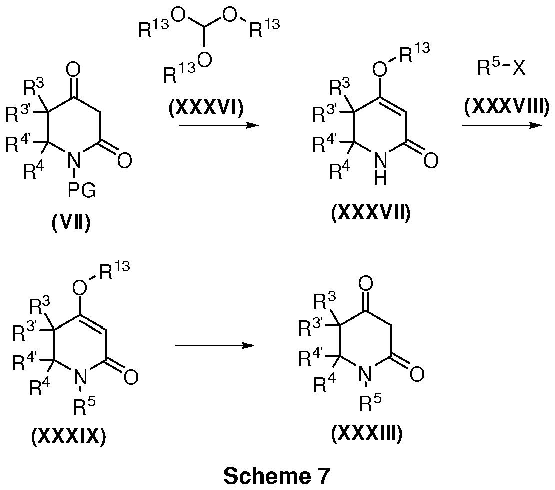

Compounds of general formula (XXXVII) wherein R¹³ represents an alkyl group such as methyl, ethyl, propyl or benzyl may be prepared from compounds of formula (VII) and an orthoformate of formula (XXXVI). The reaction is carried out by treating a compound of formula (VII) with an orthoformate of formula (XXXVI) wherein R¹³ is as hereinbefore defined in the presence of an acid such as para-toluenesulfonic acid or sulphuric acid in a solvent such as benzene or toluene, at a temperature from ambient to the boiling point of the solvent.

Compounds of general formula (XXXIX) may be prepared from compounds of formula (XXXVII) and a halide of formula (XXXVIII), in the presence of a base such as lithium hexamethyldisilazide (LiHMDS) in a solvent such as tetrahydrofuran at a temperature from -78ºC to the boiling point of the solvent to yield the compound of formula (XXXIX).

Compounds of general formula (XXXIII) may be prepared from compounds of formula (XXXIX) in the presence of an acid such as hydrochloric acid in a solvent such as tetrahydrofuran, water or mixtures thereof, at a temperature from ambient to the boiling point of the solvent.

In the particular case where R³ and R⁴ together form a bond and R⁵ is hydrogen atom, compounds of the general formula (I_{f}) may be prepared following the synthetic scheme depicted in Scheme 8.

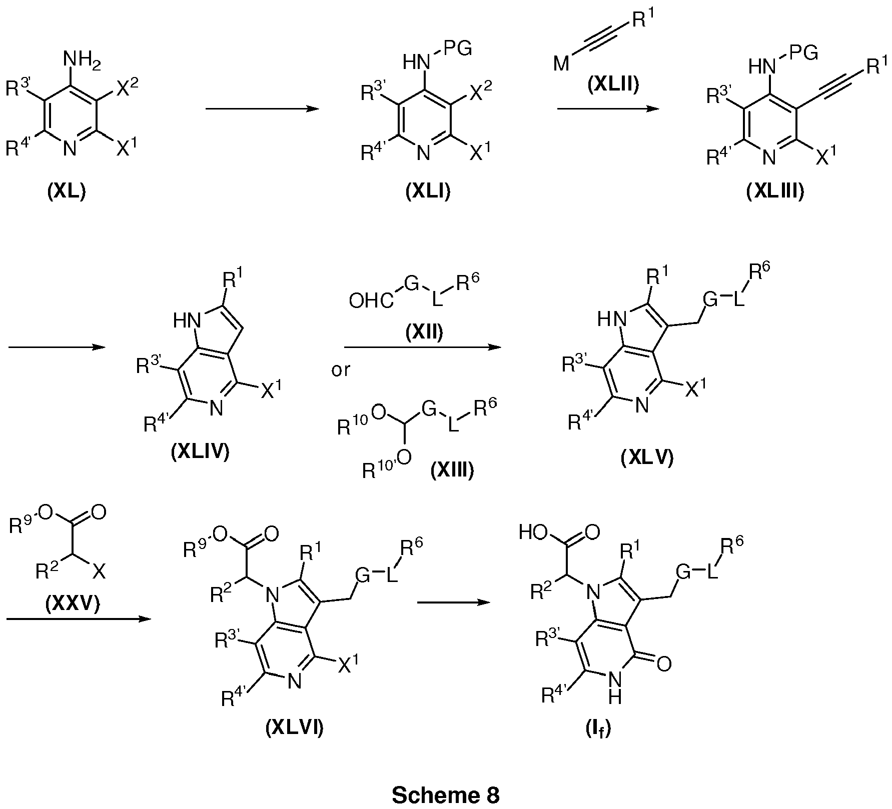

Compounds of general formula (XLI), wherein X¹ and X² independently represent a halogen atom such as chlorine, bromine or iodine, maybe prepared from compounds of formula (XL) according to standard preparative methods as recognised by one skilled in the art of amine protecting group chemistry.

For example, in one particular case the reaction is carried out by treating a compound of formula (XL) with di(tert-butyl)carbonate in a in a solvent such as tetrahydrofuran at a temperature from 0 ºC to the boiling point of the solvent to yield the compound of formula (XLI).

Compounds of general formula (XLIII) may be prepared from compounds of formula (XLI) with an alkyne of formula (XLII) wherein M represents hydrogen, lithium, magnesium salts such as magnesium chloride or magnesium bromide, zinc salts such as zinc chloride or zinc bromide, or trialkyl tin groups such as trimethyl tin or tributyl tin. In some cases for example, the reaction is carried out by treating a compound of formula (XLI) with an alkyne of formula (XLII) wherein M is hydrogen, with a one or more transition metal catalysts such as copper(I) iodide and bis(triphenylphosphine)palladium(II) dichloride, in the presence of a base such as triethylamine in a solvent such as dimethylformamide at a temperature from 0 ºC to the boiling point of the solvent to yield the compound of formula (XLIII).

In other cases for example, the reaction is carried out by treating a compound of formula (XLI) with an alkyne of formula (XLII) wherein M is tributyltin, with a one or more transition metal catalysts such as copper(I) iodide and tetrakis(triphenylphosphine)palladium in a solvent such as toluene at a temperature from ambient to the boiling point of the solvent to yield the compound of formula (XLIII).

Compounds of general formula (XLIV) may be prepared from compounds of formula (XLIII), by treating a compound of formula (XLIII) with a transition metal catalyst such as copper(I) iodide in a solvent such as dimethylformamide at a temperature from ambient to the boiling point of the solvent to yield the compound of formula (XLIV).

Compounds of general formula (XLV) may be prepared from compounds of formula (XLIV) with either an aldehyde of formula (XII) or an acetal of formula (XIII) in the presence of an acid such as trifluoroacetic acid or hydroiodic acid and in the presence of a reducing agent such as triethylsilane or hypophosphorous acid, in a solvent such as dichloromethane, chloroform, acetic anhydride, acetic acid, water or mixtures thereof at a temperature from 0ºC to the boiling point of the solvent.

Compounds of general formula (XLVI) may be prepared from compounds of formula (XLV) in the presence of a base such as sodium hydride in a solvent such as dimethylformamide at a temperature from 0ºC to the boiling point of the solvent to yield the compound of formula (XLVI).

Compounds of general formula (I_{f}) may be prepared from compounds of formula (XLVI) by treating a compound of formula (XLVI) in the presence of an acid such as acetic acid, a solvent such as water at a temperature from ambient to the boiling point of the solvent.

In the case where R³ and R⁴ together form an additional bond and R⁵ is not a hydrogen atom, compounds of the general formula (Iⱼ) may be prepared following the synthetic scheme depicted in Scheme 9.

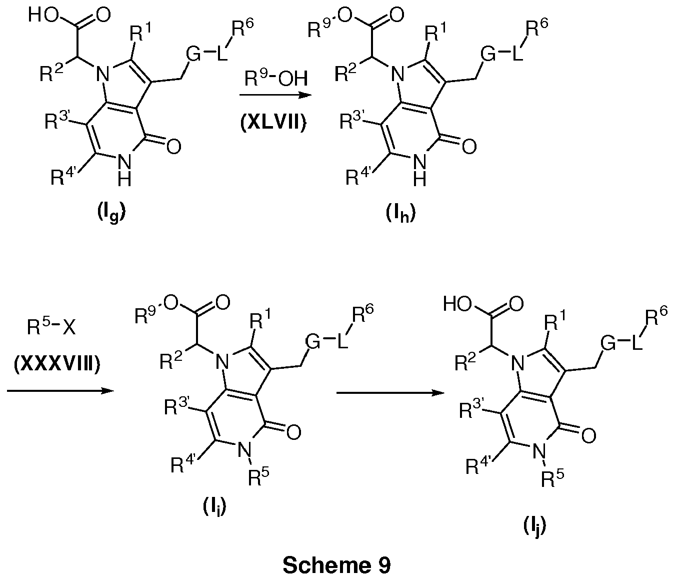

Compounds of general formula (Iₕ) may be prepared from compounds of formula (I_{g}) using suitable reagent and solvents as recognised by one skilled in the art of carboxylic acid esterification reactions.

In some cases for example, the reaction can be carried out by treating a compound of formula (I_{g}) with an alcohol of formula (XLVII) wherein R²⁰ is as hereinbefore described in the presence of an acid such as sulphuric acid at a temperature from ambient to the boiling point of the solvent.

Compounds of general formula (Iᵢ) may be prepared from compounds of formula (Iₕ) and a halide of formula (XXXVIII) in the presence of a base such as sodium hydride in a solvent such as dimethylformamide at a temperature from 0ºC to the boiling point of the solvent.

Compounds of general formula (Iⱼ) may be prepared from compounds of formula (Iᵢ) either in the presence of either an acid such as trifluoroacetic acid or hydrochloric acid and in a solvent such as chloroform, dichloromethane or dioxane, or in the presence of a base such as lithium hydroxide in a solvent such as water, tetrahydrofuran, acetonitrile or mixtures thereof, at a temperature from 0ºC to the boiling point of the solvent to yield the compound of formula (Iⱼ).

In the particular case where R³ and R⁴ together form an additional bond and R⁵ is CHF₂, compounds of the general formula (Iₘ) may be prepared following the synthetic scheme depicted in Scheme 10.

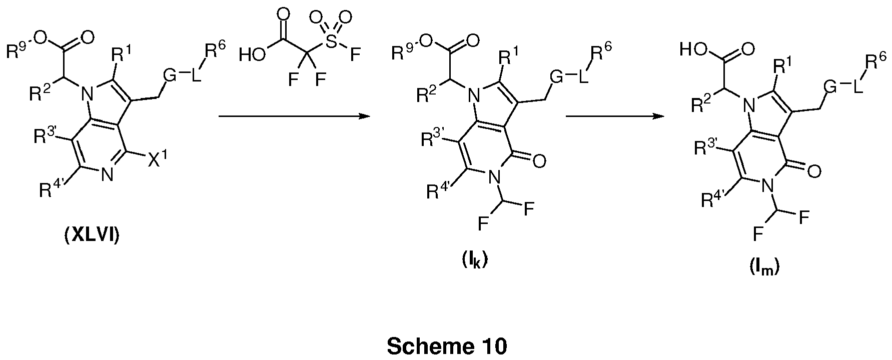

Compounds of general formula (Iₖ) may be prepared from compounds of formula (XLVI) with a suitable alkylating agent. The reaction is carried out by treating a compound of formula (XLVI) with 2,2-difluoro-2-(fluorosulfonyl)acetic acid in the presence of a base such as sodium bicarbonate and in a solvent such as water, acetonitrile or mixtures thereof at a temperature from 0ºC to the boiling point of the solvent to yield the compound of formula (Iₖ).

Compounds of general formula (Iₘ) may be prepared from compounds of formula (Iₖ) in the presence of either an acid such as trifluoroacetic acid or hydrochloric acid and in a solvent such as chloroform, dichloromethane or dioxane, or in the presence of a base such as lithium hydroxide in a solvent such as water, tetrahydrofuran, acetonitrile or mixtures thereof, at a temperature from 0ºC to the boiling point of the solvent to yield the compound of formula (Iₖ).

### PREPARATION EXAMPLES

The syntheses of the compounds of the invention are illustrated by the following Examples (1 to 84) including Preparation Examples (1 to 102) which do not limit the scope of the invention in any way.

### General

Reagents, starting materials, and solvents were purchased from commercial suppliers and used as received. Concentration or evaporation refer to evaporation under vacuum using a Büchi rotatory evaporator.

Reaction products were purified, when necessary, by flash chromatography on silica gel (40-63 µm) with the solvent system indicated. Purifications in reverse phase were made in a Biotage SP1® automated purification system equipped with a C18 column and using a gradient of water-acetonitrile/MeOH (1:1) (0.1% v/v ammonium formate both phases) from 0% to 100% acetonitrile/MeOH (1:1) in 40 column volumes. The conditions "formic acid buffer" refer to the use of 0.1 % v/v formic acid in both phases. The appropriate fractions were collected and the solvents evaporated under reduced pressure and/or liofilized.

Gas chromatography was performed using a Thermo Trace Ultra gas chromatograph, coupled to a DSQ mass detector. Injections were performed on a split/splitless injector and a HP-1MS was the capillary column. Mass spectra were obtained by electron impact ionisation at 70eV.

Preparative HPLC-MS were performed on a Waters instrument equipped with a 2767 injector/collector, a 2525 binary gradient pump, a 2996 PDA detector, a 515 pump as a make-up pump and a ZQ4000 Mass spectrometer detector.

The HPLC chromatographic separations were obtained using a Waters 2795 system equipped with a Symmetry C18 (2.1 x 50 mm, 3.5 µM) column for methods A, B and C and a Symmetry C18 (2.1 x 100 mm, 3.5 µM) for method D. The mobile phases were (B): formic acid (0.4 mL), ammonia (0.1 mL), methanol (500 mL) and acetonitrile (500 mL) and (A): formic acid (0.5 mL), ammonia (0.125 mL) and water (1000 mL) (A), the gradients are specified in the following table for each method used.

| Method | Run time | 0% B | 0 to 95% B | 95% B |
|---|---|---|---|---|
| A | 5 min | 0.2 min | 3 min | 0.8 min |
| B | 9 min | 0.5 min | 6.5 min | 1 min |
| C | 15 min | 0 min | 10.5 min | 1.5 min |
| D | 30 min | 0 min | 20 min | 4 min |

The flow rate was 0.8 mL/min for method A and 0.4 mL/min for method B, C and D. The injection volume was 5 microliter. A Waters 2996 diode array was used as a UV detector. Chromatograms were processed at 210 nM or 254 nM. Mass spectra of the chromatograms were acquired using positive and negative electrospray ionization in a Micromass ZMD or in a Waters ZQ detectors coupled to the HPLC.

The UPLC chromatographic separations were obtained using a Waters Acquity UPLC system coupled to a SQD mass spectrometer detector. The system was equipped with an ACQUITY UPLC BEH C-18 (2.1x50mm, 1.7 µm) column. The mobile phase was formic acid (0.4 mL), ammonia (0.1 mL), methanol (500 mL) and acetonitrile (500 mL) (B) and formic acid (0.5 mL), ammonia (0.125 mL) and water (1000 mL) (A). A gradient between 0 to 95% of B was used. The run time was 3 or 5 minutes The injection volume was 0.5 microliter. Chromatograms were processed at 210 nM or 254 nM. Mass spectra of the chromatograms were acquired using positive and negative electrospray ionization.

¹H Nuclear Magnetic Resonance Spectra were recorded on a Varian Gemini-2000 spectrometer operating at a frequency of 300MHz for the ¹H spectra or in a Varian Mercury plus operating at a frequency of 400MHz for the ¹H spectra. Samples were dissolved in the specified deuterated solvent. Tetramethylsilane was used as reference. The ¹H NMR spectrum for the following compounds was not recorded: Preparations 10, 16, 18, 20, 49, 52, 56, 57, 59, 60, 62, 63, 64, 70, 71, 72, 74 and 75. Examples 3, 5, 7, 9, 11, 30, 41, 57, 63, 65, 67, 73 and 74.

Mass Spectra (*m*/*z*) were recorded on a Micromass ZMD or in a Waters ZQ mass spectrometer using ESI ionization. The mass spectrum for the following compounds was not recorded: Preparations 6, 8,12,14, 22, 23, 25,29, 30, 32, 44, 67, 69, 96 and 102. Example 32.

### PREPARATIONS

### PREPARATION 1

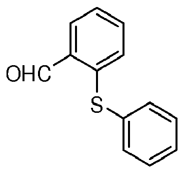

### 2-(Phenylthio)benzaldehyde

2-Fluorobenzaldehyde (9 ml, 90 mmol) and benzenethiol (8.8 ml, 90 mmol) were dissolved in 30 ml dimethylsulfoxide. Potassium carbonate (26 g, 190 mmol) was added and the mixture was heated at 100ºC for 4 h. The mixture was allowed to cool and was poured into water. The aqueous layer was extracted with ethyl acetate. The combined organics were washed with water and brine and were dried over sodium sulphate. Filtration and evaporation give 18.5 g (78.3 mmol, 92%) of the title compound as a yellow oil. Used as such without further purification. Purity 91%.
¹H NMR (400 MHz, CHLOROFORM-d) δ ppm 7.08 - 7.12 (m, 1 H), 7.29 - 7.46 (m, 7 H), 7.88 (dd, J=7.42, 1.56 Hz, 1 H), 10.40 (s, 1 H).
HPLC/MS (9 min) retention time 6.53 min.
LRMS: *m*/*z* 215 (M+1).

### PREPARATION 2

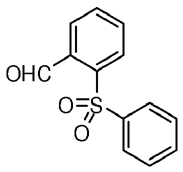

### 2-(Phenylsulfonyl)benzaldehyde

The title compound of Preparation 1 (3.5 g, 16.3 mmol) was dissolved in 50 ml dichloromethane. 3-Chloroperbenzoic acid (77% purity, 11 g, 49 mmol) was added in portions and the mixture was stirred at room temperature overnight. The mixture was washed with sodium carbonate 5% solution, water and brine. The resulting organic layer was dried over sodium sulphate, filtered and evaporated. The residue was purified using the SP1 Purification System (ethyl acetate-hexane gradient, 0:100 rising to 30:70) to give 1.9 g (7.7 mmol, 47%) of the title compound as a white solid. Purity 100%.
¹H NMR (400 MHz, DMSO-d6) δ ppm 7.67 (t, J=7.62 Hz, 2 H), 7.75 (t, J = 7.4 Hz, 1H), 7.88 - 7.99 (m, 3 H), 8.02 (d, J=7.42 Hz, 2 H), 8.20 (d, J=7.82 Hz, 1 H), 10.68 (s, 1H).
HPLC/MS (9 min) retention time 5.47 min.
LRMS: *m*/*z* 247 (M+1).

### PREPARATION 3

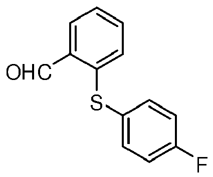

### 2-[(4-Fluorophenyl)thio]benzaldehyde

2-Fluorobenzaldehyde (1.86 g, 15.0 mmol) was treated with 4-fluorobenzenethiol (1.6 ml, 15.0 mmol) and potassium carbonate (4.67 g, 33.8 mmol) according to the method of Preparation 1 to give 2.95 g (11.8 mmol, 79%) of the title compound. Used as such without further purification. Purity 93%.
¹H NMR (400 MHz, DMSO-d6) δ ppm 6.87 (d, J=8.21 Hz, 1H), 7.27 - 7.46 (m, 3 H), 7.49 - 7.56 (m, 1H), 7.56 - 7.63 (m, 2 H), 7.97 (d, J=7.42 Hz, 1H), 10.22 (s, 1H).
HPLC/MS (9 min) retention time 6.57 min.
LRMS: *m*/*z* 233 (M+1).

### PREPARATION 4

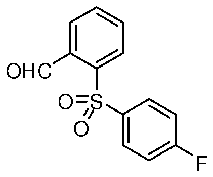

### 2-[(4-Fluorophenyl)sulfonyl]benzaldehyde

The title compound of Preparation 3 (2.0 g, 7.96 mmol) was dissolved in 30 ml dichloromethane and the mixture was cooled in an ice-bath. 3-Chloroperbenzoic acid (77% purity, 4.12 g, 23.9 mmol) was added in portions and the mixture was stirred at room temperature 2 h. The mixture was washed sequentially with 25% solution potassium hydrogen sulphate, 1 N sodium hydroxide and brine. The resulting organic layer was dried over sodium sulphate, filtered and evaporated to give 1.88 mg (7.11 mmol, 89%) of the title compound as a yellow solid. Used as such without further purification. Purity 100%.
¹H NMR (400 MHz, DMSO-d6) δ ppm 7.51 (t, J=8.79 Hz, 2 H), 7.87 - 7.99 (m, 3 H), 8.07 - 8.16 (m, 2 H), 8.20 (d, J=7.03 Hz, 1H), 10.66 (s, 1H).
GC/MS (18 min) retention time 10.0 min.
LRMS: *m*/*z* 265 (M+1).

### PREPARATION 5

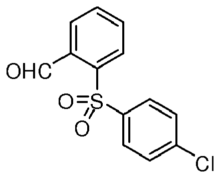

### 2-[(4-Chlorophenyl)sulfonyl]benzaldehyde

2-(4-Chlorophenylthio)benzaldehyde (1 g, 4.02 mmol) was treated with 3-chloroperbenzoic acid (77% purity, 2.45 g, 12.0 mmol) according to the method of Preparation 4 to give 650 mg (2.31 mmol, 58%) of the title compound as a white solid. Purity 100%.
¹H NMR (400 MHz, CHLOROFORM-d) δ ppm 7.52 (d, J=8.60 Hz, 2 H), 7.74 - 7.80 (m, 2 H), 7.84 (d, J=8.60 Hz, 2 H), 8.00 - 8.06 (m, 1H), 8.15 - 8.21 (m, 1H), 10.83 (s, 1H).
GC/MS (18 min) retention time 10.66 min.
LRMS: *m*/*z* 281 (M+1).

### PREPARATION 6

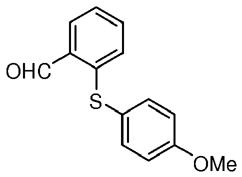

### 2-[(4-Methoxyphenyl)thio]benzaldehyde

2-Fluorobenzaldehyde (1 g, 8.06 mmol) was treated with 4-methoxybenzenethiol (1 ml, 8.13 mmol) and potassium carbonate (2.5 g, 18.09 mmol) according to the method of Preparation 1 to give 1.0 g (4.09 mmol, 51%). Used as such without further purification.
¹H NMR (400 MHz, CHLOROFORM-d) δ ppm 10.34 (1H, s), 7.82 (1H, d, J=1.6 Hz), 7.45 (2 H, d, J=8.6 Hz), 7.34 (1H, t, J=7.0 Hz), 7.24 (1H, d, J=7.0 Hz), 6.96 (2 H, d, J=8.6 Hz), 6.91 (1H, d, J=7.8 Hz), 3.85 (3 H, s).

### PREPARATION 7

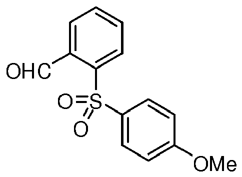

### 2-[(4-Methoxyphenyl)sulfonyl]benzaldehyde

The title compound of Preparation 6 (200 mg, 0.82 mmol) was treated with 3-chloroperbenzoic acid (77% purity, 550 mg, 0.245 mmol) according to the method of Preparation 2 to give 200 mg (0.67 mmol, 82%). Used as such without further purification. Purity 93%.
¹H NMR (400 MHz, CHLOROFORM-d) δ ppm 10.89 (1H, s), 8.13 (1H, d, J=7.4 Hz), 8.01 (1H, d, J=7.4 Hz), 7.83 (2 H, d, J=9.0 Hz), 7.58 - 7.78 (2 H, m), 6.99 (2 H, d, J=9.0 Hz), 3.86 (3 H, s).
HPLC/MS (9 min) retention time 5.68 min.
LRMS: *m*/*z* 277 (M+1).

### PREPARATION 8

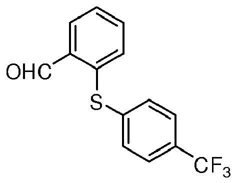

### 2-(Phenylthio)-4-(trifluoromethyl)benzaldehyde

2-Fluoro-4-(trifluoromethyl)benzaldehyde (1.5 g, 7.81 mmol) was treated with benzenethiol (0.8 ml, 7.81 mmol) and potassium carbonate (2.43 g, 17.58 mmol) according to the method of Preparation 1 to give 2.0 g (7.1 mmol, 91%) of the title compound as a yellow oil. Used as such without further purification.
¹H NMR (400 MHz, CHLOROFORM-d) δ ppm 7.27 - 7.32 (m, 1H), 7.37 - 7.51 (m, 5 H), 7.54 (d, J=7.82 Hz, 1H), 7.97 (d, J=8.21 Hz, 1H), 10.43 (s, 1 H).

### PREPARATION 9

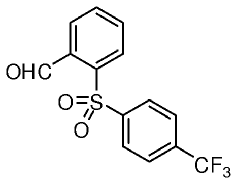

### 2-(Phenylsulfonyl)-4-(trifluoromethyl)benzaldehyde

The title compound of Preparation 8 (2.0 g, 7.1 mmol) was treated with 3-chloroperbenzoic acid (77% purity, 3.67 g, 21.3 mmol) according to the method of Preparation 4 to give 1.55 g (4.93 mmol, 70%) to the title compound as a yellow oil. Used as such without further purification. Purity 100%.
¹H NMR (400 MHz, CHLOROFORM-d) δ ppm 7.59 (t, J=7.42 Hz, 2 H), 7.66 (t, 1 H), 7.93 (d, J=7.42 Hz, 2 H), 7.98 (d, J=8.21 Hz, 1H), 8.13 (d, J=8.21 Hz, 1H), 8.45 (s, 1H), 10.88 (s, 1H).
GC/MS (18 min) retention time 9.52 min.
LRMS: *m*/*z* 315 (M+1).

### PREPARATION 10

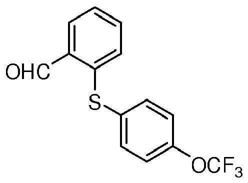

### 2-{[4-(Trifluoromethoxy)phenyl]thio}benzaldehyde

2-Fluorobenzaldehyde (0.55 ml, 5.2 mmol) was treated with 4-(trifluoromethoxy)-benzenethiol (1.0 g, 5.15 mmol) and potassium carbonate (1.58 g, 11.4 mmol) according to the method of Preparation 1 to give 1.35 g (4.5 mmol, 82%) of the title compound. Used as such without further purification. Purity 93%.
HPLC/MS (5 min) retention time 4.54 min.
LRMS: *m*/*z* 299 (M+1).

### PREPARATION 11

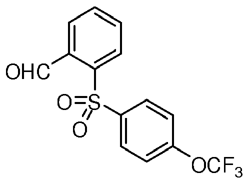

### 2-{[4-(Trifluoromethoxy)phenyl]sulfonyl}benzaldehyde

The title compound of Preparation 10 (1.35 g, 4.5 mmol) was treated with 3-Chloroperbenzoic acid (3.25 g, 11.3 mmol) according to the method of Preparation 4 to give 931 mg (2.64 mmol, 59%) of the title compound. Used as such without further purification. Purity 94%
¹H NMR (400 MHz, CHLOROFORM-d) δ ppm 7.36 (d, J=8.79 Hz, 2 H), 7.74 - 7.83 (m, 2 H), 7.94 - 8.00 (m, 2 H), 8.02 - 8.07 (m, 1H), 8.18 - 8.23 (m, 1H), 10.83 (s, 1H).
HPLC/MS (5 min) retention time 4.11 min.
LRMS: *m*/*z* 331 (M+1).

### PREPARATION 12

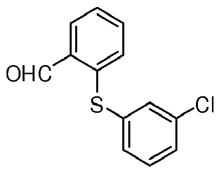

### 2-[(3-Chlorophenyl)thio]benzaldehyde

2-Fluorobenzaldehyde (1.5 g, 12.1 mmol) was treated with 3-chlorobenzenethiol (1.20 ml, 7.81 mmol) and potassium carbonate (3.76 g, 27.2 mmol) according to the method of Preparation 1 to give 2.73 g (11.0 mmol, 91 %) of the title compound. Used as such without further purification.
¹H NMR (400 MHz, CHLOROFORM-d) δ ppm 7.2 (d, J=7.8 Hz, 1H), 7.3 (m, 3 H), 7.4 (m, 2 H), 7.5 (m, 1H), 7.9 (m, 1H), 10.4 (s, 1H).

### PREPARATION 13

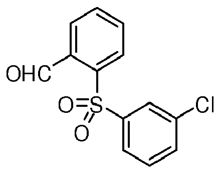

### 2-[(3-Chlorophenyl)sulfonyl]benzaldehyde

The title compound of Preparation 12 (1.5 g, 6.03 mmol) was treated with 3-chloroperbenzoic acid (77% Purity, 3.65 g, 18.0 mmol) according to the method of Preparation 4 to give 1.36 g (4.85 mmol, 80%) to the title compound. Used as such without further purification. Purity 100%.
¹H NMR (400 MHz, CHLOROFORM-d) δ ppm 7.50 (t, J=7.82 Hz, 1H), 7.58 (d, J=8.21 Hz, 1H), 7.74 - 7.84 (m, 3 H), 7.86 (s, 1H), 8.02 - 8.08 (m, 1H), 8.21 (dd, 1H), 10.82 (s, 1H).
GC/MS (18 min) retention time 10.6 min.
LRMS: *m*/*z* 281 (M+1).

### PREPARATION 14

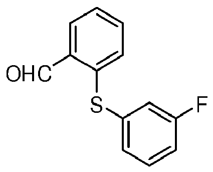

### 2-[(3-Fluorophenyl)thio]benzaldehyde

2-Fluorobenzaldehyde (500 mg, 4.03 mmol) was treated with 3-fluorobenzenethiol (0.60 ml, 5.76 mmol) and potassium carbonate (1.5 g, 10.85 mmol) according to the method of Preparation 1 to give 908 mg (3.90 mmol, 97%) of the title compound. Used as such without further purification.
¹H NMR (400 MHz, CHLOROFORM-d) δ ppm 6.96 - 7.10 (m, 2 H), 7.15 (d, J=7.42 Hz, 1H), 7.19 - 7.28 (m, 1H), 7.28 - 7.37 (m, 1H), 7.41 (t, J=7.42 Hz, 1H), 7.44 - 7.52 (m, 1H), 7.92 (d, J=7.42 Hz, 1H), 10.40 (br. s., 1H).

### PREPARATION 15

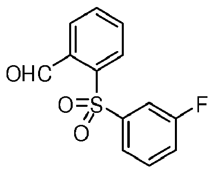

### 2-[(3-Fluorophenyl)sulfonyl]benzaldehyde

The title compound of Preparation 14 (793 mg, 3.41 mmol) was treated with 3-chloroperbenzoic acid (77% Purity, 2.08 g, 10.2 mmol) according to the method of Preparation 4 to give 540 mg (2.04 mmol, 60%) to the title compound. Used as such without further purification. Purity 100%.
¹H NMR (400 MHz, CHLOROFORM-d) δ ppm 7.32 (td, J=8.21, 2.34 Hz, 1H), 7.50 - 7.62 (m, 2 H), 7.71 (d, J=7.82 Hz, 1H), 7.76 - 7.81 (m, 2 H), 8.05 (dd, J=7.23, 1.76 Hz, 1H), 8.21 (dd, 1H), 10.82 (s, 1H).
GC/MS (18 min) retention time 9.97 min.
LRMS: *m*/*z* 265 (M+1).

### PREPARATION 16

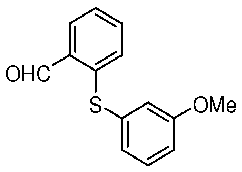

### 2-[(3-Methoxyphenyl)thio]benzaldehyde

2-Fluorobenzaldehyde (755 µl, 7.20 mmol) was treated with 3-methoxybenzenethiol (1.0 g, 7.13 mmol) and potassium carbonate (2.19 g, 15.8 mmol) according to the method of Preparation 1 to give 1.37 g of the crude title compound. Used as such without further purification. Purity 86%
HPLC/MS (5 min) retention time 4.33 min.
LRMS: *m*/*z* 245 (M+1).

### PREPARATION 17

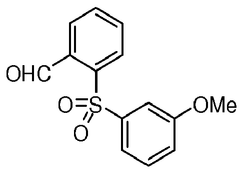

### 2-[(3-Methoxyphenyl)sulfonyl]benzaldehyde

The crude title compound of Preparation 16 (1.37 g) was treated with 3-chloroperbenzoic acid (77% Purity, 4.03 g, 14.0 mmol) according to the method of Preparation 4. The resulting residue was purified using the SP1 Purification System (ethyl acetate-hexane gradient, 0:100 rising to 100:0) to give 831 mg (2.94 mmol, 41% over two steps) of the title compound. Purity 98%.
¹H NMR (400 MHz, CHLOROFORM-d) δ ppm 3.84 (s, 3 H), 7.09 - 7.15 (m, 1 H), 7.38 - 7.41 (m, 1H), 7.42 - 7.46 (m, 2 H), 7.71 - 7.80 (m, 2 H), 8.01 - 8.05 (m, 1H), 8.17 - 8.20 (m, 1H), 10.86 (s, 1H).
HPLC/MS (5 min) retention time 3.69 min.
LRMS: *m*/*z* 277 (M+1).

### PREPARATION 18

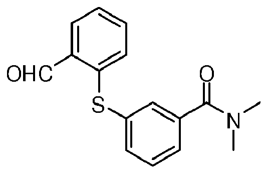

### 3-[(2-Formylphenyl)thio]-N,N-dimethylbenzamide

2-Fluorobenzaldehyde (235 µl, 2.23 mmol) was treated with 3-mercapto-N,N-dimethylbenzamide (400 mg, 2.2 mmol) and potassium carbonate (680 mg, 4.9 mmol) according to the method of Preparation 1 to give 452 mg of the crude title compound. Used as such without further purification. Purity 90%
HPLC/MS (5 min) retention time 3.82 min.
LRMS: *m*/*z* 286 (M+1).

### PREPARATION 19

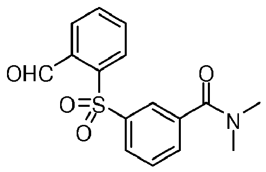

### 3-[(2-Formylphenyl)sulfonyl]-N,N-dimethylbenzamide

The crude title compound of Preparation 18 (452 mg) was treated with 3-chloroperbenzoic acid (77% purity, 1.14 g, 3.95 mmol) according to the method of Preparation 4 to give 359 mg (1.05 mmol, 47% over two steps) of the title compound. Used as such without further purification. Purity 93%
¹H NMR (400 MHz, CHLOROFORM-d) δ ppm 2.95 (s, 3 H), 3.12 (s, 3 H), 7.61 (t, J=7.72 Hz, 1H), 7.67 - 7.70 (m, 1H), 7.74 - 7.80 (m, 2 H), 7.92 - 7.94 (m, 1H), 7.96 - 7.99 (m, 1H), 8.01 - 8.04 (m, 1H), 8.16 - 8.19 (m, 1H), 10.84 (s, 1H).
HPLC/MS (5 min) retention time 3.26 min.
LRMS: *m*/*z* 318 (M+1).

### PREPARATION 20

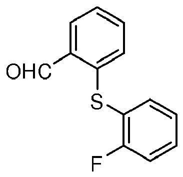

### 2-[(2-Fluorophenyl)thio]benzaldehyde

2-Fluorobenzaldehyde (415 µl, 3.94 mmol) was treated with 2-fluorobenzenethiol (500 mg, 3.9 mmol) and potassium carbonate (1.2 g, 8.67 mmol) according to the method of Preparation 1 to give 693 mg (2.86 mmol, 73%). Used as such without further purification. Purity 96%
HPLC/MS (5 min) retention time 4.21 min.
LRMS: *m*/*z* 233 (M+1).

### PREPARATION 21

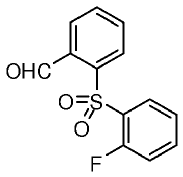

### 2-[(2-Fluorophenyl)sulfonyl]benzaldehyde

The title compound of Preparation 20 (693 mg, 2.86 mmol) was treated with 3-chloroperbenzoic acid (77% purity, 2.14 g, 7.45 mmol) according to the method of Preparation 4 to give 652 mg (2.37 mmol, 79%). Used as such without further purification. Purity 96%
¹H NMR (400 MHz, CHLOROFORM-d) δ ppm 7.09 - 7.16 (m, 1H), 7.37 - 7.43 (m, 1H), 7.61 - 7.68 (m, 1H), 7.75 - 7.84 (m, 2 H), 8.01 - 8.06 (m, 1H), 8.13 - 8.19 (m, 1H), 8.28 - 8.32 (m, 1H), 10.75 (s, 1H).
HPLC/MS (5 min) retention time 3.61 min.
LRMS: *m*/*z* 265 (M+1).

### PREPARATION 22

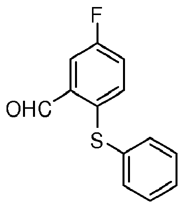

### 5-Fluoro-2-(phenylthio)benzaldehyde

2,5-Difluorobenzaldehyde (1.0 g, 7.04 mmol) was treated with benzenethiol (720 µl, 7.04 mmol) and potassium carbonate (2.19 g, 15.85 mmol) according to the method of Preparation 1 to give 1.45 g (6.24 mmol, 88%) of the crude title compound as a green oil. Used as such without further purification.
¹H NMR (400 MHz, CHLOROFORM-d) δ ppm 7.12 - 7.40 (m, 7 H), 7.62 (7 H, dd, *J*=8.4, 2.5 Hz), 10.36 - 10.54 (m, 1H).

### PREPARATION 23

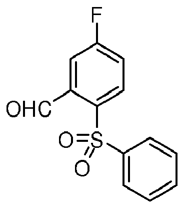

### 5-Fluoro-2-(phenylsulfonyl)benzaldehyde

The crude title compound of Preparation 22 (1.45 g, 6.24 mmol) was treated with 3-chloroperbenzoic acid (77% purity, 3.80 g, 18.7 mmol) according to the method of Preparation 012 to give 1.53 mg (5.78 mmol, 93%) of the crude title compound as a yellow solid. Used as such without further purification.
¹H NMR (400 MHz, CHLOROFORM-d) δ ppm 7.40 - 7.50 (m, 1H), 7.52 - 7.59 (m, 2 H), 7.60 - 7.67 (m, 1H), 7.70 (dd, J=8.60, 2.74 Hz, 1H), 7.86 - 7.91 (m, 2 H), 8.25 (dd, J=8.79, 4.88 Hz, 1H), 10.81 (d, J=2.74 Hz, 1H).

### PREPARATION 24

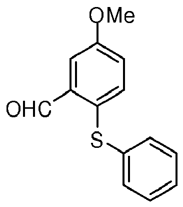

### 5-Methoxy-2-(phenylthio)benzaldehyde

2-Fluoro-5-methoxybenzaldehyde (1.5 g, 9.73 mmol) was treated with benzenethiol (1.07 ml, 9.92 mmol) and potassium carbonate (3.03 g, 21.9 mmol) according to the method of Preparation 1. The residue was purified using the SP1 Purification System (ethyl acetate-hexane 15:85) to give 2.28 g of the crude title compound. Purity 71%.
¹H NMR (400 MHz, CHLOROFORM-d) δ ppm 3.87 (s, 3 H), 7.10 (dd, J=8.60, 2.74 Hz, 1H), 7.13 - 7.33 (m, 5 H), 7.40 (d, J=8.60 Hz, 1H), 7.47 (d, J=2.34 Hz, 1H), 10.52 (s, 1H).
HPLC/MS (9 min) retention time 6.92 min.
LRMS: *m*/*z* 245 (M+1).

### PREPARATION 25

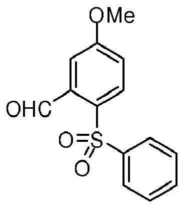

### 5-Methoxy-2-(phenylsulfonyl)benzaldehyde

The crude title compound of Preparation 24 (2.28 g) was treated with 3-chloroperbenzoic acid (4.0 g, 19.6 mmol) according to the method of Preparation 4 to give 2.26 g (8.17 mmol, 84% over two steps) of the title compound. Used as such without further purification.
¹H NMR (400 MHz, CHLOROFORM-d) δ ppm 3.90 (s, 3 H), 7.19 (d, J=8.60 Hz, 1H), 7.43 - 7.62 (m, 4 H), 7.86 (d, J=7.82 Hz, 2 H), 8.13 (d, J=8.60 Hz, 1 H), 10.81 (s, 1H).

### PREPARATION 26

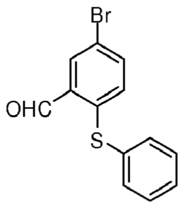

### 5-Bromo-2-(phenylthio)benzaldehyde

5-Bromo-2-fluorobenzaldehyde (2.0 g, 9.85 mmol) was treated with benzenethiol (1.0 ml, 9.74 mmol) and potassium carbonate (3.0 g, 9.74 mmol) accoriding to the method of Preparation 1. The residue was purified using the SP1 Purification System (ethyl acetate-hexane gradient, 0:100 rising to 2:98) to give 2.20 g (7.13 mmol, 73%) of the title compound. Purity 95%.
¹H NMR (400 MHz, CHLOROFORM-d) δ ppm 6.97 (d, J=8.60 Hz, 1H), 7.35 - 7.45 (m, 5 H), 7.50 (dd, J=8.60, 2.34 Hz, 1H), 7.99 (d, J=2.34 Hz, 1H), 10.33 (s, 1H).
HPLC/MS (9 min) retention time 7.18 min.
LRMS: *m*/*z* 293,295 (M+1).

### PREPARATION 27

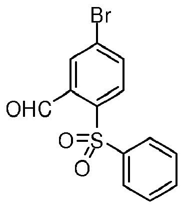

### 5-Bromo-2-(phenylsulfonyl)benzaldehyde

The title compound of Preparation 26 (2.20 g, 7.13 mmol) was treated with 3-chloroperbenzoic acid (77% purity, 5.0 g, 22.3 mmol) according to the method of Preparation 2 to give 1.70 g (5.02 mmol, 70%) of the title compound as a solid. Used as such without further purification. Purity 96%.
¹H NMR (400 MHz, CHLOROFORM-d) δ ppm 7.53 - 7.59 (m, 2 H), 7.61 - 7.67 (m, 1H), 7.86 - 7.92 (m, 3 H), 8.04 - 8.08 (m, 1H), 8.13 (d, J=1.95 Hz, 1H), 10.80 (s, 1H).
HPLC/MS (9 min) retention time 6.28 min.
LRMS: *m*/*z* 325,327 (M+1).

### PREPARATION 28

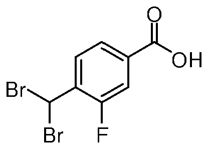

### 4-(Dibromomethyl)-3-fluorobenzoic acid

3-Fluoro-4-methylbenzoic acid (4,0 g, 26.0 mmol), *N*-bromosuccinimide (11.1 g, 62.3 mmol) and benzoyl peroxide (0.31 g, 1.3 mmol) were dissolved in 55 ml carbon tetrachloride. The mixture was heated at 77ºC for 40 h. The mixture was allowed to cool and was filtered. The resulting filtrated was evaporated under reduced pressure to give 4.55 g of the crude title compound as a yellow solid. Used as such without further purification. Purity 84%.
¹H NMR (400 MHz, CHLOROFORM-d) δ ppm 6.94 (s, 1H), 7.76 (d, J=10.55 Hz, 1H), 7.94 - 8.00 (m, 2 H).
HPLC/MS (9 min) retention time 6.1 min.
LRMS: *m*/*z* 311,313,315 (M+1).

### PREPARATION 29

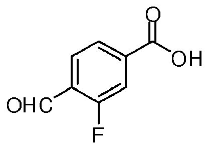

### 3-Fluoro-4-formylbenzoic acid

The crude title compound of Preparation 28 (4.55 g) was suspended in 36 ml ethanol. The suspension was heated to 50ºC and a solution of silver nitrate (4.37 g, 25.7 mmol) in 6 ml water was slowly added. The mixture was stirred at 50ºC for 45 min. The mixture was allowed to cool and was evaporated under reduced pressure. The resulting residue was partitioned between water and ethyl acetate. The aqueous layer was extracted two times with ethyl acetate. The combined organics were washed with water and brine, dried over sodium sulphate and were evaporated under reduced pressure to give 2.40 g of the crude title compound as a yellow solid. Used as such without further purification.
¹H NMR (400 MHz, CHLOROFORM-d) δ ppm 7.80 - 7.88 (m, 1H), 7.88 - 7.96 (m, 2 H), 10.40 (s, 1H).

### PREPARATION 30

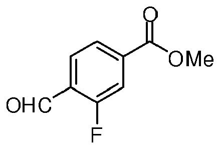

### Methyl 3-fluoro-4-formylbenzoate

The crude title compound of Preparation 29 (2.40 g) was dissolved in 40 ml dimethylformamide. A suspension of sodium hydride (60% dispersion in oil, 0.69 mg, 17.1 mmol, previously washed with pentane) in 3 ml dimethylformamide was added in portions and the mixture was stirred 45 minutes at room temperature. A solution of methyl iodide (1.07 ml, 17.2 mmol) in 2 ml dimethylformamide was slowly added and the mixture was stirred at room temperature 5 h. Hydrochloric acid 1 N was added and the mixture was extracted two times with ethyl acetate. The combined organics were washed with 4% sodium hydrogen carbonate solution, brine and dried over sodium sulphate. Filtration and evaporation gave a residue which was purified using the SP1 Purification System (ethyl acetate-hexane gradient, 0:100 rising to 8:92) to give 1.5 g (8.23 mmol, 31% over three steps) of the title compound as a yellow solid.
¹H NMR (400 MHz, CHLOROFORM-d) δ ppm 3.97 (s, 3 H), 7.83 - 7.854 (m, 1H), 7.89 - 7.98 (m, 2 H), 10.42 (s, 1H).

### PREPARATION 31

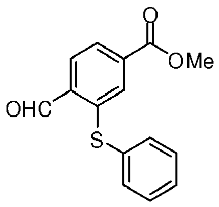

### Methyl 4-formyl-3-(phenylthio)benzoate

The title compound of Preparation 30 (1.5 g, 8.23 mmol) was treated with benzenethiol (8.77 ml, 90 mmol) and potassium carbonate (2.56 g, 8.24 mmol) according to the method of Preparation 1 give 1.72 g of the crude title compound as a yellow solid. Used as such without further purification. Purity 76%.
¹H NMR (400 MHz, CHLOROFORM-d) δ ppm 3.86 (s, 3 H), 7.26 (d, J=4.69 Hz, 1H), 7.29 - 7.44 (m, 4 H), 7.86 (br. s., 1H), 7.89 - 8.05 (m, 2 H), 10.49 (s, 1H).
HPLC/MS (9 min) retention time 6.63 min.
LRMS: *m*/*z* 273 (M+1).

### PREPARATION 32

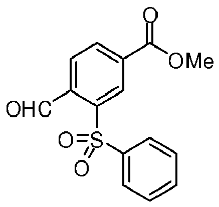

### Methyl 4-formyl-3-(phenylsulfonyl)benzoate

The crude title compound of Preparation 31 (1.72 g) was treated with 3-chloroperbenzoic acid (77% purity, 2.94 g, 14.48 mmol) according to the method of Preparation 4 to give 1.53 g (5.03 mmol, 61% over two steps). Used as such without further purification.
¹H NMR (400 MHz, CHLOROFORM-d) δ ppm 3.99 (s, 3 H), 7.56 (q, J=7.03 Hz, 2 H), 7.62 (t, J=7.42 Hz, 1H), 7.92 (d, J=7.82 Hz, 2 H), 8.05 (d, J=8.21 Hz, 1H), 8.33 (d, J=7.82 Hz, 1H), 8.78 (s, 1H), 10.89 (s, 1H).

### PREPARATION 33

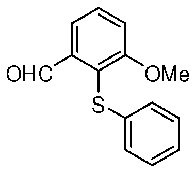

### 3-Methoxy-2-(phenylthio)benzaldehyde

2-Fluoro-3-methoxybenzaldehyde (1.5 g, 9.73 mmol) was treated with benzenethiol (1.0 g, 9.74 mmol) and potassium carbonate (3.0 g, 2.23 mmol) according to the method of Preparation 1 to give 2 g (8.19 mmol, 84%) of the title compound as a solid. Used as such without further purification. Purity 100%.
¹H NMR (400 MHz, CHLOROFORM-d) δ ppm 3.83 (s, 3 H), 7.05 - 7.16 (m, 3 H), 7.17 - 7.24 (m, 3 H), 7.50 - 7.56 (m, 1H), 7.60 - 7.65 (m, 1H), 10.69 (s, 1 H).
HPLC/MS (9 min) retention time 6.45 min.
LRMS: *m*/*z* 245 (M+1).

### PREPARATION 34

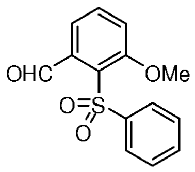

### 3-Methoxy-2-(phenylsulfonyl)benzaldehyde

The title compound of Preparation 33 (2.0 g, 8.19 mmol) was treated with 3-chloroperbenzoic acid (77% purity, 5.5 g, 24.56 mmol) according to the method of Preparation 2 to give 750 mg (2.71 mmol, 33%) of the title compound. Used as such without further purification. Purity 100%.
¹H NMR (400 MHz, CHLOROFORM-d) δ ppm 3.73 (s, 3 H), 7.09 (dd, J=8.40, 0.98 Hz, 1H), 7.32 (dd, J=7.62, 0.98 Hz, 1H), 7.49 - 7.55 (m, 2 H), 7.57 - 7.65 (m, 2 H), 7.94 - 7.99 (m, 2 H), 10.98 (s, 1H).
HPLC/MS (9 min) retention time 5.37 min.
LRMS: *m*/*z* 277 (M+1).

### PREPARATION 35

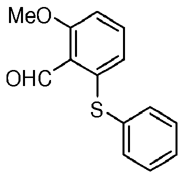

### 2-Methoxy-6-(phenylthio)benzaldehyde

2-Fluoro-6-methoxybenzaldehyde (1.5 g, 9.73 mmol) was treated with benzenethiol (1 ml, 9.71 mmol) according to the method of Preparation 1 to give 1.6 g (6.35 mmol, 65%) of the title compound. Used as such without further purification. Purity 97%.
¹H NMR (400 MHz, CHLOROFORM-d) δ ppm 3.92 (s, 3 H), 6.33 (d, J=8.21 Hz, 1H), 6.68 (d, J=8.21 Hz, 1H), 7.20 (t, J=8.21 Hz, 1H), 7.42 - 7.45 (m, 3 H), 7.55 - 7.59 (m, 2 H), 10.66 (s, 1H).
HPLC/MS (9 min) retention time 6.52 min.
LRMS: *m*/*z* 245 (M+1).

### PREPARATION 36

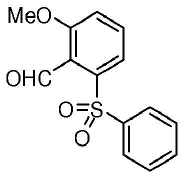

### 2-Methoxy-6-(phenylsulfonyl)benzaldehyde

The title compound of Preparation 35 (1.6 g, 6.35 mmol) was treated with 3-chloroperbenzoic acid (77% purity, 4.40 g, 19.65 mmol) according to the method of Preparation 2 to give 840 mg (1.44 mmol, 23%). Used as such without further purification. Purity 93%.
¹H NMR (400 MHz, CHLOROFORM-*d*) δ ppm 10.59 (1H, s), 7.91 (2 H, d, *J*=7.4 Hz), 7.74 (1H, d, *J*=8.2 Hz), 7.55 - 7.63 (2 H, m), 7.52 (2 H, t, *J*=7.4 Hz), 7.20 (1H, d, *J*=8.2 Hz), 3.87 (3 H, s).
HPLC/MS (30 min) retention time 11.12 min.
LRMS: *m*/*z* 277 (M+1).

### PREPARATION 37

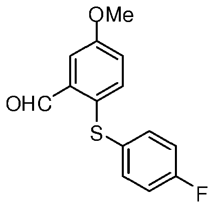

### 2-[(4-Fluorophenyl)thio]-5-methoxybenzaldehyde

2-Fluoro-5-methoxybenzaldehyde (2.5 g, 16.22 mmol) was treated with 4-fluorobenzenethiol (2.1 g, 16.38 mmol) and potassium carbonate (4.5 g, 32.56 mmol) according to the method of Preparation 1. The resulting residue was purified using the SP1 Purification System (ethyl acetate:hexane gradient, 0:100 rising 5:95) to give 2.9 g (10.8 mmol, 67%) of the title compound. Purity 98%.
¹H NMR (400 MHz, CHLOROFORM-d) δ 10.48 (s, 1H), 7.44 (d, J = 3.0 Hz, 1H), 7.28 (d, J = 8.6 Hz, 1H), 7.25 - 7.18 (m, 2H), 7.08 (dd, J = 8.7, 3.0 Hz, 1H), 7.02 - 6.95 (m, 2H), 3.86 (s, 3H).
HPLC/MS (9 min) retention time 6.80 min.
LRMS: *m*/*z* 263 (M+1).

### PREPARATION 38

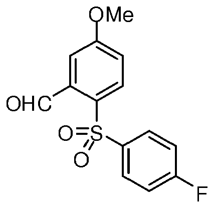

### 2-[(4-Fluorophenyl)sulfonyl]-5-methoxybenzaldehyde

The title compound of Preparation 37 (2.9 g, 10.8 mmol) was treated with 3-chloroperbenzoic acid (77% purity, 7.5 g, 33.5 mmol) according to the method of Preparation 2. The resulting residue was further purified using the SP1 Purification System (ethyl acetate:hexane gradient, 0:100 rising 25:75) to give 600 mg of the crude title compound. Purity 80%.
¹H NMR (400 MHz, CHLOROFORM-d) δ ppm 3.91 (s, 3 H), 7.17 - 7.24 (m, 3 H), 7.49 (d, J=2.74 Hz, 1H), 7.87 - 7.93 (m, 2 H), 8.14 (d, J=8.99 Hz, 1H), 10.79 (s, 1H).
HPLC/MS (9 min) retention time 5.88 min.
LRMS: *m*/*z* 295 (M+1).

### PREPARATION 39

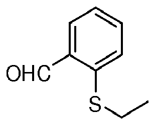

### 2-(Ethylthio)benzaldehyde

2-Fluorobenzaldehyde (2.0 g, 16.11 mmol) was treated with ethanethiol (1.19 ml, 16.1 mmol) and potassium carbonate (4.9 g, 35.5 mmol) according to the method of Preparation 1 to give 2.2 g (13.2 mmol, 82%). Used as such without further purification. Purity 100%.
¹H NMR (400 MHz, CHLOROFORM-d) δ ppm 1.37 (t, J=7.42 Hz, 3 H), 2.99 (q, J=7.29 Hz, 2 H), 7.28 - 7.33 (m, 1H), 7.40 - 7.45 (m, 1H), 7.49 - 7.54 (m, 1H), 7.84 (dd, J=7.82, 1.56 Hz, 1H), 10.39 (s, 1H).
HPLC/MS (9 min) retention time 5.73 min.
LRMS: *m*/*z* 167 (M+1).

### PREPARATION 40

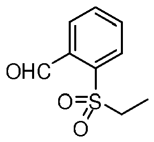

### 2-(Ethylsulfonyl)benzaldehyde

The title compound of Preparation 39 (2.2 g, 13.2 mmol) was treated with 3-chloroperbenzoic acid (77% purity, 9.0 g, 40.16 mmol) according to the method of Preparation 2 to give 2.5 g (12.6 mmol, 95%) of the title compound. Used as such without further purification. Purity 100%.
¹H NMR (400 MHz, CHLOROFORM-d) δ ppm 1.34 (t, J=7.42 Hz, 3 H), 3.31 (q, J=7.42 Hz, 2 H), 7.78 - 7.84 (m, 2 H), 8.08 - 8.14 (m, 2 H), 10.81 (s, 1H).
HPLC/MS (9 min) retention time 4.32 min.
LRMS: *m*/*z* 199 (M+1).

### PREPARATION 41

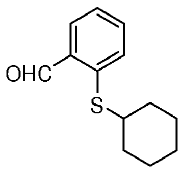

### 2-(Cyclohexylthio)benzaldehyde

2-Fluorobenzaldehyde (1.5 g, 12.09 mmol) was treated with cyclohexanethiol (1.48 ml, 12.1 mmol) and potassium carbonate (3.76 g, 27.2 mmol) according to the method of Preparation 1. The resulting residue was purified using the SP1 Purification System (ethyl acetate-hexane gradient, 0:100 rising 7:93) to give 1.71 g (7.45 mmol, 62%) of the title compound as a yellow oil. Purity 96%.
¹H NMR (400 MHz, CHLOROFORM-d) δ ppm 1.16 - 1.49 (m, 5 H), 1.55 - 1.69 (m, 1H), 1.68 - 1.86 (m, 2 H), 1.88 - 2.08 (m, 2 H), 3.05 - 3.17 (m, 1H), 7.35 (t, J=7.03 Hz, 1H), 7.42 - 7.58 (m, 2 H), 7.88 (d, J=7.82 Hz, 1H), 10.59 (s, 1 H).
HPLC/MS (9 min) retention time 7.13 min.
LRMS: *m*/*z* 221 (M+1).

### PREPARATION 42

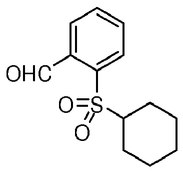

### 2-(Cyclohexylsulfonyl)benzaldehyde

The title compound of Preparation 41 (1.71 g, 7.45 mmol) was treated with 3-chloroperbenzoic acid (77% purity, 4.53 g, 22.3 mmol) according to the method of Preparation 4 to give 0.98 g (3.61 mmol, 48%) of the title compound. Used as such without further purification. Purity 93%.
¹H NMR (400 MHz, CHLOROFORM-d) δ ppm 1.07 - 1.29 (m, 4 H), 1.39 - 1.60 (m, 2 H), 1.78 - 1.94 (m, 2 H), 1.96 - 2.09 (m, 2 H), 2.90 - 3.05 (m, 1H), 7.64 - 7.77 (m, 2 H), 7.96 - 8.10 (m, 2 H), 10.85 (br. s., 1H).
HPLC/MS (9 min) retention time 5.92 min.
LRMS: *m*/*z* 253 (M+1).

### PREPARATION 43

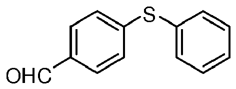

### 4-(Phenylthio)benzaldehyde

4-Fluorobenzaldehyde (1 g, 8.06 mmol) was treated with benzenethiol (830 mg, 8.06 mmol) and potassium carbonate (2.5 g, 18.1 mmol) according to the method of Preparation 1 to give 1.45 g (6.15 mmol, 76%) of the title compound. Used as such without further purification. Purity 91%.
¹H NMR (400 MHz, CHLOROFORM-d) δ ppm 7.26 (d, J=4.30 Hz, 2 H), 7.38 - 7.48 (m, 3 H), 7.48 - 7.58 (m, 2 H), 7.72 (d, J=8.21 Hz, 2 H), 9.91 (s, 1H).
HPLC/MS (9 min) retention time 6.77 min.
LRMS: *m*/*z* 215 (M+1).

### PREPARATION 44

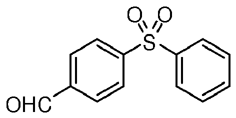

### 4-(Phenylsulfonyl)benzaldehyde

The title compound of Preparation 43 (1.45 g, 6.15 mmol) was treated with 3-chloroperbenzoic acid (77% purity, 3.74 g, 18.42 mmol) according to the method of Preparation 4 to give 1.2 g (4.87 mmol, 79%) of the title compound as a white solid. Used as such without further purification.
¹H NMR (400 MHz, CHLOROFORM-d) δ ppm 7.51 - 7.58 (m, 2 H), 7.58 - 7.66 (m, 1H), 7.95 - 8.04 (m, 4 H), 8.09 - 8.15 (m, 2 H), 10.08 (s, 1H).

### PREPARATION 45

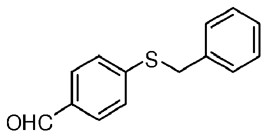

### 4-(Benzylthio)benzaldehyde

4-Fluorobenzaldehyde (1 g, 8.06 mmol) was treated with phenylmethanethiol (0.95 ml, 9.7 mmol) according to the method of Preparation 1 to give 1.4 g (5.95 mmol, 74%) of the title compound as a solid. Used as such without further purification. Purity 97%.
¹H NMR (400 MHz, CHLOROFORM-d) δ ppm 4.24 (s, 2 H), 7.24 - 7.42 (m, 7 H), 7.71 - 7.78 (m, 2 H), 9.92 (s, 1H).
HPLC/MS (9 min) retention time 6.57 min.
LRMS: *m*/*z* 229 (M+1).

### PREPARATION 46

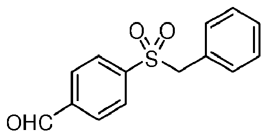

### 4-(Benzylsulfonyl)benzaldehyde

The title compound of Preparation 45 (1.4 g, 5.95 mmol) was treated with 3-chloroperbenzoic acid (77% purity, 3.08 g, 17.8 mmol) according to the method of Preparation 2 to give 1.5 g (5.76 mmol, 97%). Used as such without further purification. Purity 100%.
¹H NMR (400 MHz, CHLOROFORM-d) δ ppm 4.36 (s, 2 H), 7.06 - 7.10 (m, 2 H), 7.24 - 7.30 (m, 2 H), 7.30 - 7.37 (m, 1 H), 7.76 - 7.80 (m, 2 H), 7.92 - 7.97 (m, 2 H), 10.10 (s, 1 H).
HPLC/MS (9 min) retention time 5.35 min.
LRMS: *m*/*z* 275 (M+1).

### PREPARATION 47

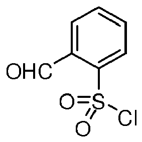

### 2-Formylbenzenesulfonyl chloride

Sodium 2-formylbenzenesulfonate (1.0 g, 4.80 mmol) was suspended in sulphurous dichloride (2.9 ml, 39.8 mmol). Dimethylformamide (20 µl, 0.29 mmol) was added and the mixture was hetead at 100ºC for 3 min. The mixture was allowed to cool. The mixture was then cooled in an ice bath and water was added slowly until there was no further reaction, and with the formation of a white precipitate. The mixture was extracted with pentane. The combined organics were dried over sodium sulphate, filtrated and evaporated under reduced pressure to give 0.73 g of the crude title compound as a yellow oil. Purity 86%.
¹H NMR (400 MHz, DMSO-d6) δ ppm 7.50 (t, J=7.42 Hz, 1 H), 7.62 (m, 1 H), 7.76 (d, J=7.42 Hz, 1 H), 7.82 (d, J=7.42 Hz, 1 H), 10.88 (s, 1 H).
GC/MS (18 min) retention time 7.75 min.
LRMS: *m*/*z* 204 (M+1).

### PREPARATION 48

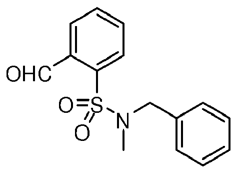

### N-Benzyl-2-formyl-N-methylbenzenesulfonamide

The crude title compound of Preparation 47 (350 mg) was dissolved in 8 ml dichloromethane. N-Methyl-1-phenylmethanamine (0.22 ml, 1.71 mmol) was added and the mixture was stirred at room temperature 2 h. The mixture was concentrated under reduced pressure and the resulting residue was purified using the SP1 Purification System (ethyl acetate-hexane gradient, 0:100 rising to 20:80) to give 200 mg (0.691 mmol, 47%) of the title compound as a colourless oil. Purity 100%.
¹H NMR (400 MHz, CHLOROFORM-d) δ ppm 2.68 (s, 3 H), 4.26 (s, 2 H), 7.22 - 7.28 (m, 2 H), 7.28 - 7.39 (m, 3 H), 7.71 - 7.79 (m, 2 H), 7.96 - 8.02 (m, 1 H), 8.09 - 8.15 (m, 1 H), 10.91 (s, 1 H).
GC/MS (18 min) retention time 10.76 min.
LRMS: *m*/*z* 290 (M+1).

### PREPARATION 49

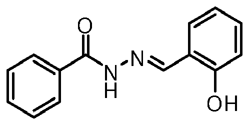

### N'-[(1E)-(2-Hydroxyphenyl)methylene]benzohydrazide

2-Hydroxybenzaldehyde (1.49 g, 12.2 mmol) was dissolved in 60 ml acetic acid. Benzoylhydrazine (1.66 g, 12.2 mmol) was then added and the mixture was stirred at room temperature 1 h. The mixture was poured into cold water and the precipitated formed was collected by filtration. The solid was washed with water and triturated with hexane. The solid was dried under reduced pressure to give 2.9 g (12.1 mmol, 99%) of the title compound. Purity 100%.
HPLC/MS (5 min) retention time 3.62 min.
LRMS: *m*/*z* 241 (M+1).

### PREPARATION 50

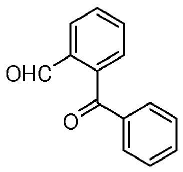

### 2-Benzoylbenzaldehyde

The title compound of Preparation 49 (2.9, 12.1 mmol) was dissolved in 40 ml tetrahydrofuran and cooled to 0ºC. Lead(IV) tetraacetate (5.4 g, 12.2 mmol) was then added and the mixture was stirred at 0ºC 4 h. The mixture was then allowed to room temperature and was stirred 2h. The mixture was filtered and the filtrated was evaporated under reduced pressure. The resulting residue was partitioned between saturated sodium hydrogen carbonate solution and ethyl acetate. The organic layer was washed with brine, was dried over magnesium sulphate, filtered and evaporated under reduced pressure. The resulting residue was purified using the SP1 Purification System (dichloromethane, 100%) to give the title compound. Purity 95%.
¹H NMR (400 MHz, CHLOROFORM-d) δ ppm 7.44 - 7.53 (m, 3 H), 7.58 - 7.64 (m, 1 H), 7.67 - 7.71 (m, 2 H), 7.78 - 7.83 (m, 2 H), 8.02 - 8.06 (m, 1 H), 10.03 (s, 1 H).
HPLC/MS (5 min) retention time 3.63 min.
LRMS: *m*/*z* 211 (M+1).

### PREPARATION 51

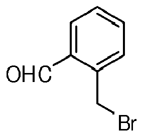

### 2-(Bromomethyl)benzaldehyde

2-Cyanobenzyl bromide (1.5 g, 7.65 mmol) was disolved in 25 ml dichloromethane and the mixture was cooled to 0ºC. Diisobutylaluminum hydride (8.2 ml, 8.26 mmol) was slowly added and the mixture was stirred at 0ºC for 3.5 h. The mixture was then poured into a cold solution of 5% hydrobromic acid. Dichloromethane was added and the layers were separated. The aqueous layer was twice extracted with dichloromethane. The combined organics were dried over magnesium sulphate, filtered and evaporated under reduced pressure to give 1.4 g (6.68 mmol, 87%) of the title compound. Purity 95%.
¹H NMR (400 MHz, CHLOROFORM-d) δ ppm 4.96 (s, 2 H), 7.47 - 7.61 (m, 3 H), 7.85 (dd, J=7.42, 1.56 Hz, 1 H), 10.26 (s, 1 H).
HPLC/MS (5 min) retention time 3.52 min.
LRMS: *m*/*z* 199, 201 (M+1).

### PREPARATION 52

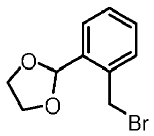

### 2-[2-(Bromomethyl)phenyl]-1,3-dioxolane

The title compound of Preparation 51 (400 mg, 1.91 mmol) was dissolved in 8 ml toluene. Ethylene glycol (0.137 ml, 2.14 mmol) and p-toluenesulfonic acid (38.2 mg, 0.194 mmol) were added and the mixture was heated at reflux overnight in a flask fitted with a Dean-Stark head. Ethyleneglycol (0.124 ml, 2.0 mmol) was added to the reaction and the mixture was heated at reflux for a further 30 min. The mixture was then allowed to cool and was partitioned between ethyl acetate and saturated sodium hydrogen carbonate solution. The organic layer was dried over magnesium sulphate, filtered and evaporated under reduced pressure to give 400 mg of the crude title compound. Purity 81%.
HPLC/MS (5 min) retention time 3.80 min.
LRMS: *m*/*z* 244 (M+1).

### PREPARATION 53

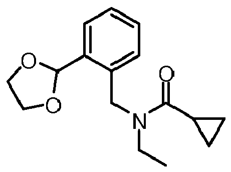

### N-[2-(1,3-Dioxolan-2-yl)benzyl]-N-ethylcyclopropanecarboxamide

The crude title compound of Preparation 52 (400 mg) was stirred with ethylamine (2M in tetrahydrofuran, 4.9 ml, 9.84 mmol) at room temperature for 10 min. Methanol was then added and the mixture was concentrated under reduced pressure to give a residue.

The residue was dissolved in 20 ml dichloromethane. Triethylamine (0.94 ml, 6.75 mmol) and a solution of cyclopropanecarbonyl chloride (0.26 ml, 2.89 mmol) in 10 ml dichloromethane were added and the mixture was stirred at room temperature for 20 min. Dichloromethane was added and the solution was washed witjh sodium hydrogen carbonate three times. The organic layer was dried over magnesium sulphate, filtered and evaporated under reduced pressure. The resulting residue was purified using the SP1 Purification System (methanol-dichloromethane gradient, 0:100 rising to 5:95) to give 270 mg (0.91 mmol, 48% over two steps) of the title compound. Purity 93%.
¹H NMR (400 MHz, CHLOROFORM-*d*) 1:1 mixture of rotamers. Spectrum reported as sum of both rotamers: δ ppm 7.57 (1 H, br. s.), 7.56 (1 H, d, *J*=2.1 Hz), 7.24 - 7.43 (5 H, m), 7.19 (1 H, d, *J*=7.2 Hz), 5.94 (1 H, s), 5.91 (1 H, s), 4.89 (2 H, s), 4.80 (2 H, s), 4.10 - 4.21 (4 H, m), 3.97 - 4.10 (4 H, m), 3.47 (2 H, q, *J*=7.2 Hz), 3.45 (2 H, q, *J*=7.2 Hz), 1.77 - 1.86 (1 H, m), 1.52 - 1.62 (1 H, m), 1.19 (3 H, t, *J*=7.1 Hz), 1.13 (3 H, t, *J*=7.0 Hz), 1.03 - 1.09 (2 H, m), 0.95 - 1.03 (2 H, m), 0.78 - 0.84 (2 H, m), 0.62 - 0.69 (2 H, m).
HPLC/MS (5 min) retention time 3.76 min.
LRMS: *m*/*z* 276 (M+1).

### PREPARATION 54

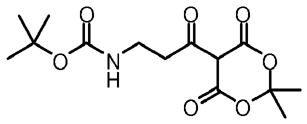

### tert-Butyl [3-(2,2-dimethyl-4,6-dioxo-1,3-dioxan-5-yl)-3-oxopropyl]carbamate

Boc-beta alanine (83 g, 0.44 mol) was dissolved in 2.7 l dichloromethane, which had been previously cooled to 5 ºC in the fridge. Meldrum's acid (66.6 g, 0.46 mol) was added and the mixture shaken to dissolve. 4-Dimethylaminopyridine (75 g, 0.61 mol) was added and shaken to dissolve. The solution was left in the fridge for 1 h to cool to approx 5 ºC. Dicyclohexylcarbodiimide (100 g, 0.48 mol) was added, the mixture shaken to dissolve and then left to stand in the fridge (5ºC) for 2 d. The mixture was filtered and the solid washed twice with cold dichloromethane. The combined filtrates were evaporated to approx 700 ml volume. The organics were washed three times with 10% w/v potassium hydrogen sulfate solution, once with brine and were dried over sodium sulfate. Filtration, evaporation of the filtrate and re-evaporation from ether gave 136 g (0.414 mol, 94%) of the title compound as a white solid. Used as such without further purification. Purity 95%.
¹H NMR (200 MHz, CHLOROFORM-*d*) δ ppm 4.85 (1 H, br. s.), 3.55 (2 H, q, *J*=5.9 Hz), 3.30 (2 H, t, *J*=6.4 Hz), 1.75 (6 H, s), 1.43 (9 H, s).
HPLC/MS (5 min) retention time 3.07 min.
LRMS: *m*/*z* 316 (M+1).

### PREPARATION 55

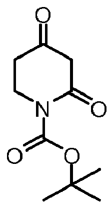

### tert-Butyl 2,4-dioxopiperidine-1-carboxylate

The title compound of Preparation 54 (16.3 g, 485 mmol) was suspended in 250 ml ethyl acetate and heated at reflux (bath temperature 120 ºC) for 2.5 h. The mixture was allowed to cool and was filtered. The filtrate was evaporated, giving an orange semisolid residue. The residue was dissolved in 200 ml diisopropyl ether. The volume of solvent was then reduced to around 40 ml, precipitating a yellow solid. The solid was collected by filtration, washed twice with diisopropyl ether and dried in a stream of air to give 6.4 g the title compound 6.4 g (30.0 mmol, 62%) as a pale yellow solid. Purity 100%.
¹H NMR (200 MHz, CHLOROFORM-*d*) δ ppm 4.11 (2 H, t, *J*=5.9 Hz), 3.52 (2 H, s), 2.63 (2 H, t, *J*=6.1 Hz), 1.56 (9 H, s).
HPLC/MS (5 min) retention time 2.41 min.
LRMS: *m*/*z* 212 (M-1).

### PREPARATION 56

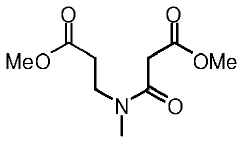

### Methyl N-(3-methoxy-3-oxopropanoyl)-N-methyl-beta-alaninate

Methyl acrylate (1.0 g, 11.6 mmol) and methylamine solution (2M in tetrahydrofuran, 4.47 ml, 8.94 mmol) were stirred together overnight. Triethylamine (1.62 ml, 11.6 mmols) was added and the mixture was then cooled to 0 ºC in an ice-bath. Methyl 3-chloro-3-oxopropionate (1.1 ml, 10.3 mmol) was added drop-wise and the mixture was agitated for 3 h, warming to room temperature. The mixture was partitioned between dichloromethane and 4% w/v sodium bicarbonate solution. The aqueous layer was extracted with dichloromethane. The combined organics were dried over magnesium sulphate, filtered and evaporated under reduced pressure. The resulting residue was purified by reverse-phase chromatography using the SP1 Purification system to give 784 mg of the crude title compound. Purity 85%.
HPLC/MS (5 min) retention time 2.20 min.
LRMS: *m*/*z* 218 (M+1).

### PREPARATION 57

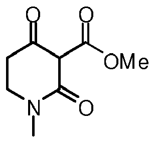

### Methyl 1-methyl-2,4-dioxopiperidine-3-carboxylate

Sodium metal (166 mg, 7.22 mmol) was slowly added in portions to 2 ml methanol and the mixture left to stand until all the solid had dissolved. The crude title compound of Preparation 56 (784 mg) and 10 ml toluene were then added and the mixture was heated at 110ºC for 2 h. The mixture was allowed to cool and was acidified to pH 3 with dilute hydrochloric acid. The aqueous was extracted twice with ethyl acetate. The combined organics were dried over magnesium sulphate, filtered and evaporated under reduced pressure to give 380 mg of the crude title compound. Used as such without further purification. Purity 81%.
HPLC/MS (5 min) retention time 2.30 min.
LRMS: *m*/*z* 186 (M+1).

### PREPARATION 58

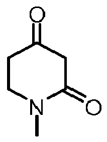

### 1-Methylpiperidine-2,4-dione

The crude title compound of Preparation 57 (380 mg) was dissolved in 2 ml acetonitrile and 2 ml water. The solution was heated at reflux for 30 min. The mixture was allowed to cool and was partitioned between water and ethyl acetate. The organic layer was dried over magnesium sulphate, filtered and evaporated under reduced pressure to give 168 mg (1.32 mmol, 11% over three steps) of the title compound. Purity 95%.
¹H NMR (400 MHz, CHLOROFORM-d) δ ppm 2.67 (t, J=6.25 Hz, 2 H), 3.09 (s, 3 H), 3.34 (s, 2 H), 3.59 (t, J=6.35 Hz, 2 H).
HPLC/MS (5 min) retention time 0.41 min.
LRMS: *m*/*z* 128 (M+1).

### PREPARATION 59

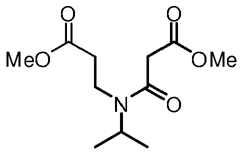

### Methyl N-isopropyl-N-(3-methoxy-3-oxopropanoyl)-beta-alaninate

Methyl acrylate (1.0 g, 11.6 mmol) and isopropylamine (0.77 ml, 8.94 mmol) were stirred together overnight. The mixture was diluted with 5 ml tetrahydrofuran and triethylamine (1.62 ml, 11.62 mmol) was added. Methyl 3-chloro-3-oxopropionate (1.1 ml, 10.3 mmol) was then added drop-wise and the mixture was agitated at room temperature for 3 h. The mixture was partitioned between ethyl acetate and 4% sodium hydrogen carbonate solution. The aqueous layer was extracted with ethyl acetate. The combined organics were dried over magnesium sulphate, filtered and evaporated under reduced pressure to give 1.8 g of the crude title compound. Used as such without further purification.
HPLC/MS (5 min) retention time 2.95 min.
LRMS: *m*/*z* 246 (M+1).

### PREPARATION 60

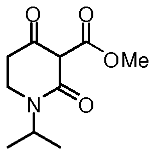

### Methyl 1-isopropyl-2,4-dioxopiperidine-3-carboxylate

The title compound of Preparation 59 (1.5 g) was treated with a solution of sodium metal (280 mg, 11.7 mmol) in 4 ml methanol according to the method of Preparation 57 to give 1.07 g (5.0 mmol, 43% over two steps) of the title compound. Used as such without further purification. Purity 95%.
HPLC/MS (5 min) retention time 3.1 min.
LRMS: *m*/*z* 214 (M+1).

### PREPARATION 61

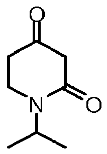

### 1-Isopropylpiperidine-2,4-dione

The title compound of Preparation 60 (1.0 g, 4.7 mmol) was dissolved in 6 ml acetonitrile and 6 ml water. The solution was heated at reflux for 2 h. The mixture was allowed to cool and was evaporated under reduced pressure. The residue was dissolved in ethyl acetate and was washed with water. The organic layer was dried over magnesium sulphate, filtered and evaporated under reduced pressure to give 410 mg (2.65 mmol, 56%) of the title compound. Purity 95%.
¹H NMR (400 MHz, CHLOROFORM-d) δ ppm 1.17 (d, J=6.84 Hz, 6 H), 2.56 (t, J=6.06 Hz, 2 H), 3.36 (s, 2 H), 3.49 (t, J=6.06 Hz, 2 H), 4.85 - 5.00 (m, 1 H).
HPLC/MS (5 min) retention time 1.87 min.
LRMS: *m*/*z* 156 (M+1).

### PREPARATION 62

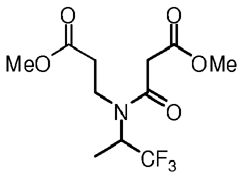

### Methyl N-(3-methoxy-3-oxopropanoyl)-N-(2,2,2-trifluoro-1-methylethyl)-beta-alaninate

β-Alanine methyl ester hydrochloride (140 mg, 1.0 mmol) was suspended in 2 ml benzene. Diisopropylethylamine (0.17 ml, 1.0 mmol) and 0.2 ml methanol were added, dissolving the solid. 1,1,1-Trifluoroecetone (0.42 ml, 4.7 mmol) was added and the mixture was capped and was stirred for 3 d. The mixture was evaporated to dryness and was resuspended in 2 ml methanol. Acetic acid (0.11 ml, 2 mmol) was added followed by sodium cyanoborohydride (250 mg, 4 mmol) and the mixture was stirred at room temperature for 4h. The mixture was partitioned between dichloromethane and 1 N sodium hydroxide solution. The aqueous phase was extracted with dichloromethane. The combined organics were washed with water, dried over sodium sulphate and evaporated to give 176 mg of a residue.

The residue (176 mg) was dissolved in 4 ml tetrahydrofuran and Triethylamine (0.31 ml, 2.21 mmols) was added. The mixture was cooled to 0ºC. Methyl 3-chloro-3-oxopropionate (0.12 ml, 1.14 mmol) was added drop-wise and the mixture was agitated at room temperature overnight. The mixture was partitioned between dichloromethane and sodium hydrogen carbonate saturated solution. The organic layer was dried over magnesium sulphate, filtered and evaporated under reduced pressure to give 160 mg of the crude title compound. Purity 57%.
HPLC/MS (5 min) retention time 3.32 min.
LRMS: *m*/*z* 300 (M+1).

### PREPARATION 63

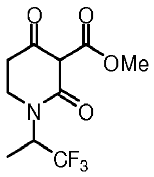

### Methyl 2,4-dioxo-1-(2,2,2-trifluoro-1-methylethyl)piperidine-3-carboxylate

The crude title compound of Preparation 62 (160 mg) was treated with a solution of sodium metal (25 mg, 1.1 mmol) in 0.5 ml methanol according to the method of Preparation 57 to give 104 mg of the crude title compound. Used as such without further purification. Purity 82%.
HPLC/MS (5 min) retention time 3.46 min.
LRMS: *m*/*z* 268 (M+1).

### PREPARATION 64

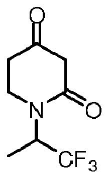

### 1-(2,2,2-Trifluoro-1-methylethyl)piperidine-2,4-dione

The crude title compound of Preparation 63 (100 mg) was dissolved in 0.5 ml acetonitrile and 0.5 ml water. The solution was heated at reflux for 30 min. The mixture was allowed to cool and was evaporated under reduced pressure to give 65 mg of the crude title compound. Purity 76%.
HPLC/MS (5 min) retention time 1.64 min.
LRMS: *m*/*z* 210 (M+1).

### PREPARATION 65

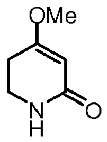

### 4-Methoxy-5,6-dihydropyridin-2(1H)-one

The title compound of Preparation 55 (2 g, 9.38 mmol), trimethyl orthoformate (2.06 ml, 10.50 mmol), and p-toluenesulphonic acid (19.5 mg, 0.1 mmol) were dissolved in 20 ml methanol. The mixture was heated to reflux for 1.5 h. Benzene (5 ml) was then added and the mixture was heated again to reflux for 5 h. Potassium carbonate (2.66 g, 19.24 mmol) was added and the mixture was stirred at room temperature overnight. The mixture was then filtered and evaporated under reduced pressure to give 500 mg (3.9 mmol, 42%) of the title compound. Used as such without further purification. Purity 95%.
¹H NMR (400 MHz, CHLOROFORM-d) δ ppm 2.46 (t, J=7.03 Hz, 2 H), 3.36 - 3.47 (m, 2 H), 3.69 (s, 3 H), 5.07 (s, 1 H), 5.58 - 5.76 (m, 1 H).
HPLC/MS (5 min) retention time 0.97 min
LRMS: *m*/*z* 128 (M+1).

### PREPARATION 66

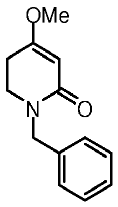

### 1-Benzyl-4-methoxy-5,6-dihydropyridin-2(1H)-one

The title compound of Preparation 65 (500 mg, 3.9 mmol) was dissolved in 4 ml tetrahydrofuran. Lithium bis(trimethylsilyl)amide solution (1M in tetrahydrofuran, 4.33 ml, 4.33 mmol) was then added and the mixture was stirred 30 min. Benzyl bromide (0.56 ml, 4.72 mmol) was added and the mixture was stirred at room temperature for 3.5 h. The mixture was then partitioned between ethyl acetate and ammonium chloride saturated solution. The organic layer was dried over magnesium sulphate, filtered and evaporated under reduced pressure. The resulting residue was further purified using the SP1 Purification System (ethyl acetate:hexane gradient, 30:70 rising 90:10) to give 140 mg (0.65 mmol, 16% yield) of the title compound. Purity 95%.
¹H NMR (400 MHz, CHLOROFORM-d) δ ppm 2.41 (t, J=7.13 Hz, 2 H), 3.22 (s, 2 H), 3.30 (t, J=7.13 Hz, 2 H), 3.68 (s, 3 H), 5.18 (s, 1 H), 7.20 - 7.26 (m, 1 H), 7.27 - 7.35 (m, 4 H).
HPLC/MS (5 min) retention time 2.28 min.
LRMS: *m*/*z* 218 (M+1).

### PREPARATION 67

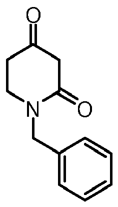

### 1-Benzylpiperidine-2,4-dione

The title compound of Preparation 66 (140 mg, 0.65) was dissolved in 4 ml tetrahydrofuran. 4ml 25% Hydrochloric acid solution was added and the mixture was agitated at room temperature for 3 h. The mixture was partitioned between water and ethyl acetate. The organic layer was dried over magnesium sulphate, filtered and evaporated under reduced pressure to give 140 mg of the title compound. Used as such without further purification.
¹H NMR (400 MHz, CHLOROFORM-d) δ ppm 2.54 (t, J=6.25 Hz, 2 H), 3.43 (s, 2 H), 3.49 (t, J=6.25 Hz, 2 H), 4.70 (s, 2 H), 7.24 - 7.39 (m, 5 H).

### PREPARATION 68

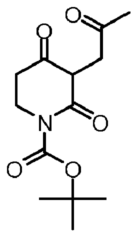

### tert-Butyl 2,4-dioxo-3-(2-oxopropyl)piperidine-1-carboxylate

The title compound of Preparation 55 (8.0 g, 37.6 mmol) was dissolved in 46 ml chloroform. 1-Chloropropan-2-one (2.90 ml, 36.3 mmol) and potassium carbonate (5.03 g, 36.4 mmol) were added and the mixture was agitated at room temperature overnight. The mixture was filtered and the resulting filtrate was washed with 2N hydrochloric acid. The organic layer was then dried over sodium sulphate. Filtration and evaporation under reduced pressure give 10.1g of the crude title compound as an amber oil. Used as such without further purification. Purity 74%.
¹H NMR (400 MHz, CHLOROFORM-d) δ ppm 1.55 (s, 9 H), 2.27 (s, 3 H), 2.65 - 2.73 (m, 2 H), 3.11 (dd, J=5.08, 2.34 Hz, 2 H), 3.68 - 3.87 (m, 1 H), 3.97 (t, J=5.08 Hz, 1 H), 4.57 (ddd, J=14.26, 5.28, 3.13 Hz, 1 H).
HPLC/MS (9 min) retention time 4.65 min.
LRMS: *m*/*z* 270 (M+1).

### PREPARATION 69

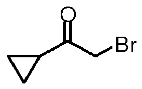

### 2-Bromo-1-cyclopropylethanone

Cyclopropyl methyl ketone (1.18 ml, 11.89 mmol) was dissolved in 2 ml metanol. Three drops of a solution of bromine (0.61 ml, 11.9 mmol) in 7 ml methanol was added. The mixture was stirred at room temperature until the total decolouration of the solution was observed. The mixture was cooled to -10ºC and the remainder of the bromine solution was slowly added. The mixture was stirred between -10ºC and 0ºC for 4 h. Saturated sodium hydrogen carbonate solution was then added and the aqueous layer was extracted with dichloromethane. The combined organics were dried over magnesium sulphate, filtered and evaporated under reduced pressure to give 0.38 g of the crude title compound.
¹H NMR (400 MHz, CHLOROFORM-d) δ ppm 1.00 - 1.05 (m, 2 H), 1.11 - 1.17 (m, 2 H), 2.19 - 2.25 (m, 1 H), 4.02 (s, 2 H).

### PREPARATION 70

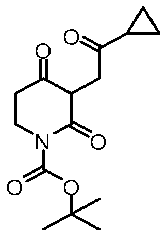

### tert-Butyl 3-(2-cyclopropyl-2-oxoethyl)-2,4-dioxopiperidine-1-carboxylate

The title compound of Preparation 55 (500 mg, 2.35 mmol) and the crude title compound of Preparation 69 (0.38 g) were dissolved in 5 ml chloroform. Potassium carbonate (324 mg, 2.35 mmol) was added and the mixture was agitated at room temperature for 22 h. Water was added and mixture was acidified to pH 3 with 2N hydrochloric acid. The aqueous layer was extracted three times with dichloromethane. The combined organics were dried over magnesium sulphate, filtered and evaporated under reduced pressure to give 535 mg of the crude title compound. Purity 50%
HPLC/MS (5 min) retention time 2.22 min.
LRMS: *m*/*z* 296 (M+1).

### PREPARATION 71

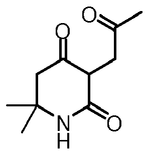

### 6,6-Dimethyl-3-(2-oxopropyl)piperidine-2,4-dione

6,6-Dimethyl-piperidine-2,4-dione (1.25 g, 8.85 mmol) was dissolved in 25 ml chloroform. 1-Chloropropan-2-one (0.7 ml, 8.79 mmol) and potassium carbonate (1.22 g, 8.83 mmol) were added and the mixture was agitated at room temperature for 3 days. The mixture was filtered and the resulting filtrated was evaporated under reduced pressure to give 1.44 g of the crude title compound. Used as such without further purification. Purity 81%.
HPLC/MS (9 min) retention time 3.43 min.
LRMS: *m*/*z* 198 (M+1).

### PREPARATION 72

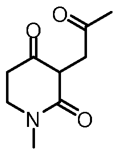

### 1-Methyl-3-(2-oxopropyl)piperidine-2,4-dione

The title compound of Preparation 58 (168 mg, 1.32 mmol) was treated with 1-chloropropan-2-one (105 µl, 1.32 mmol) and potassium carbonate (183 mg, 1.32 mmol) according to the method of Preparation 68 to give 183 mg of the crude title compound. Used as such without further purification.
HPLC/MS (5 min) retention time 1.2 min.
LRMS: *m*/*z* 184 (M+1).

### PREPARATION 73

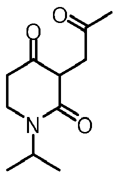

### 1-Isopropyl-3-(2-oxopropyl)piperidine-2,4-dione

The title compound of Preparation 61 (405 mg, 2.61 mmol) was treated with 1-chloropropan-2-one (208 µl, 2.61 mmol) and potassium carbonate (361 mg, 2.61 mmol) according to the method of Preparation 68 to give 550 mg of the crude title compound. Used as such without further purification. Purity 90%.
¹H NMR (400 MHz, CHLOROFORM-d) δ ppm 1.14 (d, J=6.84 Hz, 3 H), 1.17 (d, J=7.03 Hz, 3 H), 2.25 (s, 3 H), 2.47 - 2.57 (m, 1 H), 2.64 - 2.74 (m, 1 H), 3.13 (dd, J=7.91, 4.98 Hz, 2 H), 3.54 (d, J=3.91 Hz, 1 H), 3.56 (dd, J=3.71, 1.95 Hz, 1 H), 3.69 (t, J=5.08 Hz, 1 H), 4.77 - 4.91 (m, 1 H).
HPLC/MS (5 min) retention time 2.35 min.
LRMS: *m*/*z* 212 (M+1).

### PREPARATION 74

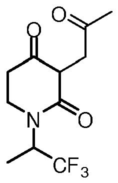

### 3-(2-Oxopropyl)-1-(2,2,2-trifluoro-1-methylethyl)piperidine-2,4-dione

The crude title compound of Preparation 64 (65 mg) was treated with 1-chloropropan-2-one (25 µl, 0.31 mmol) and potassium carbonate (43 mg, 0.31 mmol) according to the method of Preparation 68 to give the 65 mg of the crude title compound. Used as such without further purification. Purity 84%.
HPLC/MS (5 min) retention time 2.96 min.
LRMS: *m*/*z* 266 (M+1).

### PREPARATION 75

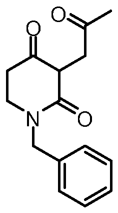

### 1-Benzyl-3-(2-oxopropyl)piperidine-2,4-dione

The title compound of Preparation 67 (140 mg) and 1-chloropropan-2-one were dissolved in 1 ml chloroform. Potassium carbonate (95.2 mg, 0.69 mmol) was added and the mixture was agitated at room temperature for 3 days. The mixture was filtered and dichloromethane was added. The mixture was acidified to pH 5 with 1 N hydrochloric acid. The organic layer was dried over magnesium sulphate, filtered and evaporated under reduced pressure to give 116 mg of the crude title compound. Used as such without further purification.
HPLC/MS (5 min) retention time 2.00 min.
LRMS: *m*/*z* 260 (M+1).

### PREPARATION 76

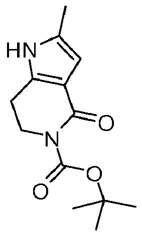

### tert-Butyl

### 2-methyl-4-oxo-1,4,6,7-tetrahydro-5H-pyrrolo[3,2-c]pyridine-5-carboxylate

The crude title compound of Preparation 68 (10.1 g) was dissolved in 65 ml ethanol. Ammonium acetate (10.6 g, 137 mmol) was then added and the mixture was agitated at room temperature overnight. The mixture was evaporated under reduced pressure. Dichloromethane was added to the residue and the mixture was filtered. The filtrate was evaporated under reduced pressure. The resulting residue was purified using the SP1 Purification System (ethyl acetate-hexane gradient, 0:100 rising to 60:40) to give 5.70 g (22.8 mmol, 63% over two steps) of the title compound as a yellow solid. Purity 100%.
¹H NMR (400 MHz, CHLOROFORM-d) δ ppm 1.55 (s, 9 H), 2.24 (s, 3 H), 2.84 (t, J=6.25 Hz, 2 H), 4.06 (t, J=6.45 Hz, 2 H), 6.25 (s, 1 H), 8.22 (br. s., 1 H).
HPLC/MS (9 min) retention time 5.17 min.
LRMS: *m*/*z* 251 (M+1).

### PREPARATION 77

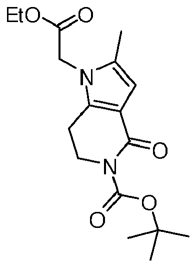

### tert-Butyl

**1-(2-ethoxy-2-oxoethyl)-2-methyl-4-oxo-1,4,6,7-tetrahydro-5H-pyrrolo[3,2-c]pyrid**i

### ne-5-carboxylate

The crude title compound of Preparation 68 (9 g), glycine ethyl ester hydrochloride (3.6 g, 25.7 mmol) and sodium acetate (4.2 g, 51 mmol) were suspended in 50 ml ethanol. The mixture was stirred at room temperature overnight. The mixture was evaporated under reduced pressure. The residue was dissolved in dichloromethane, was filtered and the filtrated was evaporated under reduced pressure. The resulting residue was purified using the SP1 Purification System (ethyl acetate-hexane gradient, 0:100 rising to 40:60) to give 6.20 g (18.4 mmol, 58% over two steps) of the title compound as a yellow solid. Purity 100%.
¹H NMR (400 MHz, CHLOROFORM-d) δ ppm 1.29 (t, J=7.03 Hz, 3 H), 1.55 (s, 9 H), 2.17 (s, 3 H), 2.74 (t, J=6.25 Hz, 2 H), 4.08 (t, J=6.25 Hz, 2 H), 4.24 (q, J=7.03 Hz, 2 H), 4.48 (s, 2 H), 6.35 (s, 1 H).
HPLC/MS (9 min) retention time 5.7 min.
LRMS: *m*/*z* 337 (M+1).

### PREPARATION 78

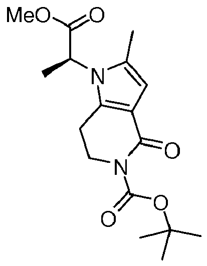

### tert-Butyl

**1-[(1*S*)-2-methoxy-1-methyl-2-oxoethyl]-2-methyl-4-oxo-1,4,6,7-tetrahydro-5H-pyr**

### rolo[3,2-c]pyridine-5-carboxylate

The crude title compound of Preparation 68 (0.5 g), L-alanine methyl ester hydrochloride (259 mg, 1.86 mmol) and sodium acetate (305 mg, 3.72 mmol) were suspended in 4 ml ethanol. The mixture was stirred at room temperature for 4 d. The mixture was evaporated under reduced pressure. The residue was dissolved in dichloromethane, was filtered and the filtrated was evaporated under reduced pressure. The resulting residue was purified using the SP1 Purification System (ethyl acetate-hexane gradient, 0:100 rising to 60:40) to give 85 mg (0.24 mmol, 13% over two steps) of the title compound. Purity 96%.
¹H NMR (400 MHz, CHLOROFORM-d) δ ppm 1.53 - 1.56 (m, 9 H), 1.66 (d, J=7.42 Hz, 3 H), 2.20 (s, 3 H), 2.69 - 2.87 (m, 2 H), 3.77 (s, 3 H), 3.93 - 4.02 (m, 1 H), 4.07 - 4.17 (m, 1 H), 4.81 (q, J=7.29 Hz, 1 H), 6.33 - 6.35 (m, 1 H).
HPLC/MS (5 min) retention time 3.83 min.
LRMS: *m*/*z* 337 (M+1).

### PREPARATION 79

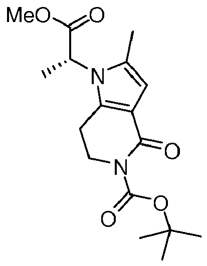

### tert-Butyl 1-[(1R)-2-methoxy-1-methyl-2-oxoethyl]-2-methyl-4-oxo-1,4,6,7-tetrahydro-5H-pyr rolo[3,2-c]pyridine-5-carboxylate

The crude title compound of Preparation 68 (0.5 g), D-alanine methyl ester hydrochloride (260 mg, 1.88 mmol) and sodium acetate (305 mg, 3.72 mmol) were treated according to the method of Preparation 77. The resulting residue was purified using the SP1 Purification System (ethyl acetate-hexane gradient, 20:80 rising to 60:40) to give 70 mg (0.20 mmol, 11 % over two steps) of the title compound. Purity 97%.
¹H NMR (400 MHz, CHLOROFORM-d) δ ppm 1.55 (s, 9 H), 1.66 (d, J=7.23 Hz, 3 H), 2.20 (s, 3 H), 2.70 - 2.77 (m, 1 H), 2.79 - 2.87 (m, 1 H), 3.77 (s, 3 H), 3.94 - 4.02 (m, 1 H), 4.08 - 4.15 (m, 1 H), 4.81 (q, J=7.29 Hz, 1 H), 6.33 - 6.35 (m, 1 H).
HPLC/MS (5 min) retention time 3.82 min.
LRMS: *m*/*z* 337 (M+1).

### PREPARATION 80

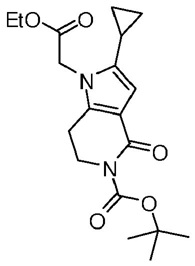

### tert-Butyl 2-cyclopropyl-1-(2-ethoxy-2-oxoethyl)-4-oxo-1,4,6,7-tetrahydro-5H-pyrrolo[3,2-c]p yridine-5-carboxylate

The crude title compound of Preparation 70 (530 mg), glycine ethyl ester hydrochloride (250 mg, 1.8 mmol) and sodium acetate (295 mg, 3.6 mmol) were suspended in 5 ml ethanol. The mixture was stirred at room temperature for 3d. The mixture was evaporated under reduced pressure. The residue was dissolved in dichloromethane, was filtered and the filtrated was evaporated under reduced pressure. The resulting residue was purified using the SP1 Purification System (ethyl acetate:hexane gradient, 40:60 rising to 100:0) to give 229 mg (0.6 mmol, 25% over 2 steps) of the title compound. Purity 95%.
¹H NMR (400 MHz, CHLOROFORM-d) δ ppm 0.55 - 0.61 (m, 2 H), 0.81 - 0.87 (m, 2 H), 1.29 (t, J=7.13 Hz, 3 H), 1.54 (s, 9 H), 1.64 - 1.75 (m, 1 H), 2.74 (t, J=6.35 Hz, 2 H), 4.08 (t, 2 H), 4.24 (q, J=7.23 Hz, 2 H), 4.66 (s, 2 H), 6.27 (d, J=1.17 Hz, 1 H).
HPLC/MS (5 min) retention time 3.97 min.
LRMS: *m*/*z* 363 (M+1).

### PREPARATION 81

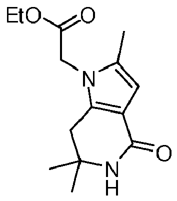

### Ethyl (2,6,6-trimethyl-4-oxo-4,5,6,7-tetrahydro-1H-pyrrolo[3,2-c]pyridin-1-yl)acetate

The crude title compound of Preparation 71 (1.44 g), glycine ethyl ester hydrochloride (1.0 g, 7.10 mmol) and sodium acetate (1.2 g, 14.63 mmol) were treated according to the method of Preparation 77. The resulting residue was purified using the SP1 Purification System (methanol-ethyl acetate gradient, 0:100 rising to 6:94) to give 1.33 g (5.03 mmol, 57% over two steps) of the title compound. Purity 100%.
¹H NMR (400 MHz, CHLOROFORM-d) δ ppm 6.30 (s, 1 H), 5.00 (s, 1 H), 4.48 (s, 2H), 4.23 (q, J = 7.1 Hz, 2H), 2.68 (s, 2H), 2.19 (s, 3H), 1.35 (s, 6H), 1.28 (t, J = 7.1 Hz, 3H).
HPLC/MS (9 min) retention time 4.8 min.
LRMS: *m*/*z* 265 (M+1).

### PREPARATION 82

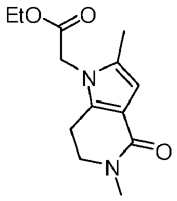

### Ethyl 2-(2,5-dimethyl-4-oxo-4,5,6,7-tetrahydro-1H-pyrrolo[3,2-c]pyridin-1-yl)acetate

The crude title compound of Preparation 72 (183 mg), glycine ethyl ester hydrochloride, (181 mg, 1.3 mmol) and sodium acetate (213 mg, 2.6 mmol) were suspended in 3 ml ethanol. The mixture was stirred at room temperature for 2 h. The mixture was evaporated under reduced pressure. The residue was dissolved in dichloromethane, filtered and the filtrate was evaporated under reduced pressure. The resulting residue was purified using the SP1 Purification System (methanol:ethyl acetate gradient, 0:100 rising to 6:94) to give 50 mg (0.20 mmol, 15% over two steps) of the title compound. Purity 97%.
¹H NMR (400 MHz, CHLOROFORM-d) δ ppm 1.28 (t, J=7.23 Hz, 3 H), 2.17 (s, 3 H), 2.78 (t, J=7.03 Hz, 2 H), 3.03 (s, 3 H), 3.56 (t, J=7.03 Hz, 2 H), 4.23 (q, J=7.03 Hz, 2 H), 4.47 (s, 2 H), 6.29 (s, 1 H).
HPLC/MS (5 min) retention time 3.02 min.
LRMS: *m*/*z* 251 (M+1).

### PREPARATION 83

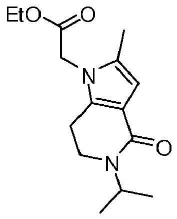

### Ethyl (5-isopropyl-2-methyl-4-oxo-4,5,6,7-tetrahydro-1H-pyrrolo[3,2-c]pyridin-1-yl)aceta te

The crude title compound of Preparation 73 (550 mg) glycine ethyl ester hydrochloride (0.36 g, 2.6 mmol) and sodium acetate (0.42 g, 5.2 mmol) were suspended in 2 ml ethanol. The mixture was stirred at room temperature for 30 min. The mixture was evaporated under reduced pressure. The residue was dissolved in dichloromethane, was filtered and the filtrated was evaporated under reduced pressure. The resulting residue was purified using the SP1 Purification System (ethyl acetate:hexane gradient, 50:50 rising to 100:0) to give 240 mg (0.86 mmol, 33% over two steps) of the title compound. Purity 95%.
¹H NMR (400 MHz, CHLOROFORM-d) δ ppm 1.14 (d, J=6.84 Hz, 6 H), 1.28 (t, J=7.13 Hz, 3 H), 2.18 (s, 3 H), 2.70 (t, J=6.84 Hz, 2 H), 3.45 (t, J=6.94 Hz, 2 H), 4.23 (q, J=7.03 Hz, 2 H), 4.47 (s, 2 H), 4.93 - 5.05 (m, 1 H), 6.27 - 6.36 (m, 1 H).
HPLC/MS (5 min) retention time 2.25 min.
LRMS: *m*/*z* 279 (M+1).

### PREPARATION 84

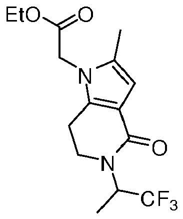

### Ethyl [2-methyl-4-oxo-5-(2,2,2-trifluoro-1-methylethyl)-4,5,6,7-tetrahydro-1H-pyrrolo[3,2

### -c]pyridin-1-yl]acetate

The crude title compound of Preparation 74 (65 mg), glycine ethyl ester hydrochloride (34 mg, 0.245 mmol) and sodium acetate (40 mg, 0.49 mmol) were treated according to the method of Preparation 77. The resulting residue was purified using the SP1 Purification System (ethyl acetate:hexane gradient, 50:50 rising to 100:0) to give 36 mg (0.108 mmol, 11 % over six steps) of the title compound. Purity 97%.
¹H NMR (400 MHz, CHLOROFORM-d) δ ppm 1.29 (t, J=7.13 Hz, 3 H), 1.37 (d, J=7.23 Hz, 3 H), 2.18 (s, 3 H), 2.65 - 2.85 (m, 2 H), 3.53 - 3.67 (m, 2 H), 4.24 (q, J=7.03 Hz, 2 H), 4.48 (s, 2 H), 5.46 - 5.59 (m, 1 H), 6.32 (s, 1 H).
HPLC/MS (5 min) retention time 3.66 min.
LRMS: *m*/*z* 333 (M+1).

### PREPARATION 85

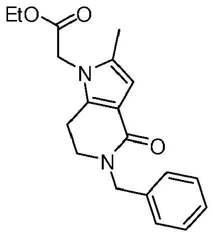

### Ethyl (5-benzyl-2-methyl-4-oxo-4,5,6,7-tetrahydro-1H-pyrrolo[3,2-c]pyridin-1-yl)acetate

The crude title compound of Preparation 75 (114 mg), glycine ethyl ester hydrochloride (61 mg, 0.44 mmol) and sodium acetate (72 mg, 0.88 mmol) were treated according to the method of Preparation 77. The resulting residue was purified using the SP1 Purification System (ethyl acetate:hexane gradient, 50:50 rising to 100:0) to give 75 mg (0.22 mmol, 34% over three steps) of the title compound. Purity 96%.
¹H NMR (400 MHz, CHLOROFORM-d) δ ppm 1.27 (t, J=7.13 Hz, 3 H), 2.19 (s, 3 H), 2.70 (t, J=6.94 Hz, 2 H), 3.47 (t, J=6.94 Hz, 2 H), 4.21 (q, J=7.03 Hz, 2 H), 4.45 (s, 2 H), 4.70 (s, 2 H), 6.33 - 6.37 (m, 1 H), 7.21 - 7.28 (m, 1 H), 7.29 - 7.33 (m, 4 H).
HPLC/MS (5 min) retention time 3.76 min.
LRMS: *m*/*z* 327 (M+1).

### PREPARATION 86

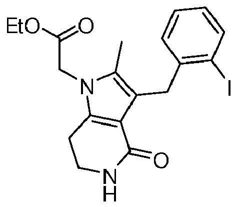

### Ethyl [3-(2-iodobenzyl)-2-methyl-4-oxo-4,5,6,7-tetrahydro-1H-pyrrolo[3,2-c]pyridin-1-yl] acetate

The title compound of Preparation 77 (387 mg, 1.15 mmol) was suspended in anhydride acetic (0.4 ml, 4.2 mmol), hydriodic Acid (57% solution, 0.4 ml, 4.2 mmol) and hypophosphorous acid (50% solution, 0.11 ml, 1.05 mmol). 2-Iodobenzaldehyde (400 mg, 1.7 mmol) was then added and the mixture was stirred at room temperature for 1 h. The mixture was poured into water and the aqueous layer was extracted with dichloromethane three times. The combined organics were dried over magnesium sulphate, filtered and evaporated under reduced pressure. The resulting residue was purified using the SP1 Purification System (methanol-dichloromethane gradient, 0:100 rising 7:93) to give 340 mg (0.751 mmol, 65%) of the title compound. Purity 100%.
¹H NMR (400 MHz, CHLOROFORM-d) δ ppm 1.28 (t, J=7.13 Hz, 3 H), 1.97 (s, 3 H), 2.78 (t, J=6.84 Hz, 2 H), 3.54 - 3.59 (m, 2 H), 4.19 (s, 2 H), 4.24 (q, J=7.23 Hz, 2 H), 4.51 (s, 2 H), 5.21 (s, 1 H), 6.78 - 6.84 (m, 1 H), 6.94 (dd, J=7.72, 1.66 Hz, 1 H), 7.12 - 7.17 (m, 1 H), 7.80 (dd, J=7.82, 1.17 Hz, 1 H).
HPLC/MS (5 min) retention time 4.11 min.
LRMS: *m*/*z* 453 (M+1).

### PREPARATION 87

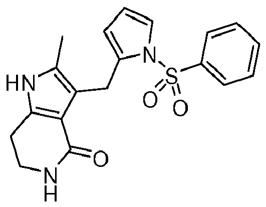

### 2-Bethyl-3-{[1-(phenylsulfonyl)-1H-pyrrol-2-yl]methyl}-1,5,6,7-tetrahydro-4H-pyrro lo[3,2-c]pyridin-4-one

The title compound of Preparation 76 (60 mg, 0.24 mmol) and 1-(phenylsulfonyl)-1H-pyrrole-2-carbaldehyde (62 mg, 0.26 mmol) were dissolved in 2 ml dichloromethane and the mixture was cooled to 0°C. Triethylsilane (0.19 ml, 1.2 mmol) and trifluoroacetic acid (55 µl, 0.72 mmol) were added and the mixture was stirred at 0ºC for 1 h and then at room temperature for 22 h. The mixture was evaporated under reduced pressure and the resulting residue was purified by reverse-phase chromatography (formic acid buffer) using the SP1 Purification system to give 29 mg (0.073 mmol, 31%) of the title compound. Purity 94%.
¹H NMR (400 MHz, DMSO-d6) δ ppm 1.68 (s, 3 H), 2.64 (t, J=6.84 Hz, 2 H), 3.25 - 3.29 (m, 2 H), 4.00 (s, 2 H), 5.41 - 5.44 (m, 1 H), 6.13 (t, J=3.32 Hz, 1 H), 6.61 - 6.66 (m, 1 H), 7.24 - 7.26 (m, 1 H), 7.59 - 7.64 (m, 2 H), 7.70 - 7.75 (m, 1 H), 7.86 - 7.90 (m, 2 H), 10.77 (s, 1 H).
HPLC/MS (5 min) retention time 3.67 min.
LRMS: *m*/*z* 370 (M+1).

### PREPARATION 88

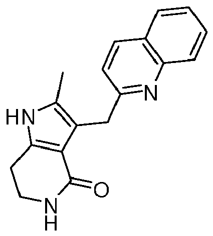

### 2-Methyl-3-(quinolin-2-ylmethyl)-1,5,6,7-tetrahydro-4H-pyrrolo[3,2-c]pyridin-4-one

The title compound of Preparation 76 (125 mg, 0.5 mmol) was suspended in anhydride acetic (0.50 ml, 5.3 mmol), hydriodic acid (57% solution, 0.50 ml, 5.3 mmol) and hypophosphorous acid (50% solution, 0.18 ml, 1.8 mmol). Quinoline-2-carbaldehyde (118 mg, 0.75 mmol) was then added and the mixture was stirred at room temperature for 2 h. Methanol was added and the mixture was purified directly by reverse-phase chromatography (formic acid buffer) using the SP1 Purification system to give 125 mg of the crude title compound. Purity 70%.
¹H NMR (partial, 400 MHz, DMSO-*d*₆) δ ppm 10.82 (1 H, br. s.), 8.13 (1 H, d, *J*=7.6 Hz), 7.91 (1 H, d, *J*=8.4 Hz), 7.84 (1 H, d, *J*=7.8 Hz), 7.67 (1 H, t, *J*=7.6 Hz), 7.49 (1 H, t, *J*=7.3 Hz), 7.43 (1 H, d, *J*=8.4 Hz), 6.74 (1 H, br. s.), 4.27 (2 H, s), 2.66 (2 H, t, *J*=6.8 Hz), 2.05 (2 H, s).
HPLC/MS (5 min) retention time 1.43 min.
LRMS: *m*/*z* 292 (M+1).

### PREPARATION 89

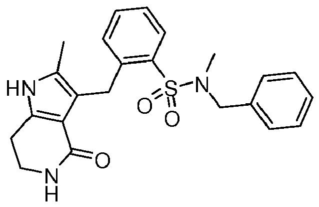

### N-Benzyl-N-methyl-2-[(2-methyl-4-oxo-4,5,6,7-tetrahydro-1H-pyrrolo[3,2-c]pyridin -3-yl)methyl]benzenesulfonamide

The title compound of Preparation 76 (145 mg, 0.58 mmol) was treated with anhydride acetic (1 ml, 10.5 mmol), hydriodic acid (57%, solution, 1.15 ml, 8.7 mmol), hypophosphorous acid (50% solution, 0.3 ml, 2.9 mmol) and the title compound of Preparation 48 (200 mg, 0.69 mmol) according to the method of Preparation 86. The resulting residue was purified by reverse-phase chromatography using the SP1 Purification system to give 20 mg (0.044 mmol, 8%) of the title compound as a yellow solid. Purity 94%.
¹H NMR (400 MHz, CHLOROFORM-d) δ ppm 2.08 (s, 3 H), 2.74 (s, 3 H), 2.88 (t, J=6.84 Hz, 2 H), 3.53 - 3.62 (m, 2 H), 4.44 (s, 2 H), 4.57 (s, 2 H), 5.06 (br. s., 1 H), 7.15 (d, J=7.42 Hz, 1 H), 7.23 - 7.31 (m, 1 H), 7.30 - 7.44 (m, 5 H), 7.96 (br. s., 1 H), 8.01 (d, J=7.82 Hz, 1 H).
HPLC/MS (9 min) retention time 6.03 min.
LRMS: *m*/*z* 424 (M+1).

### PREPARATION 90

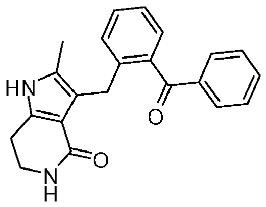

### 3-(2-Benzoylbenzyl)-2-methyl-1,5,6,7-tetrahydro-4H-pyrrolo[3,2-c]pyridin-4-one

The title compound of Preparation 76 (500 mg, 2.0 mmol) was treated with anhydride acetic (1 ml, 10.5 mmol), hydriodic acid (57% solution, 1 ml, 7.6 mmol), hypophosphorous acid (50% solution, 0.47 ml, 4.54 mmol) and a solution of the title compound of Preparation 50 (504 mg, 2.4 mmol) dissolved in 1 ml anhydride acetic according to the method of Preparation 86. The resulting residue was purified by reverse-phase chromatography (formic acid buffer) using the SP1 Purification system to give 125 mg (0.341 mmol, 17%) of the title compound. Purity 94%.
¹H NMR (400 MHz, DMSO-d6) δ ppm 1.80 (s, 3 H), 2.57 (t, J=6.84 Hz, 2 H), 3.22 - 3.27 (m, 2 H), 3.88 (s, 2 H), 5.74 (s, 2 H), 7.12 - 7.24 (m, 3 H), 7.30 - 7.36 (m, 1 H), 7.47 - 7.54 (m, 2 H), 7.60 - 7.71 (m, 3 H).
HPLC/MS (5 min) retention time 3.76 min.
LRMS: *m*/*z* 345 (M+1).

### PREPARATION 91

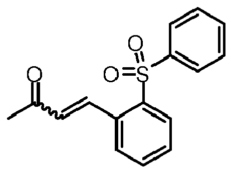

### 4-[2-(Phenylsulfonyl)phenyl]but-3-en-2-one

The title compound of Preparation 2 (100 mg, 0.41 mmol) and 1-triphenylphosphanylidene-propan-2-one (188 mg, 0.53 mmol) were stirred in 2.5 ml toluene at 90°C overnight. The mixture was allowed to cool and was evaporated. The residue was purified using the SP1 Purification System (ethyl acetate-hexane gradient, 0:100 rising to 20:80) to give 116 mg (0.41 mmol, 100%) of the title compound as a 5:3 trans:cis mixture of isomers.
¹H NMR (400 MHz, CHLOROFORM-d) cis (partial) δ ppm 6.17 (d, J=12.31 Hz, 1 H), trans (partial), 6.35 (d, J=16.22 Hz, 1 H).
HPLC/MS (5 min; cis and trans isomer unassigned) retention times 3.54 min and 3.62 min
LRMS: *m*/*z* 287 (M+1).

### PREPARATION 92

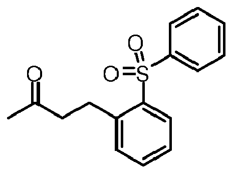

### 4-[2-(Phenylsulfonyl)phenyl]butan-2-one

The title compound of Preparation 91 (122 mg, 0.42 mmol) was dissolved in 3 ml ethyl acetate and 10% Palladium on carbon (15 mg, 0.04 mmol) was added. The mixture was stirred at 50ºC for 3 h under an atmosphere of hydrogen. The mixture was filtered and evaporated under reduced pressure to give 100 mg (0.35 mmols, 82%) of the title compound. Purity 95%.
¹H NMR (400 MHz, CHLOROFORM-d) δ ppm 2.10 (s, 3 H), 2.68 (t, 2 H), 3.04 (t, 2 H), 7.25 - 7.30 (m, 1 H), 7.39 - 7.45 (m, 1 H), 7.48 - 7.55 (m, 3 H), 7.55 - 7.61 (m, 1 H), 7.83 - 7.87 (m, 2 H), 8.20 (dd, J=8.01, 1.37 Hz, 1 H).
HPLC/MS (5 min) retention time 3.66 min.
LRMS: *m*/*z* 289 (M+1).

### PREPARATION 93

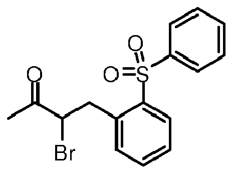

### 3-Bromo-4-[2-(phenylsulfonyl)phenyl]butan-2-one

The title compound of Preparation 92 (50 mg, 0.174 mmol) was dissolved in 0.3 ml acetonitrile and bromotrimethylsilane (25 µl, 0.19 mmol) was slowly added. Dimethylsulfoxide (13.5 µl, 0.19 mmol) was added and the mixture was stirred at room temperature for 2 h. The mixture was partitioned between ether and water. The aquous layer was extracted twice with ether and the combined organics were dried over magnesium sulphate, filtered and evaporated under reduced pressure. The resulting residue was purified using the SP1 Purification System (ethyl acetate-hexane gradient, 0:100 rising to 40:60) to give 23 mg (0.062 mmol, 36%) of the title compound as a white solid. Purity 99%.
¹H NMR (400 MHz, CHLOROFORM-d) δ ppm 2.37 (s, 3 H), 3.11 (dd, J=14.75, 8.11 Hz, 1 H), 3.72 (dd, J=14.75, 5.18 Hz, 1 H), 4.80 (dd, J=8.11, 5.18 Hz, 1 H), 7.39 - 7.43 (m, 1 H), 7.47 - 7.56 (m, 4 H), 7.57 - 7.63 (m, 1 H), 7.80 - 7.85 (m, 2 H), 8.19 (dd, J=7.82, 1.37 Hz, 1 H).
HPLC/MS (5 min) retention time 4.02 min.
LRMS: *m*/*z* 367,369 (M+1).

### PREPARATION 94

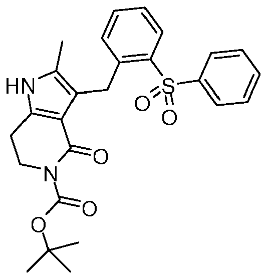

### tert-Butyl 2-methyl-4-oxo-3-[2-(phenylsulfonyl)benzyl]-1,4,6,7-tetrahydro-5H-pyrrolo[3,2-c]p yridine-5-carboxylate

The title compound of Preparation 93 (23 mg, 0.062 mmol) was dissolved in 0.3 ml ethanol. The title compound of Preparation 55 (17.3 mg, 0.08 mmol) and ammonium acetate (19.3 mg, 0.25 mmol) were then added and the mixture was stirred at room temperature for 15 h. The mixture was evaporated under reduced pressure. The resulting residue was purified using the SP1 Purification System (ethyl acetate-MeOH gradient, 0:100 rising 100:0) to give 8 mg (0.016 mmol, 27%) of the title compound. Purity 95%.
¹H NMR (400 MHz, CHLOROFORM-d) δ ppm 1.50 (s, 9 H), 1.79 (s, 3 H), 2.79 (t, J=6.35 Hz, 2 H), 4.00 (t, J=6.35 Hz, 2 H), 4.32 (s, 2 H), 5.32 (s, 1 H), 7.07 (d, J=8.01 Hz, 1 H), 7.29 - 7.34 (m, 1 H), 7.36 - 7.41 (m, 1 H), 7.47 - 7.58 (m, 3 H), 7.88 - 7.93 (m, 2 H), 8.20 (dd, J=7.82, 1.37 Hz, 1 H).
HPLC/MS (5 min) retention time 4.00 min.
LRMS: *m*/*z* 481 (M+1).

### PREPARATION 95

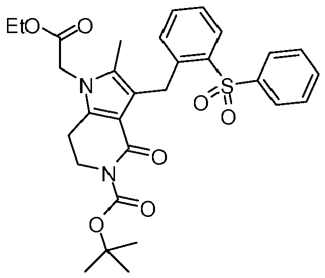

### tert-Butyl 1-(2-ethoxy-2-oxoethyl)-2-methyl-4-oxo-3-[2-(phenylsulfonyl)benzyl]-1,4,6,7-tetra hydro-5H-pyrrolo[3,2-c]pyridine-5-carboxylate

The title compound of Preparation 94 (31 mg, 0.064 mmol) was dissolved in 2 ml dimethylformamide. Sodium hydride (60% dispersion in oil, 5 mg, 0.125 mmol) previously washed with pentane was added and the mixture was stirred for 15 min at room temperature. Ethyl 2-bromoacetate (8 µl, 0.071 mmol) was then added and the mixture was agitated overnight. The mixture was partitioned between ethyl acetate and ammonium chloride saturated solution. The organic layer was washed with ammonium chloride saturated solution three times, dried over magnesium sulphate, filtered and evaporated under reduced pressure. The residue was purified using the SP1 Purification System (ethyl acetate-hexane gradient, 0:100 rising 80:20) to give 15 mg (0.026 mmol, 41 %) of the title compound. Purity 98%.
¹H NMR (400 MHz, CHLOROFORM-d) δ ppm 1.27 (t, J=7.0 Hz, 3 H), 1.50 (s, 9 H), 1.84 (s, 3 H), 2.04 (m, 2 H), 4.03 - 4.08 (m, 2 H), 4.12 (q, J=7.16 Hz, 1 H), 4.22 (q, J=7.03 Hz, 2 H), 4.39 (s, 2 H), 4.46 (s, 2 H), 7.05 (dd, J=7.62, 0.98 Hz, 1 H), 7.29 - 7.35 (m, 1 H), 7.36 - 7.41 (m, 1 H), 7.47 - 7.57 (m, 3 H), 7.90 - 7.93 (m, 2 H), 8.23 (dd, J=7.91, 1.47 Hz, 1 H).
HPLC/MS (5 min) retention time 3.32 min.
LRMS: *m*/*z* 567 (M+1).

### PREPARATION 96

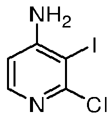

### 2-Chloro-3-iodopyridin-4-amine

2-Chloro-4-aminopyridine (1.8 g, 14 mmol) was dissolved in 28 ml dimethylformamide. N-lodosuccinimide (3.15 g, 14 mmol) was added and the mixture was stirred at room temperature overnight. Additional N-iodosuccinimide (3.15 g, 14 mmol) was added and the mixture wasa stirred at 55ºC overnight. The mixture was evaporated to dryness and the residue was partitioned between ethyl acetate and water. The aqueous was extracted with ethyl acetate and the combined organics were dried over sodium sulphate, filtered and evaporated. Purification by flash chromatography (ethyl acetate-hexane gradient, 15:85 rising to 25:75) gave 1.5 g (5.9 mmol, 42%) of the title compound as a purple solid.
¹H NMR (300 MHz, CHLOROFORM-*d*) δ ppm 8.34 (1 H, s), 6.63 (1 H, s), 4.75 (2 H, br. s.).

### PREPARATION 97

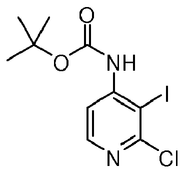

### tert-Butyl (2-chloro-3-iodopyridin-4-yl)carbamate

The title compound of Preparation 96 (6.4 g, 25 mmol) was dissolved in 25 ml tetrahydrofuran and the mixture was cooled in an ice-bath. Di-tert-butylcarbonate (6.0 g, 27.5 mmol) was added and the mixture was stirred for 1 h. The mixture was partitioned between saturated ammonium chloride solution and ethyl acetate. The organics were washed sequentially with 0.1N hydrochloric acid, water and brine. The organics were dried over sodium sulphate, filtered and evaporated. The solid was recrystallised from ethanol and was dried in the oven to give 5.0 g (14 mmol, 56%) of the title compound. Purity 100%.
¹H NMR (400 MHz, CHLOROFORM-*d*) δ ppm 8.15 (1 H, d, *J*=5.5 Hz), 8.03 (1 H, d, *J*=5.5 Hz), 7.32 (1 H, br. s.), 1.55 (9 H, s).
UPLC/MS (3 min) retention time 1.92 min.
LRMS: *m*/*z* 355 (M+1).

### PREPARATION 98

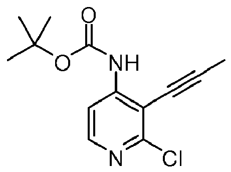

### tert-Butyl (2-chloro-3-prop-1-yn-1-ylpyridin-4-yl)carbamate

### Method A

The title compound of Preparation 97 (2.2 g, 6.2 mmol), copper(I) iodide (60 mg, 0.32 mmol) and (bis(triphenylphosphine) palladium(II) dichloride were suspended in 20 ml de-gassed dimethylformamide in a pressure tube. Nitrogen was bubbled through the mixture for several minutes and the mixture was cooled in a dry ice-acetonitrile bath. Propyne (0.9 ml, 15.8 mmol) was condensed into 2 ml triethylamine, cooled in a dry ice-acetonitrile bath and the solution was cannulated into the pressure tube. The tube was sealed and the mixture was stirred at 50 ºC for 5 h. The mixture was allowed to cool to room temperature and nitrogen was bubbled through the mixture for several minutes. The mixture was partitioned between ether and water and the organics were washed with water, dried and evaporated under reduced pressure. The residue was purified by flash chromatography (ethyl acetate-hexane gradient, 0:100 rising to 10:90) to give 1.44 g (5.4 mmol, 87%) of the title compound as a yellow solid. Purity 100%.

### Method B

The title compound of Preparation 97 (0.88 g, 2.5 mmol), tributyl(prop-1-ynyl)stannane (0.90 ml, 2.95 mmol), palladium tetrakis(triphenylphosphine) (0.20 g, 0.17 mmol) and copper(I) iodide (57 mg, 0.30 mmol) were suspended in 6 ml de-gassed toluene in a pressure tube. Argon was bubbled through the mixture for several minutes, then the tube was sealed and the mixture was stirred at 100 ºC overnight. The mixture was allowed to cool to room temperature and 1.5 ml 0.88N ammonium hydroxide solution and 1.5 ml saturated ammonium chloride solution were added. The phases were separated and the aqueous was extracted twice with dichloromethane. The combined organics were dried over sodium sulphate, filtered and evaporated. The residue was purified using the SP1 Purification System (ethyl acetate-hexane gradient, 0:100 rising to 5:95) to give 0.46 g (1.7 mmol, 70%) of the title compound as a white solid. Purity 100%.
¹H NMR (400 MHz, CHLOROFORM-*d*) δ ppm 8.13 (1 H, d, *J*=5.9 Hz), 8.05 (1 H, d, *J*=5.9 Hz), 7.42 (1 H, br. s.), 2.25 (3 H, s), 1.55 (9 H, s).
UPLC/MS (3 min) retention time 1.90 min.
LRMS: *m*/*z* 267 (M+1).

### PREPARATION 99

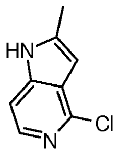

### 4-Chloro-2-methyl-1H-pyrrolo[3,2-c]pyridine

The title compound of Preparation 98 (0.35 g, 1.3 mmol) was dissolved in 9 ml de-gassed dimethylformamide in a pressure tube and nitrogen was bubbled through the mixture for several minutes. Copper(I) iodide (13 mg, 0.07 mmol) was added, the tube was sealed and the mixture was stirred at 110 ºC overnight. The mixture was allowed to cool and was partitioned between ether and water. The aqueous was extracted twice with ether and the organics were washed with water and dried over sodium sulphate. Evaporation under reduced pressure gave 225 mg (1.35 mmol, 87%) of the title compound as a pale yellow solid. Purity 96%.
¹H NMR (400 MHz, CHLOROFORM-*d*) δ ppm 10.10 (1 H, br. s.), 7.96 (1 H, d, *J*=5.5 Hz), 7.16 (1 H, d, *J*=5.5 Hz), 6.31 (1 H, s), 2.22 (3 H, s).
UPLC/MS (3 min) retention time 1.01 min.
LRMS: *m*/*z* 167 (M+1).

### PREPARATION 100

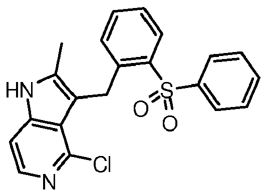

### 4-Chloro-2-methyl-3-[2-(phenylsulfonyl)benzyl]-1H-pyrrolo[3,2-c]pyridine

Hydroiodic acid (57% solution, 3.2 ml, 24.3 mmol) was added slowly to a stirred suspension of the title compound of Preparation 99 (0.4 g, 2.4 mmol) in acetic anhydride (0.6 ml) cooled in an ice-bath. The mixture was removed from the ice-bath and hypophosphorous acid (50% solution, 1.23 ml, 11.9 mmol) and the title compound of Preparation 2 (0.765 g, 3.11 mmol) were added. The mixture was stirred for 7 d. The mixture was diluted with water and extracted with ethyl acetate. The organic layer was washed with saturated sodium bicarbonate solution until the washings were neutral, brine, dried over sodium sulphate and evaporated The residue was purified by flash chromatography (ethyl acetate-hexane gradient, 0:100 rising to 100:0) to give 457 mg (1.15 mmol, 48%) of the title compound as a white solid. Purity 98%.
¹H NMR (400 MHz, DMSO-d₆) δ ppm 11.83 (1 H, br. s.), 8.23-8.26 (1 H, m), 7.93-7.96 (2 H, m), 7.73-7.83 (2 H, m), 7.63-7.69 (2 H, m), 7.50-7.53 (2 H, m), 7.29 (1 H, d, J=5.5 Hz), 6.65-6.67 (1 H, m), 4.30 (2 H, s), 2.08 (3 H, s).
UPLC/MS (3 min) retention time 1.62 min.
LRMS: *m*/*z* 397 (M+1).

### PREPARATION 101

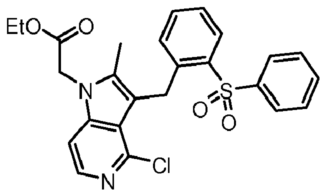

### Ethyl {4-chloro-2-methyl-3-[2-(phenylsulfonyl)benzyl]-1H-pyrrolo[3,2-c]pyridin-1-yl}acet ate

The title compound of Preparation 100 (457 mg, 1.15 mmol) was dissolved in 5 ml dimethylformamide and was cooled in an ice-bath under nitrogen. Sodium hydride (60% in oil, 48 mg, 1.20 mmol) was added. The mixture was stirred for 10 min at room temperature and then ethyl 2-bromoacetate (0.134 mL, 1.21 mmol) was added. The mixture was stirred at room temperature for 5 h and was then partitioned between water and ethyl acetate. The organic layer was washed with brine, dried and evaporated. The residue purified by flash chromatography (ethyl acetate-hexane gradient, 0:100 rising to 100:0, followed by methanol-dichloromethane, 5:95) to give 500 mg (1.04 mmol, 90%) of the title compound as a white solid. Purity 98%.
¹H NMR (400 MHz, CDCl₃) δ ppm 8.36 (1 H, dd, J=7.7, 1.6 Hz), 7.94-7.99 (3 H, m), 7.51-7.64 (3 H, m), 7.34-7.45 (2 H, m), 7.04 (1 H, d, J=5.5 Hz), 6.75 (1 H, d, J=7.2 Hz), 4.81 (2 H, s), 4.48 (2 H, s), 4.24 (2 H, q, J=7.1 Hz), 2.19 (3 H, s), 1.28 (3 H, t, J=7.1 Hz).
UPLC/MS (3 min) retention time 1.77 min.
LRMS: *m*/*z* 483 (M+1).

### PREPARATION 102

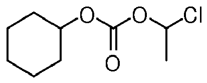

### 1-Chloroethyl cyclohexyl carbonate

Cyclohexanol (0.50 g, 5.0 mmol) and pyridine (0.40 ml, 5.0 mmol) were dissolved in 10 ml dichloromethane and the mixture cooled to -78 ºC. a solution of 1-chloroethyl carbonochloridate (0.54 ml, 5.0 mmol) dissolved in 2 ml dichloromethane was added drop-wise and with stirring. The mixture was stirred for 30 min at -78ºC and then overnight, warming to room temperature and forming a precipitate. The mixture was diluted with dichloromethane and was washed sequentially with twice with water, once with 0.1N hydrochloric acid, once with water and once with brine. The organics were dried over sodium sulphate, filtered and evaporated under reduced pressure to give 0.82 g (4.0 mmol, 80%) of the title compound as a colourless oil.
¹H NMR (400 MHz, CHLOROFORM-*d*) δ ppm 6.43 (1 H, q, *J*=5.9 Hz), 4.69 (0 H, tt, *J*=9.0, 4.5 Hz), 1.89 - 2.01 (2 H, m), 1.83 (3 H, d, *J*=5.9 Hz), 1.70 - 1.81 (2 H, m), 1.45 - 1.58 (2 H, m), 1.22 - 1.44 (4 H, m).

### EXAMPLES

### EXAMPLE 1

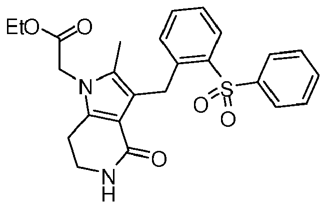

### Ethyl 2-(2-methyl-4-oxo-3-(2-(phenylsulfonyl)benzyl)-4,5,6,7-tetrahydro-1H-pyrrolo[3,2-c]pyridin-1-yl)acetate

### Method A

The title compound of Preparation 95 (15 mg, 0.026 mmol) was dissolved in 4 ml dichloromethane. Trifluoroacetic acid (500 µl, 6.49 mmol) was added and the mixture was stirred for 30 minutes at room temperature. The mixture was concentrated under reduced pressure to give 12 mg (0.026 mmol, 97%) of the title compound. Purity 98%.

### Method B

The title compound of Preparation 77 (400 mg, 1.2 mmol) was suspended in anhydride acetic (2.4 ml, 25.4 mmol), hydriodic Acid (57% solution, 2.4 ml, 25.4 mmol) and hypophosphorous acid (50% solution, 73 µl, 5.63 mmol). The title compound of Preparation 2 (440 mg, 1.79 mmol) was then added and the mixture was stirred at room temperature for 1.5 h. The mixture was partitioned between water and dichloromethane. The aqueous layer was extracted with dichloromethane two times. The combined organics were dried over magnesium sulphate, filtered and evaporated under reduced pressure. The resulting residue was purified by reverse-phase chromatography (formic acid buffer) using the SP1 Purification system to give 340 mg (0.728 mmol, 61 %) of the title compound. Purity 100%.
¹H NMR (partial, 400 MHz, DMSO-d6) δ ppm 8.12 (1 H, d, J=6.6 Hz), 7.88 (2 H, d, J=7.4 Hz), 7.69 (1 H, t, J=7.2 Hz), 7.62 (2 H, t, J=7.5 Hz), 7.51 (1 H, t, J=6.9 Hz), 7.44 (1 H, t, J=7.3 Hz), 6.92 (1 H, d, J=7.6 Hz), 6.79 (1 H, br. s.), 4.70 (2 H, s), 4.16 (2 H, s), 4.11 (2 H, q, J=7.0 Hz), 2.66 (2 H, t, J=6.8 Hz), 1.41 (3 H, s), 1.16 (3 H, t, J=7.0 Hz).
HPLC/MS (30 min) retention time 14.38 min.
LRMS: m/z 467 (M+1).

### EXAMPLE 2

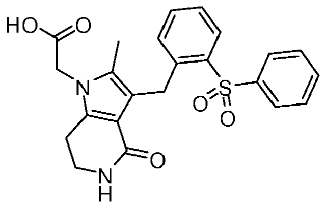

### {2-Methyl-4-oxo-3-[2-(phenylsulfonyl)benzyl]-4,5,6,7-tetrahydro-1H-pyrrolo[3,2-c] pyridin-1-yl}acetic acid

### Method A

The title compound of Example 1 (12 mg, 0.026 mmol) was dissolved in 1 ml tetrahydrofuran and 0.5 ml water. Lithium hydroxide monohydrate (1.5 mg, 0.062 mmol) was added and the mixture was agitated at room temperature for 15 min. The mixture was partitioned between dilute lithium hydroxide solution (pH 8-9) and ethyl acetate. The aqueous layer was washed with ethyl acetate and was acidified with 6N hydrochloric acid. The aqueous layer was extracted three times with ethyl acetate. The combined organics were washed with brine, dried over magnesium sulphate and evaporated to give 11 mg (0.033 mmol, 97%) of the title compound. Purity 97%.

### Method B

The title compound of Preparation 77 (400 mg, 1.2 mmol) was treated with anhydride acetic (2.4 ml, 25.4 mmol), hydriodic Acid (57% solution, 2.4 ml, 18.2 mmol) and hypophosphorous acid (50% solution, 0.58 ml, 5.6 mmol). The title compound of Preparation 2 (440 mg, 1.79 mmol) was added and the mixture was stirred at room temperature for 21 h. The mixture was partitioned between water and dichloromethane. The aqueous layer was extracted twice with dichlaramethane. The combined organics were dried over magnesium sulphate, filtered and evaporated under reduced pressure. The resulting residue was purified by reverse-phase chromatography (formic acid buffer) using the SP1 Purification system to give 136 mg (0.31 mmol, 26%) of the title compound as a white solid. Purity 99%.
¹H NMR (400 MHz, DMSO-d6) δ ppm 8.13 (1 H, d, J=7.8 Hz), 7.88 (2 H, d, J=7.4 Hz), 7.70 (1 H, t, J=7.3 Hz), 7.62 (2 H, t, J=7.8 Hz), 7.51 (1 H, t, J=7.4 Hz), 7.45 (1 H, d, J=7.4 Hz), 6.94 (1 H, d, J=7.4 Hz), 6.77 (1 H, br. s.), 4.58 (2 H, s), 4.15 (2 H, s), 3.31 (2 H, t, J=6.6 Hz), 2.66 (2 H, t, J=6.8 Hz), 1.42 (3 H, s).
HPLC/MS (30 min) retention time 12.45 min.
LRMS: *m*/*z* 439 (M+1).

### EXAMPLE 3

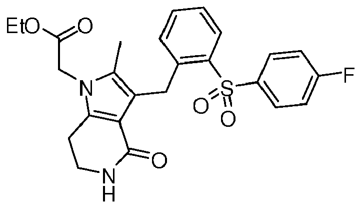

### Ethyl (3-{2-[(4-fluorophenyl)sulfonyl]benzyl}-2-methyl-4-oxo-4,5,6,7-tetrahydro-1H-pyrr olo[3,2-c]pyridin-1-yl)acetate

The title compound of Preparation 77 (50 mg, 0.15 mmol) was treated with anhydride acetic (0.30 ml, 3.17 mmol), hydriodic acid (57% solution, 0.30 ml, 2.27 mmol) and hypophosphorous acid (50% solution, 73 µl, 0.7 mmol). The title compound of Preparation 4 (59 mg, 0.223 mmol) was then added and the mixture was stirred at room temperature for 2 h. The mixture was partitioned between water and dichloromethane. The aqueous layer was extracted with dichloromethane two times. The combined organics were dried over magnesium sulphate, filtered and evaporated under reduced pressure. The residue was dissolved in dichloromethane and polystyrene-supported tosyl hydrazine resin (60 mg, loading 3.7 mmol/g, 0.22 mmol) was added and the mixture was agitated for 1 h. The mixture was filtered and the filtrated evaporated under reduced pressure to give 72 mg of the crude title compound. Used as such without further purification.
HPLC/MS (5 min) retention time 3.91 min.
LRMS: *m*/*z* 485 (M+1).

### EXAMPLE 4

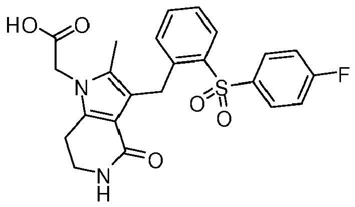

### (3-{2-[(4-Fluorophenyl)sulfonyl]benzyl}-2-methyl-4-oxo-4,5,6,7-tetrahydro-1H-pyrr olo[3,2-c]pyridin-1-yl)acetic acid

### Method A

The crude title compound of Example 3 (72 mg) was dissolved in 1 ml tetrahydrofuran and 0.5 ml of water. Lithium hydroxide monohydrate (7 mg, 0.29 mmol) was added and the mixture was agitated at room temperature for 30 min. The mixture was partitioned between water and ethyl acetate. The aqueous layer was acidified with 2N hydrochloric acid. The aqueous layer was extracted twice with ethyl acetate. The combined organics were washed with brine, dried over magnesium sulphate and evaporated. The resulting residue was purified by reverse-phase chromatography (formic acid buffer) using the SP1 Purification system to give 29 mg (0.063 mmol, 43% over two steps) of the title compound. Purity 98%.

### Method B

The title compound of Preparation 77 (450 mg, 1.34 mmol) was suspended in anhydride acetic (2.7 ml, 28.5 mmol), hydriodic acid (57% solution, 2.7 ml, 20.5 mmol) and hypophosphorous acid (50% solution, 0.66 ml, 6.33 mmol). The title compound of Preparation 4 (460 mg, 1.74 mmol) was then added and the mixture was stirred at room temperature for 4 d. The mixture was partitioned between water and dichloromethane. The aqueous layer was extracted with dichloromethane, two times. The combined organics were dried over magnesium sulphate, filtered and evaporated under reduced pressure. The resulting residue was dissolved in dichloromethane polystyrene-supported tosyl hydrazine resin (60 mg, loading 3.7 mmol/g, 0.22 mmol) was added and the mixture was agitated at room temperature for 4 h. The mixture was filtered and the filtrated evaporated under reduced pressure. The resulting residue was purified by reverse-phase chromatography (formic acid buffer) using the SP1 Purification system to give 423 mg (0.926 mmol, 69%) of the title compound. Purity 99%
¹H NMR (partial, 400 MHz, DMSO-d6) δ ppm 8.11 (1 H, d, J=1.0 Hz), 7.97 (2 H, dd, J=8.8, 5.1 Hz), 7.52 (1 H, t, J=7.6 Hz), 7.40 - 7.48 (3 H, m), 6.94 (1 H, d, J=7.6 Hz), 6.76 (1 H, br. s.), 4.60 (2 H, s), 4.16 (2 H, s), 2.66 (2 H, t, J=6.8 Hz), 1.51 (3 H, s).
HPLC/MS (30 min) retention time 12.85 min.
LRMS: *m*/*z* 457 (M+1).

### EXAMPLE 5

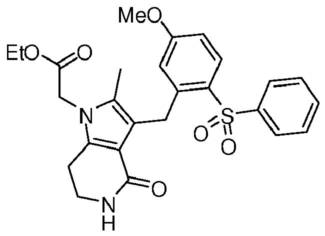

### Ethyl {3-[5-methoxy-2-(phenylsulfonyl)benzyl]-2-methyl-4-oxo-4,5,6,7-tetrahydro-1H-py rrolo[3,2-c]pyridin-1-yl}acetate

The title compound of Preparation 77 (50 mg, 0.15 mmol) was treated with anhydride acetic (0.3 ml, 3.2 mmol), hydroiodic Acid (57% solution, 0.3 ml, 2.3 mmol), hypophosphorous acid (50% solution, 73 µl, 0.7 mmol) and the title compound of Preparation 25 (62 mg, 0.223 mmol) according to the method of Example 3 to give the crude title compound. Used as such without further purification. Purity 67%.
HPLC/MS (5 min) retention time 3.86 min.
LRMS: *m*/*z* 497 (M+1).

### EXAMPLE 6

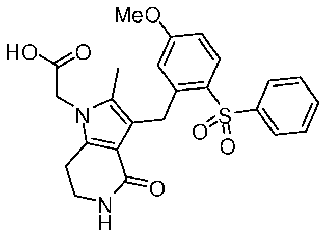

### {3-[5-Methoxy-2-(phenylsulfonyl)benzyl]-2-methyl-4-oxo-4,5,6,7-tetrahydro-1H-py rrolo[3,2-c]pyridin-1-yl}acetic acid

The crude title compound of Example 5 was treated with lithium hydroxide monohydrate (7 mg, 0.29 mmol) according to Method A of Example 4. The resulting residue was purified by reverse-phase chromatography (formic acid buffer) using the SP1 Purification system to give 20 mg (0.042 mmol, 29% over two steps) of the title compound. Purity 100%.
¹H NMR (partial, 400 MHz, DMSO-d6) δ ppm 8.09 (1 H, d, J=8.8 Hz), 7.84 (2 H, d, J=7.4 Hz), 7.66 (1 H, t, J=7.2 Hz), 7.60 (2 H, t, J=7.8 Hz), 6.99 (1 H, dd, J=8.8, 2.5 Hz), 6.75 (1 H, br. s.), 6.40 (1 H, d, J=2.1 Hz), 4.55 (2 H, s), 4.08 (2 H, s), 3.67 (3 H, s), 2.65 (2 H, t, J=6.7 Hz), 1.44 (3 H, s).
HPLC/MS (30 min) retention time 12.60 min.
LRMS: *m*/*z* 469 (M+1).

### EXAMPLE 7

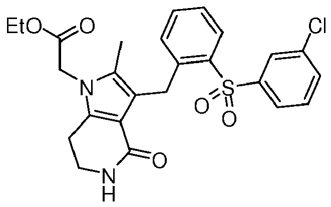

### Ethyl (3-{2-[(3-chlorophenyl)sulfonyl]benzyl}-2-methyl-4-oxo-4,5,6,7-tetrahydro-1H-pyrr olo[3,2-c]pyridin-1-yl)acetate

The title compound of Preparation 77 (50 mg, 0.15 mmol) was suspended in anhydride acetic (0.3 ml, 3.2 mmol), hydriodic Acid (57% solution, 0.3 ml, 2.3 mmol), hypophosphorous acid (50% solution, 73 µl, 0.7 mmol). The title compound of Preparation 13 (59 mg, 0.223 mmol) was then added and the mixture was stirred at room temperature for 3 h. The mixture was partitioned between water and dichloromethane. The aqueous layer was extracted twice with dichloromethane. The combined organics were dried over magnesium sulphate, filtered and evaporated under reduced pressure. The resulting residue was dissolved in dichloromethane and polystyrene-supported tosyl hydrazine resin (60 mg, loading 3.7 mmol/g, 60 mg, 0.22 mmol) was added and the mixture was agitated for 30 min. The mixture was filtered and the filtrated evaporated under reduced pressure to give 91 mg of the crude title compound. Used as such without further purification. Purity 52%.
HPLC/MS (5 min) retention time 4.12 min.
LRMS: *m*/*z* 501 (M+1).

### EXAMPLE 8

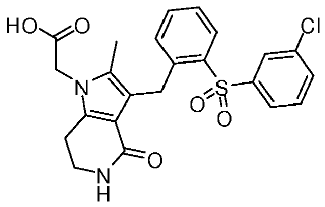

### (3-{2-[(3-Chlorophenyl)sulfonyl]benzyl}-2-methyl-4-oxo-4,5,6,7-tetrahydro-1H-pyr rolo[3,2-c]pyridin-1-yl)acetic acid

The crude title compound of Example 7 (91 mg) was treated with lithium hydroxide monohydrate (7 mg, 0.29 mmol) according to Method A of Example 4. The resulting residue was purified by reverse-phase chromatography (formic acid buffer) using the SP1 Purification system to give 40.5 mg (0.085 mmol, 58% over two steps) of the title compound. Purity 100%.
¹H NMR (partial, 400 MHz, DMSO-d6) δ ppm 8.15 (1 H, dd, J=7.8, 1.0 Hz), 7.85 - 7.91 (2 H, m), 7.80 (1 H, d, J=8.2 Hz), 7.66 (1 H, t, J=8.2 Hz), 7.55 (1 H, t, J=6.9 Hz), 7.46 (1 H, t, J=7.3 Hz), 6.96 (1 H, d, J=7.6 Hz), 6.76 (1 H, br. s.), 4.59 (2 H, s), 4.16 (2 H, s), 2.66 (2 H, t, J=6.8 Hz), 1.50 (3 H, s).
HPLC/MS (30 min) retention time 13.93 min.
LRMS: *m*/*z* 473, 475 (M+1).

### EXAMPLE 9

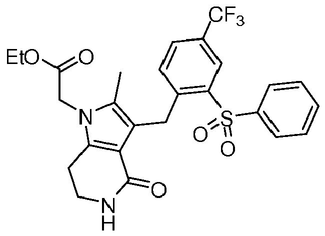

### Ethyl {2-methyl-4-oxo-3-[2-(phenylsulfonyl)-4-(trifluoromethyl)benzyl]-4,5,6,7-tetrahydr o-1H-pyrrolo[3,2-c]pyridin-1-yl}acetate

The title compound of Preparation 77 (50 mg, 0.15 mmol) was treated with anhydride acetic (0.3 ml, 3.2 mmol), hydriodic Acid (57% solution, 0.3 ml, 2.3 mmol), hypophosphorous acid (50% solution, 73 µl, 0.7 mmol) and the title compound of Preparation 9 (70 mg, 0.223 mmol) according to the method of Example 7 to give 99 mg of the crude title compound. Used as such without further purification. Purity 48%.
HPLC/MS (5 min) retention time 4.23 min.
LRMS: *m*/*z* 535 (M+1).

### EXAMPLE 10

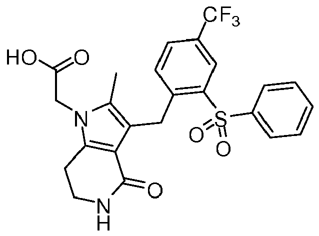

### {2-Methyl-4-oxo-3-[2-(phenylsulfonyl)-4-(trifluoromethyl)benzyl]-4,5,6,7-tetrahydr o-1H-pyrrolo[3,2-c]pyridin-1-yl}acetic acid

The crude title compound of Example 9 (99 mg) was treated with lithium hydroxide monohydrate (7 mg, 0.29 mmol) according to Method A of Example 4. The resulting residue was purified by reverse-phase chromatography (formic acid buffer) using the SP1 Purification system to give 29.5 mg (0.058 mmol, 39% over two steps) of the title compound. Purity 100%.
¹H NMR (partial, 400 MHz, DMSO-d6) δ ppm 8.38 (1 H, s), 7.98 (2 H, d, J=7.6 Hz), 7.95 (1 H, d, J=8.2 Hz), 7.74 (1 H, d, J=7.4 Hz), 7.65 (2 H, t, J=7.6 Hz), 7.16 (1 H, d, J=8.2 Hz), 6.80 (1 H, br. s.), 4.59 (2 H, s), 4.18 (2 H, s), 2.66 (2 H, t, J=6.8 Hz), 1.46 (3 H, s).
HPLC/MS (30 min) retention time 15.05 min.
LRMS: *m*/*z* 507 (M+1).

### EXAMPLE 11

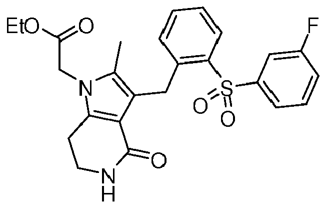

### Ethyl (3-{2-[(3-fluorophenyl)sulfonyl]benzyl}-2-methyl-4-oxo-4,5,6,7-tetrahydro-1H-pyrr olo[3,2-c]pyridin-1-y))acetate

The title compound of Preparation 77 (50 mg, 0.15 mmol) was treated with anhydride acetic (0.3 ml, 3.2 mmol), hydroiodic Acid (57% solution, 0.3 ml, 2.3 mmol), hypophosphorous acid (50% solution, 73 µl, 0.7 mmol) and the title compound of Preparation 15 (59 mg, 0.223 mmol) according to the method of Example 5 to give 87 mg of the crude title compound. Used as such without further purification. Purity 53%.
HPLC/MS (5 min) retention time 3.97 min.
LRMS: *m*/*z* 485 (M+1).

### EXAMPLE 12

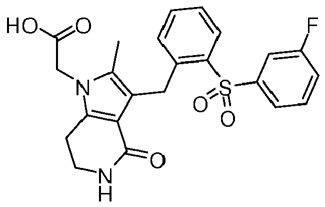

### (3-{2-[(3-Fluorophenyl)sulfonyl]benzyl}-2-methyl-4-oxo-4,5,6,7-tetrahydro-1H-pyrr olo[3,2-c]pyridin-1-yl)acetic acid

The crude title compound of Example 11 (87 mg) was treated with lithium hydroxide monohydrate (7 mg, 0.29 mmol) according to Method A of Example 4. The resulting residue was purified by reverse-phase chromatography (formic acid buffer) using the SP1 Purification system to give 26 mg (0.056 mmol, 38% over two steps) of the title compound. Purity 99%.
¹H NMR (partial, 400 MHz, DMSO-d6) δ ppm 8.13 (1 H, d, J=8.0 Hz), 7.63 - 7.80 (3 H, m), 7.58 (1 H, t, J=7.4 Hz), 7.54 (1 H, t, J=7.4 Hz), 7.45 (1 H, t, J=7.5 Hz), 6.95 (1 H, s), 6.75 (1 H, br. s.), 4.58 (2 H, s), 4.17 (2 H, s), 2.65 (2 H, t, J=6.7 Hz), 1.51 (3 H, s).
HPLC/MS (30 min) retention time 13.02 min.
LRMS: *m*/*z* 457 (M+1).

### EXAMPLE 13

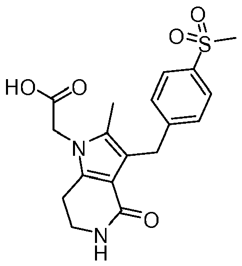

### {2-Methyl-3-[4-(methylsulfonyl)benzyl]-4-oxo-4,5,6,7-tetrahydro-1H-pyrrolo[3,2-c] pyridin-1-yl}acetic acid

The title compound of Preparation 77 (50 mg, 0.15 mmol) was suspended in anhydride acetic (0.3 ml, 3.2 mmol), hydriodic Acid (57% solution, 0.3 ml, 2.3 mmol), hypophosphorous acid (50% solution, 73 µl, 0.7 mmol). 4-(Methylsulfonyl)-benzaldehyde (41 mg, 0.223 mmol) was then added and the mixture was stirred at room temperature for 24 h. The mixture was partitioned between water and dichloromethane. The aqueous layer was extracted with dichloromethane two times. The combined organics were dried over magnesium sulphate, filtered and evaporated under reduced pressure. The resulting residue was dissolved in dichloromethane polystyrene-supported tosyl hydrazine resin (60 mg, loading 3.7 mmol/g, 0.22 mmol) was added and the mixture was agitated at room temperature for 4 h. The mixture was filtered and the filtrated evaporated under reduced pressure. The resulting residue was purified by reverse-phase chromatography (formic acid buffer) using the SP1 Purification system to give 16 mg (0.043 mmol, 29%) of the title compound. Purity 98%.
¹H NMR (400 MHz, DMSO-d6) δ ppm 7.73 (2 H, d, J=8.4 Hz), 7.47 (2 H, d, J=8.2 Hz), 6.77 (1 H, br. s.), 4.58 (2 H, s), 4.07 (2 H, s), 3.28 (2 H, t, J=6.6 Hz), 3.12 (3 H, s), 2.63 (2 H, t, J=6.8 Hz), 2.02 (3 H, s).
HPLC/MS (30 min) retention time 9.17 min.
LRMS: m/z 377 (M+1).

### EXAMPLE 14

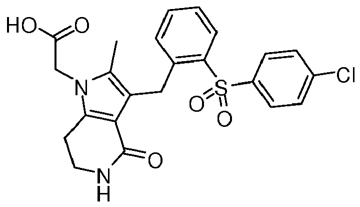

### (3-{2-[(4-Chlorophenyl)sulfonyl]benzyl}-2-methyl-4-oxo-4,5,6,7-tetrahydro-1H-pyr rolo[3,2-c]pyridin-1-yl)acetic acid

The title compound of Preparation 77 (75 mg, 0.223 mmol) was treated with anhydride acetic (0.40 ml, 4.22 mmol), hydriodic acid (57% solution, 0.40 ml, 3.0 mmol), hypophosphorous acid (50% solution, 0.11 ml, 1.05 mmol) and the title compound of Preparation 5 (94 mg, 0.334 mmol) according to the method of Example 13. The resulting residue was purified by purification by reverse-phase chromatography (formic acid buffer) using the SP1 Purification system to give 54 mg (0.113 mmol, 51 %) of the title compound. Purity 99%.
¹H NMR (400 MHz, CHLOROFORM-d) δ ppm 8.18 (1 H, d, J=7.8 Hz), 7.87 (2 H, d, J=8.4 Hz), 7.46 - 7.54 (2 H, m), 7.43 (1 H, t, J=7.4 Hz), 7.35 (1 H, t, J=7.4 Hz), 7.11 (1 H, d, J=7.6 Hz), 5.75 (1 H, br. s.), 4.48 (2 H, s), 4.34 (2 H, s), 3.54 (2 H, t, J=6.3 Hz), 2.78 (2 H, t, J=6.5 Hz), 1.82 (3 H, s).
HPLC/MS (30 min) retention time 13.83 min.
LRMS: *m*/*z* 473 (M+1).

### EXAMPLE 15

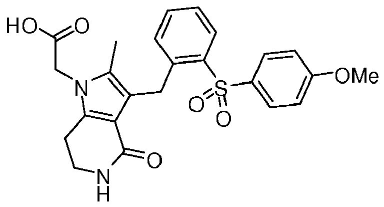

### (3-{2-[(4-Methoxyphenyl)sulfonyl]benzyl}-2-methyl-4-oxo-4,5,6,7-tetrahydro-1H-p yrrolo[3,2-c]pyridin-1-yl)acetic acid

The title compound of Preparation 77 (75 mg, 0.223 mmol) was treated with anhydride acetic (0.40 ml, 4.22 mmol), hydriodic acid (57% solution, 0.40 ml, 3.0 mmol), hypophosphorous acid (50% solution, 0.11 ml, 1.05 mmol) and the title compound of Preparation 7 (92 mg, 0.334 mmol) according to the method of Example 13. The resulting residue was purified by purification by reverse-phase chromatography (formic acid buffer) using the SP1 Purification system to give 67.6 mg (0.142 mmol, 64%) of the title compound. Purity 99%.
¹H NMR (partial, 400 MHz, DMSO-d6) δ ppm 8.07 (1 H, d, J=7.2 Hz), 7.81 (2 H, d, J=9.0 Hz), 7.47 (1 H, t, J=7.1 Hz), 7.40 (1 H, t, J=7.4 Hz), 7.13 (2 H, d, J=8.8 Hz), 6.94 (1 H, d, J=7.6 Hz), 6.77 (1 H, br. s.), 4.59 (2 H, s), 4.18 (2 H, s), 3.82 (3 H, s), 2.66 (2 H, t, J=6.7 Hz), 1.48 (3 H, s).
HPLC/MS (30 min) retention time 12.72 min.
LRMS: *m*/*z* 469 (M+1).

### EXAMPLE 16

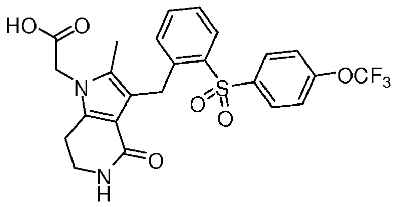

### [2-Methyl-4-oxo-3-(2-{[4-(trifluoromethoxy)phenyl]sulfonyl}benzyl)-4,5,6,7-tetrahy dro-1H-pyrrolo[3,2-c]pyridin-1-yl]acetic acid

The title compound of Preparation 77 (67.9 mg, 0.202 mmol) was suspended in anhydride acetic (0.20 ml, 2.11 mmol), hydriodic acid (57% solution, 0.40 ml, 3.0 mmol), hypophosphorous acid (50% solution, 0.11 ml, 1.05 mmol). The title compound of Preparation 11 (100 mg, 0.303 mmol) dissolved in 200 µl acetic anhydride was then added and the mixture was stirred at room temperature for 24 h. The mixture was partitioned between water and dichloromethane. The aqueous layer was extracted with dichloromethane three times. The combined organics were dried over magnesium sulphate, filtered and evaporated under reduced pressure. The resulting residue was purified by reverse-phase chromatography (formic acid buffer) using the SP1 Purification system to give 50 mg (0.0918 mmol, 45%) of the title compound. Purity 96%.
¹H NMR (400 MHz, DMSO-d6) δ ppm 8.15 (1 H, dd, J=7.9, 1.1 Hz), 8.04 (2 H, d, J=8.8 Hz), 7.61 (2 H, d, J=8.2 Hz), 7.54 (1 H, t, J=7.0 Hz), 7.46 (1 H, t, J=7.4 Hz), 6.95 (1 H, d, J=7.6 Hz), 6.75 (1 H, s), 4.58 (2 H, s), 4.14 (2 H, s), 3.30 (2 H, t, J=6.6 Hz), 2.65 (2 H, t, J=6.7 Hz), 1.43 (3 H, s).
HPLC/MS (30 min) retention time 14.93 min.
LRMS: *m*/*z* 523 (M+1).

### EXAMPLE 17

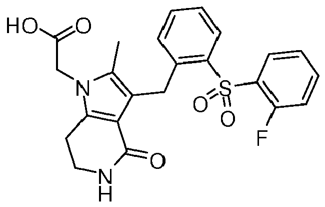

### (3-{2-[(2-Fluorophenyl)sulfonyl]benzyl}-2-methyl-4-oxo-4,5,6,7-tetrahydro-1H-pyrr olo[3,2-c]pyridin-1-yl)acetic acid

The title compound of Preparation 77 (84.8 mg, 0.252 mmol) was treated with anhydride acetic (0.40 ml, 4.22 mmol), hydriodic acid (57% solution, 0.40 ml, 3.0 mmol), hypophosphorous acid (50% solution, 0.11 ml, 1.05 mmol) and the title compound of Preparation 21 (100 mg, 0.378 mmol) according to the method of Example 16. The resulting residue was purified by reverse-phase chromatography (formic acid buffer) using the SP1 Purification system to give 27 mg (0.058 mmol, 23%) of the title compound. Purity 99%.
¹H NMR (partial, 400 MHz, DMSO-d6) d ppm 8.09 - 8.20 (2 H, m), 7.80 (1 H, dd, J=12.5, 7.5 Hz), 7.34 - 7.58 (4 H, m), 6.94 (1 H, d, J=7.6 Hz), 6.74 (1 H, br. s.), 4.55 (2 H, s), 4.09 (2 H, s), 2.64 (2 H, t, J=6.7 Hz), 1.43 (3 H, s).
HPLC/MS (30 min) retention time 12.85 min.
LRMS: *m*/*z* 457 (M+1).

### EXAMPLE 18

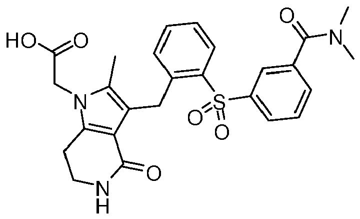

### {3-[2-({3-[(Dimethylamino)carbonyl]phenyl}sulfonyl)benzyl]-2-methyl-4-oxo-4,5,6, 7-tetrahydro-1H-pyrrolo[3,2-c]pyridin-1-yl}acetic acid

The title compound of Preparation 77 (70.6 mg, 0.21 mmol) was treated with anhydride acetic (0.40 ml, 4.22 mmol), hydriodic acid (57% solution, 0.40 ml, 3.0 mmol), hypophosphorous acid (50% solution, 0.11 ml, 1.05 mmol) and the title compound of Preparation 19 (100 mg, 0.315 mmol) according to the method of Example 5. The resulting residue was purified by reverse-phase chromatography (formic acid buffer) using the SP1 Purification system to give 21 mg (0.039 mmol, 19%) of the title compound. Purity 96%.
¹H NMR (400 MHz, DMSO-d6) δ ppm 8.14 (1 H, d, J=7.8 Hz), 7.95 (1 H, d, J=7.6 Hz), 7.83 (1 H, s), 7.71 - 7.76 (1 H, m), 7.68 (1 H, t, J=7.6 Hz), 7.53 (1 H, t, J=7.0 Hz), 7.46 (1 H, t, J=7.4 Hz), 6.94 (1 H, d, J=7.6 Hz), 6.70 (1 H, br. s.), 4.55 (2 H, s), 4.15 (2 H, s), 3.30 (2 H, t, J=6.6 Hz), 2.94 (3 H, s), 2.81 (3 H, s), 2.65 (2 H, t, J=6.7 Hz), 1.46 (3 H, s).
HPLC/MS (30 min) retention time 11.47 min.
LRMS: *m*/*z* 510 (M+1).

### EXAMPLE 19

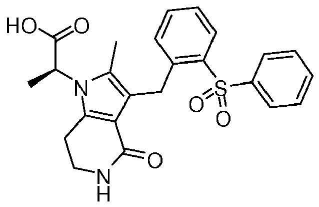

### (2S)-2-{2-Methyl-4-oxo-3-[2-(phenylsulfonyl)benzyl]-4,5,6,7-tetrahydro-1H-pyrrolo [3,2-c]pyridin-1-yl}propanoic acid

The title compound of Preparation 78 (85 mg, 0.253 mmol) was treated with anhydride acetic (0.45 ml, 4.75 mmol), hydriodic acid (57% solution, 0.45 ml, 3.4 mmol), hypophosphorous acid (50% solution, 0.125 ml, 1.2 mmol) and the title compound of Preparation 2 (93 mg, 0.379 mmol) according to Method B of Example 4. The resulting residue was purified by reverse-phase chromatography (formic acid buffer) using the SP1 Purification system to give 51.8 mg (0.108 mmol, 43%) of the title compound. Purity 95%.
¹H NMR (partial, 400 MHz, DMSO-d6) δ ppm 13.09 (1 H, br. s.), 8.13 (1 H, d, J=7.8 Hz), 7.88 (2 H, d, J=7.4 Hz), 7.70 (1 H, t, J=7.2 Hz), 7.62 (2 H, t, J=7.6 Hz), 7.52 (1 H, t, J=7.4 Hz), 7.44 (1 H, t, J=7.4 Hz), 6.92 (1 H, d, J=7.6 Hz), 6.80 (1 H, br. s.), 4.96 (1 H, q, J=7.0 Hz), 4.15 (2 H, s), 2.72 (1 H, dt, J=14.2, 6.9 Hz), 2.62 (1 H, dt, J=14.2, 6.7 Hz), 1.46 (3 H, s), 1.43 (3 H, d, J=7.2 Hz).
HPLC/MS (30 min) retention time 13.27 min.
LRMS: *m*/*z* 453 (M+1).

### EXAMPLE 20

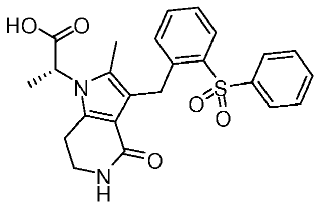

### (2R)-2-{2-Methyl-4-oxo-3-[2-(phenylsulfonyl)benzyl]-4,5,6,7-tetrahydro-1H-pyrrolo [3,2-c]pyridin-1-yl}propanoic acid

The title compound of Preparation 79 (70 mg, 0.208 mmol) was treated with anhydride acetic (0.40 ml, 4.22 mmol), hydriodic acid (57% solution, 0.40 ml, 3.0 mmol), hypophosphorous acid (50% solution, 0.11 ml, 1.05 mmol) and the title compound of Preparation 2 (77 mg, 0.31 mmol) according to Method B of Example 4. The resulting residue was purified by reverse-phase chromatography (formic acid buffer) using the SP1 Purification system to give 43 mg (0.086 mmol, 34%) of the title compound. Purity 91%.
¹H NMR (partial, 400 MHz, DMSO-d6) δ ppm 8.13 (1 H, d, J=6.8 Hz), 7.88 (2 H, d, J=7.2 Hz), 7.70 (1 H, t, J=7.4 Hz), 7.62 (2 H, t, J=7.5 Hz), 7.52 (1 H, t, J=6.9 Hz), 7.44 (1 H, t, J=7.4 Hz), 6.92 (1 H, d, J=7.4 Hz), 6.79 (1 H, br. s.), 4.96 (1 H, q, J=7.2 Hz), 4.15 (2 H, s), 2.72 (1 H, dt, J=14.3, 7.0 Hz), 2.62 (1 H, dt, J=14.3, 6.8 Hz), 1.46 (3 H, s), 1.43 (3 H, d, J=7.2 Hz).
HPLC/MS (30 min) retention time 13.27 min.
LRMS: *m*/*z* 453 (M+1).

### EXAMPLE 21

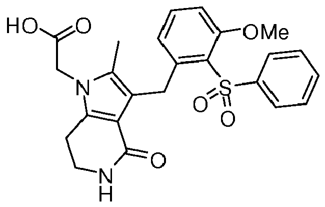

### {3-[3-Methoxy-2-(phenylsulfonyl)benzyl]-2-methyl-4-oxo-4,5,6,7-tetrahydro-1H-py rrolo[3,2-c]pyridin-1-yl}acetic acid

The title compound of Preparation 77 (60 mg, 0.178 mmol) was treated with anhydride acetic (0.35 ml, 3.7 mmol), hydriodic acid (57% solution, 0.35 ml, 2.65 mmol), hypophosphorous acid (50% solution, 0.09 ml, 0.9 mmol) and the title compound of Preparation 34 (74 mg, 0.267 mmol) according to the method of Example 13. The resulting residue was purified by purification by reverse-phase chromatography (formic acid buffer) using the SP1 Purification system to give 35 mg (0.074 mmol, 42%) of the title compound. Purity 100%.
¹H NMR (partial, 400 MHz, DMSO-d6) δ ppm 7.96 (2 H, d, J=7.4 Hz), 7.64 (1 H, t, J=7.3 Hz), 7.56 (2 H, t, J=7.8 Hz), 7.35 (1 H, t, J=8.1 Hz), 6.88 (1 H, d, J=8.2 Hz), 6.74 (1 H, br. s.), 6.56 (1 H, d, J=7.8 Hz), 4.67 (4 H, s), 3.50 (3 H, s), 2.70 (2 H, t, J=6.8 Hz), 1.94 (3 H, s).
HPLC/MS (30 min) retention time 12.12 min.
LRMS: *m*/*z* 469 (M+1).

### EXAMPLE 22

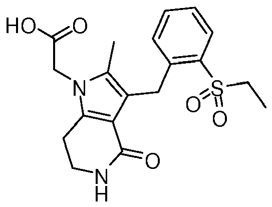

### {3-[2-(Ethylsulfonyl)benzyl]-2-methyl-4-oxo-4,5,6,7-tetrahydro-1H-pyrrolo[3,2-c]p yridin-1-yl}acetic acid

The title compound of Preparation 77 (60 mg, 0.178 mmol) was treated with anhydride acetic (0.35 ml, 3.7 mmol), hydriodic acid (57% solution, 0.35 ml, 2.65 mmol), hypophosphorous acid (50% solution, 0.09 ml, 0.9 mmol) and the title compound of Preparation 40 (53.1 mg, 0.267 mmol) according to the method of Example 13. The resulting residue was purified by reverse-phase chromatography (formic acid buffer) using the SP1 Purification system to give 14 mg (0.036 mmol, 20%) of the title compound. Purity 99%.
¹H NMR (partial, 400 MHz, DMSO-d6) δ ppm 12.96 - 13.30 (1 H, br. s), 7.84 (1 H, d, J=7.8 Hz), 7.51 (1 H, t, J=7.5 Hz), 7.38 (1 H, t, J=7.5 Hz), 6.98 (1 H, d, J=7.8 Hz), 6.76 (1 H, br. s.), 4.70 (2 H, s), 4.31 (2 H, s), 3.54 (2 H, q, J=7.3 Hz), 2.68 (2 H, t, J=6.8 Hz), 1.98 (3 H, s), 1.12 (3 H, t, J=7.2 Hz).
HPLC/MS (30 min) retention time 10.75 min.
LRMS: *m*/*z* 391 (M+1).

### EXAMPLE 23

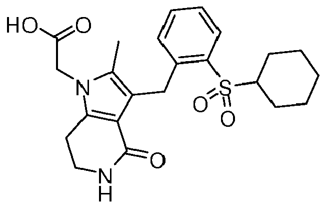

### {3-[2-(Cyclohexylsulfonyl)benzyl]-2-methyl-4-oxo-4,5,6,7-tetrahydro-1H-pyrrolo[3, 2-c]pyridin-1-yl}acetic acid

The title compound of Preparation 77 (60 mg, 0.178 mmol) was treated with anhydride acetic (0.35 ml, 3.7 mmol), hydriodic acid (57% solution, 0.35 ml, 2.65 mmol), hypophosphorous acid (50% solution, 0.09 ml, 0.9 mmol) and the title compound of Preparation 42 (65.6 mg, 0.267 mmol) according to the method of Example 13. The resulting residue was purified by reverse-phase chromatography (formic acid buffer) using the SP1 Purification system to give 48 mg (0.108 mmol, 61%) of the title compound. Purity 99%.
¹H NMR (partial, 400 MHz, DMSO-d6) δ ppm 7.79 (1 H, d, J=7.8 Hz), 7.50 (1 H, t, J=7.4 Hz), 7.37 (1 H, t, J=7.4 Hz), 6.98 (1 H, d, J=7.8 Hz), 6.76 (1 H, br. s.), 4.69 (2 H, s), 4.30 (2 H, s), 3.72 (1 H, t, J=11.3 Hz), 2.68 (2 H, t, J=6.8 Hz), 1.97 (3 H, s), 1.83 (2 H, d, J=11.5 Hz), 1.74 (2 H, d, J=12.3 Hz), 1.59 (1 H, d, J=12.7 Hz), 1.21 - 1.47 (4 H, m), 1.06 - 1.20 (1 H, m).
HPLC/MS (30 min) retention time 13.73 min.
LRMS: *m*/*z* 445 (M+1).

### EXAMPLE 24

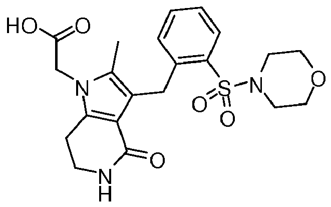

### {2-Methyl-3-[2-(morpholin-4-ylsulfonyl)benzyl]-4-oxo-4,5,6,7-tetrahydro-1H-pyrrol o[3,2-c]pyridin-1-yl}acetic acid

The title compound of Preparation 77 (60 mg, 0.178 mmol) was treated with anhydride acetic (0.35 ml, 3.7 mmol), hydriodic acid (57% solution, 0.35 ml, 2.65 mmol), hypophosphorous acid (50% solution, 0.09 ml, 0.9 mmol) and 2-(morpholinosulfonyl)benzaldehyde (68.4 mg, 0.267 mmol) according to the method of Example 13. The resulting residue was purified by reverse-phase chromatography (formic acid buffer) using the SP1 Purification system to give 18 mg (0.034 mmol, 19%) of the title compound. Purity 95%.
¹H NMR (400 MHz, DMSO-d6) δ ppm 7.82 (1 H, d, J=7.8 Hz), 7.48 (1 H, t, J=7.5 Hz), 7.35 (1 H, t, J=7.5 Hz), 6.98 (1 H, d, J=7.6 Hz), 6.77 (1 H, s), 4.65 (2 H, s), 4.39 (2 H, s), 3.64 (4 H, t, J=4.5 Hz), 3.09 (4 H, t, J=4.5 Hz), 2.69 (2 H, t, J=6.8 Hz), 1.88 (3 H, s).
HPLC/MS (30 min) retention time 11.32 min.
LRMS: *m*/*z* 448 (M+1).

### EXAMPLE 25

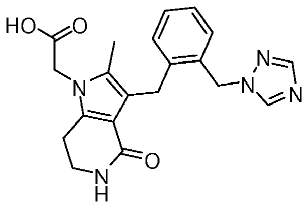

### {2-Methyl-4-oxo-3-[2-(1H-1,2,4-triazol-1-ylmethyl)benzyl]-4,5,6,7-tetrahydro-1H-py rrolo[3,2-c]pyridin-1-yl}acetic acid

The title compound of Preparation 77 (50 mg, 0.148 mmol) was treated with anhydride acetic (0.3 ml, 3.2 mmol), hydroiodic Acid (57% solution, 0.3 ml, 2.3 mmol), hypophosphorous acid (50% solution, 73 µl, 0.7 mmol) and 2-(1H-1,2,4-triazol-1-ylmethyl)benzaldehyde (42 mg, 0.223 mmol) according to the method of Example 13. The resulting residue was purified by reverse-phase chromatography (formic acid buffer) using the SP1 Purification system to give 30 mg (0.064 mmol, 43%) of the title compound. Purity 100%.
¹H NMR (partial, 400 MHz, DMSO-d6) δ ppm 8.57 (1 H, s), 7.98 (1 H, s), 7.13 (1 H, td, J=7.3, 1.3 Hz), 7.08 (1 H, t, J=7.2 Hz), 6.92 (1 H, d, J=7.0 Hz), 6.90 (1 H, d, J=7.0 Hz), 6.76 (1 H, br. s.), 5.57 (2 H, s), 4.65 (2 H, s), 4.06 (2 H, s), 2.67 (2 H, t, J=6.8 Hz), 1.90 (3 H, s).
HPLC/MS (30 min) retention time 10.03 min.
LRMS: *m*/*z* 380 (M+1).

### EXAMPLE 26

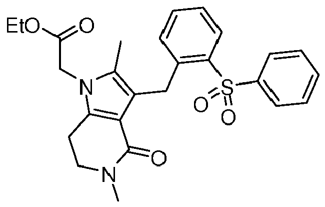

### Ethyl 2-(2,5-dimethyl-4-oxo-3-(2-(phenylsulfonyl)benzyl)-4,5,6,7-tetrahydro-1H-pyrrolo[ 3,2-c]pyridin-1-yl)acetate

The title compound of Example 1 (25 mg, 0.053 mmol) was dissolved in 1.5 ml tetrahydrofuran and the mixture was cooled to -78°C. Lithium bis(thmethylsilyl)amide solution (1M in THF, 60 µl, 0.06 mmol) was then added and the mixture was stirred 30 min. Methyl iodide (10 µl, 0.16 mmol) was added and the mixture was stirred for 3.5 h, warming to room temperature. Bis(trimethylsilyl)amide solutions (1M in THF, 60 µl, 0.06 mmol) and methyl iodide (10 µl, 0.16 mmol) were added again and the mixture was agitated at room temperature overnight. The mixture was partitioned between water and dichloromethane. The mixture was evaporated and the residue partitioned between dichloromethane and water The aqueous layer was extracted twice with dichloromethane. The combined organics were dried over magnesium sulphate, filtered and evaporated under reduced pressure to give 6 mg of the title compound. Purity 95%
¹H NMR (400 MHz, DMSO-d6) δ ppm 1.16 (t, J=7.03 Hz, 3 H), 1.43 (s, 3 H), 2.73 - 2.77 (m, 2 H), 2.78 (s, 3 H), 3.45 (t, J=7.03 Hz, 2 H), 4.11 (q, J=7.10 Hz, 2 H), 4.16 (s, 2 H), 4.69 (s, 2 H), 6.89 (d, J=6.84 Hz, 1 H), 7.41 - 7.52 (m, 2 H), 7.60 - 7.66 (m, 2 H), 7.68 - 7.73 (m, 1 H), 7.86 - 7.91 (m, 2 H), 8.12 (dd, J=7.82, 1.37 Hz, 1 H).
HPLC/MS (5 min) retention time 3.87 min.
LRMS: *m*/*z* 481 (M+1).

### EXAMPLE 27

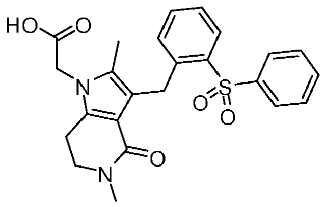

### {2,5-Dimethyl-4-oxo-3-[2-(phenylsulfonyl)benzyl]-4,5,6,7-tetrahydro-1H-pyrrolo[3, 2-c]pyridin-1-yl}acetic acid

### Method A

The crude title compound of Example 26 (5.3 mg) was treated with lithium hydroxide monohydrate (5 mg, 0.12 mmol) according to the method of Example 29 to give 3.5 mg (0.007 mmol, 39% over two steps) of the title compound. Purity 100%.

### Method B

The title compound of Preparation 82 (50 mg, 0.20 mmol) was treated with anhydride acetic (0.3 ml, 3.2 mmol), hydriodic Acid (57% solution, 0.3 ml, 2.3 mmol), hypophosphorous acid (50% solution, 73 µl, 0.7 mmol) and the title compound of Preparation 2 (59 mg, 0.24 mmol) according to the method of Example 16. The resulting residue was purified by reverse-phase chromatography (formic acid buffer) using the SP1 Purification system to give the title compound. Purity 100%.
¹H NMR (400 MHz, DMSO-d6) δ ppm 8.12 (1 H, dd, J=7.7, 1.1 Hz), 7.88 (2 H, d, J=7.4 Hz), 7.71 (1 H, t, J=7.2 Hz), 7.63 (2 H, t, J=7.5 Hz), 7.50 (1 H, td, J=7.5, 1.1 Hz), 7.44 (1 H, t, J=7.2 Hz), 6.91 (1 H, d, J=7.6 Hz), 4.56 (2 H, s), 4.15 (2 H, s), 3.45 (2 H, t, J=7.0 Hz), 2.78 (3 H, s), 2.75 (2 H, t, J=7.0 Hz), 1.43 (3 H, s).
HPLC/MS (30 min) retention time 13.05 min.
LRMS*: m*/*z* 453 (M+1).

### EXAMPLE 28

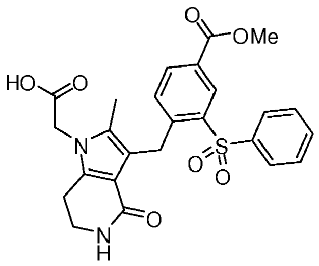

### {3-[4-(Methoxycarbonyl)-2-(phenylsulfonyl)benzyl]-2-methyl-4-oxo-4,5,6,7-tetrahy dro-1H-pyrrolo[3,2-c]pyridin-1-yl}acetic acid

The title compound of Preparation 77 (50 mg, 0.15 mmol) was treated with anhydride acetic (0.3 ml, 3.2 mmol), hydriodic Acid (57% solution, 0.3 ml, 2.3 mmol), hypophosphorous acid (50% solution, 73 µl, 0.7 mmol) and the title compound of Preparation 32 (68 mg, 0.223 mmol) according to the method B of Example 4. The resulting residue was purified by purification by reverse-phase chromatography (formic acid buffer) using the SP1 Purification system to give 28 mg (0.056 mmol, 38%) of the title compound. Purity 97%
¹H NMR (partial, 400 MHz, DMSO-d6) d ppm 8.68 (1 H, d, J=1.6 Hz), 8.07 (1 H, dd, J=8.1, 1.7 Hz), 7.92 (2 H, d, J=7.6 Hz), 7.72 (1 H, d, J=7.2 Hz), 7.63 (2 H, t, J=7.6 Hz), 7.09 (1 H, d, J=8.2 Hz), 6.80 (1 H, br. s.), 4.58 (2 H, s), 4.19 (2 H, s), 3.88 (3 H, s), 2.66 (2 H, t, J=6.7 Hz), 1.41 (3 H, s).
HPLC/MS (30 min) retention time 12.93 min.
LRMS: *m*/*z* 497 (M+1).

### EXAMPLE 29

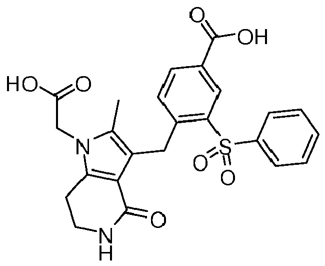

### 4-{[1-(Carboxymethyl)-2-methyl-4-oxo-4,5,6,7-tetrahydro-1H-pyrrolo[3,2-c]pyridin -3-yl]methyl}-3-(phenylsulfonyl)benzoic acid

The title compound of Example 28 (27 mg, 0.054 mmol) was dissolved in 1 ml of tetrahydrofuran and 0.5 ml of water. Lithium hydroxide monohydrate (2 mg, 0.083 mmol) was added and the mixture was agitated at room temperature for 1 h. The mixture was partitioned between water and ethyl acetate. The aqueous layer was acidified to pH 3 with 2N hydrochloric acid. The aqueous layer was extracted twice with ethyl acetate. The combined organics were washed with brine, dried over magnesium sulphate and evaporated to give 2 mg (0.003 mmol, 8%) of the crude title compound. Purity 84%.
¹H NMR (partial, 400 MHz, DMSO-d6) δ ppm 13.27 (1 H, br. s.), 8.66 (1 H, d, J=1.6 Hz), 8.03 (1 H, dd, J=8.0, 1.6 Hz), 7.92 (2 H, d, J=7.4 Hz), 7.68 - 7.77 (1 H, m), 7.63 (2 H, t, J=7.6 Hz), 7.06 (1 H, d, J=8.0 Hz), 6.79 (1 H, br. s.), 4.59 (2 H, s), 4.19 (2 H, s), 2.66 (2 H, t, J=6.7 Hz), 1.43 (3 H, s).
HPLC/MS (30 min) retention time 11.53 min.
LRMS: *m*/*z* 483 (M+1).

### EXAMPLE 30

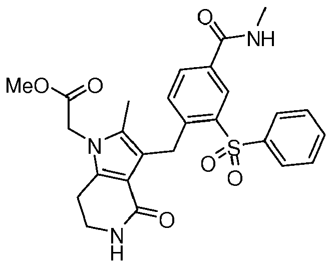

### Methyl 2-(2-methyl-3-(4-(methylcarbamoyl)-2-(phenylsulfonyl)benzyl)-4-oxo-4,5,6,7-tetra hydro-1H-pyrrolo[3,2-c]pyridin-1-yl)acetate

The title compound of Example 28 (31 mg, 0.063 mmol) was suspended in 1 ml acetonitrile. Methylamine (1M in tetrahydrofuran, 2 ml, 2 mmol) and added and the mixture was stirred at 120ºC in a sealed tube for 4 d. The mixture was evaporated under reduced pressure and re-suspended in 1ml methanol. 5 Drops of sulphuric acid were added and the mixture was stirred at room temperature for 5 h. The mixture was evaporated and partitioned between dichloromethane and saturated sodium bicarbonate solution. The aqueous layer was extracted with dichloromethane and the combined organics were dried over magnesium sulphate and evaporated. The resulting residue was purified by flash chromatography (dichloromethane-methanol gradient, 0:100 rising to 2:98) to give 4 mg (0.0079 mmol, 12%) of the title compound. Purity 95%.
HPLC/MS (5 min) retention time 2.22 min.
LRMS: *m*/*z* 510 (M+1).

### EXAMPLE 31

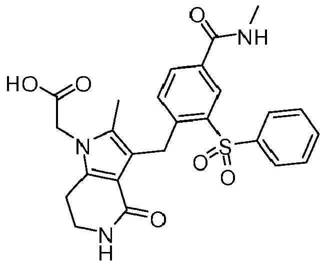

### {2-Methyl-3-[4-[(methylamino)carbonyl]-2-(phenylsulfonyl)benzyl]-4-oxo-4,5,6,7-t etrahydro-1H-pyrrolo[3,2-c]pyridin-1-yl}acetic acid

The title compound of Example 30 (4 mg, 0.0079 mmol) was treated with lithium hydroxide monohydrate (2 mg, 0.17 mmol) according to the method of Example 29 to give 2.5 mg (0.004 mmol, 63%) of the title compound. Purity 95%.
¹H NMR (400 MHz, DMSO-d6) δ ppm 8.65 (1 H, dd, J=8.2, 4.3 Hz), 8.58 (1 H, d, J=1.8 Hz), 7.93 (2 H, d, J=7.4 Hz), 7.90 (1 H, dd, J=8.2, 1.5 Hz), 7.71 (1 H, t, J=7.4 Hz), 7.63 (2 H, t, J=7.6 Hz), 6.99 (1 H, d, J=8.2 Hz), 6.79 (1 H, br. s.), 4.59 (2 H, s), 4.17 (2 H, s), 3.31 (2 H, t, J=6.8 Hz), 2.79 (3 H, d, J=4.3 Hz), 2.66 (2 H, t, J=6.8 Hz), 1.41 (3 H, s).
HPLC/MS (30 min) retention time 10.97 min.
LRMS: *m*/*z* 496 (M+1).

### EXAMPLE 32

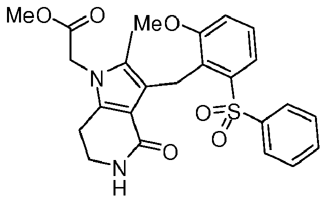

### Methyl {3-[2-methoxy-6-(phenylsulfonyl)benzyl]-2-methyl-4-oxo-4,5,6,7-tetrahydro-1H-py rrolo[3,2-c]pyridin-1-yl}acetate

The title compound of Preparation 77 (60 mg, 0.178 mmol) was suspended in anhydride acetic (0.35 ml, 3.7 mmol), hydriodic acid (57% solution, 0.35 ml, 2.65 mmol), hypophosphorous acid (50% solution, 0.09 ml 0.9 mmol). The title compound of Preparation 36 (74 mg, 0.267 mmol) was then added and the mixture was stirred at room temperature for 42 h. The mixture was partitioned between water and dichloromethane. The aqueous layer was extracted with dichloromethane, two times. The combined organics were dried over magnesium sulphate, filtered and evaporated under reduced pressure. The resulting residue was dissolved in dichloromethane and polystyrene-supported tosyl hydrazine resin (70 mg, loading 3.7 mmol/g, 0.26 mmol) was added and the mixture was agitated for 4 h. The mixture was filtered and the filtrate was evaporated under reduced pressure. The resulting residue was purified twice by reverse-phase chromatography (formic acid buffer) using the SP1 Purification system to isolate 50 mg of the crude acid of Example 33. The solid was dissolved in 1 ml methanol and was treated with five drops of sulphuric acid. The mixture was stirred then at room temperature for 30 min. The mixture was evaporated under reduced pressure and partitioned between dichloromethane and saturated sodium hydrogen carbonate solution. The aqueous layer was extracted with dichloromethane and the combined organics were dried over magnesium sulphate, filtered and evaporated. The resulting residue was purified using the SP1 Purification System (methanol-ethyl acetate gradient, 0:100 rising to 12:88) to give 30 mg of the title compound.
¹H NMR (400 MHz, CHLOROFORM-d) δ ppm 1.46 (s, 3 H), 2.70 (t, J=6.94 Hz, 2 H), 3.51 - 3.58 (m, 2 H), 3.64 (s, 3 H), 3.73 (s, 3 H), 4.34 (s, 2 H), 4.54 (s, 2 H), 5.13 (s, 1 H), 7.03 - 7.07 (m, 1 H), 7.36 - 7.43 (m, 3 H), 7.45 - 7.50 (m, 1 H), 7.80 - 7.83 (m, 2 H), 7.92 (dd, J=8.01, 0.98 Hz, 1 H).

### EXAMPLE 33

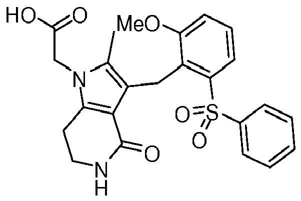

### {3-[2-Methoxy-6-(phenylsulfonyl)benzyl]-2-methyl-4-oxo-4,5,6,7-tetrahydro-1H-py rrolo[3,2-c]pyridin-1-yl}acetic acid

The title compound of Example 32 (30 mg) was treated with lithium hydroxide monohydrate (15 mg, 0.36 mmol) according to the method of Example 29 to give 27 mg (0.058 mmol, 32% over two steps) of the title compound. Purity 98%.
¹H NMR (partial, 400 MHz, DMSO-d6) δ ppm 12.91 (1 H, br. s.), 7.78 (1 H, d, J=8.0 Hz), 7.62 (2 H, d, J=7.4 Hz), 7.55 (1 H, t, J=7.3 Hz), 7.52 (1 H, t, J=8.0 Hz), 7.42 (2 H, t, J=7.7 Hz), 7.30 (1 H, d, J=8.0 Hz), 6.71 (1 H, br. s.), 4.40 (2 H, br. s.), 4.27 (2 H, s), 3.64 (3 H, s), 2.59 (2 H, t, J=6.6 Hz), 0.88 (3 H, s).
HPLC/MS (30 min) retention time 11.90 min.
LRMS: *m*/*z* 469 (M+1).

### EXAMPLE 34

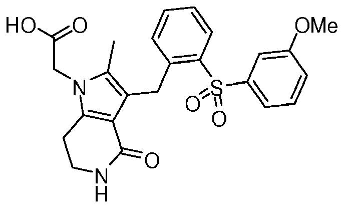

### (3-{2-[(3-Methoxyphenyl)sulfonyl]benzyl}-2-methyl-4-oxo-4,5,6,7-tetrahydro-1H-p yrrolo[3,2-c]pyridin-1-yl)acetic acid

The title compound of Preparation 77 (81 mg, 0.24 mmol) was treated with anhydride acetic (0.40 ml, 4.22 mmol), hydriodic acid (57% solution, 0.40 ml, 3.0 mmol), hypophosphorous acid (50% solution, 0.11 ml, 1.05 mmol) and the title compound of Preparation 17 (100 mg, 0.362 mmol) according to the method of Example 16. The resulting residue was partially purified by reverse-phase chromatography (formic acid buffer) using the SP1 Purification system to give 26 mg of the crude title compound. This was re-purified by preparative HPLC (aqueous mobile phase: 0.025% ammonium formate in water, organic mobile phase 0.02% ammonium formate in 50:50 methanol - acetonitrile, gradient 50:50 rising to 61:39 over 9 min) to give 10 mg (0.021 mmol, 9%) of the title compound. Purity 95%.
¹H NMR (partial, 400 MHz, DMSO-d6) δ ppm 8.12 (1 H, d, J=7.8 Hz), 7.50 - 7.61 (2 H, m), 7.45 (2 H, t, J=7.6 Hz), 7.34 (1 H, s), 7.29 (1 H, d, J=7.8 Hz), 6.98 (1 H, d, J=7.4 Hz), 6.78 (1 H, br. s.), 4.49 (2 H, br. s.), 4.20 (2 H, s), 3.81 (3 H, s), 2.66 (2 H, t, J=6.8 Hz).
HPLC/MS (30 min) retention time 13.12 min.
LRMS: *m*/*z* 469 (M+1).

### EXAMPLE 35

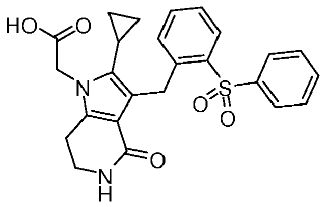

### {2-Cyclopropyl-4-oxo-3-[2-(phenylsulfonyl)benzyl]-4,5,6,7-tetrahydro-1H-pyrrolo[ 3,2-c]pyridin-1-yl}acetic acid

The title compound of Preparation 80 (60 mg, 0.165 mmol) was treated with anhydride acetic (0.35 ml, 3.7 mmol), hydriodic acid (57% solution, 0.35 ml, 2.65 mmol), hypophosphorous acid (50% solution, 0.09 ml, 0.9 mmol) and the title compound of Preparation 2 (57 mg, 0.23 mmol) according to the method of Example 13. The resulting residue was purified by reverse-phase chromatography (formic acid buffer) using the SP1 Purification system to give 21 mg (0.044 mmol, 27%) of the title compound. Purity 98%.
¹H NMR (partial, 400 MHz, DMSO-d6) δ ppm 8.36 (1 H, dd, J=7.6, 1.4 Hz), 8.12 (2 H, d, J=7.4 Hz), 7.92 (1 H, t, J=7.2 Hz), 7.84 (2 H, t, J=7.8 Hz), 7.70 (1 H, td, J=7.2, 1.5 Hz), 7.66 (1 H, t, J=7.3 Hz), 7.02 (1 H, d, J=7.2 Hz), 6.92 (1 H, br. s.), 4.91 (2 H, s), 4.46 (2 H, s), 2.86 (2 H, t, J=6.8 Hz), 1.30 - 1.41 (1 H, m), 0.33 - 0.43 (2 H, m), -0.03 - 0.05 (2 H, m).
HPLC/MS (30 min) retention time 13.52 min.
LRMS: *m*/*z* 465 (M+1).

### EXAMPLE 36

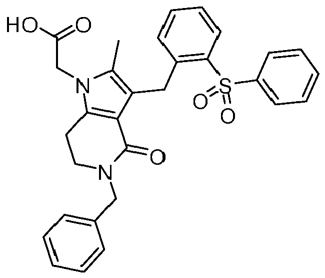

### {5-Benzyl-2-methyl-4-oxo-3-[2-(phenylsulfonyl)benzyl]-4,5,6,7-tetrahydro-1H-pyrr olo[3,2-c]pyridin-1-yl}acetic acid

The title compound of Preparation 85 (75 mg, 0.23 mmol) was treated with anhydride acetic (0.50 ml, 5.3 mmol), hydriodic acid (57% solution, 0.50 ml, 3.8 mmol), hypophosphorous acid (50% solution, 0.11 ml, 1.05 mmol) and the title compound of Preparation 2 (74 mg, 0.299 mmol) according to the method of Example 16. The resulting residue was purified by reverse-phase chromatography (formic acid buffer) using the SP1 Purification system to give 65 mg (0.118 mmol, 51%) of the title compound. Purity 96%.
¹H NMR (400 MHz, DMSO-d6) δ ppm 8.14 (1 H, d, J=7.8 Hz), 7.89 (2 H, d, J=7.2 Hz), 7.70 (1 H, t, J=7.4 Hz), 7.63 (2 H, t, J=7.5 Hz), 7.53 (1 H, td, J=7.5, 1.1 Hz), 7.45 (1 H, t, J=7.3 Hz), 7.29 (2 H, d, J=7.4 Hz), 7.16 - 7.26 (3 H, m), 6.97 (1 H, d, J=7.6 Hz), 4.57 (2 H, s), 4.49 (2 H, s), 4.19 (2 H, s), 3.39 (2 H, t, J=6.9 Hz), 2.73 (2 H, t, J=6.9 Hz), 1.45 (3 H, s).
HPLC/MS (30 min) retention time 16.07 min.
LRMS: *m*/*z* 529 (M+1).

### EXAMPLE 37

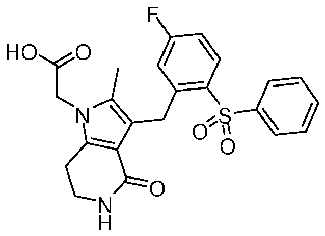

### {3-[5-Fluoro-2-(phenylsulfonyl)benzyl]-2-methyl-4-oxo-4,5,6,7-tetrahydro-1H-pyrr olo[3,2-c]pyridin-1-yl}acetic acid

The title compound of Preparation 77 (60 mg, 0.18 mmol) was treated with anhydride acetic (0.35 ml, 3.7 mmol), hydriodic acid (57% solution, 0.35 ml, 2.65 mmol), hypophosphorous acid (50% solution, 0.09 ml, 0.9 mmol) and the title compound of Preparation 23 (74 mg, 0.299 mmol) according to the method B of Example 4. The resulting residue was purified by reverse-phase chromatography using the SP1 Purification system to give 36 mg (0.078 mmol, 43%) of the title compound. Purity 99%.
¹H NMR (400 MHz, DMSO-d6) δ ppm 1.48 (s, 3 H), 2.68 (t, J=6.64 Hz, 2 H), 4.14 (s, 2 H), 4.61 (s, 2 H), 6.63 (dd, J=10.36, 2.15 Hz, 1 H), 6.85 (br. s., 1 H), 7.33 (td, J=8.40, 2.34 Hz, 1 H), 7.60 - 7.78 (m, 3 H), 7.92 (d, J=7.42 Hz, 2 H), 8.24 (dd, J=8.79, 5.67 Hz, 1 H).
HPLC/MS (30 min) retention time 12.95 min.
LRMS: *m*/*z* 457 (M+1).

### EXAMPLE 38

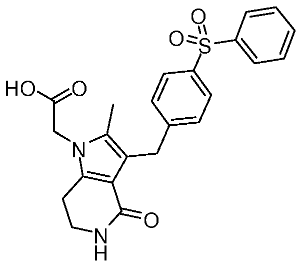

### {2-Methyl-4-oxo-3-[4-(phenylsulfonyl)benzyl]-4,5,6,7-tetrahydro-1H-pyrrolo[3,2-c] pyridin-1-yl}acetic acid

The title compound of Preparation 77 (60 mg, 0.18 mmol) was treated with anhydride acetic (0.35 ml, 3.7 mmol), hydriodic acid (57% solution, 0.35 ml, 2.65 mmol), hypophosphorous acid (50% solution, 0.09 ml, 0.9 mmol) and the title compound of Preparation 44 (57 mg, 0.23 mmol) according to the method of Example 13. The resulting residue was purified by reverse-phase chromatography using the SP1 Purification system to give 26 mg (0.058 mmol, 32%) of the title compound as a white solid. Purity 98%.
¹H NMR (400 MHz, DMSO-d6) δ ppm 2.01 (s, 3 H), 2.63 (t, J=6.84 Hz, 2 H), 3.29 (t, J=7.03 Hz, 2 H), 4.05 (s, 2 H), 4.61 (s, 2 H), 6.80 (br. s., 1 H), 7.45 (d, J=8.21 Hz, 2 H), 7.54 - 7.69 (m, 3 H), 7.80 (d, J=8.60 Hz, 2 H), 7.92 (d, J=7.42 Hz, 2 H).
HPLC/MS (30 min) retention time 12.62 min.
LRMS: m/z 439 (M+1).

### EXAMPLE 39

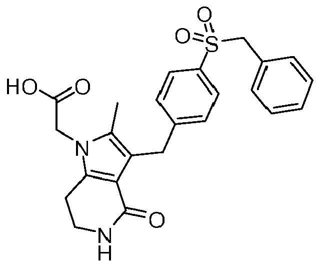

### {3-[4-(Benzylsulfonyl)benzyl]-2-methyl-4-oxo-4,5,6,7-tetrahydro-1H-pyrrolo[3,2-c] pyridin-1-yl}acetic acid

The title compound of Preparation 77 (60 mg, 0.18 mmol) was treated with anhydride acetic (0.35 ml, 3.7 mmol), hydriodic acid (57% solution, 0.35 ml, 2.65 mmol), hypophosphorous acid (50% solution, 0.09 ml, 0.9 mmol) and the title compound of Preparation 46 (607 mg, 0.23 mmol) according to the method of Example 13. The resulting residue was purified by reverse-phase chromatography using the SP1 Purification system to give 37 mg (0.080 mmol, 45%) of the title compound as a white solid. Purity 99%.
¹H NMR (400 MHz, DMSO-d6) δ ppm 1.94 (s, 3 H), 2.59 (t, J=6.06 Hz, 2 H), 4.02 (br. s., 2 H), 4.51 (s, 2 H), 4.57 (br. s., 2 H), 6.78 (br. s., 1 H), 7.06 (d, J=6.64 Hz, 2 H), 7.22 (d, J=7.03 Hz, 3 H), 7.35 (d, J=7.82 Hz, 2 H), 7.49 (d, J=7.82 Hz, 2 H).
HPLC/MS (30 min) retention time 12.86 min.
LRMS: *m*/*z* 453 (M+1).

### EXAMPLE 40

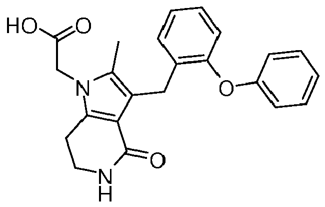

### [2-Methyl-4-oxo-3-(2-phenoxybenzyl)-4,5,6,7-tetrahydro-1H-pyrrolo[3,2-c]pyridin-1-yl]acetic acid

The title compound of Preparation 86 (50 mg, 0.11 mmol) was dissolved in 2 ml dioxane and 2 ml dimethylformamide. Caesium carbonate (4.6 mg, 0.033 mmol Copper iodide (2 mg, 0.011 mol) and N,N-dimethylglycine hydrochloride (115 mg, 0.353 mmol) were added and the mixture was degassed with argon. The mixture was stirred at 110ºC for 24 h, at room temperature for 72 h and again at 110ºC for 7 h. The mixture was allowed to cool and was poured into a solution of 0.1N hydrochloric acid with agitation. The aqueous layer was extracted three times with dichloromethane. The combined organics were dried over magnesium sulphate, filtered and evaporated under reduced pressure. The resulting residue was purified by reverse-phase chromatography (formic acid buffer) using the SP1 Purification system to give 3.9 mg (0.008 mmol, 8%) of the title compound. Purity 89%.
¹H NMR (partial, 400 MHz, DMSO-d6) δ ppm 7.34 (2 H, t, J=7.9 Hz), 7.18 (1 H, d, J=7.4 Hz), 7.11 (1 H, t, J=7.7 Hz), 7.07 (1 H, t, J=8.0 Hz), 6.98 (1 H, t, J=7.4 Hz), 6.92 (2 H, d, J=7.8 Hz), 6.80 (1 H, d, J=8.0 Hz), 6.72 (1 H, br. s.), 4.53 (2 H, br. s.), 3.95 (2 H, s), 2.63 (2 H, t, J=6.5 Hz), 1.89 (3 H, s).
HPLC/MS (30 min) retention time 15.30 min.
LRMS: *m*/*z* 391 (M+1).

### EXAMPLE 41

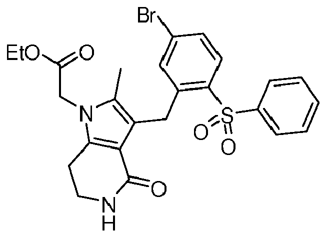

### Ethyl {3-[5-bromo-2-(phenylsulfonyl)benzyl]-2-methyl-4-oxo-4,5,6,7-tetrahydro-1H-pyrr olo[3,2-c]pyridin-1-yl}acetate

The title compound of Preparation 77 (250 mg, 0.74 mmol) was treated with anhydride acetic (2.1 ml, 22.2 mmol), hydriodic Acid (57% solution, 2.1 ml, 15.9 mmol) and hypophosphorous acid (50% solution, 0.4 ml, 3.84 mmol). The title compound of Preparation 27 (315 mg, 0.97 mmol) was added and the mixture was stirred at room temperature for 1 h. The mixture was partitioned between water and dichloromethane. The aqueous layer was extracted twice with dichloromethane. The combined organics were dried over magnesium sulphate, filtered and evaporated under reduced pressure. The resulting residue was dissolved in dichloromethane and polystyrene-supported tosylhydrazine resin (270 mg, loading 3.7 mmol/g, 1.0 mmol) was added and the mixture was agitated for 4 h. The mixture was filtered and the filtrate was washed with saturated sodium bicarbonate solution until the washes above pH 7. The combined organics were dried over magnesium sulphate, filtered and evaporated under reduced pressure to give 340 mg (0.56 mmol, 75%) of the title compound. Purity 90%.
HPLC/MS (9 min) retention time 6.33 min.
LRMS: *m*/*z* 547 (M+1).

### EXAMPLE 42

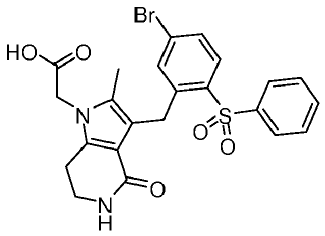

### {3-[5-Bromo-2-(phenylsulfonyl)benzyl]-2-methyl-4-oxo-4,5,6,7-tetrahydro-1H-pyrr olo[3,2-c]pyridin-1-yl}acetic acid

The title compound of Preparation 77 (50 mg, 0.15 mmol) was suspended in anhydride acetic (0.42 ml, 4.4 mmol), hydriodic acid (57% solution, 0.42 ml, 3.2 mmol), hypophosphorous acid (50% solution, 0.08 ml, 0.8 mmol). The title compound of Preparation 27 (63 mg, 0.194 mmol) was then added and the mixture was stirred at room temperature for 72 h. The mixture was partitioned between water and dichloromethane. The aqueous layer was extracted with dichloromethane, two times. The combined organics were dried over magnesium sulphate, filtered and evaporated under reduced pressure. The resulting residue was dissolved in dichloromethane and polystyrene-supported tosyl hydrazine resin (60 mg, loading 3.7 mmol/g, 0.22 mmol) was added and the mixture was agitated at room temperature for 4 h. The mixture was filtered and the filtrated evaporated under reduced pressure. The resulting residue was purified by reverse-phase chromatography without buffer using the SP1 Purification system to give 31 mg (0.059 mmol, 40%) of the title compound. Purity 99%.
¹H NMR (400 MHz, CHLOROFORM-d) δ ppm 1.77 (s, 3 H), 2.72 (t, J=6.84 Hz, 2 H), 3.49 (t, J=5.86 Hz, 2 H), 4.25 (s, 2 H), 4.44 (s, 2 H), 7.07 (s, 1 H), 7.44 - 7.51 (m, 3 H), 7.51 - 7.57 (m, 2 H), 7.87 (d, J=7.42 Hz, 2 H), 8.10 (d, J=8.60 Hz, 1 H).
HPLC/MS (30 min) retention time 14.09 min.
LRMS: *m*/*z* 517,519 (M+1).

### EXAMPLE 43

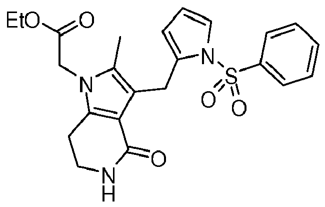

### Ethyl

### (2-methyl-4-oxo-3-{[1-(phenylsulfonyl)-1H-pyrrol-2-yl]methyl}-4,5,6,7-tetrahydro-1 H-pyrrolo[3,2-c]pyridin-1-yl)acetate

To a suspension of sodium hydride (3.8 mg, 0.0942 mmol) in 0.5 ml dimethylformamide was added the title compound of Preparation 87 (29 mg, 0.073 mmol) and the mixture was stirred at room temperature for 30 min. Ethyl bromoacetate (9.5 µl, 0.086 mmol) was then added and the mixture was stirred at room temperature for 4 h. The mixture was partitioned between ethyl acetate and water. The aqueous was extracted with ethyl acetate and the combined organics were washed with brine, dried over magnesium sulphate and filtered. Evaporation gave a residue which was purified by reverse-phase chromatography (formic acid buffer) using the SP1 Purification system to give 7.7 mg (0.017 mmol, 22%) of the title compound. Purity 95%.
¹H NMR (400 MHz, DMSO-d6) δ ppm 1.17 (t, J=7.13 Hz, 3 H), 1.63 (s, 3 H), 2.61 - 2.67 (m, 2 H), 3.26 - 3.31 (m, 2 H), 4.05 (s, 2 H), 4.12 (q, J=7.23 Hz, 2 H), 4.72 (s, 2 H), 5.37 - 5.41 (m, 1 H), 6.13 (t, J=3.32 Hz, 1 H), 6.75 - 6.80 (m, 1 H), 7.24 - 7.28 (m, 1 H), 7.59 - 7.66 (m, 2 H), 7.70 - 7.76 (m, 1 H), 7.88 - 7.92 (m, 2 H).
HPLC/MS (5 min) retention time 3.89 min.
LRMS: *m*/*z* 456 (M+1).

### EXAMPLE 44

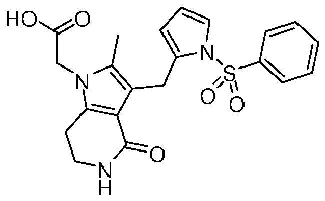

### (2-Methyl-4-oxo-3-{[1-(phenylsulfonyl)-1H-pyrrol-2-yl]methyl}-4,5,6,7-tetrahydro-1 H-pyrrolo[3,2-c]pyridin-1-yl)acetic acid

The title compound of Example 43 (7.7 mg, 0.017 mmol) was dissolved in 0.5 ml of tetrahydrofuran and 0.25 ml of water. Lithium hydroxide monohydrate (7 mg, 0.083 mmol) was added and the mixture was agitated at room temperature for 30 min. The mixture was partitioned between water and ethyl acetate. The aqueous layer was acidified to pH 3 with 1 H hydrochloric acid. The aqueous layer was extracted twice with ethyl acetate. The combined organics were washed with brine, dried over magnesium sulphate and evaporated to give 6 mg (0.013 mmol, 82%) of the title compound. Purity 99%.
¹H NMR (partial, 400 MHz, DMSO-d6) δ ppm 13.02 (1 H, br. s.), 7.90 (2 H, d, J=7.6 Hz), 7.73 (1 H, t, J=7.4 Hz), 7.62 (2 H, t, J=7.8 Hz), 7.26 (1 H, d, J=1.2 Hz), 6.75 (1 H, br. s.), 6.13 (1 H, t, J=3.3 Hz), 5.40 (1 H, br. s.), 4.60 (2 H, s), 4.05 (2 H, s), 2.64 (2 H, t, J=6.8 Hz), 1.63 (3 H, s).
HPLC/MS (30 min) retention time 13.32 min.
LRMS: *m*/*z* 428 (M+1).

### EXAMPLE 45

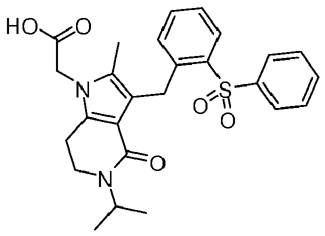

### {5-Isopropyl-2-methyl-4-oxo-3-[2-(phenylsulfonyl)benzyl]-4,5,6,7-tetrahydro-1H-p yrrolo[3,2-c]pyridin-1-yl}acetic acid

The title compound of Preparation 83 (80 mg, 0.287 mmol) was treated with anhydride acetic (0.40 ml, 4.2 mmol), hydriodic acid (57% solution, 0.40 ml, 3.0 mmol), hypophosphorous acid (50% solution, 0.11 ml, 1.05 mmol) and the title compound of Preparation 2 (85 mg, 0.345 mmol) according to the method of Example 16. The resulting residue was purified by reverse-phase chromatography (formic acid buffer) using the SP1 Purification system to give 85 mg (0.176 mmol, 62%) of the title compound. Purity 100%.
¹H NMR (400 MHz, DMSO-d6) δ ppm 13.04 (1 H, br. s.), 8.13 (1 H, dd, J=7.7, 1.1 Hz), 7.88 (2 H, d, J=7.4 Hz), 7.70 (1 H, t, J=7.2 Hz), 7.63 (2 H, t, J=7.5 Hz), 7.50 (1 H, td, J=7.5, 1.3 Hz), 7.44 (1 H, t, J=7.2 Hz), 6.92 (1 H, d, J=7.6 Hz), 4.63 (1 H, spt, J=6.8 Hz), 4.57 (2 H, s), 4.17 (2 H, s), 3.33 (2 H, t, J=6.8 Hz), 2.68 (2 H, t, J=6.7 Hz), 1.42 (3 H, s), 1.02 (6 H, d, J=6.8 Hz).
HPLC/MS (30 min) retention time 14.67 min.
LRMS: *m*/*z* 482 (M+1).

### EXAMPLE 46

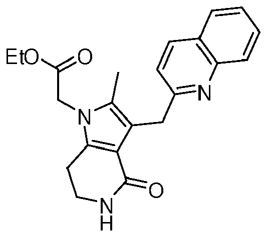

### Ethyl [2-methyl-4-oxo-3-(quinolin-2-ylmethyl)-4,5,6,7-tetrahydro-1H-pyrrolo[3,2-c]pyridi n-1-yl]acetate

The crude title compound of Preparation 88 (125 mg) was dissolved in 2 ml dimethylformamide. Sodium hydride (60% dispersion in oil, 16.5 mg, 0.41 mmol) was added and the mixture was stirred for 30 min at room temperature. Ethyl 2-bromoacetate (42 µl, 0.38 mmol) was then added and the mixture was agitated at room temperature for 1 h. Additonal sodium hydride (60% dispersion in oil, 16.5 mg, 0.41 mmol) was added and the mixture was stirred at room temperature for 30 min. A solution of ethyl 2-bromoacetate (42 µl, 0.38 mmol) in 4 ml dimethylformamide was added to the mixture and the solution was stirred at room temperature overnight. The mixture was evaporated under reduced pressure. Methanol was then added and the mixture was purified by reverse-phase chromatography (formic acid buffer) using the SP1 Purification system to give 16 mg of the crude title compound. Purity 80%.
¹H NMR (400 MHz, DMSO-*d*₆) δ ppm 8.13 (1 H, d, *J*=8.4 Hz), 7.91 (1 H, d, *J*=8.4 Hz), 7.83 (1 H, d, *J*=7.8 Hz), 7.67 (1 H, t, *J*=7.6 Hz), 7.49 (1 H, t, *J*=7.3 Hz), 7.40 (1 H, d, *J*=8.6 Hz), 6.87 (1 H, br. s.), 4.67 - 4.77 (2 H, m), 4.33 (2 H, s), 4.12 (2 H, q, *J*=7.2 Hz), 3.28 - 3.37 (2 H, m), 2.66 (2 H, t, *J*=6.8 Hz), 2.02 (2 H, s), 1.17 (3 H, t, *J*=7.0 Hz).
HPLC/MS (5 min) retention time 2.89 min.
LRMS: *m*/*z* 378(M+1).

### EXAMPLE 47

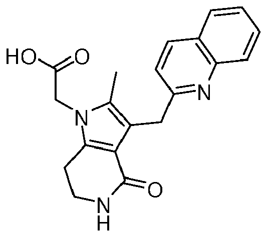

### [2-Methyl-4-oxo-3-(quinolin-2-ylmethyl)-4,5,6,7-tetrahydro-1H-pyrrolo[3,2-c]pyridi n-1-yl]acetic acid

The crude title compound of Example 46 (16 mg) was dissolved in 1 ml of tetrahydrofuran and 0.5 ml of water. Lithium hydroxide monohydrate (10 mg, 0.23 mmol) was added and the mixture was agitated at room temperature for 30 min. The mixture was partitioned between water and ethyl acetate. The aqueous layer was acidified to pH3 with 1 N hydrochloric acid. The aqueous layer was concentrated under reduced pressure and the resulting residue was purified by reverse-phase chromatography (formic acid buffer) using the SP1 Purification system to give 2.2 mg (0.005 mmol, 1% over 3 steps) of the crude title compound. Purity 81%.
¹H NMR (400 MHz, DMSO-d6) δ ppm 12.74 - 13.35 (1 H, m), 8.58 (1 H, br. s.), 7.98 - 8.18 (2 H, m), 7.90 (1 H, br. s.), 7.56 - 7.80 (2 H, m), 7.20 (1 H, br. s.), 4.66 (2 H, s), 4.41 (2 H, s), 3.34 (2 H, t, J=6.9 Hz), 2.68 (2 H, t, J=6.9 Hz), 2.11 (3 H, s).
HPLC/MS (30 min) retention time 6.43 min.
LRMS: *m*/*z* 350 (M+1).

### EXAMPLE 48

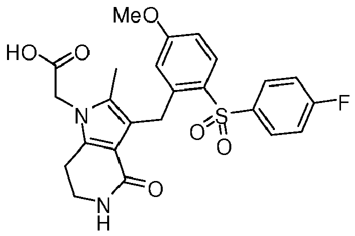

### (3-{2-[(4-Fluorophenyl)sulfonyl]-5-methoxybenzyl}-2-methyl-4-oxo-4,5,6,7-tetrahy dro-1H-pyrrolo[3,2-c]pyridin-1-yl)acetic acid

The title compound of Preparation 77 (100 mg, 0.30 mmol) was treated with anhydride acetic (0.58 ml, 6.1 mmol), hydriodic Acid (57% solution, 0.58 ml, 4.4 mmol), hypophosphorous acid (50% solution, 0.15 ml, 1.5 mmol) and the crude title compound of Preparation 38 (115 mg) according to Method B of Example 4. The resulting residue was purified by reverse-phase chromatography using the SP1 Purification system to give 125 mg (0.241 mmol, 80%) of the title compound. Purity 94%.
¹H NMR (400 MHz, DMSO-d6) δ ppm 13.37 - 12.63 (m, 1 H), 8.11 (d, J = 8.6 Hz, 1 H), 7.99 - 7.87 (m, 2H), 7.45 (t, J = 8.5 Hz, 2H), 7.00 (d, J = 8.6 Hz, 1 H), 6.81 (s, 1 H), 6.40 (s, 1 H), 4.64 (s, 2H), 4.10 (s, 2H), 3.69 (s, 3H), 2.67 (t, J = 6.5 Hz, 2H), 1.55 (s, 3H).
HPLC/MS (30 min) retention time 13.17 min.
LRMS: *m*/*z* 487 (M+1).

### EXAMPLE 49

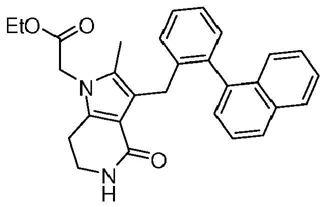

### Ethyl {2-methyl-3-[2-(1-naphthyl)benzyl]-4-oxo-4,5,6,7-tetrahydro-1H-pyrrolo[3,2-c]pyri din-1-yl}acetate

The title compound of Preparation 86 (40 mg, 0.088 mmol), 1-naphthylboronic acid (23 mg, 0.13 mmol) and 1,1'-bis(diphenylphosphino)ferrocene palladium(II) dichloride (3.6 mg, 4.4 µmol) were suspended in 1.7 ml dioxane in a Schlenk tube. A solution of caesium carbonate (2M, 0.13 ml, 0.26 mmol) was added. Argon was bubbled through the mixture for several minutes and then the mixture was stirred at 90 ºC under argon overnight. The mixture was allowed to cool and was filtered through a plug of Celite. The celite was washed several times with ethyl acetate. The combined organics were evaporated under reduced pressure and the residue was partitioned between ethyl acetate and water. The organics were washed with water, dried over magnesium sulphate, filtered and evaporated under reduced pressure. The residue was purified by flash chromatography (ethyl acetate-hexane gradient, 0:100 rising to 100:0) to give 12 mg (0.026 mmol, 30%) of the title compound. Purity 95%.
¹H NMR (400 MHz, CHLOROFORM-*d*) δ ppm 7.89 (1 H, d, *J*=8.2 Hz), 7.86 (1 H, d, *J*=8.0 Hz), 7.52 - 7.59 (2 H, m), 7.44 - 7.51 (2 H, m), 7.39 (1 H, t, *J*=7.0 Hz), 7.29 - 7.34 (2 H, m), 7.19 - 7.28 (2 H, m), 5.14 (1 H, br. s.), 4.34 (1 H, d, *J*=17.2 Hz), 4.29 (1 H, d, *J*=17.2 Hz), 4.19 (2 H, q, *J*=7.2 Hz), 3.93 (1 H, d), 3.87 (1 H, d, *J*=15.2 Hz), 3.49 (2 H, t, *J*=6.5 Hz), 2.65 (2 H, td, *J*=6.8, 2.1 Hz), 1.65 (3 H, s), 1.26 (3 H, t, *J*=7.1 Hz).
HPLC/MS (5 min) retention time 3.48 min.
LRMS: m/z 453 (M+1)

### EXAMPLE 50

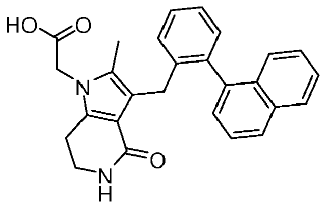

### {2-Methyl-3-[2-(1-naphthyl)benzyl]-4-oxo-4,5,6,7-tetrahydro-1H-pyrrolo[3,2-c]pyri din-1-yl}acetic acid

The title compound of Example 49 (12 mg, 0.026 mmol) was dissolved in 1 ml of tetrahydrofuran and 0.5 ml of water. Lithium hydroxide monohydrate (3 mg, 0.07 mmol) was added and the mixture was agitated at room temperature for 10 min. The mixture was evaporated under reduced pressure and was re-suspended in water. The aqueous was acidified to pH1 with 1 N hydrochloric acid. The aqueous layer was The aqueous phase was extracted three times with dichloromethane. The combined organics were dried over magnesium sulphate, filtered and evaporated under reduced pressure to give 11 mg (0.026 mmol, 98%) of the title compound. Purity 96%.
¹H NMR (400 MHz, DMSO-d6) δ ppm 8.07 (1 H, d, J=8.2 Hz), 8.03 (1 H, d, J=8.2 Hz), 7.67 (1 H, t, J=7.6 Hz), 7.59 (1 H, dd, J=8.0, 1.8 Hz), 7.46 - 7.55 (3 H, m), 7.27 - 7.39 (2 H, m), 7.21 (2 H, d, J=7.2 Hz), 6.78 (1 H, br. s.), 4.60 (2 H, s), 3.72 (2 H, s), 3.37 (3 H, t, J=6.8 Hz), 2.68 (2 H, t, J=6.8 Hz), 1.65 (3 H, s).
HPLC/MS (30 min) retention time 16.58 min.
LRMS: m/z 403 (M+1).

### EXAMPLE 51

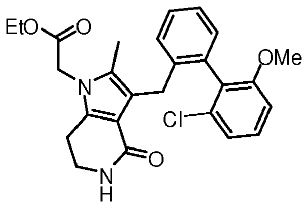

### Ethyl {3-[(2'-chloro-6'-methoxybiphenyl-2-yl)methyl]-2-methyl-4-oxo-4,5,6,7-tetrahydro-1H-pyrrolo[3,2-c]pyridin-1-yl}acetate

The title compound of Preparation 86 (100 mg, 0.22 mmol), 2-chloro-6-methoxyphenylboronic acid (62 mg, 0.33 mmol) and 1,1'-bis(diphenylphosphino)-ferrocene palladium(II) dichloride dichloromethane complex (14 mg, 17 µmol) were suspended in 2.5 ml dioxane in a Schlenk tube. A solution of caesium carbonate (2M, 0.33 ml, 0.66 mmol) was added. Argon was bubbled through the mixture for several minutes and then the mixture was stirred at 90 ºC under argon for 6 h. The mixture was allowed to cool and was filtered through a plug of Celite. The celite was washed several times with ethyl acetate. The combined organics were evaporated under reduced pressure and the residue was partitioned between ethyl acetate and water. The organics were washed with brine, dried over sodium sulphate, filtered and evaporated under reduced pressure. The residue was purified by flash chromatography (ethyl acetate-hexane gradient, 0:100 rising to 90:10) to give 33 mg (0.070 mmol, 32%) of the title compound. Purity 100%.
¹H NMR (400 MHz, CHLOROFORM-*d*) δ ppm 7.17 - 7.26 (4 H, m), 7.07 - 7.11 (1 H, m), 7.05 (1 H, d, *J*=7.8 Hz), 6.86 (1 H, d, *J*=8.2 Hz), 4.99 (1 H, br. s.), 4.43 (2 H, s), 4.20 (2 H, q, *J*=7.2 Hz), 4.00 (1 H, d, *J*=16.0 Hz), 3.91 (1 H, d, *J*=16.0 Hz), 3.77 (3 H, s), 3.50 (2 H, t, *J*=6.8 Hz), 2.72 (2 H, t, *J*=6.8 Hz), 1.79 (3 H, s), 1.26 (3 H, t, *J*=7.2 Hz).
UPLC/MS (3 min) retention time 1.90 min.
LRMS: *m*/*z* 467 (M+1).

### EXAMPLE 52

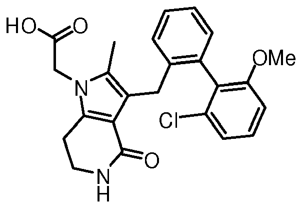

### {3-[(2'-Cloro-6'-methosybiphenyl-2-yl)menthyl]-2-methyl-4-oxo-4,5,6,7-tetrahydro-1H-pyrrolo[3,2-c]pyridin-1-yl}acetic acid

The title compound of Example 51 (30 mg, 0.064 mmol) was dissolved in 2 ml tetrahydrofuran and 1.5 ml water. Lithium hydroxide monohydrate (11 mg, 0.26 mmol) was added and the mixture was agitated at room temperature for 2 h. The organic solvent was evaporated under reduced pressure and the remaining aqueous layer was acidified to pH 4 with acetic acid, forming a precipitate. The mixture was agitated for 1.5 h. The precipitate was filtered, washes with a little water and was dried under reduced pressure to give 20 mg (0.046 mmol, 71%) of the title compound as a white solid. Purity 95%.
¹H NMR (400 MHz, DMSO-d₆) δ ppm 13.03 (1 H, br. s.), 7.39 (1 H, t, *J*=8.2 Hz), 7.05 - 7.20 (5 H, m), 6.98 (1 H, d, *J*=6.6 Hz), 6.77 (1 H, br. s.), 4.59 (2 H, s), 3.74 (1 H, d, *J*=16.0 Hz), 3.70 (3 H, s), 3.66 (1 H, d, *J*=16.0 Hz), 3.28 - 3.33 (2 H, m), 2.65 (2 H, t, *J*=6.8 Hz), 1.68 (3 H, s).
HPLC/MS (30 min) retention time 15.19 min.
LRMS: *m*/*z* 439 (M+1).

### EXAMPLE 53

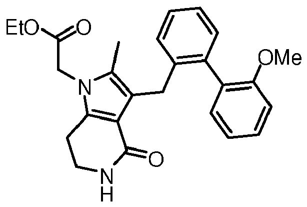

### Ethyl {3-[(2'-methoxybiphenyl-2-yl)methyl]-2-methyl-4-oxo-4,5,6,7-tetrahydro-1H-pyrrol o[3,2-c]pyridin-1-yl}acetate

The title compound of Preparation 86 (80 mg, 0.18 mmol), 2-methoxyphenylboronic acid (41 mg, 0.27 mmol) and 1,1'-bis(diphenylphosphino)-ferrocene palladium(II) dichloride dichloromethane complex (12 mg, 15 µmol) were suspended in 2 ml dioxane in a Schlenk tube. A solution of caesium carbonate (2M, 0.26 ml, 0.52 mmol) was added. Argon was bubbled through the mixture for several minutes and then the mixture was stirred at 90 ºC under argon for 6 h. The mixture was allowed to cool and was filtered through a plug of Celite. The celite was washed several times with dioxane. The combined organics were evaporated under reduced pressure and the residue was partitioned between ethyl acetate and water. The organics were washed with brine, dried over sodium sulphate, filtered and evaporated under reduced pressure. The residue was purified by flash chromatography (ethyl acetate-hexane gradient, 0:100 rising to 90:10) to give 29 mg (0.066 mmol, 37%) of the title compound as a pale yellow oil. Purity 99%.
¹H NMR (400 MHz, CHLOROFORM-*d*) δ ppm 7.32 (1 H, t, *J*=7.8 Hz), 7.21 - 7.26 (2 H, m), 7.13 - 7.19 (2 H, m), 7.00 (1 H, t, *J*=7.2 Hz), 6.95 (1 H, d, *J*=8.6 Hz), 5.02 (1 H, br. s.), 4.43 (2 H, s), 4.20 (2 H, q, *J*=7.0 Hz), 4.06 (1 H, d, *J*=16.0 Hz), 3.94 (1 H, d, *J*=16.0 Hz), 3.79 (3 H, s), 3.53 (2 H, td, *J*=6.7, 2.5 Hz), 2.72 (2 H, t, *J*=6.8 Hz), 1.75 (3 H, s), 1.26 (3 H, t, *J*=7.0 Hz).
UPLC/MS (3 min) retention time 1.84 min.
LRMS: *m*/*z* 433 (M+1).

### EXAMPLE 54

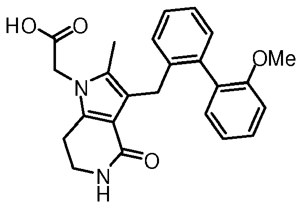

### {3-[(2'-methoxybiphenyl-2-yl)methyl]-2-methyl-4-oxo-4,5,6,7-tetrahydro-1H-pyrrol o[3,2-c]pyridin-1-yl}acetic acid

The title compound of Example 53 (28 mg, 0.065 mmol) was dissolved in 1.5 ml tetrahydrofuran and 1.75 ml water. Lithium hydroxide monohydrate (11 mg, 0.26 mmol) was added and the mixture was agitated at room temperature for 3 h. The organic solvent was evaporated under reduced pressure and the remaining aqueous layer was acidified to pH 4 with acetic acid, forming a precipitate. The mixture was agitated for 1.5 h. The precipitate was filtered, washes with a little water and was dried under reduced pressure to give 21 mg (0.052 mmol, 80%) of the title compound as a white solid. Purity 97%.
¹H NMR (400 MHz, DMSO-d₆) δ ppm 12.85 - 13.23 (1 H, m), 7.38 (1 H, t, *J*=7.8 Hz), 7.07 - 7.19 (4 H, m), 7.00 - 7.06 (3 H, m), 6.76 (1 H, br. s.), 4.58 (2 H, s), 3.83 (1 H, d, *J*=16.0 Hz), 3.73 (3 H, s), 3.71 (1 H, d, *J*=16.0 Hz), 3.26 - 3.33 (2 H, m), 2.65 (2 H, t, *J*=6.8 Hz), 1.66 (3 H, s).
HPLC/MS (30 min) retention time 14.53 min.
LRMS: *m*/*z* 405 *(M+1).*

### EXAMPLE 55

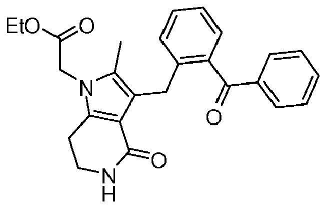

### Ethyl [3-(2-benzoylbenzyl)-2-methyl-4-oxo-4,5,6,7-tetrahydro-1H-pyrrolo[3,2-c]pyridin-1 -yl]acetate

To a suspension of sodium hydride (17.4 mg, 0.43 mmol) in 0.5 ml dimethylformamide was added the title compound of Preparation 90 (125 mg, 0.363 mmol) dissolved in 1 ml dimethylformamide and the mixture was stirred at room temperature for 30 min. Ethyl bromoacetate (44 µl, 0.40 mmol) was added and the mixture was stirred at room temperature for 1 h. The mixture was partitioned between ethyl acetate and water. The aqueous was extracted with ethyl acetate and the combined organics were washed with brine, dried over magnesium sulphate and filtered. Evaporation gave a residue which was purified using the SP1 Purification System (methanol:ethyl acetate gradient, 0:100 rising 5:95) to give 125 mg of the crude title compound. Purity 82%.
¹H NMR (400 MHz, DMSO-d6) δ ppm 1.17 (t, J=7.13 Hz, 3 H), 1.78 (s, 3 H), 2.57 (t, J=6.84 Hz, 2 H), 3.23 - 3.27 (m, 2 H), 3.93 (s, 2 H), 4.11 (q, J=7.16 Hz, 2 H), 4.58 (s, 2 H), 6.71 (s, 1 H), 7.13 - 7.24 (m, 3 H), 7.31 - 7.36 (m, 1 H), 7.51 (t, J=7.62 Hz, 2 H), 7.61 - 7.70 (m, 3 H).
HPLC/MS (5 min) retention time 3.97 min.
LRMS: *m*/*z* 431 (M+1).

### EXAMPLE 56

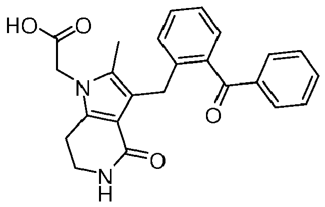

### [3-(2-Benzoylbenzyl)-2-methyl-4-oxo-4,5,6,7-tetrahydro-1H-pyrrolo[3,2-c]pyridin-1 -yl]acetic acid

The crude title compound of Example 55 (125 mg) was dissolved in 4 ml of tetrahydrofuran and 2 ml of water. Lithium hydroxide monohydrate (14 mg, 0.58 mmol) was added and the mixture was agitated at room temperature for 3 h. The mixture was partitioned between water and ethyl acetate. The aqueous layer was acidified to pH 3 with 2N hydrochloric acid. The aqueous layer was extracted twice with ethyl acetate. The combined organics were washed with brine, dried over magnesium sulphate and evaporated to give 79 mg (0.194 mmol, 45% over two steps) of the title compound. Purity 99%.
¹H NMR (400 MHz, CHLOROFORM-d) d ppm 7.70 (2 H, d, J=7.2 Hz), 7.50 (1 H, t, J=7.3 Hz), 7.46 (1 H, br. s.), 7.37 (2 H, t, J=7.6 Hz), 7.23 - 7.33 (2 H, m), 7.13 - 7.19 (2 H, m), 4.17 (2 H, s), 4.05 (2 H, s), 3.39 (2 H, t, J=6.5 Hz), 2.52 (2 H, t, J=7.0 Hz), 1.92 (3 H, s).
HPLC/MS (30 min) retention time 13.17 min.
LRMS: *m*/*z* 403 (M+1).

### EXAMPLE 57

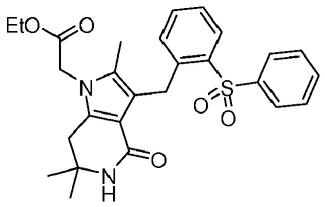

### Ethyl {2,6,6-trimethyl-4-oxo-3-[2-(phenylsulfonyl)benzyl]-4,5,6,7-tetrahydro-1H-pyrrolo[ 3,2-c]pyridin-1-yl}acetate

The title compound of Preparation 81 (250 mg, 0.95 mmol) was treated with anhydride acetic (2 ml, 21.2 mmol), hydriodic acid (57% solution, 1.25 ml, 9.5 mmol) and hypophosphorous acid (50% solution, 0.49 ml, 4.7 mmol). The title compound of Preparation 2 (303 mg, 1.23 mmol) was then added and the mixture was stirred at room temperature for 3 h. The mixture was partitioned between water and dichloromethane. The aqueous layer was extracted twice with dichloromethane. The combined organics were washed with water, dried over magnesium sulphate, filtered and evaporated under reduced pressure. The resulting residue was purified using the SP1 Purification System (ethyl acetate-hexane gradient, 0:100 rising to 100:0) to give 325 mg (0.66 mmol, 69%) of the title compound. Purity 100%.
HPLC/MS (9 min) retention time 6.3 min.
LRMS: *m*/*z* 495 (M+1).

### EXAMPLE 58

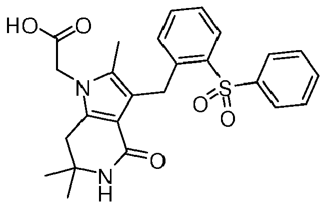

### {2,6,6-Trimethyl-4-oxo-3-[2-(phenylsulfonyl)benzyl]-4,5,6,7-tetrahydro-1H-pyrrolo[ 3,2-c]pyridin-1-yl}acetic acid

The title compound of Example 57 (325 mg, 0.66 mmol) was dissolved in 2 ml tetrahydrofuran and 2 ml water. Lithium hydroxide monohydrate (83 mg, 2.0 mmol) was added and the mixture was agitated at room temperature for 3 h. The organic solvent was evaporated under reduced pressure and the remaining aqueous layer was acidified with 2N hydrochloric acid, forming of a white precipitate. The precipitate was filtered and was dried under reduced pressure to give 289 mg (0.62 mmol, 94%) of the title compound. Purity 100%.
¹H NMR (400 MHz, CHLOROFORM-d) δ ppm 8.27 - 8.16 (m, J = 7.4, 2.0 Hz, 1 H), 7.93 - 7.83 (m, 2H), 7.57 - 7.43 (m, 3H), 7.42 - 7.29 (m, 2H), 7.03 - 6.93 (m, 1 H), 4.41 (s, 2H), 4.29 (s, 2H), 2.65 (s, 2H), 1.76 (s, 3H), 1.28 (s, 6H).
HPLC/MS (30 min) retention time 11.80 min.
LRMS: *m*/*z* 467 (M+1).

### EXAMPLE 59

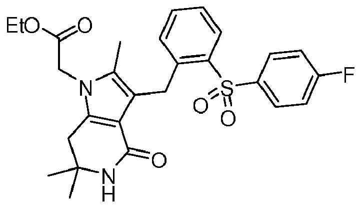

### Ethyl (3-{2-[(4-fluorophenyl)sulfonyl]benzyl}-2,6,6-trimethyl-4-oxo-4,5,6,7-tetrahydro-1 H-pyrrolo[3,2-c]pyridin-1-yl)acetate

The title compound of Preparation 81 (100 mg, 0.38 mmol) was treated with acetic anhydride (1.0 ml, 10 mmol), hydriodic acid (57% solution, 0.5 ml, 3.8 mmol), hypophosphorous acid (50% solution, 0.19 ml, 1.9 mmol) and the title compound of Preparation 4 (130 mg, 0.49 mmol) according to the method of Preparation 86. The resulting residue by flash chromatography (ethyl acetate-hexane gradient, 0:100 rising to 100:0) to give 147 mg (0.29 mmol, 75%) of the title compound. Purity 100%.
¹H NMR (400 MHz, CHLOROFORM-*d*) δ ppm 8.21 (1 H, d, *J*=7.8 Hz), 7.95 (2 H, dd, *J*=9.0, 5.1 Hz), 7.39 (1 H, t, *J*=7.0 Hz), 7.32 (1 H, t, *J*=7.4 Hz), 7.18 (2 H, t, *J*=8.6 Hz), 7.07 (1 H, d, *J*=7.4 Hz), 4.80 (1 H, s), 4.47 (2 H, s), 4.36 (2 H, s), 4.22 (2 H, q, *J*=7.2 Hz), 2.69 (2 H, s), 1.89 (3 H, s), 1.34 (6 H, s), 1.28 (3 H, t, *J*=7.0 Hz).
UPLC/MS (3 min) retention time 1.77 min.
LRMS: *m*/*z* 513 (M+1).

### EXAMPLE 60

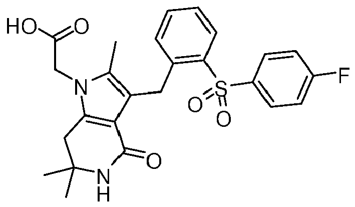

### (3-{2-[(4-Fluorophenyl)sulfonyl]benzyl}-2,6,6-trimethyl-4-oxo-4,5,6,7-tetrahydro-1 H-pyrrolo[3,2-c]pyridin-1-yl)acetic acid

The title compound of Example 59 (147 mg, 0.29 mmol) was suspended in a mixture of 2 ml tetrahydrofuran, 1 ml ethanol and 1 ml water. Lithium hydroxide monohydrate (37 mg, 0.88 mmol) was added and the mixture was agitated at room temperature for 2.5 h. The organic solvent was evaporated under reduced pressure and the remaining aqueous layer was acidified with 1 N hydrochloric acid. forming a precipitate. The precipitate was filtered and was dried under reduced pressure to give 130 mg (0.27 mmol, 93%) of the title compound as a white solid. Purity 98%.
¹H NMR (400 MHz, DMSO-*d*₆) δ ppm 13.05 (1 H, br. s.), 8.14 (1 H, d, *J*=7.8 Hz), 8.00 (2 H, dd, *J*=9.0, 5.1 Hz), 7.53 (1 H, t, *J*=7.6 Hz), 7.43 - 7.51 (3 H, m), 6.94 (1 H, d, *J*=7.4 Hz), 6.71 (1 H, s), 4.60 (2 H, s), 4.17 (2 H, s), 2.66 (2 H, s), 1.51 (3 H, s), 1.21 (6 H, s).
HPLC/MS (30 min) retention time 13.97 min.
LRMS: *m*/*z* 485 (M+1).

### EXAMPLE 61

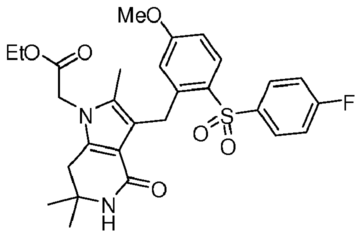

### Ethyl (3-{2-[(4-fluorophenyl)sulfonyl]-5-methoxybenzyl}-2,6,6-trimethyl-4-oxo-4,5,6,7-te trahydro-1H-pyrrolo[3,2-c]pyridin-1-yl)acetate

The title compound of Preparation 81 (100 mg, 0.38 mmol) was treated with acetic anhydride (1.0 ml, 10 mmol), hydriodic acid (57% solution, 0.5 ml, 3.8 mmol), hypophosphorous acid (50% solution, 0.19 ml, 1.9 mmol) and the title compound of Preparation 38 (145 mg, 0.49 mmol) according to the method of Preparation 86. The resulting residue was purified by flash chromatography (ethyl acetate-hexane gradient, 0:100 rising to 100:0) to give 158 mg (0.29 mmol, 77%) of the title compound. Purity 98%.
¹H NMR (400 MHz, CHLOROFORM-*d*) δ ppm 8.16 (1 H, d, *J*=8.6 Hz), 7.93 (2 H, dd, *J*=8.6, 5.1 Hz), 7.17 (2 H, t, *J*=8.6 Hz), 6.79 (1 H, dd, *J*=8.8, 2.5 Hz), 6.56 (1 H, d, *J*=2.3 Hz), 4.77 (1 H, s), 4.47 (2 H, s), 4.32 (2 H, s), 4.22 (2 H, q, *J*=7.0 Hz), 3.70 (3 H, s), 2.67 (2 H, s), 1.90 (3 H, s), 1.33 (6 H, s), 1.27 (3 H, t, *J*=7.0 Hz).
UPLC/MS (3 min) retention time 1.79 min.
LRMS: *m*/*z* 543 (M+1).

### EXAMPLE 62

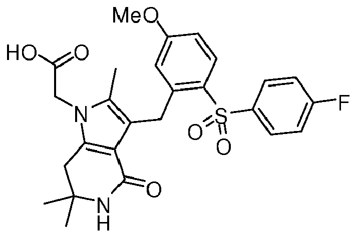

### (3-{2-[(4-Fluorophenyl)sulfonyl]-5-methoxybenzyl}-2,6,6-trimethyl-4-oxo-4,5,6,7-te trahydro-1H-pyrrolo[3,2-c]pyridin-1-yl)acetic acid

The title compound of Example 61 (158 mg, 0.29 mmol) was suspended in a mixture of 2 ml tetrahydrofuran, 1 ml ethanol and 1 ml water. Lithium hydroxide monohydrate (37 mg, 0.88 mmol) was added and the mixture was agitated at room temperature for 2.5 h. The organic solvent was evaporated under reduced pressure and the remaining aqueous layer was acidified with 1 N hydrochloric acid. forming a precipitate. The precipitate was filtered and was dried under reduced pressure to give 136 mg (0.26 mmol, 91 %) of the title compound as a white solid. Purity 100%.
¹H NMR (400 MHz, DMSO-*d*₆) δ ppm 13.05 (1 H, br. s.), 8.10 (1 H, d, *J*=8.6 Hz), 7.95 (2 H, dd, *J*=9.0, 5.1 Hz), 7.45 (2 H, t, *J*=8.8 Hz), 7.00 (1 H, dd, *J*=8.8, 2.5 Hz), 6.69 (1 H, s), 6.41 (1 H, d, *J*=2.3 Hz), 4.60 (2 H, s), 4.11 (2 H, s), 3.67 (3 H, s), 2.65 (2 H, s), 1.55 (3 H, s), 1.21 (6 H, s).
HPLC/MS (30 min) retention time 14.06 min.
LRMS: *m*/*z* 515 (M+1).

### EXAMPLE 63

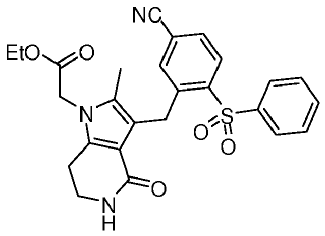

### Ethyl {3-[5-cyano-2-(phenylsulfonyl)benzyl]-2-methyl-4-oxo-4,5,6,7-tetrahydro-1H-pyrro lo[3,2-c]pyridin-1-yl}acetate

The title compound of Example 41 (340 mg, 0.56 mmol) was dissolved in 2 ml dimethylformamide under argon atmosphere. Zinc cyanide (73 mg, 0.62 mmol) and Palladium tetrakis(triphenylphosphine) (80 mg, 0.07 mmol) were added and the mixture was heated at 180 ºC in the microwave (CEM Discover) for 40 min. The reaction was diluted with ethyl acetate and was filtered over Celite. The filtrate was concentrated under reduced pressure. The residue was purified using the SP1 Purification System (ethyl acetate:hexane gradient, 0:100 rising 100:0) to give 60 mg of the crude title compound. Purity 88%
HPLC/MS (9 min) retention time 5.97 min.
LRMS: *m*/*z* 492 (M+1).

### EXAMPLE 64

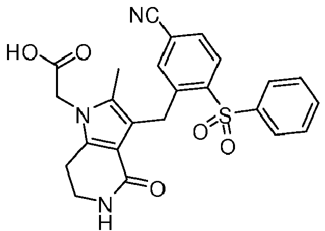

### {3-[5-Cyano-2-(phenylsulfonyl)benzyl]-2-methyl-4-oxo-4,5,6,7-tetrahydro-1H-pyrr olo[3,2-c]pyridin-1-yl}acetic acid

The crude title compound of Example 63 (60 mg) was dissolved in 5 ml of tetrahydrofuran and 5 ml of water. Lithium hydroxide monohydrate (25 mg, 0.6 mmol) was added and the mixture was agitated at room temperature for 4 h. The organic solvent was evaporated under reduced pressure and the remaining aqueous layer was acidified to pH 4-5 with 2N hydrochloric acid, forming a precipitate. The precipitate was filtered and was dried in a stream of air. The resulting solid was purified using the SP1 Purification System (methanol-dichloromethane gradient, 0:100 rising to 30:70) to give 4 mg (0.007 mmol, 1% over two steps) of the title compound. Purity 87%.
¹H NMR (400 MHz, METHANOL-d4) δ 8.24 (d, J = 8.2 Hz, 1 H), 7.87 - 7.81 (m, 2H), 7.70 - 7.65 (m, 1 H), 7.64 - 7.58 (m, 1 H), 7.56 - 7.49 (m, 3H), 7.30 - 7.28 (m, 1 H), 4.17 (s, 2H), 3.42 (t, J = 7.0 Hz, 2H), 2.71 (t, J = 7.0 Hz, 2H), 1.54 (s, 3H).
HPLC/MS (30 min) retention time 12.76 min.
LRMS: *m*/*z* 464 (M+1).

### EXAMPLE 65

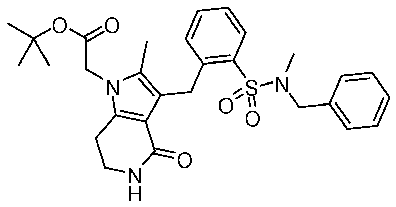

### tert-Butyl [3-(2-{[benzyl(methyl)amino]sulfonyl}benzyl)-2-methyl-4-oxo-4,5,6,7-tetrahydro-1 H-pyrrolo[3,2-c]pyridin-1-yl]acetate

The title compound of Preparation 89 (20 mg, 0.05 mmol) was dissolved in 1 ml dimethylformamide. Sodium hydride (60% dispersion in oil, 3 mg, 0.08 mmol) previously washed with pentane was added and the mixture was stirred for 45 minutes at room temperature. Tert-butyl 2-bromoacetate (9 µl, 0.06 mmol) was then added and the mixture was agitated for 5 h. The mixture was poured into water and the aqueous layer was extracted with ethyl ether two times. The combined organics were washed with water and brine and were dried over sodium sulphate. Filtration and evaporation gave 12 mg of the crude title compound as a colourless solid. Purity 83%.
HPLC/MS (9 min) retention time 7.02 min.
LRMS: *m*/*z* 538 (M+1).

### EXAMPLE 66

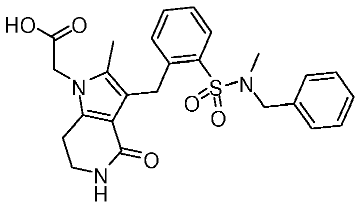

### [3-(2-{[Benzyl(methyl)amino]sulfonyl}benzyl)-2-methyl-4-oxo-4,5,6,7-tetrahydro-1 H-pyrrolo[3,2-c]pyridin-1-yl]acetic acid

The crude title compound of Example 65 (12 mg) was dissolved in 0.3 ml chloroform. Trifluoroacetic acid (0.1 ml, 1.3 mmol) was added and the mixture was stirred at room temperature for 2 d. The mixture was concentrated under reduced pressure, frozen and lyophilised. The resulting residue was triturated several times with ether, decanting each time. The solid obtained was dried under reduced pressure to give 3 mg (0.006 mmol, 12% over two steps) of the title compound as a grey solid. Purity 100%.
¹H NMR (400 MHz, DMSO-d6) δ ppm 1.88 (s, 3 H), 2.63 - 2.78 (m, 5 H), 4.43 (s, 4 H), 4.68 (br. s., 2 H), 6.84 (br. s., 1 H), 7.04 (d, J=7.82 Hz, 1 H), 7.29 - 7.45 (m, 6 H), 7.49 (t, J=7.42 Hz, 1 H), 7.86 (d, J=7.42 Hz, 1 H).
HPLC/MS (30 min) retention time 14.80 min.
LRMS: *m*/*z* 482 *(M+1).*

### EXAMPLE 67

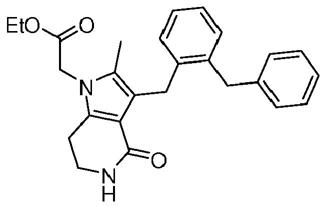

### Ethyl [3-(2-benzylbenzyl)-2-methyl-4-oxo-4,5,6,7-tetrahydro-1H-pyrrolo[3,2-c]pyridin-1-yl]acetate

The title compound of Preparation 86 (100 mg, 0.21 mmol) was dissolved in 2ml dioxane under argon atmosphere. 2-Benzyl-4,4,5,5-tetramethyl-1,3,2-dioxaborolane (70 µl, 0.315 mmol), bis(diphenylphosphino)ferrocene]dichloropalladium(II), complex with dichloromethane (9 mg, 0.01 mmol) and caesium carbonate solution (2M, 0.31 ml, 0.63 mmol) were added and the mixture was heated at 90ºC for 24 h. Additional 2-benzyl-4,4,5,5-tetramethyl-1,3,2-dioxaborolane (70 µl, 0.315 mmol) and bis(diphenyl-phosphino)ferrocene]dichloropalladium(II), complex with dichloromethane (9 mg, 0.01 mmol) were added and the mixture was heated at 90ºC for 24 h. The mixture was allowed to cool and was filtered through Celite. The filtrate was concentrated under reduced pressure and the residue was taken up in ethyl acetate and was washed with water and brine. The organic layer was dried over sodium sulphate, filtered and evaporated under reduced pressure. The resulting residue was purified by reverse-phase chromatography using the SP1 Purification system to give 8 mg (0.019 mmol, 9%) of the title compound as an amber oil. Purity 100%.
HPLC/MS (9 min) retention time 6.83 min.
LRMS: *m*/*z* 417 (M+1).

### EXAMPLE 68

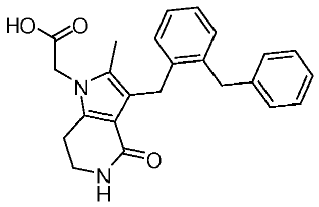

### [3-(2-Benzylbenzyl)-2-methyl-4-oxo-4,5,6,7-tetrahydro-1H-pyrrolo[3,2-c]pyridin-1-yl]acetic acid

The title compound of Example 67 (8 mg, 0.02 mmol) was dissolved in 0.5 ml of tetrahydrofuran and 0.5 ml of water. Lithium hydroxide monohydrate (4 mg, 0.10 mmol) was added and the mixture was agitated at room temperature for 90 min. The organic solvent was evaporated under reduced pressure and the remaining aqueous layer was acidified to pH 4 with acetic acid, forming a precipitate. The mixture was agitated for 4 h. The precipitate was filtered and was dried under reduced pressure. The resulting precipitate was triturated several times with ether, decanting each time and was dried again under reduced pressure to give 1.0 mg (0.002 mmol, 13%) of the title compound as a grey solid. Purity 98%.
¹H NMR (400 MHz, DMSO-d6) δ ppm 1.73 (br. s., 3 H), 2.61 - 2.69 (m, 2 H), 3.97 (br. s., 2 H), 4.10 (br. s., 2 H), 4.58 (br. s., 2 H), 6.74 (br. s., 1 H), 6.84 (br. s., 1 H), 6.99 - 7.36 (m, 8 H).
HPLC/MS (30 min) retention time 13.82 min.
LRMS: *m*/*z* 389 (M+1).

### EXAMPLE 69

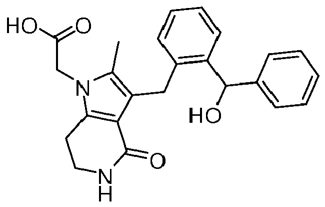

### (3-{2-[Hydroxy(phenyl)methyl]benzyl}-2-methyl-4-oxo-4,5,6,7-tetrahydro-1H-pyrro lo[3,2-c]pyridin-1-yl)acetic acid

The title compound of Example 56 (80 mg, 0.20 mmol) was dissolved in 3 ml methanol. Sodium borohydride (150 mg, 4.0 mmol) was added and the mixture stirred at room temperature for 5 h. The mixture was evaporated under reduced pressure and the resulting residue was purified by reverse-phase chromatography (formic acid buffer) using the SP1 Purification system to give 27 mg (0.065 mmol, 33%) of the title compound. Purity 98%.
¹H NMR (partial, 400 MHz, DMSO-d6) δ ppm 7.24 - 7.34 (5 H, m), 7.19 (1 H, t, J=6.7 Hz), 7.08 (1 H, t, J=7.0 Hz), 7.02 (1 H, td, J=7.4, 1.2 Hz), 6.78 (1 H, d, J=7.6 Hz), 6.75 (1 H, br. s.), 6.09 (1 H, d, J=3.3 Hz), 5.80 (1 H, d, J=3.9 Hz), 4.60 (2 H, s), 3.99 - 4.12 (1 H, m), 3.82 - 3.95 (1 H, m), 2.65 (2 H, t, J=6.7 Hz), 1.72 (3 H, s).
HPLC/MS (30 min) retention time 13.17 min.
LRMS: m/z 403 (M-1)

### EXAMPLE 70

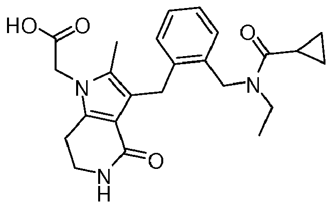

### [3-(2-{[(Cyclopropylcarbonyl)(ethyl)amino]methyl}benzyl)-2-methyl-4-oxo-4,5,6,7-tetrahydro-1H-pyrrolo[3,2-c]pyridin-1-yl]acetic acid

The title compound of Preparation 77 (125 mg, 0.371 mmol) was treated with anhydride acetic (0.40 ml, 4.2 mmol), hydriodic acid (57% solution, 0.40 ml, 3.0 mmol), hypophosphorous acid (50% solution, 0.11 ml, 1.05 mmol) and the title compound of Preparation 53 (123 mg, 0.45 mmol) according to the method of Example 16. The resulting residue was purified by reverse-phase chromatography (formic acid buffer) using the SP1 Purification system to give 44 mg (0.10 mmol, 27%) of the title compound. Purity 97%.
50:50 Mixture of 2 rotamers. The data refers to the sum of the two spectra:
¹H NMR (400 MHz, DMSO-d6) δ ppm 7.02 - 7.18 (4 H, m), 6.91 - 7.02 (3 H, m), 6.89 (1 H, d, J=6.1 Hz), 6.73 (2 H, br. s.), 4.86 (2 H, s), 4.55 - 4.69 (6 H, m), 4.01 (2 H, s), 3.97 (2 H, s), 3.36 - 3.46 (4 H, m), 3.24 - 3.35 (4 H, m), 2.59 - 2.71 (4 H, m), 1.95 - 2.01 (1 H, m), 1.93 (3 H, s), 1.87 (3 H, s), 1.64 (1 H, tt, J=8.0, 4.0 Hz), 1.10 (3 H, t, J=6.9 Hz), 0.99 (3 H, t, J=6.9 Hz), 0.66 - 0.82 (6 H, m), 0.58 (2 H, dd, J=7.2, 3.3 Hz).
HPLC/MS (30 min) retention time 12.63 min.
LRMS: *m*/*z* 423 (M+1).

### EXAMPLE 71

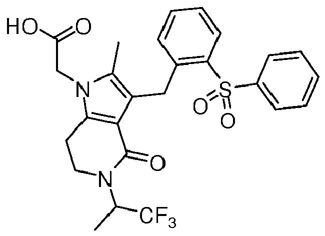

### [2-Methyl-4-oxo-3-[2-(phenylsulfonyl)benzyl]-5-(2,2,2-trifluoro-1-methylethyl)-4,5, 6,7-tetrahydro-1H-pyrrolo[3,2-c]pyridin-1-yl]acetic acid

The title compound of Preparation 84 (45 mg, 0.135 mmol) was treated with anhydride anhydride acetic (0.3 ml, 3.2 mmol), hydriodic acid (57% solution, 0.3 ml, 2.3 mmol), hypophosphorous acid (50% solution, 73 µl, 0.7 mmol) and the title compound of Preparation 2 (40 mg, 0.162 mmol) according to the method of Example 16. The resulting residue was purified by reverse-phase chromatography (formic acid buffer) using the SP1 Purification system to give 32 mg (0.06 mmol, 44%) of the title compound. Purity 99%.
¹H NMR (400 MHz, CHLOROFORM-d) δ ppm 8.22 (1 H, dd, J=7.8, 1.0 Hz), 7.90 (2 H, d, J=7.2 Hz), 7.55 (1 H, t, J=7.2 Hz), 7.48 (2 H, t, J=7.6 Hz), 7.38 (1 H, td, J=7.5, 1.1 Hz), 7.31 (1 H, t, J=7.4 Hz), 7.05 (1 H, d, J=7.4 Hz), 5.38 (1 H, spt, J=7.6 Hz), 4.49 (2 H, s), 4.38 (1 H, d, J=17.0 Hz), 4.30 (1 H, d, J=17.0 Hz), 3.48 - 3.65 (2 H, m), 2.68 - 2.86 (2 H, m), 1.79 (3 H, s), 1.32 (3 H, d, J=7.2 Hz).
HPLC/MS (30 min) retention time 15.83 min.
LRMS: *m*/*z* 535 (M+1).

### EXAMPLE 72

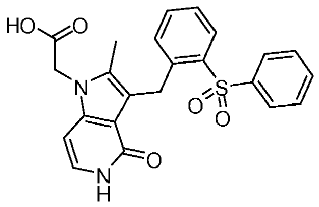

### {2-Methyl-4-oxo-3-[2-(phenylsulfonyl)benzyl]-4,5-dihydro-1H-pyrrolo[3,2-c]pyridin -1-yl}acetic acid

The title compound of Preparation 101 (0.20 g, 0.41 mmol) was suspended in 7.5 ml glacial acetic acid and 2.5 ml water and was heated in a pressure tube at 130°C for 4 d. The mixture was evaporated and the residue was resuspended in 2N sodium hydroxide solution. The solution acidified to pH 2-3 with concentrated hydrochloric acid, forming a precipitate. The solid was filtered and dried in vacuo to give 171 mg (0.39 mmol, 95%) of the title compound as a white solid. Purity 94%.
¹H NMR (400 MHz, DMSO-d₆) δ ppm 10.71 (1 H, d, J=5.5 Hz), 8.16 (1 H, dd, J=7.4, 1.9 Hz), 7.90-7.97 (2 H, m), 7.63-7.76 (3 H, m), 7.44-7.54 (2 H, m), 6.89-6.95 (2 H, m), 6.48 (1 H, d, J=7.1 Hz), 4.83 (2 H, s), 4.33 (2 H, s), 1.56 (3 H, s).
HPLC/MS (9 min) retention time 5.42 min.
LRMS: *m*/*z* 437 (M+1).

### EXAMPLE 73

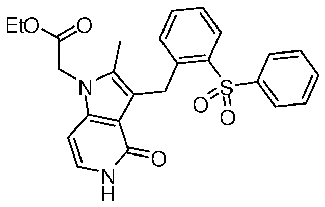

### Ethyl {2-methyl-4-oxo-3-[2-(phenylsulfonyl)benzyl]-4,5-dihydro-1H-pyrrolo[3,2-c]pyridin -1-yl}acetate

The title compound of Example 72 (135 mg, 0.31 mmol) was suspended in 2.5 ml ethanol. A drop of concentrated sulphuric acid was added and the mixture was capped and agitated at 75 ºC overnight. The mixture was partitioned between ethyl acetate and saturated sodium bicarbonate solution. The organics were washed with brine, dried over sodium sulphate and evaporated to give 116 mg (0.25 mmol, 81%) of the title compound as a cream solid. Purity 96%.
UPLC/MS (3 min) retention time 1.62 min.
LRMS: *m*/*z* 465 (M+1).

### EXAMPLE 74

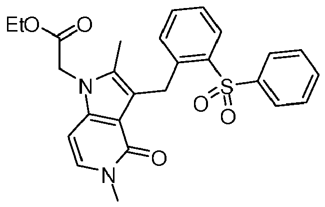

### Ethyl {2,5-dimethyl-4-oxo-3-[2-(phenylsulfonyl)benzyl]-4,5-dihydro-1H-pyrrolo[3,2-c]pyr idin-1-yl}acetate

The title compound of Example 73 (100 mg, 0.22 mmol) was dissolved in 1.2 ml dimethylformamide and was cooled in an ice-bath under nitrogen. Sodium hydride (60% in oil, 9 mg, 0.23 mmol) was added and the mixture was stirred for 30 min. Methyl iodide (14 µL, 0.22 mmol) was added the mixture was stirred overnight. The mixture was diluted with water and brine and was extracted with ethyl acetate. The organic layer was washed with brine, dried over sodium sulfate and evaporated. The residue was purified by flash chromatography (methanol-dichloromethane, 2:98) to give 54 mg (0.11 mmol, 52%) as an off-white foam. Purity 93%.
UPLC/MS (3 min) retention time 1.64 min.
LRMS: *m*/*z* 479 (M+1).

### EXAMPLE 75

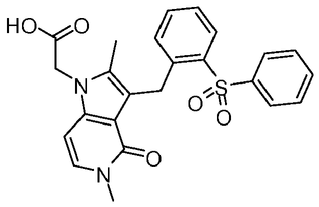

### {2,5-Dimethyl-4-oxo-3-[2-(phenylsulfonyl)benzyl]-4,5-dihydro-1H-pyrrolo[3,2-c]pyr idin-1-yl}acetic acid

The title compound of Example 74 (54 mg, 0.11 mmol) was dissolved in a mixture of 1 ml tetrahydrofuran and 0.5 ml water. Lithium hydroxide monohydrate (15 mg, 0.36 mmol) was added and the mixture was stirred at room temperature for 1 h. The organic solvents were removed in vacuo. The solution was diluted with 3ml water and 2 ml 2N sodium hydroxide. The aquous was washed with ethyl acetate and then acidified to pH 4-5 with 5N hydrochloric acid, precipitating a white solid. The precipitate was filtered, washed with water and dried in vacuo to give 27 mg (0.060 mmol, 53%) of the title compound as a white solid. Purity 96%.
¹H NMR (400 MHz, DMSO-d₆) δ ppm 8.16 (1 H, dd, J=6.9, 2.2 Hz), 7.93 (2 H, dd, J=7.7 Hz), 7.63-7.76 (3 H, m), 7.44-7.52 (2 H, m), 7.24 (1 H, d, J=7.4 Hz), 6.87 (1 H, d, J=6.6 Hz), 6.51 (1 H, d, J=7.1 Hz), 4.76 (2 H, s), 4.34 (2 H, s), 3.40 (3H, s), 1.57 (3 H, s).
UPLC/MS (3 min) retention time 1.38 min.
LRMS: *m*/*z* 451 (M+1).

### EXAMPLE 76

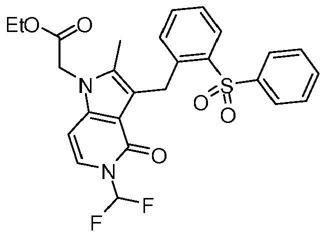

### Ethyl {5-(difluoromethyl)-2-methyl-4-oxo-3-[2-(phenylsulfonyl)benzyl]-4,5-dihydro-1H-p yrrolo[3,2-c]pyridin-1-yl}acetate

The title compound of Preparation 101 (0.15 g, 0.32 mmol) dissolved in 2 ml anhydrous acetonitrile. 2-(Fluorosulfonyl)difluoroacetic acid (96 µl, 0.93 mmol) was added, the vial was capped and the mixture was stirred at 40 °C overnight. The mixture was allowed to cool and was stirred with saturated sodium bicarbonate solution for 15 min. The mixture was extracted with ethyl acetate. The organics were washed with brine, dried over sodium sulphate and evaporated. The residue was purified by flash chromatography (ethyl acetate-hexane gradient, 33:67) to give 59 mg (0.11 mmol, 36%) of the title compound. Purity 95%.
¹H NMR (400 MHz, CDCl₃) δ ppm 8.20-8.30 (1 H, m), 7.88-8.04 (2 H, m), 7.30-7.75 (6 H, m), 7.13-7.20 (1 H, m), 7.00 (1 H, d, J=6.0 Hz), 6.35 (1 H, d, J=7.7 Hz), 4.68 (2 H, s), 4.50 (2 H, s), 4.24 (2 H, q, J=7.1 Hz), 2.00 (3 H, s), 1.28 (3 H, t, J=7.1 Hz).
UPLC/MS (3 min) retention time 1.83 min.
LRMS: *m*/*z* 515 (M+1).

### EXAMPLE 77

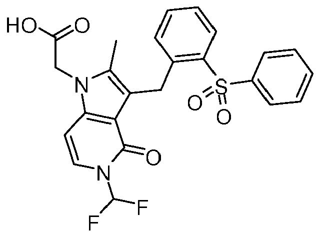

### {5-(Difluoromethyl)-2-methyl-4-oxo-3-[2-(phenylsulfonyl)benzyl]-4,5-dihydro-1H-p yrrolo[3,2-c]pyridin-1-yl}acetic acid

The title compound of Example 76 (59 mg, 0.11 mmol) was dissolved in a mixture of 1 ml tetrahydrofuran and 1 ml water. Lithium hydroxide monohydrate (15 mg, 0.36 mmol) was added and the mixture was stirred at room temperature for 1 h. The organics were evaporated in vacuo and the remaining slolution was dilted with 3 ml water. The solution was acidified to pH 3-4 with 2N hydrochloric acid, precipitating a solid. The precipitate was collected by filtration, was washed successively with water and 5M hydrochloric acid and was dried in the oven to give 49 mg (0.10 mmol, 87%) of the title compound. Purity 95%.
¹H NMR (400 MHz, DMSO-d₆) δ ppm 8.16 (1 H, dd, J=9.3, 1.3 Hz), 7.93 (2 H, d, J=7.4 Hz), 7.83 (1 H, t, J = 60.7 Hz), 7.63-7.76 (3 H, m), 7.46-7.55 (2 H, m), 7.37 (1 H, d, J=7.7 Hz), 6.85 (1 H, d, J=7.7 Hz), 5.00 (2 H, s), 4.40 (2 H, s), 1.70 (3 H, s).
UPLC/MS (3 min) retention time 1.59 min.
LRMS: *m*/*z* 487 (M+1).

### EXAMPLE 78

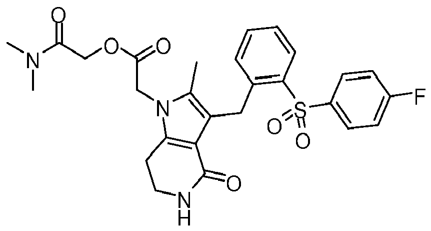

### 2-(Dimethylamino)-2-oxoethyl (3-{2-[(4-fluorophenyl)sulfonyl]benzyl}-2-methyl-4-oxo-4,5,6,7-tetrahydro-1H-pyrr olo[3,2-c]pyridin-1-yl)acetate

The title compound of Example 4 (100 mg, 0.22 mmol) and potassium carbonate (78 mg, 0.56 mmol) were suspended in 2 ml dimethylformamide. 2-Chloro-N,N-dimethylacetamide (54 µl, 0.52 mmol) was added and the mixture was stirred at room temperature for 4 d. The mixture was partitioned between ethyl acetate and water. The organics were washed with brine, dried over sodium sulphate, filtered and evaporated under reduced pressure. The residue was triturated with ether to give 34 mg (0.060 mmol, 27%) of the title compound as a pale yellow solid. Purity 95%.
¹H NMR (400 MHz, DMSO-d6) δ ppm 1.58 (s, 3 H) 2.72 (t, J=6.06 Hz, 2 H) 2.81 (s, 3 H) 2.90 (s, 3 H) 4.18 (s, 2 H) 4.84 (s, 2 H) 4.87 (s, 2 H) 6.81 (br. s., 1 H) 6.95 (d, J=7.42 Hz, 1 H) 7.41 - 7.58 (m, 4 H) 7.99 (dd, J=8.99, 5.08 Hz, 2 H) 8.14 (d, J=7.82 Hz, 1 H).
HPLC/MS (30 min) retention time 12.95 min.
LRMS: *m*/*z* 542 (M+1).

### EXAMPLE 79

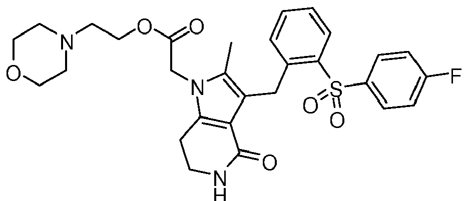

### 2-Morpholin-4-ylethyl (3-{2-[(4-fluorophenyl)sulfonyl]benzyl}-2-methyl-4-oxo-4,5,6,7-tetrahydro-1H-pyrr olo[3,2-c]pyridin-1-yl)acetate

The title compound of Example 4 (100 mg, 0.22 mmol) was dissolved in 2 ml dichloromethane. 2-Morpholinoethanol (35 mg, 0.27 mmol), 4-dimethylaminopyridine (13 mg, 0.11 mmol) and dicyclohexylcarbodiimide (63 mg, 0.31 mmol) were added and the mixture was stirred for 6 h at room temperature. The mixture was filtered and the filtrate was washed with water, dried over sodium sulphate, filtered and evaporated under reduced pressure. The residue was purified by reverse-phase chromatography using the SP1 Purification system to give 47 mg (0.080 mmol, 37%) of the title compound as a white solid. Purity 97%.
¹H NMR (400 MHz, DMSO-d6) δ ppm 1.53 (s, 3 H) 2.32 (br. s., 4 H) 2.70 (t, J=6.64 Hz, 2 H) 3.48 (t, J=4.30 Hz, 4 H) 4.17 (s, 2 H) 4.20 (t, J=5.28 Hz, 2 H) 4.75 (s, 2 H) 6.82 (br. s., 1 H) 6.95 (d, J=7.82 Hz, 1 H) 7.38 - 7 .81 (m, 4 H) 7.98 (dd, J=8.60, 5.08 Hz, 2 H) 8.14 (d, J=7.82 Hz, 1 H).
HPLC/MS (30 min) retention time 9.23 min.
LRMS: *m*/*z* 570 (M+1).

### EXAMPLE 80

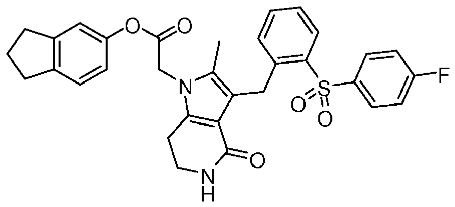

### 2,3-Dihydro-1H-inden-5-yl (3-{2-[(4-fluorophenyl)sulfonyl]benzyl}-2-methyl-4-oxo-4,5,6,7-tetrahydro-1H-pyrr olo[3,2-c]pyridin-1-yl)acetate

The title compound of Example 4 (150 mg, 0.33 mmol) was suspended in 3 ml dichloromethane. 2,3-Dihydro-1H-inden-5-ol (53 mg, 0.39 mmol), 4-dimethylamino-pyridine (20 mg, 0.16 mmol) and dicyclohexylcarbodiimide (93 mg, 0.45 mmol) were added and the mixture was stirred for 2.5 h at room temperature. The mixture was diluted with dichloromethane and filtered. The filtrate was washed with water, dried over sodium sulphate, filtered and evaporated under reduced pressure. The residue was partially purified by flash chromatography (methanol-dichloromethane gradient, 0:100 rising to 3:97) to give the crude product. The crude was further purified by reverse-phase chromatography using the SP1 Purification system to give 96 mg (0.17 mmol, 51%) of the title compound. Purity 99%.
¹H NMR (400 MHz, CHLOROFORM-*d*) δ ppm 8.22 (1 H, d, *J*=7.8 Hz), 7.95 (2 H, dd, *J*=9.0, 5.1 Hz), 7.37 (1 H, t, *J*=6.8 Hz), 7.32 (1 H, t, *J*=7.0 Hz), 7.12 - 7.23 (2 H, m), 7.07 (1 H, d, *J*=7.4 Hz), 6.91 (1 H, s), 6.80 (1 H, d, *J*=7.8 Hz), 4.98 (1 H, br. s.), 4.73 (2 H, s), 4.38 (2 H, s), 3.56 (2 H, td, *J*=6.6, 2.3 Hz), 2.90 (4 H, q, *J*=7.3 Hz), 2.84 (2 H, t, *J*=6.8 Hz), 2.10 (2 H, quin, *J*=7.4 Hz), 1.97 (3 H, s).
HPLC/MS (30 min) retention time 17.86 min.
LRMS: *m*/*z* 573 (M+1).

### EXAMPLE 81

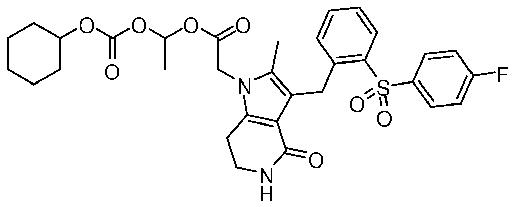

### 1-{[(Cyclohexyloxy)carbonyl]oxy}ethyl (3-{2-[(4-fluorophenyl)sulfonyl]benzyl}-2-methyl-4-oxo-4,5,6,7-tetrahydro-1H-pyrr olo[3,2-c]pyridin-1-yl)acetate

The title compound of Example 4 (150 mg, 0.33 mmol) and potassium carbonate (114 mg, 0.82 mmol) were suspended in 3 ml dimethylformamide. The title compound of Preparation 102 (163 mg, 0.79 mmol) was added and the mixture was stirred at room temperature overnight. Additional potassium carbonate (114 mg, 0.82 mmol) and title compound of Preparation 102 (163 mg, 0.79 mmol) and the mixture was stirred at room temperature for 6 h. The mixture was partitioned between ethyl acetate and water. The organics were washed sequentially three times with water and once with brine, dried over sodium sulphate, filtered and evaporated under reduced pressure. The residue was triturated with ether to give 120 mg (0.19 mmol, 58%) of the title compound as a white solid. Purity 96%.
¹H NMR (400 MHz, CHLOROFORM-d) δ ppm 8.21 (1 H, d, *J*=7.8 Hz), 7.95 (2 H, dd, *J*=8.8, 4.9 Hz), 7.41 (1 H, t, *J*=7.0 Hz), 7.32 (1 H, t, *J*=7.6 Hz), 7.18 (2 H, t, *J*=8.6 Hz), 7.06 (1 H, d, *J*=7.4 Hz), 6.76 (1 H, q, *J*=5.2 Hz), 4.96 (1 H, br. s.), 4.61 (1 H, tt, *J*=9.0, 4.5 Hz), 4.52 (2 H, s), 4.36 (2 H, s), 3.50 - 3.58 (2 H, m), 2.75 (2 H, t, *J*=6.8 Hz), 1.89 (3 H, s), 1.83 - 1.95 (2 H, m), 1.68 - 1.78 (1 H, m), 1.53 (3 H, d, *J*=5.5 Hz), 1.39 - 1.50 (2 H, m), 1.17 - 1.41 (4 H, m).
HPLC/MS (30 min) retention time 17.98 min.
LRMS: *m*/*z* 627 (M+1).

### EXAMPLE 82

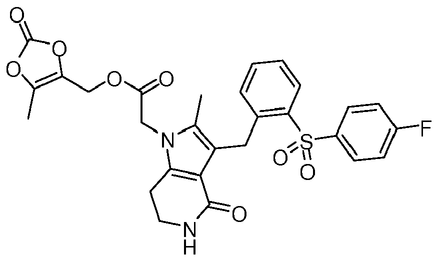

### (5-Methyl-2-oxo-1,3-dioxol-4-yl)methyl (3-{2-[(4-fluorophenyl)sulfonyl]benzyl}-2-methyl-4-oxo-4,5,6,7-tetrahydro-1H-pyrr olo[3,2-c]pyridin-1-yl)acetate

The title compound of Example 4 (150 mg, 0.33 mmol) and potassium carbonate (114 mg, 0.82 mmol) were suspended in 2 ml dimethylformamide. 4-(Chloromethyl)-5-methyl-1,3-dioxol-2-one (117 mg, 0.79 mmol) was added and the mixture was stirred at room temperature overnight. The mixture was partitioned between ethyl acetate and water. The organics were washed sequentially three times with water and once with brine, dried over sodium sulphate, filtered and evaporated under reduced pressure. The residue was purified by reverse-phase chromatography using the SP1 Purification system to give a solid. The solid was triturated with ether to give 140 mg (0.24 mmol, 75%) of the title compound. Purity 98%.
¹H NMR (400 MHz, CHLOROFORM-*d*) δ ppm 8.22 (1 H, d, *J*=7.0 Hz), 7.94 (2 H, dd, *J*=8.8, 4.9 Hz), 7.41 (1 H, t, *J*=6.8 Hz), 7.33 (1 H, t, *J*=7.2 Hz), 7.18 (2 H, t, *J*=8.6 Hz), 7.08 (1 H, d, *J*=7.4 Hz), 5.03 (1 H, br. s.), 4.94 (2 H, s), 4.56 (2 H, s), 4.37 (2 H, s), 3.55 (2 H, td, *J*=6.7, 2.5 Hz), 2.75 (2 H, t, *J*=6.8 Hz), 2.18 (3 H, s), 1.89 (3 H, s).
HPLC/MS (30 min) retention time 14.55 min.
LRMS: *m*/*z* 569 (M+1).

### EXAMPLE 83

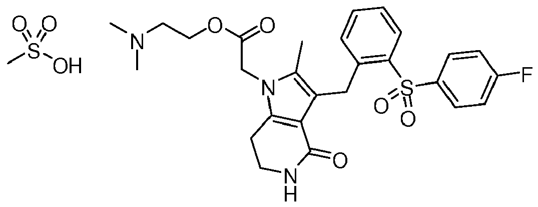

### 2-(Dimethylamino)ethyl (3-{2-[(4-fluorophenyl)sulfonyl]benzyl}-2-methyl-4-oxo-4,5,6,7-tetrahydro-1H-pyrr olo[3,2-c]pyridin-1yl)acetate mesylate salt

The title compound of Example 4 (200 mg, 0.44 mmol) was dissolved in 5 ml dichloromethane. 2-(Dimethylamino)ethanol (53 µl, 0.53 mmol), 4-dimethylamino-pyridine (27 mg, 0.22 mmol) and dicyclohexylcarbodiimide (126 mg, 0.61 mmol) were added and the mixture was stirred for 5 h at room temperature. The mixture was diluted with dichloromethane, the mixture was filtered and the filtrate evaporated under reduced pressure. The residue was purified by reverse-phase chromatography using the SP1 Purification system to give 128 mg (0.25 mmol, 55%) of the title compound free base.

The free base (128 mg, 0.25 mmol) was dissolved in 6 ml dry tetrahydrofuran. Methanesulfonic acid (16 µl, 0.25 mmol) was added, forming a waxy solid. A further 30 ml dry tetrahydrofuran was added and the mixture was sonicated to give a white precipitate. The precipitate was collected by filtration and was dried at 40 ºC under reduced pressure to give 43 mg (0.069 mmol, 27%) of the title compound as a pale yellow solid. Purity 93%.
¹H NMR (400 MHz, DMSO-*d*₆) δ ppm 9.53 (1 H, br. s.), 8.14 (1 H, d, *J*=7.4 Hz), 7.99 (2 H, dd, *J*=8.6, 5.1 Hz), 7.55 (1 H, t, *J*=7.2 Hz), 7.42 - 7.51 (2 H, m), 6.92 - 6.98 (2 H, m), 6.88 (1 H, br. s.), 4.79 (2 H, s), 4.41 (1 H, t, *J*=4.7 Hz), 4.18 (2 H, s), 3.38 - 3.44 (2 H, m), 3.34 (2 H, t, *J*=6.6 Hz), 2.80 (3 H, s), 2.79 (3 H, s), 2.71 (2 H, t, *J*=6.6 Hz), 2.32 (3 H, s), 1.55 (3 H, s).
HPLC/MS (30 min) retention time 8.71 min.
LRMS: *m*/*z* 528 (M+1).

### EXAMPLE 84

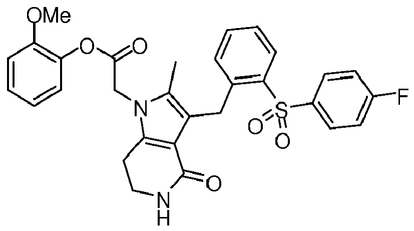

### 2-Methoxyphenyl (3-{2-[(4-fluorophenyl)sulfonyl]benzyl}-2-methyl-4-oxo-4,5,6,7-tetrahydro-1H-pyrr olo[3,2-c]pyridin-1-yl)acetate

The title compound of Example 4 (150 mg, 0.33 mmol) was dissolved in 3 ml dichloromethane. 2-Methoxyphenol (49 mg, 0.45 mmol), 4-dimethylamino-pyridine (20 mg, 0.16 mmol) and dicyclohexylcarbodiimide (95 mg, 0.46 mmol) were added and the mixture was stirred for 5 h at room temperature. The mixture was diluted with dichloromethane and was filtered. The filtrate was washed with water, dried over sodium sulphate, filtered and evaporated under reduced pressure. The residue was purified by reverse-phase chromatography using the SP1 Purification system to give 60 mg (0.11 mmol, 65%) of the title compound as a white solid. Purity 98%.
¹H NMR (partial, 400 MHz, DMSO-*d*₆) δ ppm 8.15 (1 H, d, *J*=7.4 Hz), 7.96 - 8.06 (2 H, m), 7.43 - 7.59 (4 H, m), 7.26 (1 H, t, *J*=7.0 Hz), 7.14 (2 H, t, *J*=7.4 Hz), 6.92 - 7.03 (2 H, m), 6.87 (1 H, br. s.), 5.06 (2 H, br. s.), 4.19 (2 H, br. s.), 3.73 (3 H, s), 2.77 (2 H, t, *J*=6.1 Hz), 1.61 (3 H, s).
HPLC/MS (30 min) retention time 16.12 min.
LRMS: *m*/*z* 563 (M+1).

### PHARMACOLOGICAL ACTIVITY

### CRTh2 radioligand binding assay

The assay is performed using membranes obtained from CHO.K1 cells stably overexpressing the CRTh2 receptor and ³[H]PGD₂ as radioligand. The assay is performed incubating 2-4 µg of membranes per well with the compound to be tested and 5 nM ³[H]PGD₂ in incubation buffer (50 mM HEPES pH 7.0, 10 mM MgCl₂, 1 mM EDTA, 0.2% BSA) for 2 h at room temperature. The reaction is terminated by filtering in GF/C plates pre-treated with 0.3% de polyetilenimine buffer and washing 3 times with wash buffer (50 mM HEPES pH 7.4, 0.5 M NaCl). After washing, plates are dried and scintillation cocktail Optiphase Hisafe II added. The radioactivity retained in the filter is counted using a Microbeta liquid scintillation counter. Compound IC₅₀s are determined using Excel XL-fit for calculations.

### CRTh2 GTPγS antagonism binding assay

The assay is performed using membranes obtained from CHO.K1 cells stably overexpressing the CRTh2 receptor and [³⁵S]-GTPγS as radioligand. Assay is performed pre-incubating 4-8 µg of membranes per well with the compound to be tested for 1 h, followed by incubation with PGD₂ at 50 nM (EC₈₀), 0.1 nM [³⁵S]-GTPγS in incubation buffer (20 mM HEPES, 10 mM MgCl₂, 100 mM NaCl, 10 µM GDP, 10 µg/ml Saponine and 0.2% BSA) for 2 h at room temperature. The reaction is terminated by filtering in GF/C plates pre-treated with 20 mM HEPES, 10 mM MgCl₂, 100 mM NaCl and washing 6 times with wash buffer (20 mM NaH₂PO4, 20 mM Na₂HPO4). After washing, plates are dried and scintillation buffer Optiphase added. The radioactivity retained in the filter is counted using a Microbeta liquid scintillation counter. Compound IC₅₀s are determined using Excel XL-fit for calculations.

### CRTh2 Isolated cell eosinophil cell shape assay

Citrated whole blood is obtained from consenting donors and polymorphonuclear leukocytes obtained by gradient centrifugation on Polymorphprep for 40 min at 500G brake off. PMN fraction is resuspended in calcium free buffer (10 mM HEPES, 10 mM glucose, 0.1 % BSA in calcium free PBS) and centrifuged for 10 min at 400G at room temperature. Lysis of red blood cells is performed by adding 20 ml of 0.2% saline buffer (0.2% NaCl) for 40 s and stopped by adding 20 ml of 1.6% saline buffer (1.6% NaCl). For compound treatment 90 µl of PMNs cells resuspended at a density of 5 x 10⁶ cells/ml in 10 mM HEPES, 10 mM glucose, 0.1 % BSA in calcium containing PBS are incubated for 10 min with 10 µl of compound to be tested followed by incubation with 10µl of 500 nM PGD₂ for exactly 4 min at 37°C. Fixation is performed by addition of 0.2 ml of ice-cold 1:5 diluted CellFix (BD Biosciences) and immediately analysed on a FACSCalibur (BD Biosciences). Eosinophils are gated on the basis of autofluorescence in the FL2 channels and shape change of 600 cells is assayed by forward scatter and side scatter analysis. Compound IC₅₀s are determined using Excel XL-fit for calculations.

### CRTh2 whole blood eosinophil cell shape assay

Whole blood is obtained from consenting donors mixed with heparin and kept in rotation until use. For compound treatment, 90 µl of blood are mixed with 10 µl of compound to be tested and incubated for 10 min at room temperature followed by addition of 10 µl of 500 nM PGD₂ for exactly 4 min at 37ºC. Reaction was stopped by placing the tubes on ice and adding of 0.25 ml of ice-cold 1:5 diluted CellFix (BD Biosciences). Red blood cells are lysed in two steps by adding 2 ml of lysis solution (150mM ammonium chloride, 10mM potassium hydrogencarbonate), incubating for 20 and 10 min respectively and centrifuging at 300G for 5 min at 6ºC. The pellet is resuspended in 0.2 ml of fixative solution and immediately analized on a FACSCalibur (BD Biosciences). Eosinophils are gated on basis of autofluorescence in the FL2 channels and shape change of 600 cells is assayed by forward scatter and side scatter analysis. Compound IC₅₀s are determined using Excel XL-fit for calculations.

Preferred compounds of the invention show a IC₅₀ value for the inhibition of CRTH2 (determined as defined above) of less than 1 µM, preferably less than 100 nM and most preferably less than 20 nM.

| **Example** | **GTPγ Binding (nM)** |
|---|---|
| 2 | 4.6 |
| 4 | 3.2 |
| 6 | 3.6 |
| 8 | 4 |
| 10 | 13 |
| 12 | 6.2 |
| 14 | 8.4 |
| 15 | 3.9 |
| 16 | 6.9 |
| 17 | 3.6 |
| 20 | 9.2 |
| 21 | 20 |
| 23 | 27 |
| 24 | 20 |
| 27 | 5.4 |
| 28 | 11 |
| 29 | 18 |
| 31 | 9.5 |
| 34 | 12 |
| 36 | 1.5 |
| 37 | 3.7 |
| 39 | 2.9 |
| 42 | 3.9 |
| 44 | 5.9 |
| 45 | 9 |
| 58 | 4.5 |
| 60 | 5 |
| 62 | 4.8 |
| 71 | 5.8 |
| 72 | 8.6 |
| 75 | 4.9 |

### COMBINATION PRODUCT

The pyrazole derivatives of the invention may also be combined with other active compounds in the treatment of diseases indicated above. For example the compounds of the present invention can be combined with active substances which are known to be useful in the treatment of these diseases.

Examples of such active substances are (a) anticholinergics, such as Aclidinium Bromide or Tiotropium, (b) corticosteroids, or gluococorticoids such as prednisone or methylprednisolone; (c) antihistamines, such as Ebastine, Ranitidine, ABT-239 or JNJ 7777120, (d) beta-2 agonists, such as salmeterol or formoterol, (e) chemokine receptor antagonists, such as Maraviroc or Enfuvirtide, (f) Leukotriene receptor antagonists, (g) JAK inhibitors such as tasocitinib citrate or INCB018424, (h) Syk inhibitors such as R-112, (i) Phosphosdiesterase IV inhibitors such as GRC-4039, (j) Dual beta-2 adrenoceptor agonist/M3 receptor antagonist (MABA compounds) such as GSK-961081, (k) p38 Inhibitors such as ARRY-797; (I) PKC inhibitors such as NVP-AEB071; (m) 5-lipoxygenase activating protein inhibitors, such as Veliflapon; (n) 5-lipoxygenase inhibitors, (o) CYSLTR1 antagonists, such as Montelukast, Pranlukast or Zafirlukast; (p) CYSLTR2 antagonists, such as Pranlukast, Zafirlukast or Tipilukast; (q) BLT1 antagonists, (r) BLT2 antagonists, (s) Thromboxane A2 antagonists such as Ramatroban; (t) DP1 receptor antagonists, such as Laropiprant; (u) DP1 receptor agonists, such as BW-245C; (v) IP receptor agonists, such as RO-1138452; (w) anti-IgE, such as Omalizumab; (x) IL5 antibody, such as Mepolizumab; (y) leukotriene formation inhibitors, (z) bronchodilators, such as pirbuterol, epinephrine, ephedrine, salbutamol or salmeterol; (aa) decongestants, such as ephedrine, Levo-methamphetamine, Naphazoline, Oxymetazoline, Phenylephrine, Phenylpropanolamine, Propylhexedrine, Pseudoephedrine, Synephrine or Tetrahydrozoline; (bb) mucolytics such as Acetylcysteine, Ambroxol, Bromhexine, Carbocisteine, Domiodol, Eprazinone, Erdosteine, Letosteine, Neltenexine, Sobrerol, Stepronin or Tiopronin; (cc) antitussives, such as dectromethorphan; (dd) analgesics such as aspirin, paracetamol, rofecoxid, celecoxib, morphine, codeine, oxycodone, hydrocodone, dihydromorphine or flupirtine and (ee) Expectorants such Antimony pentasulfide, Guaiacolsulfonate, Guaifenesin, Potassium iodide or Tyloxapol.

Accordingly, another embodiment of the invention is a combination product comprising (i) at least a compound of formula (I) as defined previously, and (ii) one or more active ingredients as described above, for simultaneous, separate or sequential use in the treatment of the human or animal body.

The combinations of the invention may be used in the treatment of disorders which are susceptible to amelioration by CRTh2 receptor antagonists. Thus, the present application encompasses methods of treatment of these disorders, as well as the use of the combinations of the invention in the manufacture of a medicament for the treatment of these disorders.

Preferred examples of such disorders are asthma, chronic obstructive pulmonary disease (COPD), allergic rhinitis, atopic dermatitis, psoriasis, emphysema, eosinophilic bronchitis, being more preferably asthma, chronic obstructive pulmonary disease (COPD), allergic rhinitis and atopic dermatitis.

The amount of each active which is required to achieve a therapeutic effect will, of course, vary with the particular active, the route of administration, the subject under treatment, and the particular disease or disorder being treated.

The active ingredients may be administered from 1 to 6 times a day, sufficient to exhibit the desired activity. Preferably, the active ingredients are administered once or twice a day, most preferably once a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-α-day dosing occurred, or separately. Preferably at least two, and more preferably all, of the actives would be taken together at the same time. Preferably, at least two, and more preferably all actives would be administered as an admixture.

Examples of suitable β-agonists that can be combined with the compounds of formula (I) are terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, formoterol, (R,R)-Formoterol tartrate; Arformoterol tartrate, sulfonterol, Bedoradrine sulphate, Indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in PCT patent applications Nos. WO 2007/124898, WO 2006/122788A1, WO 2008/046598, WO 2008095720, WO 2009/068177 and WO 2010/072354.

Examples of suitable corticosteroids and glucocorticoids that can be combined with the compounds of formula (I) are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RS-85095, CGP-13774, GW-250495, deltacortisone, NO-Prednisolone, etiprednol dicloacetate, QAE-397, 7beta-OH-EPIA, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acetoxy]-4-pregnene-3,20-dione, Desisobutyrylciclesonide, hydrocortisone acetate, hydrocortisone sodium succinate, prednisolone sodium phosphate and hydrocortisone probutate, Prednisolone sodium metasulfobenzoate and clobetasol propionate.

Examples of suitable anticholinergics that can be combined with the compounds of formula (I) are tiotropium salts, oxitropium salts, flutropium salts, ipratropium salts, glycopyrronium salts, trospium salts, zamifenacin, revatropate, espatropate, darotropium bromide, CI-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1 (R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable anti-histamines that can be combined with the compounds of formula (I) are Methapyrilene, Mequitazine, Azelastine hydrochloride, Ranitinide, Acrivastine, Emedastine difumarate, Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride, Epinastine hydrochloride, Olopatadine hydrochloride, Bepotastine besilate,Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, ABT-239, JNJ7777120, Vapitadine hydrochloride, AZD-1744, GSK-1004723D, GSK-835726, SUN-1334H.

Examples of suitable PDE4 inhibitors that can be combined with the compounds of formula (I) are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, revamilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1 H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, MK-0873, CDC-801, UK-500001, BLX-914, 2-carbomethoxy-4-cyano-4-(3-cyclopropylmethoxy-4-difluroromethoxyphenyl)cyclohexan1-one, cis [4-cyano-4-(3-cyclopropylmethoxy-4-difluoromethoxyphenyl)cyclohexan-1-ol, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the PCT patent applications number WO 03/097613, WO 2004/058729, WO 2005/049581, WO 2005/123693, WO 2005/123692, and WO 2010/069504.

Examples of suitable Syk kinase inhibitors that can be combined with the compounds of formula (I) are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Particularly preferred combination products according to the invention comprise a compound of formula (I) and a therapeutically effective amount of one or more additional therapeutic agents selected from the group consisting of mometasone furoate, ciclesonide, budesonide, fluticasone propionate, fluticasone furoate, betamethasone valerate, clobetasol propionate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, formoterol, salmeterol, indacaterol, carmoterol, LAS 100977, compounds described in PCT patent applications Nos. WO 2008/077639, WO 2009/021696, WO 2009/153043, and WO 2010/083975 (in particular amino(iso)nicotinic acid derivatives selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid and 2-(3,5-difluoro-2-methylbiphenyl-4-ylamino)nicotinic acid; and azabiphenylaminobenzoic acid derivatives selected from the group consisting of 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid and 5-methyl-2-((6-(2,3-difluorophenyl)pyridin-3-yl)amino)benzoic acid), methapyrilene, cetirizine, loratadine, ebastine, desloratadine, fexofenadine, azelastine, levocabastine, olopatadine, Montelukast, picremolimus, tacrolimus, mupiricin, retapamulin, clotrimazole, ketoconazole and terbinafine.

Preferred combination products according to the invention comprises a compound of formula (I) and a therapeutically effective amount of one or more additional therapeutic agents selected from the group consisting of mometasone furoate, ciclesonide, budesonide, fluticasone propionate, fluticasone furoate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, rolipram, roflumilast, cilomilast and the compounds claimed in the PCT patent applications number WO 03/097613, WO 2004/058729, WO 2005/049581, WO 2005/123693, WO 2005/123692, and WO 2010/069504.

Thus, in one aspect of the invention, the combination product comprises a compound of formula (I) and a corticosteroid. Particularly preferred corticosteroids are those selected from the group consisting of mometasone furoate, ciclesonide, budesonide, fluticasone furoate, fluticasone propionate, betamethasone valerate and clobetasol propionate.

In another aspect of the invention, the combination product comprises a compound of formula (I) and an anticholinergic agent. Particulary preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]-octane salts. The combination product may further comprise a corticosteroid selected from the group consisting of mometasone furoate, ciclesonide, budesonide, fluticasone furoate and fluticasone propionate.

In a still other aspect of the invention, the combination product comprises a compound of formula (I) and a suitable β2-agonist. Particularly preferred β2-agonists are those selected from the group consisting of formoterol, salmeterol, indacaterol, carmoterol, LAS100977 and compounds described in PCT patent applications Nos. WO 2007/124898, WO 2006/122788A1, WO 2008/046598, WO 2008095720, WO 2009/068177 and WO 2010/072354. The composition may further comprise a corticosteroid selected from the group consisting of mometasone furoate, ciclesonide, budesonide, fluticasone furoate, and fluticasone propionate. In addition to the compound of formula (I) and to the β2-agonist, the combination product may further comprise an anticholinergic agent selected from the group consisting of tiotropium salts, glycopyrronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

In a still other aspect of the invention, the combination product comprises a compound of formula (I) and a PDE4 inhibitor. Particularly preferred PDE4 inhibitors are those selected from the group consisting of rolipram, roflumilast, cilomilast and the compounds claimed in the PCT patent applications number WO 03/097613, WO 2004/058729, WO 2005/049581, WO 2005/123693, WO 2005/123692, and WO 2010/069504. The combination product may further comprise a corticosteroid selected from the group consisting of mometasone furoate, ciclesonide, budesonide, fluticasone furoate and fluticasone propionate. In addition to the compound of the invention and to the PDE4 inhibitor, the combination product may further comprise an anticholinergic agent selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

In a preferred embodiment of the present invention, the combination product comprises a compound of formula (I) and a therapeutically effective amount of a 3-[2-Hydroxy-2,2-bis(2-thienyl)acetoxy]-1-(3-phenoxypropyl)-1-azoniabicyclo[2.2.2]octane salts. Optionally, the combination product further comprises a β2 agonist, a corticosteroid and/or a PDE4 inhibitor.

In another preferred embodiment of the present invention, the combination product comprises a compound of formula (I) and a therapeutically effective amount of a mometasone furoate. Optionally, the combination product further comprises a β2 agonist, an anticholinergic salt and/or a PDE4 inhibitor.

In another preferred embodiment of the present invention, the combination product comprises a compound of formula (I) and a therapeutically effective amount of a formoterol or LAS-100977. Optionally, the combination product further comprises a corticosteroid, an anticholinergic salt and/or a PDE4 inhibitor.

The active compounds in the combination product may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be administered in the morning and the other (s) later in the day. Or in another scenario, one or two actives could be administered twice daily and the other (s) once daily, either at the same time as one of the twice-a-day dosing occurred, or separately. Preferably at least two, and more preferably all, of the actives would be administered together at the same time. Preferably, at least two, and more preferably all actives would be administered as an admixture.

### PHARMACEUTICAL COMPOSITIONS

Pharmaceutical compositions according to the present invention comprise the compounds of the invention in association with a pharmaceutically acceptable diluent or carrier.

As used herein, the term pharmaceutical composition refers to a mixture of one or more of the compounds described herein, or physiologically/pharmaceutically acceptable salts, solvates, N-oxides, stereoisomers, deuterated derivatives thereof or prodrugs thereof, with other chemical components, such as physiologically/pharmaceutically acceptable carriers and excipients. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

A pharmaceutically acceptable excipient refers to an inert substance added to a pharmaceutical composition to further facilitate administration of a compound.

The invention further provides pharmaceutical compositions comprising the compounds of the invention in association with a pharmaceutically acceptable diluent or carrier together with one or more other therapeutic agents such as the previously described for use in the treatment of a pathological condition or disease susceptible to amelioration by CRTh2 receptor antagonists.

The invention is also directed to pharmaceutical compositions of the invention for use in the treatment of a pathological disease or disorder susceptible to amelioration by CRTh2 receptor antagonists, in particular wherein the pathological disease or disorder is as described above.

The invention also provides a method of treatment of a pathological condition or disease susceptible to amelioration by CRTh2 receptor antagonists in particular wherein the pathological condition or disease described above, comprising administering a therapeutically effective amount of a pharmaceutical composition of the invention.

The present invention also provides pharmaceutical compositions which comprise, as an active ingredient, at least a compound of formula (I) or a pharmaceutically acceptable salt, solvate, N-oxide or deuterated derivative thereof in association with a pharmaceutically acceptable excipient such as a carrier or diluent. The active ingredient may comprise 0.001% to 99% by weight, preferably 0.01% to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

Pharmaceutical compositions suitable for the delivery of compounds of the invention and methods for their preparation will be readily apparent to those skilled in the art. Such compositions and methods for their preparation can be found, for example, in Remington: The Science and Practice of Pharmacy, 21 st Edition, Lippincott Williams & Wilkins, Philadelphia, Pa., 2001.

The pharmaceutically acceptable excipients which are admixed with the active compound or salts of such compound, to form the compositions of this invention are well-known per se and the actual excipients used depend inter alia on the intended method of administering the compositions. Examples, without limitation, of excipients include calcium carbonate, calcium phosphate, various sugars and types of starch, cellulose derivatives, gelatin, vegetable oils and polyethylene glycols.

Additional suitable carriers for formulations of the compounds of the present invention can be found in Remington: The Science and Practice of Pharmacy, 21 st Edition, Lippincott Williams & Wilkins, Philadelphia, Pa., 2001.

### i) Oral Administration

The compounds of the invention may be administered orally (peroral administration; per os (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y.,, 1980. Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule. Solid formulations for oral administration may be formulated to be immediate and/or modified release. Modified release formulations include delayed-, sustained-, pulsed-, controlled-, targeted and programmed release. Suitable modified release formulations are described in U.S. Patent No. 6,106,864. Details of other suitable release technologies such as high energy dispersions and osmotic and coated particles can be found in Verma et al, Pharmaceutical Technology On-line, 25(2),1-14 (2001). The use of chewing gum to achieve controlled release is described in WO 00/35298. The disclosures of these references are incorporated herein by reference in their entireties.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.

Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tetrafluoroethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-100 mg, more preferably 0.01-5 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000703, WO03/000325 and W02006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the Respimat® which is described, for example, in PCT Patent Applications Nos. WO 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. Particles having a size above 20 µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

### iv) Nasal mucosal administration

The compounds of the invention may also be administered via the nasal mucosal.

Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/Intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable {e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

The amount of the active compound administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Preferably the composition is in unit dosage form, for example a tablet, capsule or metered aerosol dose, so that the patient may administer a single dose.

The following preparations forms are cited as formulation examples:

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Examples 5 (Formulation for a MDI)

| **Ingredient** | **Amount** |
|---|---|
| Active Compound (micronized) | 10g |
| 1,1,1,2,3,3,3-heptafluoro-n-propane | q.s. to 200 ml |

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, solvate, N-oxide, stereoisomer or deuterated derivative thereof: 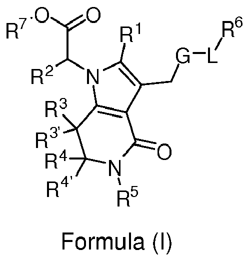 wherein:
• R¹ is selected from the group consisting of a linear or branched C₁₋₄ alkyl group and a C₃₋₇ cycloalkyl group;
• R² is selected from the group consisting of a hydrogen atom and a linear or branched C₁₋₄ alkyl group;
• R³, R^{3'}, R⁴ and R^{4'} are independently selected from the group consisting of a hydrogen atom and a linear or branched C₁₋₄ alkyl group;
or R³ and R^{3'} together with the carbon atom to which they are attached form a C₃₋₇ cycloalkyl group and R⁴ and R^{4'} are independently selected from the group consisting of a hydrogen atom and a linear or branched C₁₋₄ alkyl group;
or R⁴ and R^{4'} together with the carbon atom to which they are attached form a C₃₋₇ cycloalkyl group and R³ and R^{3'} are independently selected from the group consisting of a hydrogen atom and a linear or branched C₁₋₄ alkyl group;
or R³ together with R⁴ form an additional bond and R^{3'} and R^{4'} are independently selected from the group consisting of a hydrogen atom and a linear or branched C₁₋₄ alkyl group;
• R⁵ is selected from the group consisting of a hydrogen atom, a linear or branched C₁₋₄ alkyl group; a linear or branched C₁₋₄ haloalkyl group and a benzyl group;
• R⁶ is selected from the group consisting of a hydrogen atom, a linear or branched C₁₋₄ alkyl group, a C₃₋₇ cycloalkyl group, a benzyl group, a -N(R^{b})COR^{a} group, a C₆₋₁₀ aryl group, a 5- to 10-membered , mono- or bicyclic heteroaryl group containing at least one heteroatom selected from N, S and O, and a 5- to 10-membered, mono- or bicyclic heterocyclyl group containing at least one heteroatom selected from N, S and O;
wherein the aryl, heteroaryl and heterocyclyl groups independently are optionally substituted by one or more substituents selected from the group consisting of a halogen atom, a cyano group, a nitro group, a -CF₃ group, a linear or branched C₁₋₄ alkyl group, a C₃₋₇ cycloalkyl group, a -CO₂H group, a -CO₂R^{a} group, a -CONR^{d}R^{e} group, a -NR^{d}R^{e} group, a -NCOR^{a} group, a -NCO₂R^{a} group, a -NCONR^{d}R^{e} group, a -NSO₂R^{a} group, a -NSO₂NR^{d}R^{e} group, a -OH group, a -OR^{a} group, -SR^{a} group, a -SOR^{a} group, a -SO₂R^{a} group and a -SO₂NR^{d}R^{e} group;
• L is selected from the group consisting of a direct bond, a -CR^{b}R^{c}-, -O-, -CH(OH)-, -CO-, -S-, -SO-, -SO₂- and -SO₂N(CH₃)-;
• G is selected from a C₆₋₁₀ aryl group and a 5- to 10-membered, mono- or bicyclic heteroaryl group containing at least one heteroatom selected from N, S and O; wherein the aryl group and the heteroaryl groups independently are optionally substituted by one or more substituents selected from the group consisting of a halogen atom, a cyano group, a nitro group, a -CF₃ group, a linear or branched C₁₋₄ alkyl group, a C₃₋₇ cycloalkyl group, a -CO₂H group, a -CO₂R^{a} group, a -CONR^{d}R^{e} group, a -NR^{d}R^{e} group, a -NCOR^{a} group, a -NCO₂R^{a} group, a -NCONR^{d}R^{e} group, a -NSO₂R^{a} group, a -NSO₂NR^{d}R^{e} group, a -OH group, a -OR^{a} group, -SR^{a} group, a -SOR^{a} group, a -SO₂R^{a} group and a -SO₂NR^{d}R^{e} group;
• R⁷ is selected from the group consisting of a hydrogen atom, a linear or branched C₁₋₄ alkyl group, a mono- or bicyclic saturated or unsaturated C₃₋₁₀ cycloalkyl group, a C₁₋₄ alkoxy-C₁₋₄ alkyl, a -(CH₂)₂O(CH₂)₂OCH₃ group, a -CH₂CH(OH)CH₂OH group, a phenyl group, a benzyl group, a -CH₂(CHR^{a})₀₋₁CO₂H group, a -CH₂CONR^{d}R^{e} group, a -CHR^{b}OC(O)R^{a} group, a -CHR^{b}OC(O)O-cyclohexyl group, a -(CH₂)₂NR^{f}R^{g} group, a -(CH₂)₀₋₁(CR^{b}R^{c})CH₂NR^{f}R^{g} and a -(CH₂)₀₋₁-(saturated or unsaturated 5- to 10-membered heterocyclyl) group;
wherein the cycloalkyl, phenyl, benzyl and heterocyclyl groups are optionally substituted by one or more substituents selected from the group consisting of a C₁₋₄ alkyl group, a C₁₋₄ alkoxy group, an oxo group, a halogen atom, a carboxy group and a benzyl group;
• R^{a} is selected from the group consisting of a linear or branched C₁₋₄ alkyl group, a -CF₃ group, a C₃₋₇ cycloalkyl group, a 3 to 7-membered heterocycyl group containing at least one heteroatom selected from N, S and O, a -(C₁₋₄ alkyl)-(C₆₋₁₀ aryl) group, a C₆₋₁₀ aryl group and a 5- to 10-membered heteroaryl group containing at least one heteroatom selected from N, S and O; wherein the aryl and the heteroaryl groups are optionally substituted by one or more substituents selected from halogen atom, a C₁₋₂ alkyl group or a cyano group;
• R^{b}, R^{c}, R^{d} and R^{e} are independently selected from the group consisting of a hydrogen atom, a linear or branched C₁₋₄ alkyl group, a C₃₋₇ cycloalkyl group and a 3- to 7-membered heterocyclyl group containing at least one heteroatom selected from N, S and O;
or R^{b} and R^{c} together with the carbon atom to which they are attached form a 3-C₃₋₇ cycloalkyl or a 3- to 7-membered heterocycyl group containing one additional heteroatom selected from N, S and O;
or R^{d} and R^{e} together with the nitrogen atom to which they are attached form a 3- to 7-membered N-containing heterocyclyl group and optionally containing one additional heteroatom selected from N, S and O;
• R^{f} and R^{g} are independently selected from the group consisting of a hydrogen atom, a C₁₋₄ alkyl group, a phenyl group, a diphenylpropyl group, a -CO-pyridyl group and a benzyl group optionally substituted by a halogen atom; or R^{f} and R^{g} together with the nitrogen atom to which they are attached form a 6-membered N-containing heterocyclyl group and optionally containing one additional heteroatom selected from N, S and O.

2. A compound according to claim 1, wherein R¹ represents a -CH₃ group or a cyclopropyl group.

3. A compound according to claim 1 or 2, wherein R³, R^{3'} , R⁴ and R^{4'} are independently selected from the group consisting of a hydrogen atom and a -CH₃ group; or R³ together with R⁴ form an additional bond and R^{3'} and R^{4'} are independently selected from the group consisting of a hydrogen atom and a -CH₃ group.

4. A compound according to any one of claims 1 to 3, wherein R⁵ represents a hydrogen atom, a -CH₃ group, an isopropyl group, a -CHF₂ group, a -CH(CH₃)CF₃ group or a benzyl group.

5. A compound according to any one of claims 1 to 4, wherein L represents -SO₂-, -SO₂NCH₃ or -CH(OH)-, preferably -SO₂-.

6. A compound according to any one of claims 1 to 5, wherein G represents a phenyl group, a pyrrolyl group or a quinolinyl group, wherein the phenyl, pyrrolyl and quinolinyl groups are optionally substituted by one or more substitutents selected from the group consisting of a halogen atom, a cyano group, a -CF₃ group, a -CO₂H group, a -CO₂R^{a} group, a -CONR^{d}R^{e} group and a -OR^{a} group.

7. A compound according to any one of claims 1 to 6, wherein R⁶ represents a phenyl group, a benzyl group, a cyclohexyl group or a morpholinyl group, wherein the phenyl, benzyl, cyclohexyl and morpholinyl groups are optionally substituted by one or more substituents selected from the group consisting of a halogen atom and a -OR^{a} group.

8. A compound according to any one of claim 1 to 7, wherein R⁷ represents a hydrogen atom, a linear or branched C₁₋₄ alkyl group, a -CH₂CONR^{d}R^{e} group, a -(CH₂)₂NR^{f}R^{g} group, a -CHR^{b}OC(O)O-cyclohexyl group, a phenyl group, a mono- or bicyclic saturated or unsaturated C₆₋₁₀ cycloalkyl group and a -(CH₂)-(saturated or unsaturated 5-membered heterocyclyl) group, wherein the phenyl and heterocyclyl groups are optionally substituted by one or more substituents selected from the group consisting of a C₁₋₄ alkyl group, a C₁₋₄ alkoxy group and an oxo group.

9. A compound according to any one of the preceding claims, wherein
• R¹ represents a -CH₃ group or a cyclopropyl group;
• R³, R^{3'} , R⁴ and R^{4'} are independently selected from the group consisting of a hydrogen atom and a -CH₃ group; or R³ together with R⁴ form an additional bond and R^{3'} and R^{4'} are independently selected from the group consisting of a hydrogen atom and a -CH₃ group;
• R⁵ represents a hydrogen atom, a -CH₃ group, an isopropyl group, a -CHF₂ group, a -CH(CH₃)CF₃ group or a benzyl group;
• L represents -SO₂-, -SO₂NCH₃ or -CH(OH)-, preferably -SO₂-;
• G represents a phenyl group, a pyrrolyl group or a quinolinyl group, wherein the phenyl, pyrrolyl and quinolinyl groups are optionally substituted by one or more substitutents selected from the group consisting of a halogen atom, a cyano group, a -CF₃ group, a -CO₂H group, a -CO₂R^{a} group, a -CONR^{d}R^{e} group and a -OR^{a} group;
• R⁶ represents a phenyl group, a benzyl group, a cyclohexyl group or a morpholinyl group, wherein the phenyl, benzyl, cyclohexyl and morpholinyl groups are optionally substituted by one or more substituents selected from the group consisting of a halogen atom and a -OR^{a} group; and
• R⁷ represents a hydrogen atom, a linear or branched C₁₋₄ alkyl group, a -CH₂CONR^{d}R^{e} group, a -(CH₂)₂NR^{f}R^{g} group, a -CHR^{b}OC(O)O-cyclohexyl group, a phenyl group, a mono- or bicyclic saturated or unsaturated C₆₋₁₀ cycloalkyl group and a -(CH₂)-(saturated or unsaturated 5-membered heterocyclyl) group, wherein the phenyl and heterocyclyl groups are optionally substituted by one or more substituents selected from the group consisting of a C₁₋₄ alkyl group, a C₁₋₄ alkoxy group and an oxo group.

10. A compound according to claim 1, wherein
• R¹ represents a -CH₃ group or a cyclopropyl group;
• R² represents a hydrogen atom or a -CH₃ group;
• R³, R^{3'}, R⁴ and R^{4'} are independently selected from the group consisting of a hydrogen atom and a -CH₃ group; or R³ together with R⁴ form an additional bond and R^{3'} and R^{4'} are independently selected from the group consisting of a hydrogen atom and a -CH₃ group;
• R⁵ represents a hydrogen atom, a -CH₃ group, an isopropyl group, a -CHF₂ group, a -CH(CH₃)CF₃ group or a benzyl group;
• L represents a direct bond, -CH₂-, -O-, -CH(OH)-, -CO-, -SO₂- and -SO₂N(CH₃)-;
• G represents a phenyl group, a pyrrolyl group or a quinolinyl group, wherein the phenyl, pyrrolyl and quinolinyl groups are optionally substituted by one or more substitutents selected from the group consisting of a fluorine atom, a bromine atom, a cyano group, a -CF₃ group, a -CO₂H group, a -CO₂CH₃ group, a -CONHCH₃ group and a -OCH₃ group;
• R⁶ represents a hydrogen atom, a -CH₃ group, a -CH₂CH₃ group, a phenyl group, a benzyl group, a cyclohexyl group, a morpholinyl group, a triazolyl group, a naphthyl group and a -N(Et)C(O)cyclopropyl group, wherein the phenyl group is optionally substituted by one or more substituents selected from the group consisting of a fluorine atom, a chlorine atom, a -OCF₃ group, a - OCH₃ group and a -CON(CH₃)₂ group; and
• R⁷ represents a hydrogen atom, a -CH₃ group, a -CH₂CH₃ group, a tert-butyl group, a -CH₂CON(CH₃)₂ group, a -(CH₂)₂N-morpholinyl group, -(CH₂)₂N(CH₃)₂ group, a -CH(CH₃)OC(O)O-cyclohexyl group, a 2-methoxyphenyl group, a 2,3-dihydro-1H-inden-5-yl group and a (5-methyl-2-oxo-1,3-dioxol-4-yl)methyl group.

11. A compound according to claim 1 which is one of:
• Ethyl 2-(2-methyl-4-oxo-3-(2-(phenylsulfonyl)benzyl)-4,5,6,7-tetrahydro-1H-pyrrolo-[3,2-c]pyridin-1-yl)acetate;
• {2-Methyl-4-oxo-3-[2-(phenylsulfonyl)benzyl]-4,5,6,7-tetrahydro-1H-pyrrolo-[3,2-c]pyridin-1-yl}acetic acid;
• Ethyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
• (3-{2-[(4-Fluorophenyl)sulfonyl]benzyl}-2-methyl-4-oxo-4,5,6,7-tetrahydro-1H-pyrrolo[3,2-c]pyridin-1-yl)acetic acid;
• Ethyl {3-[5-methoxy-2-(phenylsulfonyl)benzyl]-2-methyl-4-oxo-4,5,6,7-tetrahydro-1H-pyrrolo[3,2-c]pyridin-1-yl}acetate;
• {3-[5-Methoxy-2-(phenylsulfonyl)benzyl]-2-methyl-4-oxo-4,5,6,7-tetrahydro-1H-pyrrolo[3,2-c]pyridin-1-yl}acetic acid;
• Ethyl (3-{2-[(3-chlorophenyl)sulfonyl]benzyl}-2-methyl-4-oxo-4,5,6,7-tetrahydro-1H-pyrrolo[3,2-c]pyridin-1-yl)acetate;
• (3-{2-[(3-Chlorophenyl)sulfonyl]benzyl}-2-methyl-4-oxo-4,5,6,7-tetrahydro-1H-pyrrolo[3,2-c]pyridin-1-yl)acetic acid;
• Ethyl {2-methyl-4-oxo-3-[2-(phenylsulfonyl)-4-(trifluoromethyl)benzyl]-4,5,6,7-tetrahydro-1H-pyrrolo[3,2-c]pyridin-1-yl}acetate;
• {2-Methyl-4-oxo-3-[2-(phenylsulfonyl)-4-(trifluoromethyl)benzyl]-4,5,6,7-tetrahydro-1H-pyrrolo[3,2-c]pyridin-1-yl}acetic acid;
• Ethyl (3-{2-[(3-fluorophenyl)sulfonyl]benzyl}-2-methyl-4-oxo-4,5,6,7-tetrahydro-1H-pyrrolo[3,2-c]pyridin-1-yl)acetate;
• (3-{2-[(3-Fluorophenyl)sulfonyl]benzyl}-2-methyl-4-oxo-4,5,6,7-tetrahydro-1H-pyrrolo[3,2-c]pyridin-1-yl)acetic acid;
• {2-Methyl-3-[4-(methylsulfonyl)benzyl]-4-oxo-4,5,6,7-tetrahydro-1H-pyrrolo-[3,2-c]pyridin-1-yl}acetic acid;
• (3-{2-[(4-Chlorophenyl)sulfonyl]benzyl}-2-methyl-4-oxo-4,5,6,7-tetrahydro-1H-pyrrolo[3,2-c]pyridin-1-yl)acetic acid;
• (3-{2-[(4-Methoxyphenyl)sulfonyl]benzyl}-2-methyl-4-oxo-4,5,6,7-tetrahydro-1H-pyrrolo[3,2-c]pyridin-1-yl)acetic acid;
• [2-Methyl-4-oxo-3-(2-{[4-(trifluoromethoxy)phenyl]sulfonyl}benzyl)-4,5,6,7-tetrahydro-1H-pyrrolo[3,2-c]pyridin-1-yl]acetic acid;
• (3-{2-[(2-Fluorophenyl)sulfonyl]benzyl}-2-methyl-4-oxo-4,5,6,7-tetrahydro-1H-pyrrolo[3,2-c]pyridin-1-yl)acetic acid;
• {3-[2-({3-[(Dimethylamino)carbonyl]phenyl}sulfonyl)benzyl]-2-methyl-4-oxo-4,5,6,7-tetrahydro-1H-pyrrolo[3,2-c]pyridin-1-yl}acetic acid;
• (2S)-2-{2-Methyl-4-oxo-3-[2-(phenylsulfonyl)benzyl]-4,5,6,7-tetrahydro-1H-pyrrolo[3,2-c]pyridin-1-yl}propanoic acid;
• (2R)-2-{2-Methyl-4-oxo-3-[2-(phenylsulfonyl)benzyl]-4,5,6,7-tetrahydro-1H-pyrrolo[3,2-c]pyridin-1-yl]propanoic acid;
• {3-[3-Methoxy-2-(phenylsulfonyl)benzyl]-2-methyl-4-oxo-4,5,6,7-tetrahydro-1H-pyrrolo[3,2-c]pyridin-1-yl}acetic acid;
• {3-[2-(Ethylsulfonyl)benzyl]-2-methyl-4-oxo-4,5,6,7-tetrahydro-1H-pyrrolo[3,2-c] pyridin-1-yl}acetic acid;
• {3-[2-(Cyclohexylsulfonyl)benzyl]-2-methyl-4-oxo-4,5,6,7-tetrahydro-1H-pyrrolo-[3,2-c]pyridin-1-yl}acetic acid;
• {2-Methyl-3-[2-(morpholin-4-ylsulfonyl)benzyl]-4-oxo-4,5,6,7-tetrahydro-1H-pyrrolo[3,2-c]pyridin-1-yl}acetic acid;
• {2-Methyl-4-oxo-3-[2-(1H-1,2,4-thazol-1-ylmethyl)benzyl]-4,5,6,7-tetrahydro-1H-pyrrolo[3,2-c]pyridin-1-yl]acetic acid;
• Ethyl 2-(2,5-dimethyl-4-oxo-3-(2-(phenylsulfonyl)benzyl)-4,5,6,7-tetrahydro-1 H-pyrrolo[3,2-c]pyridin-1-yl)acetate;
• {2,5-Dimethyl-4-oxo-3-[2-(phenylsulfonyl)benzyl]-4,5,67-tetrahydro-1H-pyrrolo-[3,2-c]pyridin-1-yl}acetic acid;
• {3-[4-(Methoxycarbonyl)-2-(phenylsulfonyl)benzyl]-2-methyl-4-oxo-4,5,6,7-tetrahydro-1H-pyrrolo[3,2-c]pyridin-1-yl]acetic acid;
• 4-{[1-(Carboxymethyl)-2-methyl-4-oxo-4,5,6,7-tetrahydro-1H-pyrrolo[3,2-c]-pyridin-3-yl]methyl]-3-(phenylsulfonyl)benzoic acid;
• Methyl 2-(2-methyl-3-(4-(methylcarbamoyl)-2-(phenylsulfonyl)benzyl)-4-oxo-4,5,6,7-tetrahydro-1H-pyrrolo[3,2-c]pyridin-1-yl)acetate;
• (2-Methyl-3-[4-[(methylamino)carbonyl]-2-(phenylsulfonyl)benzyl]-4-oxo-4,5,6,7-tetrahydro-1H-pyrrolo[3,2-c]pyridin-1-yl}acetic acid;
• Methyl {3-[2-methoxy-6-(phenylsulfonyl)benzyl]-2-methyl-4-oxo-4,5,6,7-tetrahydro-1H-pyrrolo[3,2-c]pyridin-1-yl]acetate;
• {3-[2-Methoxy-6-(phenylsulfonyl)benzyl]-2-methyl-4-oxo-4,5,6,7-tetrahydro-1H-pyrrolo[3,2-c]pyridin-1-yl}acetic acid;
• (3-{2-[(3-Methoxyphenyl)sulfonyl]benzyl}-2-methyl-4-oxo-4,5,6,7-tetrahydro-1H-pyrrolo[3,2-c]pyridin-1-yl)acetic acid;
• {2-Cyclopropyl-4-oxo-3-[2-(phenylsulfonyl)benzyl]-4,5,6,7-tetrahydro-1H-pyrrolo [3,2-c]pyridin-1-yl}acetic acid;
• {5-Benzyl-2-methyl-4-oxo-3-[2-(phenylsulfonyl)benzyl]-4,5,6,7-tetrahydro-1H-pyrrolo[3,2-c]pyridin-1-yl]acetic acid;
• {3-[5-Fluoro-2-(phenylsulfonyl)benzyl]-2-methyl-4-oxo-4,5,6,7-tetrahydro-1H-pyrrolo[3,2-c]pyridin-1-yl}acetic acid;
• {2-Methyl-4-oxo-3-[4-(phenylsulfonyl)benzyl]-4,5,6,7-tetrahydro-1H-pyrrolo-[3,2-c]pyridin-1-yl}acetic acid;
• {3-[4-(Benzylsulfonyl)benzyl]-2-methyl-4-oxo-4,5,6,7-tetrahydro-1H-pyrrolo-[3,2-c]pyridin-1-yl}acetic acid;
• [2-Methyl-4-oxo-3-(2-phenoxybenzyl)-4,5,6,7-tetrahydro-1H-pyrrolo[3,2-c]-pyridin-1-yl]acetic acid;
• Ethyl {3-[5-bromo-2-(phenylsulfonyl)benzyl]-2-methyl-4-oxo-4,5,6,7-tetrahydro-1H-pyrrolo[3,2-c]pyridin-1-yl]acetate;
• {3-[5-Bromo-2-(phenylsulfonyl)benzyl]-2-methyl-4-oxo-4,5,6,7-tetrahydro-1 H-pyrrolo[3,2-c]pyridin-1-yl}acetic acid;
• Ethyl (2-methyl-4-oxo-3-{[1-(phenylsulfonyl)-1H-pyrrol-2-yl]methyl]-4,5,6,7-tetrahydro-1H-pyrrolo[3,2-c]pyridin-1-yl)acetate;
• (2-Methyl-4-oxo-3-{[1-(phenylsulfonyl)-1H-pyrrol-2-yl]methyl}-4,5,6,7-tetrahydro-1H-pyrrolo[3,2-c]pyridin-1-yl)acetic acid;
• {5-Isopropyl-2-methyl-4-oxo-3-[2-(phenylsulfonyl)benzyl]-4,5,6,7-tetrahydro-1H-pyrrolo[3,2-c]pyridin-1-yl}acetic acid;
• Ethyl [2-methyl-4-oxo-3-(quinolin-2-ylmethyl)-4,5,6,7-tetrahydro-1 H-pyrrolo-[3,2-c]pyridin-1-yl]acetate;
• [2-Methyl-4-oxo-3-(quinolin-2-ylmethyl)-4,5,6,7-tetrahydro-1 H-pyrrolo[3,2-c]-pyridin-1-yl]acetic acid;
• (3-{2-[(4-Fluorophenyl)sulfonyl]-5-methoxybenzyl}-2-methyl-4-oxo-4,5,6,7-tetrahydro-1H-pyrrolo[3,2-c]pyridin-1-yl)acetic acid;
• Ethyl {2-methyl-3-[2-(1-naphthyl)benzyl]-4-oxo-4,5,6,7-tetrahydro-1H-pyrrolo-[3,2-c]pyridin-1-yl}acetate;
• {2-Methyl-3-[2-(1-naphthyl)benzyl]-4-oxo-4,5,6,7-tetrahydro-1H-pyrrolo[3,2-c]-pyridin-1-yl}acetic acid;
• Ethyl {3-[(2'-chloro-6'-methoxybiphenyl-2-yl)methyl]-2-methyl-4-oxo-4,5,6,7-tetrahydro-1H-pyrrolo[3,2-c]pyridin-1-yl]acetate;
• {3-[(2'-Chloro-6'-methoxybiphenyl-2-yl)methyl]-2-methyl-4-oxo-4,5,6,7-tetrahydro-1H-pyrrolo[3,2-c]pyridin-1-yl}acetic acid;
• Ethyl {3-[(2'-methoxybiphenyl-2-yl)methyl]-2-methyl-4-oxo-4,5,6,7-tetrahydro-1H-pyrrolo[3,2-c]pyridin-1-yl}acetate;
• {3-[(2'-Methoxybiphenyl-2-yl}methyl]-2-methyl-4-oxo-4,5,6,7-tetrahydro-1H-pyrrolo[3,2-c]pyridin-1-yl}acetic acid;
• Ethyl [3-(2-benzoylbenzyl)-2-methyl-4-oxo-4,5,6,7-tetrahydro-1H-pyrrolo[3,2-c]-pyridin-1-yl]acetate;
• [3-(2-Benzoylbenzyl)-2-methyl-4-oxo-4,5,6,7-tetrahydro-1H-pyrrolo[3,2-c]pyridin -1-yl]acetic acid;
• Ethyl {2,6,6-trimethyl-4-oxo-3-[2-(phenylsulfonyl)benzyl]-4,5,6,7-tetrahydro-1H-pyrrolo[3,2-c]pyridin-1-yl}acetate;
• {2,6,6-Trimethyl-4-oxo-3-[2-(phenylsulfonyl)benzyl]-4,5,6,7-tetrahydro-1H-pyrrolo[3,2-c]pyridin-1-yl}acetic acid;
• Ethyl (3-{2-[(4-fluorophenyl)sulfonyl]benzyl}-2,6,6-trimethyl-4-oxo-4,5,6,7-tetrahydro-1H-pyrrolo[3,2-c]pyridin-1-yl)acetate;
• (3-{2-[(4-Fluorophenyl)sulfonyl]benzyl}-2,6,6-trimethyl-4-oxo-4,5,6,7-tetrahydro-1H-pyrrolo[3,2-c]pyridin-1-yl)acetic acid;
• Ethyl (3-{2-[(4-fluorophenyl)sulfonyl]-5-methoxybenzyl}-2,6,6-trimethyl-4-oxo-4,5,6,7-tetrahydro-1H-pyrrolo[3,2-c]pyridin-1-yl)acetate;
• (3-{2-[(4-Fluorophenyl)sulfonyl]-5-methoxybenzyl}-2,6,6-trimethyl-4-oxo-4,5,6,7-tetrahydro-1H-pyrrolo[3,2-c]pyridin-1-yl)acetic acid;
• Ethyl {3-[5-cyano-2-(phenylsulfonyl)benzyl]-2-methyl-4-oxo-4,5,6,7-tetrahydro-1 H-pyrrolo[3,2-c]pyridin-1-yl]acetate;
• {3-[5-Cyano-2-(phenylsulfonyl)benzyl]-2-methyl-4-oxo-4,5,6,7-tetrahydro-1H-pyrrolo[3,2-c]pyridin-1-yl}acetic acid;
• tert-Butyl [3-(2-{[benzyl(methyl)amino]sulfonyl}benzyl)-2-methyl-4-oxo-4,5,6,7-tetrahydro-1H-pyrrolo[3,2-c]pyridin-1-yl]acetate;
• [3-(2-{[Benzyl(methyl)amino]sulfonyl]benzyl)-2-methyl-4-oxo-4,5,6,7-tetrahydro-1H-pyrrolo[3,2-c]pyridin-1-yl]acetic acid;
• Ethyl [3-(2-benzylbenzyl)-2-methyl-4-oxo-4,5,6,7-tetrahydro-1 H-pyrrolo[3,2-c]-pyridi n-1-yl]acetate;
• [3-(2-Benzylbenzyl)-2-methyl-4-oxo-4,5,6,7-tetrahydro-1 H-pyrrolo[3,2-c]-pyridin-1-yl]acetic acid;
• (3-{2-[Hydroxy(phenyl)methyl]benzyl}-2-methyl-4-oxo-4,5,6,7-tetrahydro-1H-pyrrolo[3,2-c]pyridin-1-yl)acetic acid;
• [3-(2-{[(Cyclopropylcarbonyl)(ethyl)amino]methyl]benzyl)-2-methyl-4-oxo-4,5,6,7-tetrahydro-1H-pyrrolo[3,2-c]pyridin-1-yl]acetic acid;
• [2-Methyl-4-oxo-3-[2-(phenylsulfonyl)benzyl]-5-(2,2,2-trifluoro-1-methylethyl)-4,5,6,7-tetrahydro-1H-pyrrolo[3,2-c]pyridin-1-yl]acetic acid;
• {2-Methyl-4-oxo-3-[2-(phenylsulfonyl)benzyl]-4,5-dihydro-1H-pyrrolo[3,2-c]-pyridin-1-yl}acetic acid;
• Ethyl {2-methyl-4-oxo-3-[2-(phenylsulfonyl)benzyl]-4,5-dihydro-1 H-pyrrolo-[3,2-c]pyridin-1-yl}acetate;
• Ethyl {2,5-dimethyl-4-oxo-3-[2-(phenylsulfonyl)benzyl]-4,5-dihydro-1 H-pyrrolo-[3,2-c]pyridin-1-yl}acetate;
• {2,5-Dimethyl-4-oxo-3-[2-(phenylsulfonyl)benzyl]-4,5-dihydro-1H-pyrrolo-[3,2-c]pyridin-1-yl}acetic acid;
• Ethyl {5-(difluoromethyl)-2-methyl-4-oxo-3-[2-(phenylsulfonyl)benzyl]-4,5-dihydro-1H-pyrrolo[3,2-c]pyridin-1-yl}acetate;
• {5-(Difluoromethyl)-2-methyl-4-oxo-3-[2-(phenylsulfonyl)benzyl]-4,5-dihydro-1H-pyrrolo[3,2-c]pyridin-1-yl}acetic acid;
• 2-(Dimethylamino)-2-oxoethyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
• 2-Morpholin-4-ylethyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
• 2,3-Dihydro-1 H-inden-5-yl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
• 1-{[(Cyclohexyloxy)carbonyl]oxy}ethyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
• (5-Methyl-2-oxo-1,3-dioxol-4-yl)methyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
• 2-(Dimethylamino)ethyl (3-{2-[(4-fluorophenyl)sulfonyl]benzyl}-2-methyl-4-oxo-4,5,6,7-tetrahydro-1H-pyrrolo[3,2-c]pyridin-1-yl)acetate;
• 2-Methoxyphenyl (3-{2-[(4-fluorophenyl)sulfonyl]benzyl}-2-methyl-4-oxo-4,5,6,7-tetrahydro-1H-pyrrolo[3,2-c]pyridin-1-yl)acetate.

12. A compound according to any one of claims 1 to 11 for use in the treatment of a pathological condition or disease mediated by prostaglandin D₂, which condition or disease is preferably selected from asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome, myocardial infarction, aneurysm, stroke, cancer, wound healing, endotoxic shock, pain, inflammatory conditions, eosinophilic esophagitis, eosinophil-associated gastrointestinal disorders (EGID), idiopathic, otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, Crohn's disease, ulcerative colitis, irritable bowel disease and inflammatory bowel disease, more preferably selected from asthma, chronic obstructive pulmonary disease (COPD), allergic rhinitis and atopic dermatitis.

13. A pharmaceutical composition comprising a compound as defined in any one of claims 1 to 11 in association with a pharmaceutically acceptable diluent or carrier.

14. A method for treating a subject afflicted with a pathological condition or disease as defined in claim 12, which comprises administering to said subject an effective amount of a compound as defined in any one of claims 1 to 11.

15. A combination product comprising (i) a compound according to any one of claims 1 to 11; and (ii) another compound selected from
a. Anticholinergics, such as aclidinium bromide or tiotropium,
b. Corticosteroids, or gluococorticoids such as prednisone or methylprednisolone,
c. Antihistamines, such as ebastine, ranitidine, ABT-239 or JNJ 7777120;
d. Beta-2 agonists, such as salmeterol or formoterol,
e. Chemokine receptor antagonists, such as maraviroc or enfuvirtide,
f. Leukotriene receptor antagonists,
g. JAK inhibitors such as tasocitinib citrate or INCB018424,
h. Syk inhibitors such as R-112,
i. Phosphosdiesterase IV inhibitors such as roflumilast or revamilast,
j. Dual beta-2 adrenoceptor agonist/M3 receptor antagonist (MABA compounds) such as GSK-961081,
k. p38 Inhibitors such as ARRY-797,
l. PKC inhibitors such as NVP-AEB071,
m. 5-lipoxygenase activating protein inhibitors, such as veliflapon,
n. 5-lipoxygenase inhibitors,
o. CYSLTR1 antagonists, such as montelukast, pranlukast or zafirlukast;
p. CYSLTR2 antagonists, such as pranlukast, zafirlukast or tipilukast;
q. BLT1 antagonists,
r. BLT2 antagonists,
s. Thromboxane A2 antagonists such as ramatroban,
t. DP1 receptor antagonists, such as laropiprant,
u. DP1 receptor agonists, such as BW-245C,
v. IP receptor agonists, such as RO-1138452,
w. Anti-IgE, such as omalizumab,
x. IL5 antibody, such as mepolizumab,
y. Leukotriene formation inhibitors,
z. Bronchodilators, such as pirbuterol, epinephrine, ephedrine or salbutamol;
aa. Decongestants, such as ephedrine, levo-methamphetamine, naphazoline, oxymetazoline, phenylephrine, phenylpropanolamine, propylhexedrine, pseudoephedrine, synephrine or tetrahydrozoline;
bb. Mucolytics such as acetylcysteine, ambroxol, bromhexine, carbocisteine, domiodol, eprazinone, erdosteine, letosteine, neltenexine, sobrerol, stepronin or tiopronin;
cc. Antitussives, such as dextromethorphan,
dd. Analgesics such as aspirin, paracetamol, rofecoxid, celecoxib, morphine, codeine, oxycodone, hydrocodone, dihydromorphine or flupirtine; and
ee. Expectorants such antimony pentasulfide, guaiacolsulfonate, guaifenesin, potassium iodide or tyloxapol,
for simultaneous, separate or sequential use in the treatment of the human or animal body.
